(19) Europäisches Patentamt
European Patent Office
Office européen des brevets

(11) Veröffentlichungsnummer: **0 313 512 B1**

(12) # EUROPÄISCHE PATENTSCHRIFT

(45) Veröffentlichungstag der Patentschrift: **25.11.92**

(51) Int. Cl.⁵: **A01N 43/82**, C07D 285/14, C07F 7/08, C07F 9/6518, C07D 417/12, C07H 13/08

(21) Anmeldenummer: **88810561.6**

(22) Anmeldetag: **17.08.88**

(54) **Benzothiadiazole und ihre Verwendung in Verfahren und Mitteln gegen Pflanzenkrankheiten.**

(30) Priorität: **21.08.87 CH 3229/87**

(43) Veröffentlichungstag der Anmeldung:
**26.04.89 Patentblatt 89/17**

(45) Bekanntmachung des Hinweises auf die Patenterteilung:
**25.11.92 Patentblatt 92/48**

(84) Benannte Vertragsstaaten:
**AT BE CH DE ES FR GB GR IT LI LU NL SE**

(56) Entgegenhaltungen:
**DE-A- 1 695 786**
**US-A- 4 177 054**

**Journal of the Chemical Society (C), 1971, Seiten 3994-3999, London, GB, E. Haddock et al.**

(73) Patentinhaber: **CIBA-GEIGY AG**
**Klybeckstrasse 141**
**CH-4002 Basel(CH)**

(72) Erfinder: **Schurter, Rolf, Dr.**
**Holzmattstrasse 45**
**CH-4102 Binningen(CH)**
Erfinder: **Kunz, Walter, Dr.**
**Buchenstrasse 9**
**CH-4104 Oberwil(CH)**
Erfinder: **Nyfeler, Robert, Dr.**
**Bärenfelserstrasse 8**
**CH-4057 Basel(CH)**

EP 0 313 512 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren und Mittel zur artifiziellen Erzeugung von Abwehrmechanismen bei Pflanzen gegen den Befall durch Krankheiten, sowie Massnahmen und Stoffe zur Durchführung dieses Verfahrens.

Pflanzen sind vielfältigen mikrobiellen Einflüssen durch Bakterien, Viren und Pilzen ausgesetzt, die an der Pflanze parasitieren.

Die bisherigen Bemühungen im Pflanzenschutz beschränken sich in der Regel darauf, die Pflanze allgemein, z.B. durch Kultivierung und Düngung, zu stärken und drohenden oder erfolgten Krankheitsbefall durch Applikation von direkt einwirkenden Gegenmitteln (= Mikrobiziden) zu verhüten oder zu bekämpfen.

Die vorliegende Erfindung hat sich die Aufgabe gestellt, in schonender Weise die in einer Pflanze latent vorhandenen eigenen Abwehrmechanismen zu aktivieren, so dass die Pflanze aus sich selbst heraus den Angriff eines Krankheitserregers erkennen und bekämpfen kann. Dieser Vorgang lässt sich als Immunisierung bezeichnen.

Die vorliegende erfinderische Lösung des Problems der Krankheitsbekämpfung an Pflanzen besteht in der artifiziellen chemischen Aktivierung der pflanzeneigenen Abwehrkräfte gegen pathogene mikrobiologische Einflüsse. Bereits eine einmalige chemische Applikation kann eine langanhaltende, mehrwöchige bis mehrmonatige Resistenz der Pflanze gegenüber bestimmten pathogenen Erregern bewirken. Der wesentliche Unterschied zur herkömmlichen Krankheitsbekämpfung besteht somit in der Verwendung von Substanzen, die selbst keine mikrobizide Wirkung entfalten, sondern die Abwehrbereitschaft der Pflanze gegen mikrobielle Infektionen anregen und aufgrund ihrer geringen Aufwandmengen für diese selbst und ihren Lebensbereich keine Belastung darstellen.

Die Erfindung betrifft ein Verfahren zur Immunisierung von Pflanzen gegen Krankheitsbefall, welches gekennzeichnet ist durch Applikation geringer Mengen eines 7-Cyano-1,2,3-benzothiadiazol-Derivats bzw. eines Derivats der 1,2,3-Benzothiadiazol-7-carbonsäure der nachfolgend angegebenen Formel I auf die zu schützende Pflanze, auf Pflanzenteile oder ihren Lebensbereich.

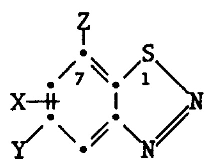

(I)

In dieser Formel bedeuten:

X Wasserstoff, Halogen, Hydroxy, Methyl, Methoxy, HOOC oder MOOC;

Y Wasserstoff, Halogen, $SO_3H$, $SO_3M$, Nitro, Hydroxy, oder Amino, wobei M das Moläquivalent eines Alkali- oder Erdalkali-Ions ist, das aus einer entsprechenden Base oder basischen Verbindung gebildet wird; und

Z Cyano oder -CO-A, wobei

A entweder OH oder SH bedeutet, deren H auch durch das Moläquivalent eines anorganischen oder organischen kationischen Restes ersetzt sein kann.

Zu einem wesentlichen Teil stellen die X und Y enthaltende 1,2,3-Benzothiadiazol-7-carbonsäure der Formel I und ihre Salze das Wirkungsprinzip dar, das in der Pflanze die Abwehr gegen pathogene Krankheitserreger auslöst. Es gehören auch die von der Formel I umfassten 7-Cyano-Verbindungen dazu, die in metabolischer Wechselwirkung zur entsprechenden 7-Carbonsäure stehen können. Es ist daher verständlich, dass der Beitrag zur biologischen Aktivität, den der Substituent A der Formel I leistet, von geringerer Bedeutung sein wird. Es ist auch verständlich, dass trotz grösserer Strukturenvariation im Substituenten A mit Verbindungen der Formel I weitgehend äquivalente biologische Wirkungen erzielt werden. Ein Substituent A sollte jedoch die 7-substituierte 1,2,3-Benzothiadiazol-Molekülstruktur nicht zu stark abschirmen.

Bevorzugt als Substituent A sind daher kationische oder sonstige organische Reste mit einem Molgewicht von weniger als 600, besonders bevorzugt weniger als 400.

Bevorzugt sind ferner Verbindungen der Formel I mit Z = CN und X Wasserstoff, Halogen oder Methyl und Y Wasserstoff oder Halogen.

Ein wichtiger Aspekt vorliegender Erfindung sind Verbindungen und entsprechende Pflanzenschutzmittel und Applikationsverfahren auf Basis der Formel I, worin bedeuten:

X Wasserstoff, Halogen, Hydroxy, Methyl, Methoxy, HOOC oder MOOC;

Y Wasserstoff, Halogen, $SO_3H$, $SO_3M$, Nitro, Hydroxy, oder Amino, wobei M das Moläquivalent eines Alkali- oder Erdalkali-Ions ist, das aus einer entsprechenden Base oder basischen Verbindung gebildet wird; und
Z Cyano oder -CO-A,
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, U-$C_1$-$C_3$-Alkyl oder $C_2$-$C_4$-Dialkylamino substituiertes oder durch die CO-Gruppe unterbrochenes $C_1$-$C_8$-Alkyl, (T)-COOH oder (T)-COO$C_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

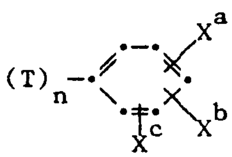 , $(T)_n$-Napht, $(T)_n$-Si($C_1$-$C_8$-Alkyl$)_3$,

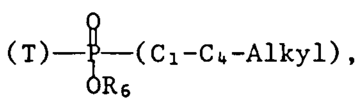 , 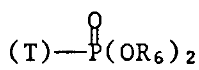 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder $X^a$ $C_1$-$C_2$-Halogenalkoxy mit bis zu 5 Halogenatomen, Nitro, Dimethylamino, Phenyl, Phenyloxy, Benzyloxy, Sulfamoyl und $X^b$ und $X^c$ gleichzeitig Wasserstoff; oder
$X^a$ Phenyl, Phenyloxy oder Benzyloxy und
$X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;
W einen 5- bis 7-gliedrigen gesättigten oder ungesättigten unsubstituierten oder durch Halogen, Trifluormethyl, Cyano, $C_1$-$C_2$-Alkyl oder einen $C_1$-$C_2$-Alkoxycarbonyl-$C_2$-$C_4$-alkylen-amino(imino)-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$, $-CH(CH_3)-$, $-CCH_3(CH_3)-$, $-CH_2CH_2CH_2-$ oder $-CH_2CH_2O-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, Methoxy, $CF_3$, Cyano, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen einen Heterocyclus W oder einen carbocyclischen Ring W';
W' einen carbocyclischen Rest mit 3- bis 7 Ringkohlenstoffatomen;
$R_5$ Wasserstoff oder Methyl;

$R_6$ Wasserstoff oder $C_1$-$C_4$-Alkyl; und
n Null oder 1;
wobei in den Verbindungen der Formel I der organische Rest A ein Molgewicht von weniger als 900 besitzt; und umfassen im Falle von U als Sauerstoff oder Schwefel die Salze der pflanzenphysiologisch verträglichen 7-Carbonsäure mit primären, sekundären oder tertiären Aminen oder mit anorganischen Basen.

Eine besondere Gruppe von Wirkstoffen des erfindungsgemässen Vefahrens stellen folgende Verbindungen der Formel I dar; worin bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, Alkoxy oder U-$C_1$-$C_3$-Alkyl substituiertes $C_1$-$C_8$-Alkyl, (T)-COOH oder (T)-COO$C_1$-$C_4$-Alkyl, $C_2$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

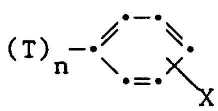 , $(T)_n$-Naphthyl, $(T)_n$-Si($C_1$-$C_8$-Alkyl)$_3$,

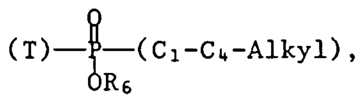 , 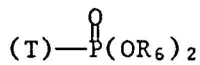 ; oder $(T)_n$—W ;

W einen 5- bis 7-gliedrigen gesättigten oder ungesättigten unsubstituierten oder durch Halogen, Trifluormethyl, Cyano, $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$, $-CH(CH_3)-$ oder $-CCH_3(CH_3)-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, Methoxy, $CF_3$, Cyano, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
und umfassen die pflanzenphysiologisch verträglichen Salze der 7-Carbonsäuren und 7-Thiocarbonsäuren mit primären, sekundären und tertiären Aminen.

Die vorliegende Erfindung betrifft ferner Mittel gegen Pflanzenkrankheiten, die als Wirkstoffe Verbindungen der Formel I enthalten. Die Erfindung betrifft weiter die Herstellung der genannten Mittel sowie die Herstellung derjenigen Wirkstoffe, die neu sind. Die Erfindung betrifft darüber hinaus die Verwendung der Wirkstoffe oder der Mittel zum Schutz von Pflanzen gegen den Befall durch phytopathogene Mikroorganismen, beispielsweise Fungi, Bakterien und Viren.

Wie erwähnt, stellen Verbindungen der Formel I keine Mikrobizide im herkömmlichen Sinne einer Direktwirkung auf den Krankheitserreger dar, sondern sind, wie weiter unten gezeigt werden kann, im Prinzip gegen derartige Erreger bei Abwesenheit der Pflanze (= in vitro) wirkungslos. Wo solche direkte Mikrobizidwirkung zuweilen dennoch auftritt, wird sie in der Regel durch gewisse Strukturelemente im Molekül zusätzlich hervorgerufen, die mit einer solchen Sekundärwirkung den bestehenden Immunisierungseffekt überlagern, jedoch nicht ersetzen.

Das immunisierende Wirkungsprinzip beruht im wesentlichen auf der speziellen in 7-Stellung durch eine Säurefunktion substituierten 1,2,3-Benzothiadiazol-Grundstruktur der Formel I, während die Fähigkeit der 1,2,3-Benzothiadiazolderivate in die Pflanzen bzw. ihren Metabolismus einzudringen, von den unter A definierten Resten und den Salzen der 7-Säure abhängig ist (Vehikel-Funktion).

Bevorzugt sind daher hinsichtlich ihres Immunisierungspotentials Verbindungen der Formel I, bei denen der organische Rest A ein Molgewicht von weniger als 600 und besonders bevorzugt ein Molgewicht von weniger als 400 besitzen. Bevorzugt sind ferner Verbindungen mit Z = CN.

In einer speziellen Variante werden Verbindungen der Formel I zur Pflanzenimmunisierung bevorzugt, bei denen der Substituent Z Cyano oder aber einen Rest A bedeutet, dessen Molgewichtsanteil am Gesamtmolekül der Formel I zwischen 5,0 % und 85 % liegt, bevorzugt zwischen 7,8 % und 60 %.

Die Aufwandmenge solcher Immunisierungsagenzien der Formel I liegt bei weniger als 1 kg AS/ha, bevorzugt bei weniger als 500 g AS/ha, besonders bevorzugt bei 50-300 g AS/ha.

Der Begriff Heteroatome schliesst auch andere Elemente als N, O oder S ein, z.B. Si oder P.

Als kationische Reste für eine M-OOC-Gruppe kommen Metalle und organische Basen in Frage. Als Metalle sind Alkali- und Erdalkali-Metalle zweckmässig, es kommen aber auch beliebige andere in Betracht. Als organische Basen gelten Amine, insbesondere mit aliphatischen, aromatischen, araliphatischen und/oder cycloaliphatischen Resten.

Halogen selbst oder als Bestandteil eines anderen Substituenten bedeutet Fluor, Chlor, Brom oder Jod, vorzugsweise Fluor, Chlor oder Brom. Unter Alkyl selbst oder als Bestandteil eines anderen Substituenten sind gerad- oder verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl, Butyl, Pentyl, Hexyl, Heptyl oder Octyl wie z.B Isopropyl, Isobutyl, tert.-Butyl, sek.-Butyl oder Isopentyl. Cycloalkyl bedeutet z.B. Cyclopropyl, Cyclobutyl, Cyclopentyl oder Cyclohexyl.

Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl.

Als Basen oder Verbindungen basischen Charakters kommen anorganische Basen oder Basenbildner in Betracht, wie z.B. Hydroxide, Carbonate und Hydrogencarbonate der Alkali- und Erdalkalimetalle, vorzugsweise LiOH, NaOH, KOH, $Mg(OH)_2$ oder $Ca(OH)_2$; und ferner $NaHCO_3$, $KHCO_3$, $Na_2CO_3$ und $K_2CO_3$.

Unter Heterocyclen sind zu verstehen z.B.: Furan, Tetrahydrofuran, Thiophen, Tetrahydropyran, Pyrrol, Pyrrolidin, Imidazol, 1,2,4-Triazol, Piperidin, Pyridin, Pyrimidin, Morpholin oder Azacycloheptan. Insbesondere stellen sie dar: Furan-2-yl, Tetrahydrofuran-2-yl, Tetrahydrofuran-3-yl, Tetrahydropyran-2-yl, 1,3-Dioxolan-5-yl, Pyrrol-1-yl, Pyrrol-2-yl, Pyrrolidin-1-yl, Isoxazol-3-yl, Isoxazol-4-yl, 1,2-Dithiazolin-5-yl, Imidazol-1-yl, 1,2,4-Triazol-1-yl, 1,3,4-Triazol-1-yl, Thiophen-2-yl, Piperidin-1-yl, Piperidin-4-yl, Pyridin-2-yl, Pyridin-3-yl, Pyridin-4-yl, Pyrimidin-2-yl, Pyrimidin-4-yl, Pyrimidin-5-yl, Morpholin-1-yl, Azacycloheptan-1-yl oder Benzo-1,2,3-thiadiazol-7′-yl.

Als salzbildende Amine sind z.B. anzusehen:
Trimethylamin, Triethylamin, Tributylamin, Tribenzylamin, Tricyclohexylamin, Triamylamin, Trihexylamin, N,N-Dimethylanilin, N,N-Dimethyltoluidin, N,N-Dimethyl-p-amino-pyridin, N-Methylpyrrolidin, N-Methylpiperidin, N-Methyl-pyrrolidin, N-Methylimidazol, N-Methylpyrrol, N-Methylmorpholin, N-Methylhexamethylenimin, Pyridin, Chinolin, alpha-Picolin, β-Picolin, Isochinolin, Pyrimidin, Acridin, N,N,N′,N′-Tetra-methylethylendiamin, N,N,N′,N′-Tetraethylethylendiamin, Chinoxalin, N-Propyldiisopropylamin, N,N-Dimethylcyclohexylamin, 2,6-Lutidin, 2,4-Lutidin, Triethylendiamin sowie heterocyclische Amine vom Morpholintyp.

Unter einem Monosaccharid-Rest sind beispielsweise zu verstehen Glucofuranosyl, Galaktopyranosyl, Allofuranosyl oder Mannityl, wobei die OH-Gruppen frei oder acetyliert oder mit Methyl, Benzyl oder Isopropylidenyl verethert sind. Unter den genannten Resten sind die Diisopropylidenyl-Derivate bevorzugt, während von diesen wiederum besonders bevorzugte Reste sind: Diaceton-D-glucosidyl, 1,2,3,4-Di-O-isopropyliden-D-galactopyranos-6-yl, 1,2,5,6-Di-O-isopropyliden-D-mannit-3-yl, 1,2,5,6-Di-O-isopropyliden-α-

D-allofuranos-3-yl, D-Glucofuranos-3-yl, D-Galactopyranos-6-yl, D-Mannit-3-yl, D-Allofuranos-4-yl, Mannopyranos-1-yl, 2-Methyl-D-glucosid-6-yl, 1,2,5,6-Tetraacetyl-D-galactopyranos-3-yl und 2,3,5-Tribenzylribofuranos-1-yl.

Aufgrund ihrer ausgeprägten gegen den Befall durch phytopathogene Mikroorganismen schützenden Eigenschaften sind diejenigen Wirksubstanzen bevorzugt, deren Strukturen folgende Substituenten oder Kombinationen dieser Substituenten untereinander aufweisen:
X und Y für Wasserstoff;
Z für Cyano oder COA;
A für UR oder $U^1N(=C)_n(R_3)R_4$;
U für Sauerstoff;
$U^1$ für Sauerstoff oder $-N(R_5)-$;
R für Wasserstoff, $C_1$-$C_8$-Alkyl, durch Halogen oder $C_1$-$C_3$-Alkoxy substituiertes $C_1$-$C_8$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, Benzyl, halogeniertes Benzyl, Methoxybenzyl, $(T)_n$-$Si(CH_3)_3$, (T)-P(O)($C_1$-$C_4$-Alkyl)$CH_3$, (T)-P(O)($OC_1$-$C_4$-Alkyl)$_2$ oder die Gruppe $(T)_n$-W;
W für einen 5- bis 7-gliedrigen gesättigten oder ungesättigten unsubstituierten Heterocyclus mit 1 bis 3 Heteroatomen aus der Gruppe O, N und S sowie für Diaceton-D-glucosidyl;
T für die Brückenglieder $-CH_2-$, $-CH_2CH_2-$, $-CH(CH_3)-$, $-CCH_3(CH_3)-$ oder $-CH_2CH_2CH_2-$;
$R_3$ für Wasserstoff, Cyano, $C_1$-$C_6$-Alkyl, Phenyl oder W;
$R_4$ für Wasserstoff, $C_1$-$C_6$-Alkyl, $CONH_2$, CONH-CONH-$C_1$-$C_3$-Alkyl, $C_1$-$C_3$-Alkanoyl oder $C_3$-$C_5$-Alkenoyl;
$R_3$ und $R_4$ zusammen mit dem Atom, an welches sie gebunden sind, W oder W';
W für einen 5- bis 7-gliedrigen gesättigten oder ungesättigten Heterocyclus mit 1 bis 3 Heteroatomen aus der Gruppe O, N und S;
W' für einen Rest aus der Gruppe

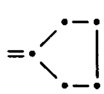 , 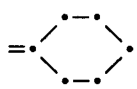 und 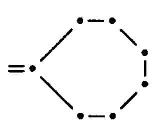 ;

n für Null oder 1.

Eine besondere Gruppe von Wirkstoffen mit bevorzugter mikrobizider Wirkung gegen phytopathogene Mikroorganismen stellen Verbindungen mit folgenden Substituenten oder Kombinationen dieser Substituenten untereinander dar:
X und Y Wasserstoff;
Z Cyano oder COA;
A UR oder $U^1N(=C)_n(R_3)R_4$;
U Sauerstoff;
$U^1$ Sauerstoff oder $-N(R_5)-$;
R Wasserstoff, $C_1$-$C_8$-Alkyl, durch Halogen oder $C_1$-$C_3$-Alkoxy substituiertes $C_1$-$C_8$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, Benzyl, halogeniertes Benzyl, $(T)_n$-$Si(CH_3)_3$, (T)-P(O)($C_1$-$C_4$-Alkyl)$CH_3$, (T)-P(O)($OC_1$-$C_4$-Alkyl)$_2$ oder die Gruppe $(T)_n$-W;
W einen 5- bis 7-gliedrigen gesättigten oder ungesättigten unsubstituierten Heterocyclus mit 1 bis 3 Heteroatomen aus der Gruppe O, N und S sowie für Diaceton-D-glucosidyl;
T die Brückenglieder $-CH_2-$, $-CH_2CH_2-$, $-CH(CH_3)-$ oder $-CCH_3(CH_3)-$
$R_3$ Wasserstoff, Cyano, $C_1$-$C_6$-Alkyl, Phenyl oder W;
$R_4$ Wasserstoff, $C_1$-$C_6$-Alkyl, $CONH_2$, CONH-CONH-$C_1$-$C_3$-Alkyl, $C_1$-$C_3$-Alkanoyl oder $C_3$-$C_5$-Alkenoyl;
$R_3$ und $R_4$ zusammen mit dem Atom, an welches sie gebunden sind, W oder W';
W einen 5- bis 7-gliedrigen gesättigten oder ungesättigten Heterocyclus mit 1 bis 3 Heteroatomen aus der Gruppe O, N und S;
W' einen Rest aus der Gruppe

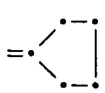 , 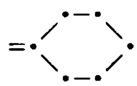 und 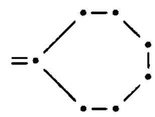 ; und

n Null oder 1.

Die unter die Formel I fallenden Verbindungen sind überwiegend neu; die übrigen sind bekannt. So sind in der Deutschen Offenlegungsschrift Nr. 1 695 786 und in der französichen Patentschrift No. 1 541 415 einige Verbindungen in allgemeiner Form als biozide Wirkstoffe zur Anwendung in herbiziden, insektiziden und fungiziden Mitteln offenbart. Für keine dieser unter die Formel I der vorliegenden Erfindung fallenden bekannten Einzelverbindungen ist jedoch speziell eine fungizide Aktivität beschrieben. Darüber hinaus ist die Benzo-1,2,3-thiadiazol-7-carbonsäure ohne Angabe biologischer Eigenschaften aus J. Chem. Soc. (C) 1971, 3997 bekannt.

Die neuen Verbindungen der vorliegenden Erfindung sind in folgenden Gruppen definiert:
Verbindungen der Formel I′

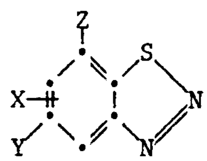

(I')

in welcher bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-$COOC_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

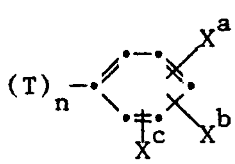 , $(T)_n$-Napht, $(T)_n$-Si($C_1$-$C_8$-Alkyl)$_3$,

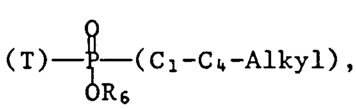 , 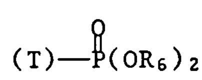 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder $X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder
$X^a$ Phenyl, Phenyloxy oder Benzyloxy und $X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;

T die Brückenglieder $-CH_2-$, $-CH_2CH_2-$, $-CH(CH_3)-$, $-CH_2CH_2CH_2-$ oder $-CH_2CH_2O-$;

$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;

$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;

<u>$R_1$ und $R_2$</u> zusammen mit dem Atom, an welches sie gebunden sind, einen Heterocyclus W;

$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;

$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;

<u>$R_3$ und $R_4$</u> zusammen mit dem Atom, an welches sie gebunden sind, einen Heterocyclus W oder einen <u>carbocyclischen</u> Ring W';

W' einen carbocyclischen Rest mit 3- bis 7 Ringkohlenstoffatomen;

$R_5$ Wasserstoff oder Methyl;

$R_6$ Wasserstoff oder $C_1$-$C_4$-Alkyl; und

n Null oder 1;

1) mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure; oder

2) mit der Massgabe, dass falls
Z Cyano, HOOC oder Methoxycarbonyl bedeutet, X und Y unabhängig voneinander nicht Wasserstoff, Chlor oder Brom sind; oder

3) mit der Massgabe, dass falls
Z Cyano, Methoxycarbonyl, Ethoxycarbonyl oder HOOC bedeutet, X nicht Wasserstoff, Halogen, Hydroxy, Methyl oder Methoxy und Y nicht Wasserstoff, Halogen, Nitro oder Amino sind; oder

4) mit der Massgabe, dass falls Z Cyano, $C_1$-$C_4$-Alkoxycarbonyl oder HOOC bedeutet, X nicht Wasserstoff, Halogen, Hydroxy, Methyl oder Methoxy und Y nicht Wasserstoff, Halogen, Nitro oder Amino sind.

Es sind auch solche Verbindungen der Formel I' neu, bei denen Z = COA ist und R eine andere Bedeutung hat als Wasserstoff und unsubstituiertes Alkyl. Die Erfindung betrifft auch solche Verbindungen sowie entsprechende Mittel und Verfahren zur Immunisierung.

Eine besondere Gruppe von neuen Wirkstoffen, stellen folgende Verbindungen der Formel I' dar, worin bedeuten:

X Wasserstoff, Halogen, Hydroxy, Methyl, Methoxy, HOOC oder MOOC;

Y Wasserstoff, Halogen, $SO_3H$, $SO_3M$, Nitro, Hydroxy oder Amino;

Z Cyano oder COA;

A UR, $N(R_1)R_2$ oder $U^1N(=C)_n(R_3)R_4$;

M Moläquivalent eines Alkali- oder Erdalkali-Ions, das aus einer entsprechenden Base oder basischen Verbindung gebildet ist;

U Sauerstoff oder Schwefel;

$U^1$ Sauerstoff oder $-N(R_5)-$;

R Wasserstoff, $C_1$-$C_8$-Alkyl, durch Halogen, Cyano, Nitro, Hydroxy, Alkoxy oder U-$C_1$-$C_3$-Alkyl substituiertes

$C_1$-$C_8$-Alkyl, (T)-COOH oder (T)-$COOC_1$-$C_4$-Alkyl, $C_2$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

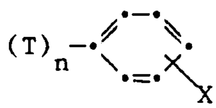 , $(T)_n$-Naphthyl, $(T)_n$-Si($C_1$-$C_8$-Alkyl$)_3$,

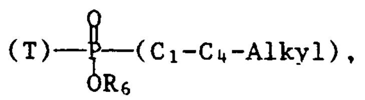 , 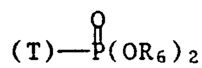 ; oder $(T)_n$—W ;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder -$CH_2$-, -$CH_2CH_2$- oder -$CH(CH_3)$-;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester.
6-Chlor-7-cyano-benzo-1,2,3-thiadiazol;
6-Chlor-benzo-1,2,3-thiadiazol-7-carbonsäure.

Wegen ihrer hervorragenden biologischen Aktivität sind als Wirkstoffe folgende Verbindungen bevorzugt:

Gruppe A (bekannte Verbindungen)

7-Carbonsäure-benzo-1,2,3-thiadiazol (Verb. 1.1);
7-Methoxycarbonyl-benzo-1,2,3-thiadiazol (Verb. 1.2;)

Gruppe B 1 (neue Verbindungen)

7-Ethoxycarbonyl-benzo-1,2,3-thiadiazol (Verb. 1.3 );
7-n-Propoxycarbonyl-benzo-1,2,3-thiadiazol (Verb. 1.4);
7-iso-Propoxycarbonyl-benzo-1,2,3-thiadiazol (Verb. 1.5);
7-n-Butoxycarbonyl-benzo-1,2,3-thiadiazol (Verb. 1.6);
7-sek.Butoxycarbonyl-benzo-1,2,3-thiadiazol (Verb. 1.7);
7-tert.Butoxycarbonyl-benzo-1,2,3-thiadiazol (Verb. 1.8);
7-Cyclopropylmethoxycarbonyl-benzo-1,2,3-thiadiazol (Verb. 1.28);
7-(2'-Phenethoxycarbonyl)-benzo-1,2,3-thiadiazol (Verb. 1.33);
7-Benzyloxycarbonyl-benzo-1,2,3-thiadiazol (Verb. 1.34);
7-Allyloxycarbonyl-benzo-1,2,3-thiadiazol (Verb. 1.44);
7-Propin-2-yloxycarbonyl-benzo-1,2,3-thiadiazol (Verb. 1.46);
2-(7-Benzo-1,2,3-thiadiazoyloxy-imino)-2-cyano-N-(ethylaminocarbonyl)-acetamid (Verb. 1.78);
Na-Salz der Benzo-1,2,3-thiadiazol-7-carbonsäure (Verb. 1.112);
K-Salz der Benzo-1,2,3-thiadiazol-7-carbonsäure (Verb. 1.113);
Triethylammoniumsalz der Benzo-1,2,3-thiadiazol-7-carbonsäure (Verb. 1.114);
7-(1-Phenethoxycarbonyl)-benzo-1,2,3-thiadiazol (Verb. 1.119);
7-(1-Naphthylmethoxycarbonyl)-benzo-1,2,3-thiadiazol (Verb. 1.116);
7-(Methylthio-carbonyl)-benzo-1,2,3-thiadiazol (Verb. 2.1);
7-(Ethylthio-carbonyl)-benzo-1,2,3-thiadiazol (Verb. 2.2);
7-(Benzylthio-carbonyl)-benzo-1,2,3-thiadiazol (Verb. 2.5);
7-[(Di-cyanomethyl)-amino-carbonyl]-benzo-1,2,3-thiadiazol (Verb. 3.13);
3-[(N-Benzo-1,2,3-thiadiazol-7-ylcarbonyl)-1,3,4-thiadiazol-2-ylamino]-2-methyl-propencarbonsäuremethylester (Verb. 3.28);
3-[(N-Benzo-1,2,3-thiadiazol-7-ylcarbonyl)-1,3,4-thiadiazol-2-ylamino]-2-ethyl-propencarbonsäuremethylester (Verb. 3.29);
1-(Benzo-1,2,3-thiadiazol-7-carbonyl)-2-(α-methylpropyliden)-hydrazin (Verb. 4.2);
1-(Benzo-1,2,3-thiadiazol-7-carbonyl)-2-(cyclobutyliden)-hydrazin (Verb. 4.8);
1-(Benzo-1,2,3-thiadiazol-7-carbonyl)-2-(cyclopentyliden)-hydrazin (Verb. 4.9);
1-(Benzo-1,2,3-thiadiazol-7-carbonyl)-2-(cyclohexyliden)-hydrazin (Verb. 4.10);
2-(Benzo-1,2,3-thiadiazol-7-carbonyl)-1-(2'-sec-butyl)-hydrazin (Verb. 5.2);
1-(Benzo-1,2,3-thiadiazol-7-carbonyl)-2-(cyclopentyl)-hydrazin (Verb. 5.7);
1-(Benzo-1,2,3-thiadiazol-7-carbonyl)-2-(cyclohexyl)-hydrazin (Verb. 5.8);
1-(Benzo-1,2,3-thiadiazol-7-carbonyl)-2-(cycloheptyl)-hydrazin (Verb. 5.9);
1-(Benzo-1,2,3-thiadiazol-7-carbonyl)-1,2-diacetyl-hydrazin (Verb. 6.7);
1-(Benzo-1,2,3-thiadiazol-7-carbonyl)-2-phenyl-hydrazin (Verb. 6.8)
1-(Benzo-1,2,3-thiadiazol-7-carbonyl)-2-pyridin-2′-yl-hydrazin (Verb. 6.9).

Gruppe B2 (neue Verbindungen)

7-n-Pentoxycarbonyl-benzo-1,2,3-thiadiazol (Verb. 1.9);
7-(4-Methoxy-benzyloxycarbonyl)-benzo-1,2,3-thiadiazol (Verb. 1.39);
7-(Cycloheximino-oxycarbonyl)-benzo-1,2,3-thiadiazol (Verb. 1.72);
7-(3-Hydroxy-n-propoxycarbonyl)-benzo-1,2,3-thiadiazol (Verb. 1.79);
1,2,5,6-Di-O-isopropyliden-3-(7-benzo-1,2,3-thiadiazoyl)-D-glucofuranose (Verb. 1.86);
7-Furfuryloxycarbonyl-benzo-1,2,3-thiadiazol (Verb. 1.96);
7-(1,2,4-Triazol-1-yl)-methoxycarbonyl-benzo-1,2,3-thiadiazol (Verb. 1.100);
7-(2-Pyridylmethoxycarbonyl)-benzo-1,2,3-thiadiazol (Verb. 1.101);
7-Trimethylsilyl-methoxycarbonyl-benzo-1,2,3-thiadiazol (Verb. 1.103);
7-[2-(Trimethylsilyl)-ethoxycarbonyl]-benzo-1,2,3-thiadiazol (Verb. 1.104);
7-Dimethylphosphono-ethoxycarbonyl-benzo-1,2,3-thiadiazol (Verb. 1.108);
7-Cyclohexyloxycarbonyl-benzo-1,2,3-thiadiazol (Verb. 1.135);
7-(1-Phenethyloxycarbonyl)-benzo-1,2,3-thiadiazol (Verb. 1.140);
7-(3-Methoxybenzyl)-benzo-1,2,3-thiadiazol (Verb. 1.144);
7-(Ethylthio-carbonyl)-benzo-1,2,3-thiadiazol (Verb. 2.2);
7-(n-Propylthio-carbonyl)-benzo-1,2,3-thiadiazol (Verb. 2.3);
7-(Benzylthio-carbonyl)-benzo-1,2,3-thiadiazol (Verb. 2.5);
7-(Carbamoyl-benzo-1,2,3-thiadiazol (Verb. 3.1);
7-N-Phenylcarbamoyl-benzo-1,2,3-thiadiazol (Verb. 3.6);

N-(7-Benzo-1,2,3-thiadiazoyl)-glycin (Verb. 3.9);
7-(N-Diallylcarbamoyl)-benzo-1,2,3-thiadiazol (Verb. 3.26);
6-Fluor-7-methoxycarbonyl-benzo-1,2,3-thiadiazol (Verb. 7.6);
6-Fluor-7-carboxy-benzo-1,2,3-thiadiazol (Verb. 7.8);
5-Fluor-7-benzyloxycarbonyl-benzo-1,2,3-thiadiazol (Verb. 7.52);
5-Fluor-7-carboxy-benzo-1,2,3-thiadiazol (Verb. 7.59);
5-Fluor-7-ethoxycarbonyl-benzo-1,2,3-thiadiazol (Verb. 7.61);
Untersuchungen über die Immunisierung von Pflanzen zum Schutz gegen Krankheiten sind in jüngerer Zeit in der Literatur publiziert worden (z.B. T. Kosuge et al., Plant-Microbe Interactions, S. Gianinazzi, Chapter 13, MacMillan, New York 1984; Pesticides, March 1985, Seiten 14, 15, A.N. Mukhopadhyay et al.).

Es wurde nun überraschenderweise gefunden, dass die Verbindungen der Formel I durch ihre erfindungsgemässe Verwendung den Befall von gesunden Pflanzen durch schädliche Mikroorganismen von vornherein verhindern und damit den befallsbedingten Schädigungen an Pflanzen vorbeugen. Der grosse Vorteil des erfindungsgemässen Verfahrens zur Behandlung von Pflanzen besteht darin, dass anstelle der direkten Einwirkung von chemischen Substanzen auf die pflanzenschädigenden Mikroorganismen eine Aktivierung und Stimulierung des pflanzeneigenen biologischen Abwehrsystems vor dem Befall der Pflanzen eintritt, so dass eine Gesunderhaltung der behandelten Pflanzen aus eigener Kraft ohne weiteren direkten Einsatz mikrobizider Substanzen während der Vegetationsperiode gewährleistet werden kann. Für die Wirkstoffe der Formel I ist somit vor allem charakteristisch, dass sie bei Anwendung umweltschonender Aufwandmengen keine direkte Wirkung auf die Schadorganismen ausüben, sondern stattdessen eine immunisierende Wirkung auf gesunde Pflanzen gegen Pflanzenkrankheiten besitzen. Direkte Wirkungen gegen Vertreter der wichtigsten Pilzgruppen (z.B. Fungi Imperfecti, Oomyceten) konnten in diesem Zusammenhang nicht nachgewiesen werden. Demzufolge werden durch die erfindungsgemässe Verwendung der Verbindungen der Formel I nachteilige Nebeneffekte, wie sie sonst bei der direkten Parasitenbekämpfung auf Pflanzen durch chemische Substanzen mehr oder weniger zu beobachten sind, vermieden, was vorteilhafterweise ein völlig ungestörtes Wachstum der Pflanzen zur Folge hat. Darüber hinaus kann bei einzelnen der unter die Formel I' fallenden neuen Verbindungen sowohl zusätzlich zu der pflanzenimmunisierenden Wirksamkeit als auch unabhängig davon eine mikrobizide, insbesondere pflanzenfungizide, Aktivität auftreten.

Die der Erfindung zugrundeliegende Wirkungsweise der Verbindungen der Formel I ist gleichzeitig auf eine allgemeine Erhöhung der Abwehrbereitschaft der behandelten Pflanzen gerichtet, so dass dadurch eine generelle antimikrobielle Resistenz gegen ein breites Spektrum von schädlichen Mikroorganismen erzielt wird. Das erfindungsgemässe Verfahren ist deshalb besonders für praktische Anwendungen geeignet. Die den Verbindungen der Formel I eigene systemische Aktivität bewirkt, dass sich der Schutzeffekt auch auf zuwachsende Pflanzenteile der behandelten Pflanzen erstreckt.

Das erfindungsgemässe immunisierende Verfahren ist gegen die den folgenden Klassen angehörenden phytopathogenen Pilze wirksam: Fungi imperfecti (z.B Botrytis, Helminthosporium, Fusarium, Septoria, Cercospora und Alternaria); Basidiomyceten (z.B. die Gattungen Hemileia, Rhizocotonia, Puccinia); Ascomyceten (z.B. Venturia, Podosphaera, Erysiphe, Monilinia, Uncinula).

Besonders vorteilhaft kann das Verfahren zur Immunisierung gegen folgende Schadorganismen eingesetzt werden:
Pilze, wie z.B. Oomyceten (z.B. Plasmopara viticola, Phytophthora infestans), Fungi imperfecti (z.B. Colletotrichum lagenarium, Piricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria; Erwinia (z.B. Erwinia amylovora; und Viren, wie z.B. das Tabakmosaikvirus.

Das erfindungsgemässe Verfahren kann zum Schutz von Pflanzen aus unterschiedlichen Nutzkulturen eingesetzt werden.

Für die hierin offenbarten Indikationsgebiete gelten im Rahmen dieser Erfindung z.B. folgende Pflanzenarten:
Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen)
Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Getreide (z.B. Weizen), Birne, Pfeffer, Kartoffeln, Tomate und Apfel.

Die Verbindungen der Formel I sind nach folgenden Verfahren herstellbar:

1. Verbindungen der Formel Ia

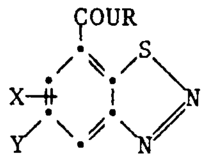

(Ia)

in welcher R, X, Y und U die unter Formel I angegebenen Bedeutungen besitzen, werden hergestellt, indem man umsetzt:

1.1 Verbindungen der Formel II

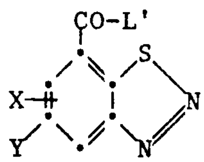

(II)

worin L′ eine Abgangsgruppe, z.B. Halogen, O-Acyl, wie z.B. der zum symmetrischen Anhydrid der Säure Ib gehörende Acylrest, oder 1-Imidazoyl bedeutet,
mit Verbindungen der Formel III

RUH (III)

a) im Ueberschuss des Reaktanden RUH oder
b) in Gegenwart einer organischen Base entweder mit oder ohne 4-Dialkylaminopyridin als Katalysator in inerten Lösungsmitteln oder
c) in Gegenwart anorganischer Basen, wobei jeweils im Temperaturbereich von -10° bis 180°C, vorzugsweise 0° bis 100°C, gearbeitet wird; und

1.2 Verbindungen der Formel Ib

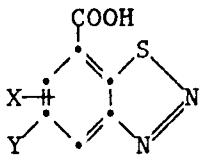

(Ib)

mit Verbindungen der Formel III im Ueberschuss oder in einem inerten Lösungsmittel in Gegenwart einer Säure, wie Schwefelsäure, Salzsäure, p-Toluolsulfonsäure oder Bortrifluorid-Diethylether-Komplex, oder von Dicyclohexylcarbodiimid bei Temperaturen von -10° bis 180°C, vorzugsweise 0° bis 140°C; und

2. Verbindungen der Formel Ic

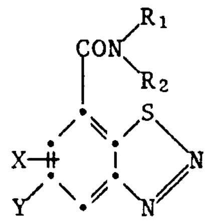

(Ic)

in welcher die Symbole $R_1$, $R_2$, X und Y die unter Formel I angegebenen Bedeutungen besitzen, werden hergestellt, indem man umsetzt:

2.1 Verbindungen der Formel II mit Verbindungen der Formel IV

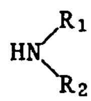

(IV)

a) im Ueberschuss des Reaktanden $HN(R_1)R_2$ oder

b) in Gegenwart einer organischen Base entweder mit oder ohne 4-Dialkylaminopyridin als Katalysator in inerten Lösungsmitteln oder

c) in Gegenwart anorganischer Basen, wobei jeweils im Temperaturbereich von -10° bis 160°C, vorzugsweise 0° bis 100°C, gearbeitet wird; und

3. Verbindungen der Formel Id

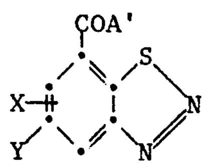

(Id)

worin A′ den Rest $U^1N(=C)_n(R_3)R_4$ darstellt und X, Y, $R_3$, $R_4$, $R_5$ und n die unter Formel I angegebenen Bedeutungen besitzen, werden hergestellt, indem man

3.1 Verbindungen der Formel Ie

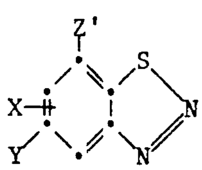

(Ie)

in welcher Z′ die Gruppe COOH, COCl, $COOAlk^1$ oder einen Acyloxycarbonylrest, wie z.B.

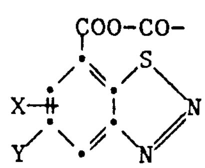

,

Benzoyloxycarbonyl oder Acetyloxycarbonyl darstellt, worin $Alk^1$ $C_1$-$C_4$-Alkyl bedeutet, in Gegenwart einer Base mit Hydrazin-Derivaten der Formeln V oder VI

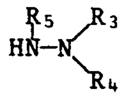 (V) oder 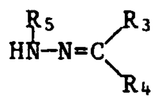 (VI)

in einem inerten Lösungsmittel bei Temperaturen von -10° bis 180°C, bevorzugt 0° bis 100°C; oder
3.2 Verbindungen der Formel Ie schrittweise zuerst mit Hydrazin umsetzt und dann die erhaltenen Hydrazinverbindungen

3.2.1 mit dem Alkylierungsmittel $R_3$-L oder $R_4$-L, worin L eine Abgangsgruppe darstellt, in einem inerten Lösungsmittel bei Temperaturen von 0° bis 160°C, bevorzugt 20° bis 120°C; oder

3.2.2 mit einem Aldehyd oder Keton der Formel $R_3(R_4)C=0$, worin $R_3$ und $R_4$ die unter Formel I angegebenen Bedeutungen haben, mit oder ohne Zusatz einer organischen oder anorganischen Säure bei Temperaturen von -10° bis 150°C, vorzugsweise bei 20° bis 100°C, und anschliessend gewünschtenfalls

3.2.3 mit einem Alkylierungsmittel L-$R_5$, worin L eine Abgangsgruppe darstellt, in Gegenwart einer starken Base in einem inerten Lösungsmittel bei Temperaturen von -80° bis 120°C, vorzugsweise -40° bis 80°C; oder gewünschtenfalls

3.2.4 die unter (3.2.1) hergestellten Hydrazonderivate

a) mit Wasserstoff unter einem Druck von 1 bis $30 \cdot 10^5$ Pa in Gegenwart eines Katalysators im Gemisch mit Aktivkohle in einem inerten Lösungsmittel bei Temperaturen von 0° bis 100°C hydriert oder

b) mit einem komplexen Metallhydrid, wie z.B. Na-Cyanoborhydrid, in einem inerten Lösungsmittel bei Temperaturen von -10° bis 80°, vorzugsweise 0° bis 50°C, behandelt; und

4. Verbindungen der Formel If

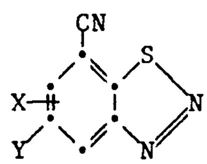 (If)

in welcher die Symbole X und Y die unter Formel I angegebenen Bedeutungen besitzen, werden hergestellt, indem man
Verbindungen der Formel Ig [hergestellt nach Verfahren (2)]

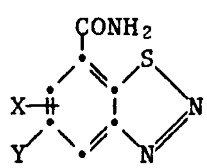 (Ig)

mit einem Dehydratisierungsagens in einem inerten Lösungsmittel oder ohne Lösungsmittel bei Temperaturen von -10° bis 250°C behandelt, wobei als Dehydratisierungsmittel in Frage kommt:

a) Trifluoressigsäureanhydrid in Gegenwart einer Base, wie z.B. Pyridin, in einem inerten Lösungsmittel, wie z.B. Tetrahydrofuran oder Dioxan, bei Temperaturen von -10° bis 40°C; oder

b) Chlorsulfonylisocyanat in einem inerten Lösungsmittel, wie z.B. Tetrahydrofuran, bei Temperaturen von 0° bis 65°C und anschliessender Behandlung mit Dimethylformamid (vgl. Org. Synth. 50, 18 oder Chem. Ber. 100, 2719); oder

c) Phosphorpentoxid mit oder ohne inertem Lösungsmittel, wie z.B. 1,2-Dichlorethan, Xylol oder Chlorbenzol, gegebenenfalls im Bombenrohr unter erhöhtem Druck, bei 50° bis 250°C (vgl. Fieser, Reagents for Organic Synthesis 1, 871);

5.1 Verbindungen der Formel $IL^1$

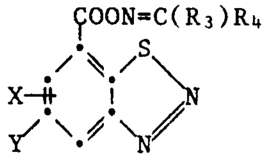

(IL[1]),

in welcher $R_3$, $R_4$, X und Y die unter Formel I angegebenen Bedeutungen besitzen, werden hergestellt, indem man Oximderivate der Formel

HO-N = C($R_3$)$R_4$

mit aktivierten Säurederivaten der Formel

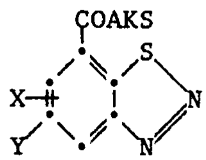

,

worin AKS ein Halogen, ein O-Acyl, wie z.B. der O-Acylrest der freien Säure vorstehender Formel, Acetoxy oder Benzoyloxy, oder 1-Imidazolyl darstellt, in einem inerten Lösungsmittel und einer Base bei -20°C bis 120°C, bevorzugt bei 0° bis 50°C, oder die freie Säure (= Ib) in Gegenwart von Dicyclohexylcarbodiimid unter den gleichen Bedingungen umsetzt (Lit. Ber. 83, 186 (1950); Houben-Weyl E5, S. 773).

5.2 Verbindungen der Formel IL[2]

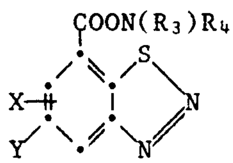

(IL[2])

in welcher $R_3$, $R_4$, X und Y die unter Formel I angegebenen Bedeutungen besitzen, werden hergestellt durch Reduktion von Verbindungen der Formel IL[1]

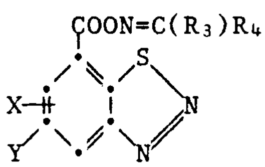

(IL[1])

a) mit einem Silan, wie z.B. Triethylsilan, in Gegenwart einer Säure, wie z.B. Trifluoressigsäure, bei 0° bis 80°C, oder

b) mit Natriumcyanoborhydrid in Gegenwart einer organischen Säure, wie z.B. Essigsäure, bei 0° bis 80°C, oder

c) auf katalytischen Wege, z.B. mit Pt/$H_2$.

In einem speziellen Syntheseverfahren werden Verbindungen der Formel IL[2], worin $R_3$ und $R_4$ Wasserstoff bedeuten, hergestellt, indem man ein Säurehalogenid oder ein Säureanhydrid einer Säure der Formel Ib

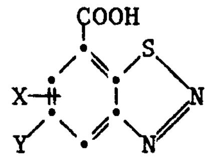

(Ib)

mit N,O-Bis-trimethylsilylhydroxylamin in Gegenwart einer Base, wie z.B. Butyllithium, in einem inerten Lösungsmittel bei -80° bis 60°C, bevorzugt -50° bis 50°C, umsetzt.

Die in den vorstehenden Formeln beschriebenen 7-Carbonsäureester, worin U Sauerstoff bedeutet und R die unter Formel I angegebenen Bedeutungen besitzen soweit sie keine Reste darstellen, die OH-Gruppen, Silizium-oder Phosphor-haltige Gruppen umfassen, können nach in der Literatur beschriebenen Umesterungsmethoden ineinander überführt werden.

Die Vorstufen-Verbindungen der Formeln Ib, Ie, und II lassen sich nach bekannten Methoden, z.B. im Rahmen der folgenden Synthese herstellen:

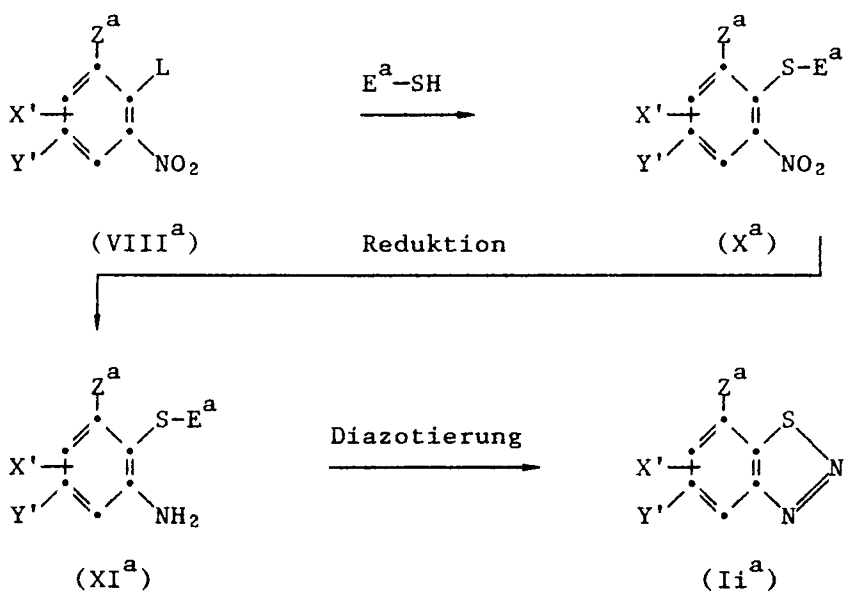


worin bedeuten:

Y′ Wasserstoff, Halogen, $SO_3H$, $SO_3M$ oder Hydroxy;

X′ Wasserstoff, Halogen, Methyl, Methoxy oder COOH;

$E^a$ eine leicht abspaltbare Gruppe wie z.B. Wasserstoff oder $C_1$-$C_4$-Alkyl, z.B. Methyl, Ethyl oder Isopropyl, oder Benzyl;

L Halogen oder Nitro;

$Z^a$ die Gruppe COOH oder $COOC_1$-$C_4$-Alkyl.

Falls $Z^a$ eine freie Säuregruppe (-COOH) bedeutet, kann diese mit den üblichen Methoden in eine Estergruppe, z.B. den Methylester, in ein Säurehalogenid, z.B. das Säurechlorid, oder in das symmetrische oder ein gemischtes Säureanhydrid, z.B. mit dem Acetyl- oder Benzoylrest, umgewandelt werden.

Auf einem parallelen Syntheseweg werden diejenigen Verbindungen der Formel I hergestellt, die durch die Formel I′[b]

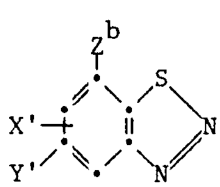

(I′[b])

dargestellt werden, worin
$Z^b$ die unter Formel I angegebenen Bedeutungen von Z besitzt soweit sie keine Reste darstellen, die primäre oder sekundäre Aminogruppen, UH- oder Nitro-Gruppen, $Si(C_1\text{-}C_8\text{-Alkyl})_3$ oder Phosphor-haltige Gruppen enthalten und X′ und Y′ die vorstehend unter Formel $Ii^a$ angegebenen Bedeutungen besitzen.

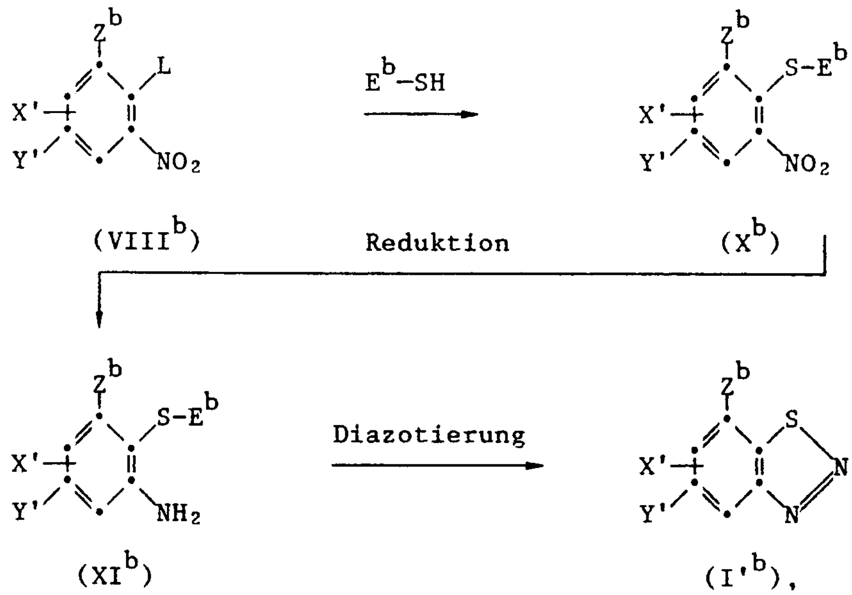


($VIII^b$) ($X^b$) ($XI^b$) ($I'^b$),

worin $E^b$ eine leicht abspaltbare Gruppe wie z.B. Wasserstoff, $C_1$-$C_{16}$-Alkyl, z.B. Methyl, Ethyl, Isopropyl, n-Dodecyl, oder Benzyl oder Acyl, z.B. Acetyl oder den Sulfonsäure-Rest ($-SO_3H-$) oder Cyano oder darüber hinaus als Teil einer Disulfid-Brücke einen zweiten Rest

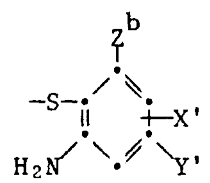

bedeutet, und L die unter Formel $VIII^a$ angegebene Bedeutung besitzt.

Darüber hinaus können Verbindungen der Formel Ik

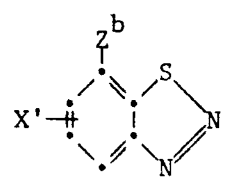

(Ik)

nach einem speziellen Verfahren (C) hergestellt werden, indem man eine Verbindung der Formel XI′

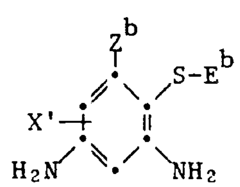

(XI')

in welcher X′, $E^b$ und $Z^b$ die vorstehend unter Formel XI′ angegebenen Bedeutungen besitzen, in saurem Medium mit einer Nitritverbindung bei -40° bis 30°C diazotiert und im gleichen oder einem zweiten Reaktionsgefäss mit einem Reduktionsmittel bei -40° bis 80°C, vorzugsweise bei -30° bis 30°C, behandelt, wobei das Reduktionsmittel vor, nach oder gleichzeitig mit der Nitritverbindung hinzugefügt werden kann.

In einer besonderen Ausführungsform des Verfahrens (C) stellt $Z^b$ in den vorstehenden Formeln XI′ und Ik einen Säureester ($COOR^a$) dar, X′ bedeutet Wasserstoff und $R^a$ in seinen Bedeutungen R entspricht mit Ausnahme von Resten mit UH- oder Nitro-Gruppen sowie von Silizium- oder Phosphor-haltigen Resten, wobei Verbindungen der Formel

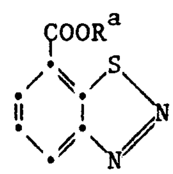

hergestellt werden, indem man eine Verbindung der Formel

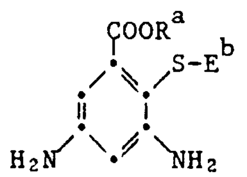

in saurem Medium mit einer Nitritverbindung bei Temperaturen von -20° bis 30°C diazotiert und im gleichen Reaktionsgefäss bei Temperaturen von -20° bis 80°C, vorzugsweise 20° bis 30°C reduziert, wobei das Reduktionsmittel vor, nach oder gleichzeitig mit der Nitritverbindung hinzugefügt werden kann.

Mit einem weiteren speziellen Verfahren können Verbindungen der Formel Ik′

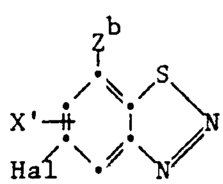 (Ik')

hergestellt werden, indem man eine Verbindung der Formel XI′

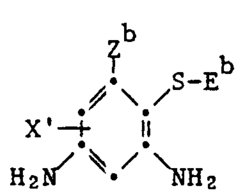 (XI')

in welcher X′, $E^b$ und $Z^b$ die vorstehend unter Formel XI′ angegebenen Bedeutungen besitzen, in saurem Medium mit einer Nitritverbindung bei -40° bis 30°C diazotiert und das Diazoniumsalz

a) mit einem Kupfer-Halogenid bei Temperaturen von -30° bis 180°C umsetzt oder

b) falls Hal Fluor bedeutet, mit Flusssäure oder Tetrafluoroborsäure, gegebenenfalls in Gegenwart von Kupferfluoridsalzen, behandelt (Lit. Houben-Weyl 5/3, 216).

Im vorstehend beschriebenen Verfahren (C) finden als saures Reaktionsmedium wässrige Verdünnungen von anorganischen Säuren, wie z.B. Halogenwasserstoffsäuren, Phosphorsäure, Schwefelsäure oder Borfluorwasserstoffsäure, Anwendung; jedoch können auch geeignete organische Säuren verwendet werden, wobei den Säuren inerte organische Lösungsmittel, wie z.B. Tetrahydrofuran oder Dioxan, zugesetzt werden können. Als Nitrite sind sowohl anorganische Nitrit-Verbindungen, wie z.B. die Alkali- und Erdalkalisalze, wie auch organische Nitrit-Verbindungen, wie z.B. Alkylnitrite, geeignet. Als Reduktionsmittel können beispielsweise Alkohole, wie z.B. Ethanol, oder Unterphosphorige Säure, metallisches Kupfer oder Trialkylsilane, wie z.B. Triethylsilan, sowie Ferrocyanide oder Ferrocene, wie z.B. Decamethylferrocen dienen. Der Reduktionsschnitt kann falls erwünscht auch in Gegenwart weiterer Zusätze wie z.B. Kronenether oder Polyethylenglykol durchgeführt werden.

Die beschriebene Methode (C) stellt ein neues chemisch eigenartiges Verfahren dar, mit welchem annellierte Thiadiazolverbindungen in vorteilhafter Weise zugänglich sind.

Die Synthese der Vorstufen der Verbindung XI′

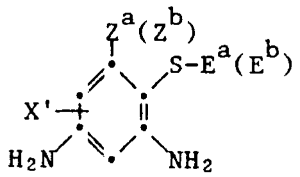

(XI′)

wird durchgeführt, indem man Verbindungen der Formeln VIII′, VIII″ und IX

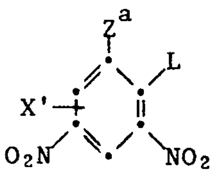

(VIII′) ,

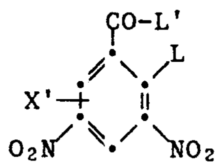

(VIII″) ,

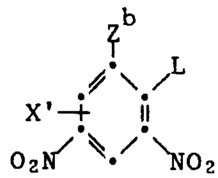

(IX) ,

wobei in den vorstehenden Formeln die Substituenten folgende Bedeutungen besitzen:

X′ Wasserstoff, Halogen, Methyl, Methoxy oder COOH;

$E^a$ eine leicht abspaltbare Gruppe wie z.B. Wasserstoff, $C_1$-$C_4$-Alkyl, z.B. Methyl, Ethyl oder Isopropyl, oder Benzyl;

$E^b$ eine leicht abspaltbare Gruppe wie z.B. Wasserstoff, $C_1$-$C_{16}$-Alkyl, z.B. Methyl, Ethyl, Isopropyl, n-Dodecyl, oder Benzyl oder Acyl, z.B. Acetyl oder den Sulfonsäure-Rest ($-SO_3H-$) oder Cyano oder darüber hinaus als Teil einer Disulfid-Brücke einen zweiten Rest

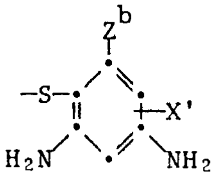

;

L Halogen oder Nitro;

L′ eine Abgangsgruppe, z.B. Halogen, O-Acyl, wie z.B. der zum symmetrischen Anhydrid der Säure Ib gehörenden Acylrest, oder 1-Imidazoyl

$Z^a$ die Gruppe COOH oder $COOC_1$-$C_4$-Alkyl;

$Z^b$ die unter Formel I angegebenen Bedeutungen von Z besitzt soweit sie keine Reste darstellen, die primäre oder sekundäre Aminogruppen, Nitro-oder UH-Gruppen, $Si(C_1$-$C_8$-$Alkyl)_3$ oder Phosphor-haltige Gruppen enthalten;

mit Verbindungen der Formeln HS-$E^a$ oder HS-$E^b$ in Gegenwart einer Base, wie z.B. Alkalicarbonat (z.B. $Na_2CO_3$ oder $K_2CO_3$) oder Erdalkalicarbonat (z.B. $MgCO_3$), Alkoxiden (z.B. Na-Alkoholaten oder K-tert.Butylat), oder Alkalihydriden (z.B. Na-Hydrid) in einem inerten, vorzugsweise dipolar aprotischen Lösungsmittel (z.B. Dimethylsulfoxid, Dimethylformamid, Hexamethylphosphorsäuretriamid, N-Methylpyrrolidon, Acetonitril, Dioxan oder Tetrahydrofuran) bei Temperaturen von -10° bis 120°C, vorzugsweise 0° bis 60°C, zu Verbindungen der Formel X′

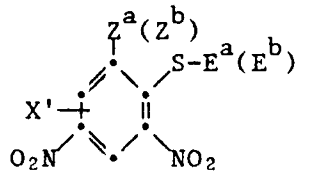

(X')

umsetzt, welche entweder durch katalytische oder metallische Reduktion in die Verbindungen der Formel XI′

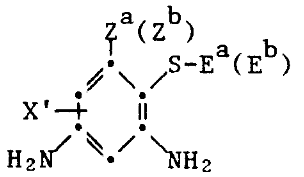

(XI')

übergeführt werden.

Für die katalytischen Reduktion können beispielsweise Raney-Nickel sowie Palladium-, Platin- oder Rhodium-Katalysatoren verwendet werden; die Reduktion kann bei Normaldruck oder leicht erhöhtem Druck bei Temperaturen von 0° bis 150°C durchgeführt werden. Als Lösungsmittel kommen z.B. Tetrahydrofuran oder Dioxan in Frage.

Für die metallische Reduktion können z.B. Eisen/Salzsäure, Eisen/Essigsäure, Zinn/Salzsäure, Zink/Salzsäure, Zink/Essigsäure, Kupfer/Ameisensäure verwendet werden.

Ferner kommen Zinn(II)chlorid/Salzsäure, Nickel/Hydrazin, Titantrichlorid, Alkalisulfide oder Na-Dithionit als reduzierende Agentien in Frage. Die Reduktion kann bei Temperaturen von 0° bis 120°C durchgeführt werden. Dabei können als Lösungsmittel Wasser oder Alkohole, wie z.B. Methanol, Ethanol, n-Propanol oder iso-Propanol eingesetzt werden.

Darüber hinaus lassen sich, falls $Z^a$ die Säurefunktion (-COOH) darstellt, spezielle Derivate der Formel IX, in denen $Z^b$ die Reste -COUR′-, -CON($R_1$)$R_2$ und -CON($R_5$)N($R_3$)$R_4$ bedeuten, herstellen, indem man

a) in bekannter Weise das vorerst synthetisierte Derivat der Formel VIII$^a$ (Y′ = $NO_2$) mit einem Alkohol der Formel HUR′ oder einem Amin der Formel HN($R_1$)$R_2$ oder einem Hydrazid der Formel HN($R_5$)N($R_3$)-$R_4$ in Gegenwart einer geeigneten Base, gegebenenfalls katalysiert durch Zusatz von Dimethylaminopyridin, in einem inerten Lösungsmittel bei Temperaturen von -20° bis 170°C, bevorzugt 0° bis 110°C umsetzt, oder

b) eine Verbindung der Formel VIII' ($Z^a$ = COOH) in Gegenwart von Dicyclohexylcarbodiimid mit einem Alkohol der Formel HUR' oder einem Amin der Formel HN($R_1$)$R_2$ oder einem Hydrazid der Formel HN-($R_5$)N($R_3$)$R_4$ in einem inerten Lösungsmittel bei Temperaturen von 0° bis 120°C, bevorzugt 10° bis 80°C, umsetzt.

Die Bedeutungen der Reste $R_1$ bis $R_5$ und das Symbol U sind im vorstehenden Text bereits beschrieben und R' hat die Bedeutung von R mit Ausnahme der Reste (T)-P(O)($OR_6$)-($C_1$-$C_4$-Alkyl), (T)-PO-($OR_6$)$_2$ und $(T)_n$-Si($C_1$-$C_8$-Alkyl)$_3$.

Die Verbindungen der Formel X' sind bekannt oder lassen sich mit in der Literatur bekannten Verfahren herstellen. Nach einem speziellen Verfahren sind einige dieser Derivate wie folgt herstellbar:

Verbindungen der Formel

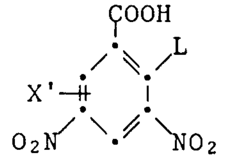

werden zuerst in Gegenwart von 2 Aequivalenten einer Base, wie beispielsweise einem Alkalicarbonat (z.B. $Na_2CO_3$ oder $K_2CO_3$) oder einem Erdalkalicarbonat (z.B. $MgCO_3$) oder einem Metallhydrid (z.B. NaH oder LiH) mit einer Verbindung der Formel HS-$E^a$ oder HS-$E^b$, in einem inerten, vorzugsweise dipolar aprotischen Lösungsmittel, wie z.B. Dimethylsulfoxid, Dimethylformamid, Hexamethylphosphonsäuretriamid oder N-Methylpyrrolidon, umgesetzt und das gebildete Derivat mit einem Alkylierungsmittel $R^a$-L", worin L" eine Abgangsgruppe, wie z.B. Halogen, vorzugsweise Jod, oder $-OSO_2R^a$, bedeutet, wobei $R^a$-L" z.B. Dimethylsulfat sein kann, verestert:

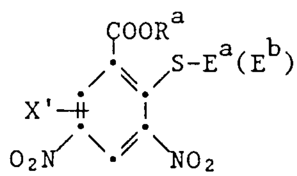

In den vorstehenden Formeln bedeuten $R^a$ ein unter R für Formel I definierter aliphatischer oder araliphatischer Rest und die Reste L, S-$E^a$,S-$E^b$ und X' die unter den Formeln VIII', VIII" und IX angegebenen Bedeutungen.

In den vorstehend beschriebenen Verfahren sind unter Basen, wenn nicht speziell angegeben, sowohl anorganische als auch organische Basen zu verstehen. Dazu zählen als anorganische Basen beispielsweise Hydroxide, Hydrogencarbonate, Carbonate und Hydride des Lithiums, Natriums, Kaliums, Calciums und Bariums, sowie Alkaliamide, wie z.B. $NaNH_2$ oder Alkyllithium verbindungen, wie z.B. n-Butyllithium. Als organische Basen sind beispielsweise zu nennen: Amine, insbesondere tertiäre Amine wie z.B. Trimethylamin, Triethylamin, Tripropylamin, Tributylamin, Tribenzylamin, Tricyclohexylamin, Triamylamin, Trihexylamin, N,N-Dimethylanilin, N,N-Dimethyltoluidin, N,N-Dimethyl-p-amino-pyridin, N-Methylpyrrolidin, N-Methylpiperidin, N-Methyl-pyrrolidin, N-Methylimidazol, N-Methylpyrrol, N-Methylmorpholin, N-Methylhexamethylenimin, Pyridin, Chinolin, alpha-Picolin, β-Picolin, Isochinolin, Pyrimidin, Acridin, N,N,N'N'-Tetra-methylethylendiamin, N,N,N'N'-Tetraethylethylendiamin, Chinoxalin, N-Propyldiisopropylamin, N,N-Dimethylcyclohexylamin, 2,6-Lutidin, 2,4-Lutidin oder Triethylendiamin.

Als inerte Lösungsmittel sind in Anpassung an die jeweiligen Reaktionsbedingungen beispielsweise zu nennen:

Halogenkohlenwasserstoffe, insbesondere Chlorkohlenwasserstoffe, wie z.B. Tetrachorethylen, Tetrachlorethan, Dichlorpropan, Methylenchlorid, Dichlorbutan, Chloroform, Chlornaphthalin, Dichlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Jodbenzol, Dichlorbenzol, Dibrombenzol, Chlortoluol, Trichlorbenzol; Ether, wie z.B. Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, Dichlordiethylether, Methylcellosolve; Alkohole; wie z.B. Methanol, Ethanol, n-Propanol, iso-Propanol, n-Butanol; iso-Butanol; Nitrokohlenwasserstoffe wie z.B. Nitromethan, Nitroethan, Nitrobenzol, Chlornitrobenzol, o-Nitrotoluol; Nitrile wie z.B. Acetonitril, Butyronitril, Isobutyronitril, Benzonitril, m-Chlorbenzonitril; aliphatische oder cycloaliphatische Kohlenwasserstoffe, wie z.B. Heptan, Pinan, Nonan, Cymol, Benzinfraktionen innerhalb eines Siedepunktintervalls von 70° bis 190°C, Cyclohexan, Methylcyclohexan, Dekalin, Petrolether, Hexan, Ligroin, Trimethylpentan, Trimethylpentan, 2,3,3-Trimethylpentan, Octan; Ester wie z.B. Ethylacetat, Acetessigester, Isobutylacetat; Amide, wie z.B. Formamid, Methylformamid, Dimethylformamid; Ketone, wie Aceton, Methylethylketon, gegebenenfalls auch Wasser. Auch Gemische der genannten Lösungs- und Verdünnungsmittel kommen in Betracht.

Die vorstehend beschriebenen Herstellungsverfahren basieren, soweit nicht neu, auf bekannten Synthesemethoden, die der folgenden Literatur entnommen werden können:

The Chemistry of Heterocyclic Compounds with Nitrogen and Sulfur or Nitrogen, Sulfur and Oxygen, Interscience Publ., New York 1952; P. Kirby et al. J. Chem. Soc. (C) 321 (1967) und 2250 (1970) und 3994 (1971); FR-PS 1 541 415; J. Org. Chem. 27, 4675 (1962); Deutsche Offenlegungsschriften 2 400 887 und 2 504 383; SU-PS 400 574 [Chem. Abstr. 80(9)47661 h]; Org. Synth. Coll. Vol. I, 125; Tetrahedr. 21, 663 (1965).

Bei den Verbindungen der Formeln X, X', XI und XI' handelt es sich um neue Stoffe, während die Verbindungen der Formeln VIII, VIII' und IX teilweise neu sind. Die neuen Verbindungen stellen einen Teil der vorliegenden Erfindung dar.

Die im Rahmen der Erfindung zur Anwendung gelangenden pflanzenschützenden Mittel, welche die Verbindungen der Formel I als Aktivstoffe enthalten, sind ebenfalls als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden. Besonders vorteilhafte applikationsfördernde Zuschlagstoffe sind ferner natürliche (tierische oder pflanzliche) oder synthetische Phospholipide aus der Reihe der Kephaline und Lecithine.

Als oberflächenaktive Verbindungen kommen je nach Art des zu formulierenden Wirkstoffes der Formel I nichtionogene, kation-und/oder anionaktive Tenside mit gutem Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-% insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung.

1. Herstellungsbeispiele

Beispiel 1.1: Herstellung von 2-Chlor-3-nitrobenzoesäuremethylester (Zwischenprodukt).

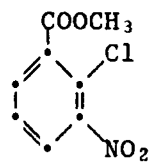

50,0 g (0,248 Mol) 2-Chlor-3-nitrobenzoesäure werden in 500 ml Methanol gelöst und mit 20 ml konzentrierter Schwefelsäure versetzt. Nach 24 Stunden Rückfluss wird das Gemisch auf Eis/Wasser gegossen und der weisse Niederschlag filtriert, mit Wasser gewaschen und getrocknet.
Ausbeute: 53 g (99 % d.Th.); Smp. 68 ° C.

Beispiel 1.2: Herstellung von 2-Benzylthio-3-nitrobenzoesäuremethylester (Zwischenprodukt)

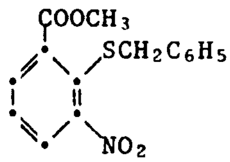

45,4 g (0,21 Mol) 2-Chlor-3-nitro-benzoesäuremethylester und 24,8 ml (0,21 Mol) Benzylmercaptan werden in 420 ml Dimethylformamid gelöst, mit 29,2 g (0,21 Mol) Kaliumcarbonat versetzt und 8 Stunden bei 80 ° C gerührt. Dann wird auf Eis/Wasser gegossen, zweimal mit Essigsäureethylester extrahiert, die Extrakte mit Wasser gewaschen, über Magnesiumsulfat getrocknet und eingedampft.
Ausbeute: 62,7 g (98,5 % d.Th.) Oel.

Beispiel 1.3: Herstellung von 3-Amino-2-benzylthio-benzoesäuremethylester (Zwischenprodukt)

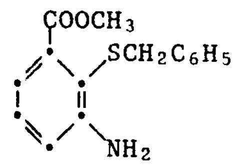

62,7 g (0,207 Mol) 2-Benzylthio-3-nitrobenzoesäuremethylester werden in 700 ml Tetrahydrofuran gelöst, mit 14 g Raney-Nickel versetzt und bei 20-28°C hydriert.
Ausbeute: 54,5 g (96 % d.Th.)

Beispiel 1.4: Herstellung von 5-Brom-2-chlor-3-nitrobenzoesäuremethylester (Zwischenprodukt)

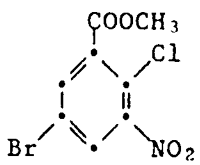

Zu 51,0 g (0,182 Mol) 5-Brom-2-chlor-3-nitrobenzoesäure und 500 ml Methanol werden 20 ml konzentrierte Schwefelsäure getropft. Das Gemisch wird dann 16 Stunden am Rückfluss gekocht. Anschliessend wird mit einem Eisbad gekühlt und der ausgefallene Niederschlag filtriert. Die Mutterlauge wird eingeengt, mit Wasser versetzt und der ausgefallene Niederschlag filtriert.
Ausbeute: 50,2 g (94 % d.Th.). Smp. 69°C

Beispiel 1.5: Herstellung von 2-Benzylmercapto-5-brom-3-nitrobenzoesäuremethylester (Zwischenprodukt)

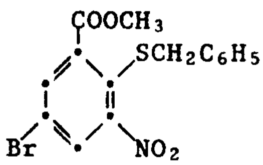

71,6 g (0,58 Mol) Benzylmercaptan werden in 2,9 l Methanol/Wasser (8:2) gelöst und 79,8 g (0,58 Mol) Kaliumcarbonat zugegeben. Bei 0-5°C werden unter Rühren 170 g (0,58 Mol) 5-Brom-2-chlor-3-nitrobenzoesäuremethylester portionenweise während 2,5 Stunden zugegeben. Dann noch zwei weitere Stunden gerührt und anschliessend die Innentemperatur auf 20°C steigen gelassen. Darauf wird der ausgefallene Niederschlag filtriert, mit etwas Wasser und anschliessend mit 500 ml Methanol/Wasser gewaschen. Nach Trocknung wurden 208 g (94 %) hellgelbes Produkt erhalten. Die Umkristallisation in 400 ml Methanol ergab 190 g (86 % d.Th.) Produkt mit einem Smp. von 65-66°C.

Beispiel 1.6: Herstellung von 3-Amino-2-benzylthio-benzoesäuremethylester (Zwischenprodukt)

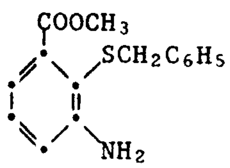

156,25 g (0,408 Mol) 2-Benzylthio-5-brom-3-nitro-benzoesäuremethylester werden in 3 l Tetrahydrofuran in Gegenwart von 60 g Pd/C (5 %) hydriert. Nach Reduktion der Nitrogruppe werden weitere 30 g Pd/C (5

%) und 45,4 g (0,448 Mol) Triethylamin zugegeben und die Hydrierung fortgesetzt. Dann wird der Katalysator abfiltriert und die Lösung eingeengt. Der ölige Rückstand wird in Essigsäureethylester aufgenommen, dreimal mit Wasser gewaschen, über Magnesiumsulfat getrocknet, filtriert und die Lösung eingedampft. Das erhaltene Produkt (111 g) wurde direkt weiter verarbeitet.

Beispiel 1.7: Herstellung von 7-Methoxycarbonylbenzo-1,2,3-thiadiazol

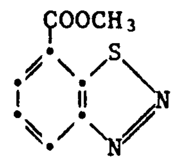

475 g (1,74 Mol) 3-Amino-2-benzylthiobenzoesäuremethylester werden bei 35°C langsam zu 1,18 l konzentrierter Salzsäure in 520 ml Wasser gegeben, wobei sich das Hydrochlorid bildet. Man rührt 15 Minuten bei der gleichen Temperatur weiter und kühlt dann auf -5°C ab. Darauf wird eine Lösung von 120 g Natriumnitrit in 520 ml Wasser während 2,5 Stunden zugetropft. Nach Beendigung des Zutropfens wird 2 Stunden bei 0° und weitere 2 Stunden bei 20°C gerührt. Das Reaktionsgut wird filtriert, mit Wasser ausgewaschen und abgepresst. Nach Umkristallisation in Essigsäureethylester/Hexan wurden 292 g (86 % d.Th) vom Smp. 134-135°C erhalten.

Beispiel 1.8: Herstellung von Benzo-1,2,3-thiadiazol-7-carbonsäure

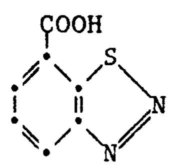

100 g (0,51 Mol) 7-Methoxycarbonylbenzo-1,2,3-thiadiazol werden in 1000 ml Wasser suspendiert. Dann werden 310 ml 2 N Natronlauge und 5 ml Dioxan dazugegeben. Das Reaktionsgemisch wird auf 40°C erwärmt und 4 Stunden bei dieser Temperatur gerührt. Dann wird auf 10°C gekühlt, nochmals 1000 ml Wasser zugegeben und mit 310 ml 2N Salzsäure neutralisiert. Der erhaltene Niederschlag wird filtriert, leicht luftgetrocknet, in Tetrahydrofuran gelöst, die Lösung über Magnesiumsulfat getrocknet, filtriert und eingeengt. Die Kristalle werden in Hexan aufgeschlämmt, filtriert und getrocknet.
Ausbeute: 91 g (98 % d.Th.); Smp. 261-263°C.

Beispiel 1.9a: Herstellung von Benzo-1,2,3-thiadiazol-7-carbonsäurechlorid (Zwischenprodukt)

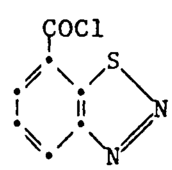

12,54 g (0,070 Mol) Benzo-1,2,3-thiadiazol-7-carbonsäure werden mit 80 ml Thionylchlorid gemischt. Das Gemisch wird erwärmt und während 8 Stunden bei einer Badtemperatur von 90°C gehalten. Anschliessend wird das überschüssige Thionylchlorid am Rotavapor bei einer Badtemperatur von 40°C abdestilliert. Das erhaltene Oel wurde fest; Smp. 107°C.

Für die weiteren Umsetzungen wird das erhaltene Säurechlorid in Toluol gelöst und direkt weiter verwendet.

Beispiel 1.9b: Herstellung des symmetrischen Anhydrids der 1,2,3-Benzthiadiazol-7-carbonsäure

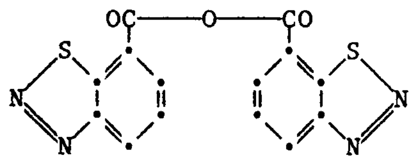

3 g 1,2,3-Benzthiadiazol-7-carbonsäure werden in 50 ml Acetanhydrid während 24 Stunden unter Rückfluss gekocht. Dann wird die dünne Suspension unter Vakuum eingedampft, der feste Rückstand mit Ether aufgeschlämmt und abfiltriert. Es resultieren 4,3 g Anhydrid vom Smp. 117-119°C. Die gleiche Verbindung wird z.B. auch durch Erwärmen der Carbonsäure mit Bis-(2-oxo-3-oxazolidinyl)-phosphinsäurechlorid in trockenem Tetrahydrofuran erhalten (vgl. Synthesis 1981, 616).

Beispiel 1.10: Herstellung von 7-Cyano-benzo-1,2,3-thiadiazol

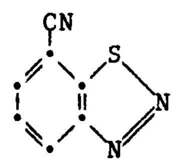

4,0 g (0,022 Mol) Benzo-1,2,3-thiadiazol-7-yl-carbonsäureamid werden in 35 ml Tetrahydrofuran gelöst und mit 3,6 ml (0,045 Mol) Pyridin versetzt. Dann wird auf 3° gekühlt und eine Lösung von 3,9 ml (0,028 Mol) Trifluoressigsäureanhydrid in 12 ml Tetrahydrofuran zugetropft. Darauf wird das Reaktionsgemisch 22 Stunden bei Raumtemperatur gerührt. Anschliessend wird auf Eis/Wasser gegossen, zweimal mit Essigsäureethylester extrahiert, die Extrakte mit Wasser gewaschen, über Magnesiumsulfat getrocknet, und durch eine Kieselgelschicht filtriert. Nach dem Eindampfen erhielt man 3,5 g (99 % d.Th.) kristallines Produkt mit einem Smp. von 119-122°.

Beispiel 1.11: Herstellung von Benzo-1,2,3-thiadiazol-7-carbonsäurehydrazid

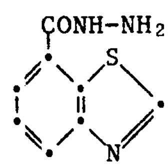

9,7 g 7-Methoxycarbonylbenzo-1,2,3-thiadiazol werden 19 Stunden mit 4,8 g Hydrazinhydrat in 30 ml Wasser bei 50°C und anschliessend weitere 6 Stunden bei 80-90°C umgesetzt. Die Suspension wird leicht abgekühlt, warm abfiltriert und mit Wasser gewaschen. Es verblieben 8,8 g weisse Kristalle vom Smp. 270-272°.

Beispiel 1.12: Herstellung von 2-(Benzo-1,2,3-thiadiazol-7-carbonyl)-1-(α-methylpropyliden)-hydrazin

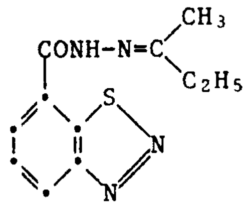

21,5 g Benzthiadiazol-7-carbonsäurehydrazid und 150 ml Methylethylketon werden in 150 ml Eisessig während 8 Stunden auf 70 ° C erwärmt. Anschliessend wird das Reaktionsgemisch am Vakuum eingedampft, der Rückstand in 1 l Dichlormethan aufgenommen und die Lösung zweimal mit 700 ml Eiswasser gewaschen. Dann wird über Natriumsulfat getrocknet, filtriert und eingedampft, der Rückstand mit Essigester angeschlämmt, filtriert und getrocknet. Das oben angegebene Produkt schmilzt bei 159-162 ° C.

Beispiel 1.13: Herstellung von 2-(Benzo-1,2,3-thiadiazol-7-carbonyl)-1-(2′-n-butyl)-hydrazin

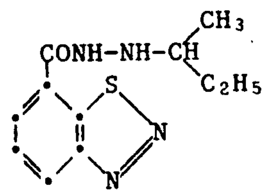

10,5 g 2-(Benzo-1,2,3-thiadiazol-7-carbonyl)-1-(α-methylpropylidenhydrazin werden in 150 ml Dimethylformamid und 100 ml Methylcellosolve gelöst und über 7 g Platin/Kohle bei Raumtemperatur und unter Normaldruck hydriert. Anschliessend wird vom Katalysator abfiltriert und das nach Eindampfen des Filtrats verbleibende Produkt mit Essigsäureethylester an Kieselgel chromatographiert. Das Produkt wurde in Form weisser Kristalle vom Smp. 148-150 ° erhalten.

Beispiel 1.14: Herstellung von 2-Benzylthio-3,5-dinitro-benzoesäuremethylester (Zwischenprodukt)

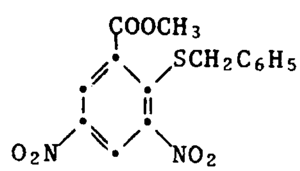

332 g (2,40 Mol) Kaliumcarbonat werden in 500 ml Dimethylformamid vorgelegt und auf -5 ° C gekühlt. Dann lässt man 375 g (1,14 Mol) 2-Chlor-3,5-dinitrobenzoesäure (75 %ig mit 25 % Wasser; Transport-und Lagerform) in 1,1 l Dimethylformamid während 30 Minuten zulaufen. Die Innentemperatur wird dabei auf -5 ° C bis 4 ° C gehalten. Anschliessend werden 142 g (1,14 Mol) Benzylmercaptan bei 0 bis 3 ° C während 2,5 Stunden zugetropft. Darauf lässt man die Temperatur während 16 Stunden auf 20 ° C steigen. Dann werden bei Raumtemperatur 170 g (1,2 Mol) Methyljodid während 5 Stunden zugetropft. Anschliessend wird das Reaktionsgemich 16 Stunden bei Raumtemperatur gerührt und danach auf 3 l Eis/Wasser gegossen, gerührt und filtriert. Das abgesaugte Gut wird viermal mit je 700 ml Wasser gewaschen noch feucht in der nächsten Stufe (vgl. Beispiel 1.15) hydriert. Das getrocknete Produkt hat einen Smp. von 113-114 ° C.

Beispiel 1.15: Herstellung von 2-Benzylthio-3,5-diamino-benzoesäuremethylester (Zwischenprodukt)

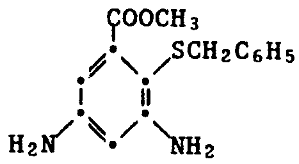

Der in der vorhergehenden Stufe (vgl. Beispiel 1.14) erhaltene, noch feuchte 2-Benzylthio-3,5-dinitrobenzoesäuremethylester wird in 2 l Tetrahydrofuran gelöst und unter Zusatz von dreimal 40 g Raney-Nickel bei 30° bis 35°C hydriert. Daruaf wird der Katalysator abfiltriert, das Filtrat eingeengt und der Rückstand in Essigsäureethylester aufgenommen. Nach Trocknung über Magnesiumsulfat und Behandlung mit Aktivkohle und Bleicherde wird das Filtrat eingeengt und das Produkt durch Zusatz von Diethylether zur Kristallisation gebracht.
Ausbeute: 253 g (77 % d.Th. über drei Stufen); Smp. 84-86°C.

Beispiel 1.16 Herstellung von 7-Methoxycarbonylbenzo-1,2,3-thiadiazol

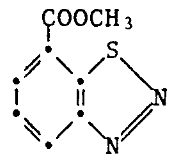

a) 100 g (0,35 Mol) 2-Benzylthio-3,5-diaminobenzoesäuremethylester werden portionsweise zu 250 ml konz. Salzsäure und 110 ml Wasser gegeben und 1,5 Stunden bei Raumtemperatur gerührt. Dann wird das Gemisch auf -5°C gekühlt und während 2,5 Stunden unter Rühren eine Lösung von 48,5 g (0,70 Mol) Natriumnitrit in 210 ml Wasser zugetropft. Der Rührvorgang wird weitere 2 Stunden bei 0°C fortgesetzt. Anschliessend werden 190 ml 50 %ige unterphosphorige Säure während 2 1/2 Stunden zugetropft. Darauf lässt man die Temperatur während 19 Stunden auf 20°C steigen. Das erhaltene Produkt wird abfiltriert, mit Wasser gewaschen und getrocknet. Zur Reinigung wird das Produkt in Essigsäureethylester/Methylenchlorid gelöst, durch Kieselgel filtriert, evaporiert und durch Zugabe von Hexan kristallisiert. Ausbeute: 44,4 g (65 % d.Th.); Smp. 132°C.

b) 576 g (2 Mol) 3,5-Diamino-2-benzylthio-benzoesäure-methylester werden in 500 ml 1,4-Dioxan gelöst und unter Rühren und Kühlen bei 0° bis 5°C zu vorgelegter 5N Salzsäure (3 l) getropft. Anschliessend wird die feine Suspension auf -17° bis -20°C abgekühlt und innerhalb 1,25 Stunden unter Niveau tropfenweise mit 294 g Natriumnitrit in 500 ml Waser versetzt. Unter weiterem Rühren wird die Innentemperatur innerhalb 1 Stunde auf -5°C ansteigen gelassen und während 2 Stunden gehalten. Dann wird auf -15°C abgekühlt und die Suspension unter Rühren portionenweise in auf -10° bis -15°C gekühlte Unterphosphorige Säure (1,1 l) eingetragen, wobei Stickstoff entweicht. Nach beendeter Zugabe wird die Innentemperatur innerhalb 5-6 Stunden auf Raumtemperatur ansteigen gelassen, der gebildete Niederschlag abfiltriert, diese mit 2,5 l Methylenchlorid verrührt, wieder vom nichtgelösten Teil abfiltriert und das Filtrat vom Wasser getrennt. Anschliessend wird die organische Phase über Natriumsulfat getrocknet, mit 300 g Kieselgel verrührt, erneut filtriert, mit Methylenchlorid nachgewaschen und das Filtrat eingedampft. Aus Methanol umkristallisiert resultieren insgesamt 244,8 g (63,1 % d. Th.) beige Kristalle vom Smp. 130°C-133°C.

c) 183 g (0,5 Mol) 3,5-Diamino-2-n-dodecylthio-benzoesäure-methylester, gelöst in 200 ml Dioxan, werden unter Kühlen und Rühren bei 0°-5°C zu vorgelegten 1,2 l 5N Salzsäure getropft. Zur Erzielung eines feinen Niederschlags wird noch ca. 1 Stunde weiter gerührt. Dann wird auf -15° bis -21°C abgekühlt und unter weiterem Rühren bei dieser Temperatur innerhalb 1 Stunde tropfenweise unter Niveau eine Lösung von 73,5 g Natriumnitrit in 130 ml Wasser zugefügt. Anschliessend wird die Innentemperatur innerhalb 1 Stunde auf - 5°C ansteigen gelassen und bei dieser Temperatur noch 3 Stunden weitergerührt. Sodann wird die Suspension wieder auf -10°C gekühlt und innerhalb 1,5 Stunden protionenweise zu ebenfalls gekühlter Unterphosphoriger Säure (280 ml) gegeben, wobei Stickstoff entweicht. Schliesslich wird während 6 Stunden weitergerührt, wobei die Raumtemperatur erreicht ist, der Niederschlag dann abfiltriert und wie unter 1.16 b zum Rohprodukt aufgearbeitet. Dieses kann zur

weiteren Reinigung auch durch einen mit Kieselgel gefüllten Saugfilter filtriert werden, wobei mit Methylenchlorid/Hexan (10:1) nachgewaschen wird. Eindampfen und Verrühren des Rückstandes mit 300 ml Methanol liefert 46,3 g beige Kristalle. Aus dem Filtrat können nach Kristallisation aus Essigsäureethylester weitere 7,2 g Rohprodukt erhalten werden. Somit beträgt die Gesamtbeute 53,5 g (55,2 % d. Th.), Smp. 130°-133°C

d) 1,48 g 1,2,3-Benzthiazol-7-carbonsäure werden unter Stickstoffatmosphäre in 40 ml abs. Tetrahydrofuran vorgelegt und bei 0-3°C unter Kühlen tropfenweise mit 1,46 g 1-Chlor-N,N-2-trimethylpropenylamin versetzt. Ueber Nacht wird bei Raumtemperatur gerührt, anderntags wieder abgekühlt und eine Lösung von 1,18 g Pyridin und 0,64 g Methanol abs. zugetropft. Anschliessend wird während 7 Stunden bei Raumtemperatur gerührt, mit Methylenchlorid verdünnt und mit Eiswasser versetzt. Die organische Phase wird abgetrennt, die wässrige Phase noch dreimal mit Methylenchlorid extrahiert, die Extrakte mit Wasser gewaschen, getrocknet und eingedampft. Der kristalline Rückstand wird am Hochvakuum bei 50°C getrocknet, mit etwas Hexan verrieben, filtriert und der Niederschlag mit Hexan gut gewaschen. Es resultieren 1,38 g (87 % d. Th.) reines Produkt vom Smp. 128-130°C.

e) Nach der gleichen Methode wie in Beispiel 1.16b beschrieben, wird 3,5-Diamino-2-methylthiobenzoesäure-methylester in die Titelverbindung umgewandelt.

Beispiel 1.17: Herstellung von 7-(2-Trimethylsilylethoxycarbonyl)-benzo-1,2,3-thiadiazol

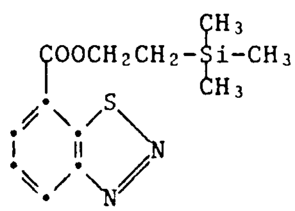

1,99 g (0,01 Mol) 7-Benzothiadiazolcarbonsäurechlorid in 18 ml Toluol werden während 25 Minuten zu einer Lösung von 1,9 ml (0,013 Mol) 2-Trimethylsilyl-ethanol, 2,4 ml (0,017 Mol) Triethylamin und 18 ml Toluol getropft. Anschliessend wird 16 Stunden bei Raumtemperatur gerührt. Dann wird das Reaktionsgemisch auf Eis/Wasser gegossen und zweimal mit Essigsäureethylester extrahiert. Die Extrakte werden vereinigt, mit Wasser gewaschen, über Magnesiumsulfat getrocknet, filtriert, eingeengt, mit der gleichen Menge Hexan versetzt und durch Kieselgel filtriert. Nach dem Einengen werden 2,0 g (71 % d.Th.) Produkt erhalten; Smp. 37-39°C.

Beispiel 1.18: Herstellung von 7-(Carbonyloxymethyl-O-ethyl-methylphospinsäureester)-benzo-1,2,3-thiadiazol

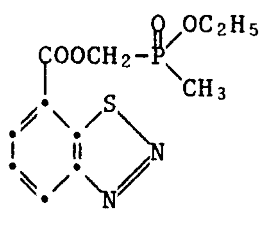

2,6 g (0,013 Mol) Benzo-1,2,3-thiadiazol-7-carbonsäurechlorid in 26 ml Dioxan werden zu einer Lösung von 2,2 g Hydroxymethyl-methyl-phosphinsäureethylester, 3,2 ml (0,023 Mol)Triethylamin und 26 ml Dioxan getropft. Anschliessend wird 16 Stunden bei Raumtemperatur gerührt. Dann wird das Reaktionsgemisch durch eine Kieselgelschicht filtriert und eingeengt. Das Produkt wird in Essigsäureethylester/Hexan umkristallisiert.
Ausbeute: 2,2 g (56 %); Smp. 89-92°C.

Beispiel 1.19: Herstellung von 2-(Benzo-1,2,3-thiadiazol-7-carbonyl-1-(2'-butyl)-hydrazin

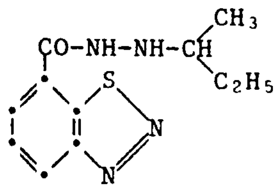

10,5 g 2-(Benzo-1,2,3-thiadiazol-7-carbonyl)-1-(α-methylpropylidenhydrazin werden in 150 ml Dimethylformamid und 100 ml Methylcellosolve gelöst und über 7 g Platin/Kohle bei Raumtemperatur und Normaldruck hydriert. Anschliessend wird die Lösung vom Katalysator abfiltriert und das nach Eindampfen des Filtrats verbleibende Produkt an Kieselgel (Essigester) chromatographiert. Das Produkt wurde in Form weisser Kristalle vom Smp. 148-150 ° C erhalten.

Beispiel 1.20: Herstellung von 2-(Benzo-1,2,3-thiadiazol-7-carbonyl-1-(2′-butyl)-hydrazin

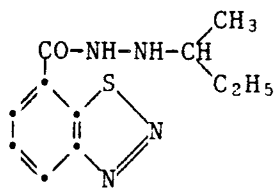

4,8 g Benzo-1,2,3-thiadiazol-7-carbonsäurehydrazid werden in 300 ml Tetrahydrofuran gelöst und mit 9,3 g Methylethylketon und 0,6 g 5%igem Platinkohle-Katalysators versetzt. Dann wird bei 20 ° bis 25 ° bei Normaldruck bis zum Stillstand der Reaktion hydriert, wobei 3 weitere Katalysator-Zusätze zu je 2 g gemacht wurden. Anschliessend wird vom Katalysator abfiltriert, eingedampft und aus Essigester umkristallisiert:
weisse Kristalle vom Smp. 147-150 ° C.

Beispiel 1.21: Herstellung von 2-Benzylthio-3-nitrobenzoesäure (Zwischenprodukt)

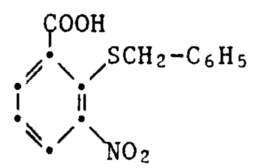

6,85 g (0,055 Mol) Benzylmercaptan werden in 150 ml Dimethylformamid gelöst. Dann wird auf 0 ° C gekühlt und 15,2 g (0,11 Mol) Kaliumcarbonat zugegeben. Bei 0 ° bis 5 ° C werden 10,6 g (0,050 Mol) 2,3-Dinitrobenzoesäure portionenweise zugegeben. Anschliessend lässt man die Innentemperatur während 24 Stunden auf Raumtemperatur steigen. Daruaf wird das Reaktionsgemisch auf Eis/Wasser gegeben und mit Salzsäure angesäuert. Das erhaltene Produkt wird filtriert, mit Wasser gewaschen und getrocknet.
Ausbeute: 11,8 g (82 % d.Th.); Smp. 152-153 ° C.

Beispiel 1.22: Herstellung von 3-Amino-2-benzylthiobenzoesäure

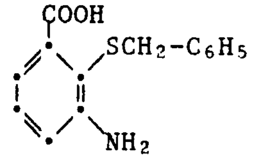

11,0 g (0,038 Mol) 2-Benzylthio-3-nitrobenzoesäure werden in 110 ml Tetrahydrofuran gelöst und bei 20° bis 25°C in Gegenwart von Raney-Nickel bei Normaldruck hydriert. Darauf wird der Katalysator abfiltriert, das Filtrat eingeengt und das erhaltene Produkt direkt in der nächsten Stufe eingesetzt (Beispiel 1.23).

Beispiel 1.23: Herstellung von Benzo-1,2,3-thiadiazol-7-carbonsäure

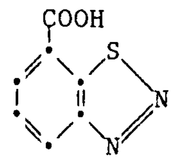

Das erhaltene Produkt aus Beispiel 1.22 wird analog Beispiel 1.7 mit Salzsäure und Natriumnitrit umgesetzt. Es wurde die Titelverbindung mit einem Smp. von 260-262°C erhalten.

Beispiel 1.24: Herstellung von 3,5-Dinitro-2-isopropylthio-benzoesäuremethylester

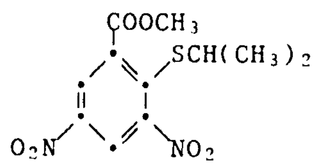

33,2 g (0,240 mol) Kaliumcarbonat werden in 100 ml Dimethylformamid vorgelegt und auf -5°C gekühlt. Dann lässt man 37,5 g (0,114 mol) 2-Chlor-3,5-dinitrobenzoesäure (75%ig mit 25 % Wasser) in 110 ml Dimethylformamid während 20 Minuten zulaufen. Die Innentemperatur wird dabei auf -7° bis -3°C gehalten. Anschliessend werden 11,0 ml (0,114 mol) Isopropyl-mercaptan (97%ig) in 20 ml Dimethylformamid bei -8° bis -1°C während 45 Minuten zugetropft. Darauf rührt man eine Stunde bei 0°C und lässt dann die Temperatur während 24 Stunden auf 25°C steigen. Dann werden bei Raumtemperatur 7,5 ml (0,12 mol) Methyljodid während 30 Minuten zugetropft. Anschliessend wird das Reaktionsgemisch 16 Stunden bei Raumtemperatur gerührt und danach auf 500 ml Eis/Wasser gegoseen, gerührt und filtriert. Das abgesaugte Gut wird mit Wasser gewaschen und bei Raumtemperatur getrocknet.
Ausbeute: 32,6 g (95 % d. Th.); Smp. 62-63°C.

Beispiel 1.25: Herstellung von 3,5-Diamino-2-isopropylthio-benzoesäure-methylester

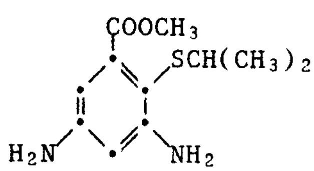

26,6 g (0,0886 mol) 3,5-Dinitro-2-isopropylthio-benzoesäure-methylester werden in 270 ml Tetrahydrofuran gelöst und unter Zusatz von 10 g Raney-Nickel bei 30°C bis 35°C hydriert. Darauf wird der Katalysator abfiltriert, das Filtrat eingeengt und der Rückstand aus Essigsäureethylester/Hexankristallisiert.
Ausbeute: 20,1 g (94 % d. Th.); Smp. 109-111°C.

Beispiel 1.26: Herstellung von 7-Methoxycarbonylbenzo-1,2,3-thiadiazol

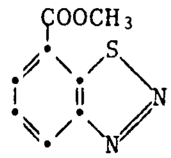

17,0 g (0,0707 mol) 3,5-Diamino-2-isopropylthio-benzoesäure-methylester werden portionenweise zu 100 ml konzentrierter Salzsäure und 50 ml Wasser gegeben und eine Stunde bei Raumtemperatur gerührt. Dann wird auf -5 °C gekühlt und während 2 Stunden unter Rühren eine Lösung von 9,80 g (0,142 mol) Natriumnitrit in 20 ml Wasser zugetropft. Anschliessend wird weitere 2 Stunden bei 0 °C gerührt. Darauf werden 23 ml (0,21 mol) 50%ige unterphosphorige Säure während 30 Minuten zugetropft. Dann lässt man die Temperatur während 24 Stunden auf 20 °C steigen. Zum Reaktionsgemisch werden 150 ml Wasser gegeben, das Produkt wird abfiltriert, mit Wasser gewaschen und getrocknet. Zur Reinigung wird das Produkt in 300 ml Essigsäureethylester aufgenommen, am Rückfluss gekocht und heiss filtriert. Zum eingeengten Filtrat wird Hexan gegeben. Das erhaltene Produkt wird abfiltriert und getrocknet.
Ausbeute: 7,5 g (55 % d. Th.); Smp. 130-131 °C.

Beispiel 1.27: Herstellung von 3,5-Dinitro-2-ethylthio-benzoesäureethylester

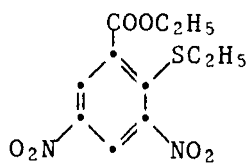

33,2 g (0,240 mol) Kaliumcarbonat werden in 100 ml Dimethylformamid vorgelegt und auf -5 °C gekühlt. Dann lässt man 37,5 g (0,114 mol) 2-Chlor-3,5-dinitrobenzoesäure (75%ig mit 25 % Wasser) gelöst in 120 ml Dimethylformamid während 20 Minuten zulaufen. Die Innentemperatur wird dabei auf -5° bis 0 °C gehalten. Anschliessend werden 8,9 ml (0,12 mol) Ethylmercaptan in 20 ml Dimethylformamid bei -8 °C während 20 Minuten zugetropft. Darauf rührt man eine Stunde bei gleicher Temperatur und lässt dann während 19 Stunden auf Raumtemperatur steigen. Dann werden 9,0 ml (0,12 mol) Ethylbromid in 20 ml Dimethylformamid während 10 Minuten zugetropft. Anschliessend wird das Reaktionsgemisch 24 Stunden bei Raumtemperatur gerührt und danach auf 500 ml Eis/Wasser gegossen, gerührt und filtriert. Das abgesaugte Gut wird mit Wasser gewaschen und darauf bei Raumtemperatur unter Vakuum und in Gegenwart von Phosphorpentoxid getrocknet.
Ausbeute: 28,7 g (84 % d. Th.); Smp. 80-81 °C.

Beispiel 1.28: Herstellung von 3,5-Diamino-2-ethylthio-benzoesäureethylester

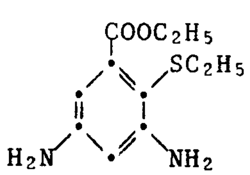

25,9 g (0,0862 mol) 3,5-Dinitro-2-ethylthio-benzoesäure-ethylester werden in 260 ml Tetrahydrofuran gelöst und in Gegenwart von 10 g Raney-Nickel bei 30°-35 °C hydriert. Dann wird der Katalysator abfiltriert, der Rückstand in Essigsäureethylester aufgenommen, über Magnesiumsulfat getrocknet, filtriert und eingeengt.
Ausbeute: 19,3 g (93 % d. Th.).

Beispiel 1.29: Herstellung von 5-Fluor-1,2,3-benzothiadiazol-7-carbonsäure-ethylester

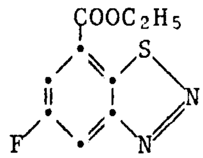

18,7 g (0,078 mol) 3,5-Diamino-2-ethylthio-benzoesäure-ethylester werden in 100 g wasserfreien Fluorwasserstoff bei 0 bis -12 ° C eingetragen. Dann werden bei 0 bis 5 ° C 12,9 g (0,187 mol) Natriumnitrit während zwei Stunden zudosiert und das Reaktionsgemisch zwei weitere Stunden gerührt. Anschliessend wird die Diazoniumlösung in einen mit Teflon beschichteten Autoklaven gezogen und darin auf 146 ° C erwärmt. Nach der Reaktion wird der Fluorwasserstoff abdestilliert und der Rückstand in Methylenchlorid aufgenommen. Nach dem Waschen mit Natriumbicarbonat-Lösung und Trocknen mit Magnesiumsulfat wird die Lösung filtriert und eingeengt. Das erhaltene Rohprodukt wird über eine Kieselgelsäule gereinigt (Lösungsmittel: Petrolether/Diethylether 2:1).
Ausbeute: 1,5 g gelbe Kristalle vom Smp. 68-69 ° C.

Beispiel 1.30: Herstellung von 2-Benzylthio-3,5-diamino-benzoesäuremethylester

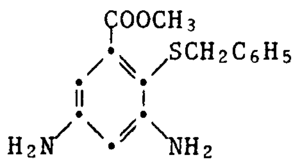

17,7 g Eisenspäne werden unter gutem Rühren in 80 ml Essigsäure (5%ig) auf 66 ° C erwärmt. Dann wird langsam eine Lösung von 12,0 g (0,034 mol) 2-Benzylthio-3,5-dinitro-benzoesäure-methylester in 20 ml Tetrahydrofuran zugetropft. Nach dem Abkühlen wird mit gesättigter Natriumbikarbonatlösung neutralisiert und dreimal mit Essigsäureethylester extrahiert. Die Extrakte werden mit Wasser gewaschen, über Magnesiumsulfat getrocknet, filtriert und eingeengt. Nach der Kristallisation aus Diethylether erhält man 8,1 g (82 % d. Th.) Produkt; Smp. 80-82 ° C.

Beispiel 1.31: Herstellung von 3,5-Diamino-2-methylthio-benzoesäure-methylester

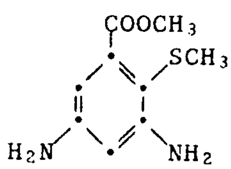

Nach der in Beispiel 1.30 beschriebenen Methode wird 3,5-Dinitro-2-methylthio-benzoesäure-methylester mit Eisenspänen reduziert. Man erhält die Titelverbindung mit Smp. 102-104 ° C.

Wie in den vorstehenden Beispielen beschrieben lassen sich folgende Verbindungen herstellen.

In den Verbindungstabellen werden für folgende Reste die dazugehörigen Symbole verwendet:

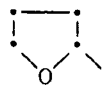
Q11

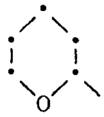
Q12

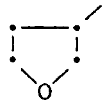
Q13

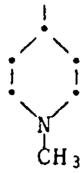
Q14

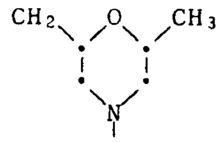
Q15

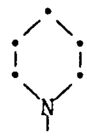
Q16

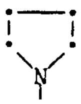
Q17

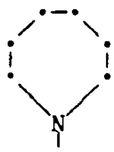
Q18

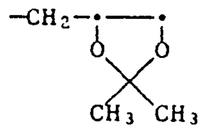
Q19

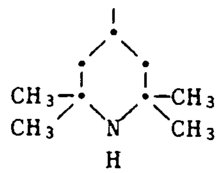
Q20

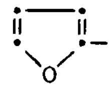
Q21

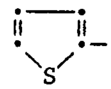
Q22

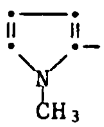
Q23

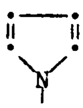
Q24

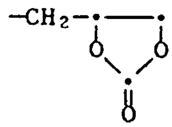
Q25

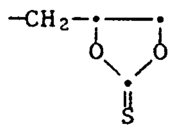
Q26

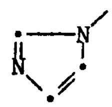
Q31

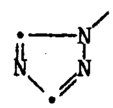
Q32

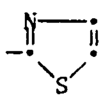
Q33

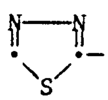
Q34

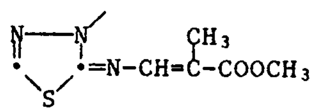
Q35

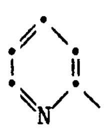
Q41

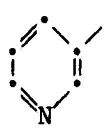
Q42

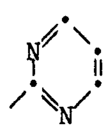
Q43

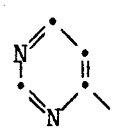
Q44

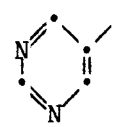
Q45

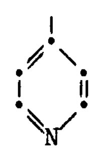
Q46

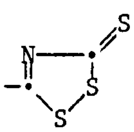
Q47

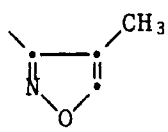
Q48

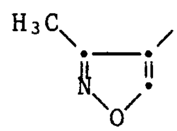
Q48

Die vorstehend beschriebenen Heterocyclen können durch niedermolekulare Reste, wie aliphatische Reste mit bis zu 6 C-Atomen, oder durch Halogenatome oder andere Reste substituiert sein.

Tabelle 1

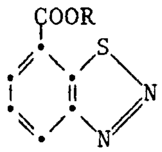

| Verb. Nr. | R | Physikal. Daten |
|---|---|---|
| 1.1 | H | Smp. 262°C |
| 1.2 | $CH_3$ | Smp. 134-135°C |
| 1.3 | $C_2H_5$ | Smp. 62-63°C |
| 1.4 | n-$C_3H_7$ | Smp. 36-38°C |
| 1.5 | i-$C_3H_7$ | Smp. 78-79°C |
| 1.6 | n-$C_4H_9$ | Oel |
| 1.7 | s-$C_4H_9$ | |
| 1.8 | t-$C_4H_9$ | |
| 1.9 | n-$C_5H_{11}$ | Smp. 35-37°C |
| 1.10 | n-$C_6H_{13}$ | |
| 1.11 | n-$C_8H_{17}$ | Smp. 41-44°C |
| 1.12 | 2-Bromethyl | |
| 1.13 | 2-Chlorethyl | |
| 1.14 | 2-Fluorethyl | |
| 1.15 | 2-Cyanoethyl | |
| 1.16 | 2-Methoxyethyl | Smp. 30-32°C |
| 1.17 | 2-n-Butoxyethyl | |
| 1.18 | 2-Allyloxyethyl | |
| 1.19 | 2,2,2-Trichlorethyl | |
| 1.20 | 3-Aethoxypropyl | |
| 1.21 | 3-Acetylpropyl | |
| 1.22 | 3-Chlorpropyl(n) | |
| 1.23 | 3-Brompropyl(n) | |
| 1.24 | 1-Chlorprop-2-yl | |
| 1.25 | 1-Bromprop-2-yl | |
| 1.26 | 2,3-Dibrompropyl(n) | |
| 1.27 | 2-Nitroethyl | |
| 1.28 | Cyclopropylmethyl | Smp. 46-48°C |

Tabelle 1 (Fortsetzung)

| Verb. Nr. | R | Physikal. Daten |
|---|---|---|
| 1.29 | 1-Cyclopropyl-eth-1-yl | Smp. 57-60°C |
| 1.30 | Cyclohexylmethyl | Smp. 62-64°C |
| 1.31 | Cyclooctylmethyl | |
| 1.32 | 3-Phenylpropyl | Sdp.150°C/0,01 Torr |
| 1.33 | 2-Phenylethyl | Smp. 77-79°C |
| 1.34 | Benzyl | Smp. 94-95°C |
| 1.35 | 2-Chlorbenzyl | Smp. 126-127°C |
| 1.36 | 3-Chlorbenzyl | |
| 1.37 | 4-Chlorbenzyl | Smp. 106-108°C |
| 1.38 | 4-Methylbenzyl | |
| 1.39 | 4-Methoxybenzyl | Smp. 98-100°C |
| 1.40 | 4-Nitrobenzyl | |
| 1.41 | 2-(4-Methoxyphenyl)ethyl | |
| 1.42 | 2-Phenoxyethyl | Smp. 60-62°C |
| 1.43 | 2-(4-Chlorphenoxy)ethyl | |
| 1.44 | Allyl | Smp. 57-58°C |
| 1.45 | 4-Pentenyl | |
| 1.46 | 2-Propinyl | Smp. 129-130°C |
| 1.47 | 3-Hexinyl | |
| 1.48 | 3-Chlor-but-2-enyl | |
| 1.49 | Cyclopropyl | |
| 1.50 | Cyclopentyl | Smp. 62-64°C |
| 1.51 | Cyclooctyl | |
| 1.52 | Phenyl | |
| 1.53 | 2-Chlorphenyl | Smp. 108-110°C |
| 1.54 | 3-Bromphenyl | Smp. 121-123°C |
| 1.55 | 3,4-Dichlorphenyl | |
| 1.56 | 4-Chlor-2-methyl-phenyl | |
| 1.57 | 4-t-Butylphenyl | Smp. 142-144°C |
| 1.58 | 3-Nitrophenyl | |
| 1.59 | 4-Nitrophenyl | Smp. 214-216°C |
| 1.60 | 3-Cyanophenyl | Smp. 181-183°C |

Tabelle 1 (Fortsetzung)

| Verb. Nr. | R | Physikal. Daten |
|---|---|---|
| 1.61 | 3-Trifluormethylphenyl | Smp.107-109°C |
| 1.62 | 3-N,N-Dimethylaminophenyl | |
| 1.63 | 2-Methoxycarbonyl-phenyl | |
| 1.64 | 3-Jod-prop-2-in-yl | |
| 1.65 | $-CH_2-COOCH_3$ | |
| 1.66 | $-CH_2-COOC_2H_5$ | |
| 1.67 | $-CH(CH_3)-COOCH_3$ | |
| 1.68 | $-CH(CH_3)-COOC_2H_5$ | Smp.118-122°C (S)-Enantiomer |
| 1.69 | $-CH_2CH_2N(CH_3)_2$ | |
| 1.70 | 3-N,N-Dimethylaminopropyl | |
| 1.71 | $-N=C(CH_3)_2$ | Smp. 127-130°C |
| 1.72 | -N= 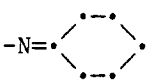 | Smp. 125°C |
| 1.73 | -N= 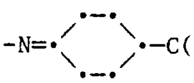 $-C(CH_3)_3$ | Smp. 112-114°C |
| 1.74 | $-N=C(CH_3)CH_2OCH_3$ | |
| 1.75 | $-N=C(CN)CONH_2$ | |
| 1.76 | $-N=C(CN)-C_6H_5$ | |
| 1.77 | $-N=C(CN)-CONHC_2H_5$ | |
| 1.78 | $-N=C(CN)-CONH-CONHC_2H_5$ | |
| 1.79 | $-CH_2CH_2CH_2OH$ | Smp. 26°C |
| 1.80 | $-CH_2CH_2OH$ | Smp. 76-79°C |
| 1.81 | 3-Fluorbenzyl | Smp. 100-102°C |
| 1.82 | 4-Trifluormethylbenzyl | |
| 1.83 | $CH_2CH_2Q_{21}$ | |
| 1.84 | $CH_2CH_2Q_{41}$ | |
| 1.85 | $CH_2CH_2Q_{24}$ | |
| 1.86 | Diaceton-D-glucos-3-yl | Smp. 121-123°C |
| 1.87 | 2-Fluorbenzyl | Smp. 113-115°C |
| 1.88 | 4-Fluorbenzyl | Smp. 107-109°C |
| 1.89 | 4-Methylphenyl | Smp. 141-143°C |
| 1.90 | 2-Methoxycarbonylphenyl | Smp.120-122°C |

Tabelle 1 (Fortsetzung)

| Verb. Nr. | R | Physikal. Daten |
|---|---|---|
| 1.91 | 2-Carboxyphenyl | |
| 1.92 | $CH_2Q_{11}$ | |
| 1.93 | $CH_2Q_{12}$ | |
| 1.94 | $CH_2Q_{13}$ | |
| 1.95 | $CH_2Q_{14}$ | |
| 1.96 | $CH_2Q_{21}$ | Smp. 67°C |
| 1.97 | $CH_2Q_{22}$ | |
| 1.98 | $CH_2Q_{23}$ | |
| 1.99 | $CH_2Q_{31}$ | |
| 1.100 | $CH_2Q_{32}$ | Smp. 166-168°C |
| 1.101 | $CH_2Q_{41}$ | Smp. 91-93°C |
| 1.102 | $CH_2Q_{42}$ | Smp. 97-99°C |
| 1.103 | $CH_2Si(CH_3)_3$ | Smp. 59-61°C |
| 1.104 | $CH_2CH_2Si(CH_3)_3$ | Smp. 37-39°C |
| 1.105 | $CH_2P(O)(CH_3)OC_2H_5$ | Smp. 92°C |
| 1.106 | $CH_2P(O)(OCH_3)_2$ | |
| 1.107 | $CH_2CH_2P(O)(OC_2H_5)_2$ | |
| 1.108 | $CH_2CH_2P(O)(OCH_3)_2$ | Smp. 86-88°C |
| 1.109 | $CH_2P(O)(CH_3)OCH_3$ | |
| 1.110 | $CH(CH_3)P(O)(OCH_3)_2$ | |
| 1.111 | $Si(CH_3)_2C(CH_3)_2CH(CH_3)_2$ | |
| 1.112 | $Na^{\oplus}$ | Smp. >250°C |
| 1.113 | $K^{\oplus}$ | Smp. >250°C |
| 1.114 | $(HN(C_2H_5)_3)^{\oplus}$ | Smp. 86-89°C |
| 1.115 | $(H_2N(CH_2CH_2OH)_2)^{\oplus}$ | Smp. 130°C |
| 1.116 | $CH_2$-Napth-1-yl | Smp. 123-125°C |
| 1.117 | $CH_2$-Naphth-2-yl | Smp. 94-96°C |
| 1.118 | $CH_2CH_2$-Naphth-1-yl | |
| 1.119 | 1-Phenethyl | Smp. 50-52°C |
| 1.120 | 2-Phenyl-prop-2-yl | |
| 1.121 | $CH_2CH_2CN$ | |
| 1.122 | (2-Sulfamoyl)-benzyl | Smp. 198-200°C |
| 1.123 | $CH_2CH_2SCH_3$ | |

Tabelle 1 (Fortsetzung)

| Verb. Nr. | R | Physikal. Daten |
|---|---|---|
| 1.124 | 1,2,3,4-Di-O-isopropyliden-D-galacto-pyranos-6-yl | |
| 1.125 | 1,2,5,6-Di-O-isopropyliden-D-mannit-3-yl | |
| 1.126 | 1,2,5,6-Di-O-isopropyliden-α-D-allo-furanos-3-yl | |
| 1.127 | D-Glucofuranos-3-yl | |
| 1.128 | D-Galactopyranos-6-yl | |
| 1.129 | D-Mannit-3-yl | |
| 1.130 | D-Allofuranos-4-yl | |
| 1.131 | Mannopyranos-1-yl | |
| 1.132 | 2-Methyl-D-glucosid-6-yl | |
| 1.133 | 1,2,5,6-Tetraacetyl-D-galactopyranos-3-yl | |
| 1.134 | 2,3,5-Tribenzylribofuranos-1-yl | |
| 1.135 | Cyclohexyl | Smp. 44-46°C |
| 1.136 | $CH_2$-$Q_{46}$ | Smp. 116-118°C |
| 1.137 | 2,6-Difluorbenzyl | Smp. 117-119°C |
| 1.138 | $CH_2$-$CCl_2CF_3$ | Smp. 71-73°C |
| 1.139 | 2-Nitrobenzyl | Smp. 196-198°C |
| 1.140 | 2-Methylbenzyl | Smp. 95-97°C |
| 1.141 | $-CH_2C(OCH_3)_2CH_3$ | |
| 1.142 | 3-Methyl-2-nitrobenzyl | Smp. 143-145°C |
| 1.143 | Cycloheptyl | $n_D^{31}$ = 1,5787 |
| 1.144 | 3-Methoxybenzyl | Smp. 73-75°C |
| 1.145 | 2,4-Dichlorbenzyl | Smp. 118-120°C |
| 1.146 | $Q_{19}$ | Smp. 82-83°C |
| 1.147 | $-CH_2$-$CH(OH)$-$CH_2OH$ | Smp. 75-77°C |
| 1.148 | $-CH_2$-$CH(OH)$-$CH_2OCH_3$ | |
| 1.149 | $-CH_2COC_4H_9(n)$ | |
| 1.150 | $Q_{20}$ | Smp. 83-84°C |
| 1.151 | $-CH_2$-$Q_{25}$ | Smp.113-116°C |
| 1.152 | $-CH_2$-$CH(OCH_3)$-$CH_2OCH_3$ | |
| 1.153 | $-CH_2$-$Q_{26}$ | |
| 1.154 | $-CH_2$-$COC(CH_3)_3$ | Smp. 98-100°C |

Tabelle 1 (Fortsetzung)

| Verb. Nr. | R | Physikal. Daten |
|---|---|---|
| 1.155 | $-CH_2-CHOH-CH_2OC_2H_5$ | |
| 1.156 | $-1/2Mg^{2\oplus}$ | |
| 1.157 | $-CH_2CH_2-Q_{16}$ | |
| 1.158 | $-CH_2CH_2-Q_{15}$ | |
| 1.159 | $-CH_2CH_2-Q_{42}$ | |
| 1.160 | $-CH_2CH_2-N(C_2H_5)_2$ | |
| 1.161 | $-CH_2CH_2-Q_{46}$ | |
| 1.162 | $-CH_2CH_2-Q_{21}$ | |
| 1.163 | $-CH_2CH_2-Q_{11}$ | |
| 1.164 | 4'-Trifluormethoxy-benzyl | |
| 1.165 | $-CH_2CH_2-Q_{22}$ | |
| 1.166 | $-CH(CH_3)-Q_{42}$ | |
| 1.167 | $-CH_2CH_2CH_2-Si(CH_3)_3$ | |
| 1.168 | -4-Phenoxy-phenyl | Smp. 97-99°C |
| 1.169 | -3-Diphenyl | Smp.108-110°C |
| 1.170 | $-CH(CH_3)-Q_{41}$ | |
| 1.171 | -4-Benzyl-benzyl | Smp.117-119°C |
| 1.172 | $-CH_2-COCH_3$ | |
| 1.173 | $-CH_2COC_5H_{11(n)}$ | |
| 1.174 | $-CH(CH_3)-Q_{21}$ | |
| 1.175 | $-C(CH_3)_2-Q_{46}$ | |
| 1.176 | 2-($OCF_3$)-phenyl | |
| 1.177 | 3-($OCF_2CF_3$)-phenyl | |
| 1.178 | 2-Naphthyl | Smp. 136-137°C |

Tabelle 2

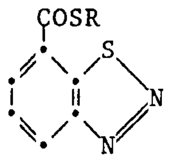

| Verb. Nr. | R | Physikal. Daten |
|---|---|---|
| 2.1 | $CH_3$ | |
| 2.2 | $C_2H_5$ | Smp. 87°C |
| 2.3 | $C_3H_7(n)$ | Oel |
| 2.4 | $C_4H_9(n)$ | |
| 2.5 | Benzyl | Smp. 101-104°C |
| 2.6 | Phenyl | Smp. 137-140°C |
| 2.7 | 4-Chlorphenyl | Smp. 53-55°C |
| 2.8 | $CH_2COOCH_3$ | Smp. 126-129°C |
| 2.9 | $CH_2COOC_2H_5$ | |
| 2.10 | 4-Methylphenyl | |
| 2.11 | n-Hexyl | |
| 2.12 | Cyclohexyl | |
| 2.13 | Cyclopentyl | |
| 2.14 | H | |
| 2.15 | $Na^{\oplus}$ | |
| 2.16 | $K^{\oplus}$ | |

Tabelle 3

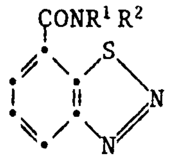

| Verb. Nr. | $NR^1R^2$ | Physikal. Daten |
|---|---|---|
| 3.1 | $NH_2$ | Smp. >270°C |
| 3.2 | $NHCH_3$ | Smp. 243-247°C |
| 3.3 | Piperidinyl | Smp. 91,5-93,5°C |
| 3.4 | Morpholinyl | Smp. 138-141°C |
| 3.5 | $NHCH(CH_3)C_2H_5$ | Smp. 134°C |
| 3.6 | $NH-C_6H_5$ | Smp. 180-183°C |
| 3.7 | $NH-CH_2COOC_2H_5$ | Smp. 119-122°C |
| 3.8 | $N(CH_3)_2$ | Smp. 83-85°C |
| 3.9 | $NH-CH_2COOH$ | Smp. 207°C |
| 3.10 | Pyrrolidinyl | |
| 3.11 | $Q_{15}$ | Smp. 150-153°C |
| 3.12 | $Q_{47}$ | |
| 3.13 | $N(CH_2CN)_2$ | Smp. 197-199°C |
| 3.14 | $N(CH_2CH_2CN)_2$ | |
| 3.15 | $NHCH_2CH_2OCH_3$ | |
| 3.16 | $Q_{24}$ | |
| 3.17 | $Q_{31}$ | Smp. 119-121°C |
| 3.18 | $Q_{32}$ | |
| 3.19 | $NH-Q_{33}$ | Smp. 225-227°C |
| 3.20 | $NH-Q_{34}$ | Zers. 303°C |
| 3.21 | $NH-Q_{41}$ | |
| 3.22 | $NH-Q_{43}$ | |
| 3.23 | $NH-Q_{44}$ | |
| 3.24 | $NHCH_2C\equiv CH$ | Smp. 229-231°C |
| 3.25 | $NH-CH(CH_3)COOCH_3$ | |
| 3.26 | $N(CH_2CH=CH_2)_2$ | Smp. 119°C |
| 3.27 | NH(5-Aethyl-6-chlor-pyrimidin-4-yl) | Smp.185-187°C |

Tabelle 3 (Fortsetzung)

| Verb. Nr. | $N(R_1)R_2$ | Physikal. Daten |
|---|---|---|
| 3.28 | 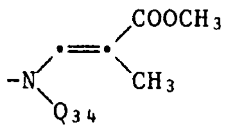 | Smp.140-142°C |
| 3.29 | 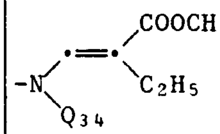 | Smp. 142-145°C |
| 3.30 | 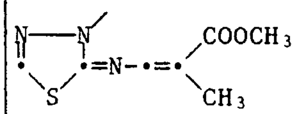 | Smp.203-206°C |
| 3.31 | $N(CH_3)OCH_3$ | Smp.115-117°C |
| 3.32 | $N(CH_3)OCH(CH_3)_2$ | |
| 3.33 | $N(C_2H_5)OCH_3$ | |
| 3.34 | $N(i\text{-}C_4H_9)OCH_3$ | |
| 3.35 | $NHCH_2CN$ | |
| 3.36 | NH-Benzyl | Smp.148-150°C |
| 3.37 | NH-4-Chlorbenzyl | |
| 3.38 | NH-3-Chlorbenzyl | |
| 3.39 | NH-2-Chlorbenzyl | Smp. 173-175°C |
| 3.40 | NH-2,4-Dichlorbenzyl | Smp. 171-174°C |
| 3.41 | NH-3,4-Dichlorbenzyl | Smp. 185-188°C |
| 3.42 | NH-2-Fluorbenzyl | Smp. 145-147°C |
| 3.43 | NH-4-Fluorbenzyl | |
| 3.44 | NH-2-Methylbenzyl | Smp. 164-165°C |
| 3.45 | NH-CH(Methyl)-$Q_{21}$ | Smp. 127-129°C |
| 3.46 | 2-Methylpiperidine-1-yl | Smp. 94-96°C |
| 3.47 | NH-4-Methylbenzyl | |
| 3.48 | N(Methyl)-benzyl | Smp. 101-103°C |
| 3.49 | NH-$CH_2$-$Q_{21}$ | Smp. 141-143°C |
| 3.50 | NH-$Q_{48}$ | Smp. 278-281°C |
| 3.51 | NHOH | Zers. >87°C |
| 3.52 | $Q_{18}$ | |

Tabelle 4

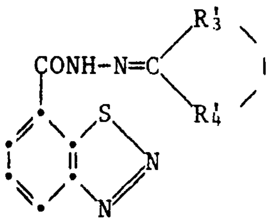

| Verb. Nr. | $R_3'$ | $R_4'$ | Physikal. Daten |
|---|---|---|---|
| 4.1 | $CH_3$ | $CH_3$ | Smp.166-168°C |
| 4.2 | $CH_3$ | $C_2H_5$ | Smp.159-162°C |
| 4.3 | $C_2H_5$ | $C_2H_5$ | |
| 4.4 | $C_3H_7$-n | $C_3H_7$-n | |
| 4.5 | $C_4H_9$-n | $C_4H_9$-n | |
| 4.6 | $C_4H_9$-s | $C_4H_9$-s | |
| 4.7 | $C_6H_{13}$-n | $C_6H_{13}$-n | |
| 4.8 | $-CH_2CH_2CH_2-$ | | |
| 4.9 | $-CH_2CH_2CH_2CH_2-$ | | Smp.154-157°C |
| 4.10 | $-CH_2-CH_2-CH_2-CH_2-CH_2-$ | | |
| 4.11 | $-CH_2CH_2CH_2CH_2CH_2-CH_2-$ | | Smp.134-136°C |
| 4.12 | H | $CH_3$ | |
| 4.13 | H | $C_2H_5$ | |
| 4.14 | H | $CH=CH_2$ | |
| 4.15 | H | $C_6H_5$ | Smp.266-268°C |
| 4.16 | H | $C_6H_4Cl(2)$ | |
| 4.17 | H | $C_6H_4Cl(4)$ | |
| 4.18 | $CH_3$ | $C_6H_5$ | |
| 4.19 | $CH_3$ | $C_6H_3Cl_2(2,4)$ | Smp.209-210°C |
| 4.20 | H | $Q_{41}$ | Smp.226-228°C |
| 4.21 | H | $Q_{42}$ | |
| 4.22 | $CH_3$ | $Q_{41}$ | |
| 4.23 | H | $Q_{21}$ | Smp.231-232°C |
| 4.24 | H | $CCl_3$ | Smp.174-175°C |
| 4.25 | $CH_3$ | $CH_2OCH_3$ | |

Tabelle 5

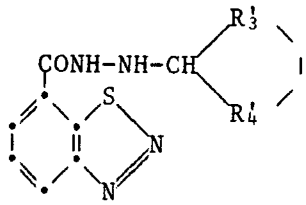

| Verb. No. | $R_3'$ | $R_4'$ | Physikal. Daten |
|---|---|---|---|
| 5.1 | $CH_3$ | $CH_3$ | Smp. 145-147°C |
| 5.2 | $CH_3$ | $C_2H_5$ | Smp. 148-150°C |
| 5.3 | $C_2H_5$ | $C_2H_5$ | Smp. 148-150°C |
| 5.4 | $C_3H_7$-n | $C_3H_7$-n | |
| 5.5 | $C_4H_9$-n | $C_4H_9$-n | |
| 5.6 | $C_6H_{13}$-n | $C_6H_{13}$-n | |
| 5.7 | $CH_2(CH_2)_2CH_2$ | | |
| 5.8 | $CH_2(CH_2)_3CH_2$ | | Smp.154-156°C |
| 5.9 | $CH_2(CH_2)_4CH_2$ | | Smp.166-168°C |
| 5.10 | H | $CH_3$ | |
| 5.11 | H | $C_2H_5$ | |
| 5.12 | H | $C_6H_5$ | Smp. > 166°C |
| 5.13 | H | o-Cl-$C_6H_4$ | |
| 5.14 | $CH_3$ | $C_6H_5$ | |
| 5.15 | $CH_3$ | 2,4-Di-Cl-$C_6H_3$ | |
| 5.16 | H | $Q_{41}$ | |
| 5.17 | H | $Q_{42}$ | |
| 5.18 | $CH_3$ | $Q_{41}$ | |
| 5.19 | H | $Q_{21}$ | |
| 5.20 | $CH_3$ | $CH_2OCH_3$ | |
| 5.21 | $CH_3$ | $CH_2CH_2OCH_3$ | |
| 5.22 | H | $Q_{24}$ | |

Tabelle 6

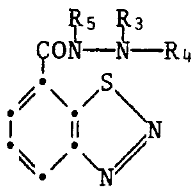

| Verb. Nr. | $R_5$ | $R_4$ | $R_3$ | Physikal. Daten |
|---|---|---|---|---|
| 6.1 | H | H | H | Smp.270-272°C |
| 6.2 | H | $CH_3$ | H | |
| 6.3 | H | $COCH_3$ | H | |
| 6.4 | H | $COC_2H_5$ | H | |
| 6.5 | H | $COCH=CH_2$ | H | |
| 6.6 | H | | H | |
| 6.7 | $COCH_3$ | $COCH_3$ | H | |
| 6.8 | H | H | $C_6H_5$ | |
| 6.9 | H | H | $Q_{41}$ | |
| 6.10 | H | $COCH_3$ | $Q_{21}$ | |
| 6.11 | H | H | $Q_{34}$ | |
| 6.12 | $CH_3$ | $COCH_3$ | $Q_{41}$ | |
| 6.13 | H | $COCH_3$ | $Q_{41}$ | |
| 6.14 | H | $COCH_2OCH_3$ | H | |
| 6.15 | H | $COCH_2OCH_3$ | $Q_{41}$ | |
| 6.16 | H | | $Q_{41}$ | |
| 6.17 | $COCH_3$ | $COCH_3$ | $Q_{41}$ | |
| 6.18 | H | H | $Q_{43}$ | |
| 6.19 | H | $COCH_3$ | $Q_{44}$ | |
| 6.20 | H | $COCH_3$ | $Q_{43}$ | |
| 6.21 | H | H | $Q_{44}$ | |
| 6.22 | H | H | $Q_{21}$ | |

Tabelle 6 (Fortsetzung)

| Verb. Nr. | $R_5$ | $R_4$ | $R_3$ | Physikal. Daten |
|---|---|---|---|---|
| 6.23 | H | sec-Butyl | sec-Butyl | Smp. 92-95°C |
| 6.24 | H | $COCH_3$ | sec-Butyl | Smp. 100-102°C |
| 6.25 | H | $CH_3$ | $CH_3$ | |
| 6.26 | H | $CH(CH_3)CH_2OCH_3$ | $CH(CH_3)CH_2OCH_3$ | |
| 6.27 | H | $C_2H_5$ | $C_2H_5$ | |
| 6.28 | $CH_3$ | sec-Butyl | sec-Butyl | |
| 6.29 | H | $COCH_3$ | $CH_3$ | |
| 6.30 | H | $COCH_3$ | $CH_2OCH_3$ | |
| 6.31 | H | $COCH_3$ | $C_2H_5$ | |
| 6.32 | H | $COC_2H_5$ | sec-Butyl | |
| 6.33 | H | $COCH_2OCH_3$ | sec-Butyl | |
| 6.34 | H | $COCH_2OCH_3$ | $C_2H_5$ | |
| 6.35 | H | $COCH_3$ | n-Propyl | |
| 6.36 | H | $COCH_3$ | i-Propyl | |
| 6.37 | H | n-Propyl | n-Propyl | |
| 6.38 | H | i-Propyl | i-Propyl | Smp. 173-175°C |

Tabelle 7

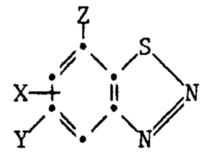

| Verb. Nr. | Y | X | Z | Physikal. Daten |
|---|---|---|---|---|
| 7.1 | Br | H | $COOCH_3$ | Smp. 138-141°C |
| 7.2 | Cl | H | $COOCH_3$ | Smp. 142°C |
| 7.3 | Cl | H | COOH | |
| 7.4 | H | 6-Cl | $COOCH_3$ | Smp. 111-114°C |
| 7.5 | H | 6-Cl | COOH | Smp. 255-260°C |
| 7.6 | H | 6-F | $COOCH_3$ | Smp. 122-125°C |
| 7.7 | Br | H | COOH | |
| 7.8 | H | 6-F | COOH | |
| 7.9 | H | 6-F | $COOC_2H_5$ | |
| 7.10 | H | 6-F | $COOC_3H_7(n)$ | |
| 7.11 | H | 6-F | $COOCH(CH_3)COOC_2H_5$ | |
| 7.12 | Cl | H | CN | |
| 7.13 | H | 4-Br | CN | |
| 7.14 | H | 4-Cl | CN | |
| 7.15 | H | 6-F | CN | |
| 7.16 | F | H | CN | |
| 7.17 | H | 4-F | CN | |
| 7.18 | H | 4-COOH | COOH | |
| 7.19 | H | $4-COOCH_3$ | $COOCH_3$ | |
| 7.20 | H | 6-OH | COOH | |
| 7.21 | H | 6-OH | $COOCH_3$ | |
| 7.22 | H | $6-OCH_3$ | $COOCH_3$ | |
| 7.23 | H | $4-CH_3$ | $COOCH_3$ | |
| 7.24 | F | 4-F | $COOCH_3$ | |
| 7.25 | F | 6-F | $COOCH_3$ | |
| 7.26 | H | H | CN | Smp. 116-118°C |
| 7.27 | $SO_3H$ | H | CN | |
| 7.28 | $SO_3H$ | H | COOH | |

Tabelle 7 (Fortsetzung)

| Verb. Nr. | Y | X | Z | Physikal. Daten |
|---|---|---|---|---|
| 7.29 | $SO_3H$ | H | $COOCH_3$ | |
| 7.30 | $NO_2$ | H | COOH | |
| 7.31 | $NO_2$ | H | $COOCH_3$ | |
| 7.32 | $SO_3Na$ | H | COONa | |
| 7.33 | $SO_3Na$ | H | CN | |
| 7.34 | $NH_2$ | H | CN | |
| 7.35 | $NH_2$ | H | COOH | |
| 7.36 | $NH_2$ | H | $COOCH_3$ | |
| 7.37 | $SO_3H$ | 6-F | $COOCH_3$ | |
| 7.38 | H | 6-F | CONHOH | |
| 7.39 | H | 6-F | $CONHNH_2$ | |
| 7.40 | H | 6-Cl | $CONHNH_2$ | Zers. 240°C |
| 7.41 | H | 6-Cl | 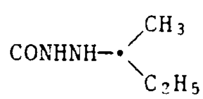 | |
| 7.42 | H | 6-F | $COOCH_2-Q_{21}$ | |
| 7.43 | H | 6-Cl | $CONHQ_{34}$ | |
| 7.44 | H | 6-COOH | COOH | |
| 7.45 | H | 6-Cl | 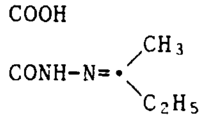 | |
| 7.46 | H | 6-Cl | 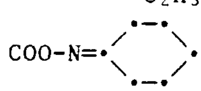 | |
| 7.47 | F | H | 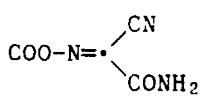 | |
| 7.48 | F | H | $COOQ_{46}$ | |
| 7.49 | H | 6-Cl | $COOCH_2-Q_{32}$ | |
| 7.50 | $NO_2$ | H | $COQ_{16}$ | |
| 7.51 | H | 6-Cl | CN | |
| 7.52 | F | H | COO-benzyl | |
| 7.53 | H | 6-F | COO-benzyl | |
| 7.54 | H | 4-F | COO-benzyl | |
| 7.55 | $NH_2$ | H | COO-benzyl | |
| 7.56 | $NO_2$ | H | COO-benzyl | |
| 7.57 | OH | H | $COO-CH_3$ | |

Tabelle 7 (Fortsetzung)

| Verb. Nr. | Y | X | Z | Physikal. Daten |
|---|---|---|---|---|
| 7.58 | F | H | $CONH_2$ | |
| 7.59 | F | H | COOH | |
| 7.60 | F | H | $COOCH_3$ | |
| 7.61 | F | H | $COOC_2H_5$ | Smp. 68-69°C |
| 7.62 | F | H | $COOCH_2CH_2CH_3$ | |
| 7.63 | F | H | $COOCH(CH_3)_2$ | |
| 7.64 | F | H | $COOCH_2C_6H_4$-o-Cl | |
| 7.65 | F | H | $COOCH_2CH_2Si(CH_3)_3$ | |
| 7.66 | F | H | $CON(OCH_3)CH_3$ | |

Tabelle 8

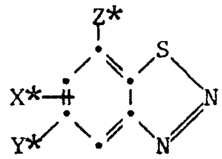

| Verb. Nr. | Y* | X* | Z* | Physikal. Daten |
|---|---|---|---|---|
| 8.1 | H | H | COCl | Smp. 107°C |
| 8.2 | H | H | COBr | |
| 8.3 | H | H | COF | |
| 8.4 | H | H | COJ | |
| 8.5 | H | H | $CO-OCOCH_3$ | |
| 8.6 | H | H | COO-CO- 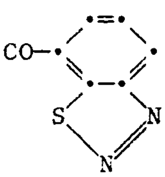 | Smp. 117-119°C |
| 8.7 | H | H | COOCO-Phenyl | |
| 8.8 | F | 6-F | COCl | |
| 8.9 | H | 6-F | COCl | |
| 8.10 | F | H | COCl | |
| 8.11 | H | 6-F | COO-CO- 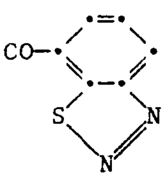 | |
| 8.12 | F | H | COO-CO- 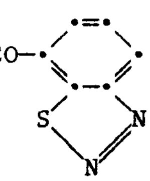 | |
| 8.13 | H | H | $COOSO_2-CH_3$ | |
| 8.14 | H | H | $COOOSO_2$-Phenyl | |

In der folgenden Tabelle 9 werden beispielhaft Zwischenprodukte der Formel I aufgezählt, die Teil der vorliegenden Erfindung sind:

Tabelle 9

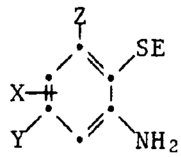

| Verb. Nr. | X | Y | Z | E | Physikal. Daten |
|---|---|---|---|---|---|
| 9.1 | H | $NH_2$ | COOH | $CH_2C_6H_5$ | Smp. 124-125°C |
| 9.2 | H | $NH_2$ | $COOCH_3$ | $CH_2C_6H_5$ | Smp. 84-86°C |
| 9.3 | H | $NH_2$ | $COOC_2H_5$ | $CH_2C_6H_5$ | |
| 9.4 | H | $NH_2$ | $COOCH(CH_3)_2$ | $CH_2C_6H_5$ | |
| 9.5 | H | $NH_2$ | $COOCH_2CH_2CH_3$ | $CH_2C_6H_5$ | |
| 9.6 | H | $NH_2$ | $COO(CH_2)_3CH_3$ | $CH_2C_6H_5$ | |
| 9.7 | H | $NH_2$ | $COOCH_2C_6H_5$ | $CH_2C_6H_5$ | |
| 9.8 | H | H | COOH | $CH_2C_6H_5$ | Smp. 98°C |
| 9.9 | H | H | $COOCH_3$ | $CH_2C_6H_5$ | |
| 9.10 | H | H | $COOC_2H_5$ | $CH_2C_6H_5$ | |
| 9.11 | H | H | $COOCH(CH_3)_2$ | $CH_2C_6H_5$ | |
| 9.12 | H | H | $COOCH_2CH_2CH_3$ | $CH_2C_6H_5$ | |
| 9.13 | H | H | $COO(CH_2)_3CH_3$ | $CH_2C_6H_5$ | |
| 9.14 | H | H | $COOCH_2C_6H_5$ | $CH_2C_6H_5$ | |
| 9.15 | H | $NH_2$ | COOH | $CH(CH_3)_2$ | |
| 9.16 | H | $NH_2$ | $COOCH_3$ | $CH(CH_3)_2$ | Smp. 109-110°C |
| 9.17 | H | $NH_2$ | $COOC_2H_5$ | $CH(CH_3)_2$ | |
| 9.18 | H | $NH_2$ | $COOCH(CH_3)_2$ | $CH(CH_3)_2$ | |
| 9.19 | H | $NH_2$ | $COOCH_2CH_2CH_3$ | $CH(CH_3)_2$ | |
| 9.20 | H | $NH_2$ | $COO(CH_2)_3CH_3$ | $CH(CH_3)_2$ | |
| 9.21 | H | $NH_2$ | $COOCH_2C_6H_5$ | $CH(CH_3)_2$ | |
| 9.22 | H | $NH_2$ | COOH | H | |
| 9.23 | H | $NH_2$ | $COOCH_3$ | H | |
| 9.24 | H | $NH_2$ | $COOC_2H_5$ | H | |
| 9.25 | H | $NH_2$ | $COOCH(CH_3)_2$ | H | |
| 9.26 | H | $NH_2$ | $COOCH_2CH_2CH_3$ | H | |
| 9.27 | H | $NH_2$ | $COOCH_2C_6H_5$ | H | |
| 9.28 | H | H | COOH | $CH(CH_3)_2$ | |

Tabelle 9 (Fortsetzung)

| Verb. Nr. | X | Y | Z | E | Physikal. Daten |
|---|---|---|---|---|---|
| 9.29 | H | H | $COOCH_3$ | $CH(CH_3)_2$ | |
| 9.30 | H | H | $COOC_2H_5$ | $CH(CH_3)_2$ | |
| 9.31 | H | H | $COOCH(CH_3)_2$ | $CH(CH_3)_2$ | |
| 9.32 | H | H | $COOCH_2CH_2CH_3$ | $CH(CH_3)_2$ | |
| 9.33 | H | H | $COOCH_2C_6H_5$ | $CH(CH_3)_2$ | |
| 9.34 | H | Br | COOH | $CH_2C_6H_5$ | |
| 9.35 | H | Br | $COOCH_3$ | $CH_2C_6H_5$ | |
| 9.36 | H | Br | $COOC_2H_5$ | $CH_2C_6H_5$ | |
| 9.37 | H | Br | $COOCH(CH_3)_2$ | $CH_2C_6H_5$ | |
| 9.38 | H | Br | $COOCH_2CH_2CH_3$ | $CH_2C_6H_5$ | |
| 9.39 | H | Br | $COOCH_2C_6H_5$ | $CH_2C_6H_5$ | |
| 9.40 | H | Cl | COOH | $CH_2C_6H_5$ | |
| 9.41 | H | Cl | $COOCH_3$ | $CH_2C_6H_5$ | |
| 9.42 | H | Cl | $COOC_2H_5$ | $CH_2C_6H_5$ | |
| 9.43 | H | Cl | $COOCH(CH_3)_2$ | $CH_2C_6H_5$ | |
| 9.44 | H | Cl | $COOCH_2CH_2CH_3$ | $CH_2C_6H_5$ | |
| 9.45 | H | Cl | $COOCH_2C_6H_5$ | $CH_2C_6H_5$ | |
| 9.46 | Cl | H | $COOCH_3$ | $CH_2C_6H_5$ | |
| 9.47 | Cl | H | COOH | $CH_2C_6H_5$ | |
| 9.48 | F | H | COOH | $CH_2C_6H_5$ | |
| 9.49 | F | H | $COOCH_3$ | $CH_2C_6H_5$ | |
| 9.50 | F | H | $COOC_2H_5$ | $CH_2C_6H_5$ | |
| 9.51 | F | H | $COOCH(CH_3)_2$ | $CH_2C_6H_5$ | |
| 9.52 | F | H | $COOCH_2CH_2CH_3$ | $CH_2C_6H_5$ | |
| 9.53 | F | H | $COOCH_2C_6H_5$ | $CH_2C_6H_5$ | |
| 9.54 | F | H | $COOCH_3$ | $CH(CH_3)_2$ | |
| 9.55 | F | H | $COOC_2H_5$ | $C_2H_5$ | |
| 9.56 | F | H | $COOCH_3$ | $CH_3$ | |

Tabelle 9 (Fortsetzung)

| Verb. Nr. | X | Y | Z | E | Physikal. Daten |
|---|---|---|---|---|---|
| 9.57 | H | $NH_2$ | COOH | $CH_3$ | |
| 9.58 | H | $NH_2$ | $COOCH_3$ | $CH_3$ | Smp. 102-104°C |
| 9.59 | H | $NH_2$ | $COOC_2H_5$ | $CH_3$ | |
| 9.60 | H | $NH_2$ | $COOCH(CH_3)_2$ | $CH_3$ | |
| 9.61 | H | $NH_2$ | $COOCH_2CH_2CH_3$ | $CH_3$ | |
| 9.62 | H | $NH_2$ | $COOCHC_6H_5$ | $CH_3$ | |
| 9.63 | H | $NH_2$ | COOH | $C_2H_5$ | |
| 9.64 | H | $NH_2$ | $COOCH_3$ | $C_2H_5$ | |
| 9.65 | H | $NH_2$ | $COOC_2H_5$ | $C_2H_5$ | Oel |
| 9.66 | H | $NH_2$ | $COOCH(CH_3)_2$ | $C_2H_5$ | |
| 9.67 | H | $NH_2$ | $COOCH_2CH_2CH_3$ | $C_2H_5$ | |
| 9.68 | H | $NH_2$ | $COOCH_2C_6H_5$ | $C_2H_5$ | |
| 9.69 | H | $NH_2$ | $COOCH_3$ | 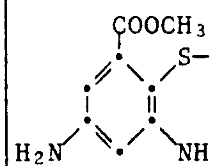 | |
| 9.70 | H | $NH_2$ | $COOCH_2C_6H_5$ | 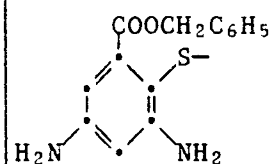 | |
| 9.71 | H | $NH_2$ | $COO(CH_2)_2CH_3$ | 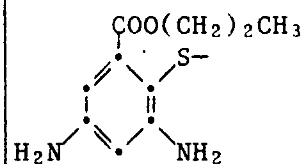 | |
| 9.72 | H | $NH_2$ | $COOCH_3$ | $CH_3(CH_2)_{11}$ | |
| 9.73 | H | $NH_2$ | $COOCH_2C_6H_5$ | $CH_3(CH_2)_{11}$ | |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 1 bis 8 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen 1 bis 8 | 80 % | 10 % | 5 % | 95 % |
| Ethylenglykolmonomethylether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 °C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus den Tabellen 1 bis 8 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1 bis 8 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen 1 bis 8 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1 bis 8 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1 bis 8 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 2.8 Extruder Granulat | |
|---|---|
| Wirkstoff aus den Tabellen 1 bis 8 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen 1 bis 8 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus den Tabellen 1 bis 8 | 40 % |
| Ethylenglykol | 10 % |
| Nonylphenolpolyethylenglykolether (15 Mol Ethylenoxid) | 6 % |
| N-Lingninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Immunisierende Wirkung gegen Colletotrichum lagenarium an Cucumis sativus L.

(A) Blattapplikation

Gurkenpflanzen werden nach 2-wöchiger Anzucht mit einer aus Spritzpulver des Wirkstoffes hergestellten Spritzbrühe besprüht (Konzentration: 0,02 % Aktivsubstanz).

Nach 1 Woche werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23° C im Dunkeln inkubiert. Die Inkubation wird dann bei normaler Luftfeuchtigkeit und bei 22° bis 23° C weitergeführt.

Die Beurteilung der Schutzwirkung erfolgt aufgrund des Pilzbefalls 7-8 Tage nach der Infektion.

B) Bodenapplikation

Gurkenpflanzen werden nach 2-wöchiger Anzucht mit einer aus Spritzpulver des Wirkstoffes hergestellten Spritzbrühe durch Bodenapplikation behandelt (Konzentration: 0,002 % Aktivsubstanz bezogen auf das Bodenvolumen).

1 Woche später werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23° C im Dunkeln inkubiert. Die Inkubation wird dann bei normaler Luftfeuchtigkeit und bei 22°-23° C weitergeführt.

Die Beurteilung der Schutzwirkung erfolgt aufgrund des Pilzbefalls 7-8 Tage nach der Infektion.

C) Beizapplikation

Gurkensamen werden mit einer Lösung des Wirkstoffes gebeizt (Konzentration: 180 g Aktivsubstanz/100 kg Samen). Die Samen werden ausgesät. Nach 4 Wochen werden die Pflanzen mit einer Sporensuspension $1.5 \cdot 10^5$ Sporen/ml) der Pilzes infiziert und für 36 Stunden bei hoher Luftfeuchtigkeit und einer Temperatur von 23° C inkubiert. Die Inkubation wird dann bei normaler Luftfeuchtigkeit und bei 22° bis 23° C weitergeführt. Die Beurteilung der Schutzwirkung erfolgt aufgrund des Pilzbefalls 7-8 Tage nach der Infektion.

Unbehandelte aber infizierte Kontrollpflanzen weisen in den Tests A und B sowie infizierte Pflanzen, deren Samen nicht behandelt waren, im Test C einen Pilzbefall von 100 % auf.

Verbindungen aus den Tabellen 1 bis 7 bewirken eine gute Immunisierung gegen Colletotrichum lagenarium. So blieben Pflanzen, die z.B. mit der Verbindung Nr. 1.1, 1.2, 1.3, 1.4, 1.5, 1.34, 1.39, 1.46, 1.79, 1.81, 1.86, 1.101, 1.116, 1.136, 1.139, 1.140, 1.144, 2.5, 3.29, 7.6 oder 7.26 behandelt wurden, fast völlig frei von Colletotrichum (Befall 20 bis 0 %).

Beispiel 3.2: Vergleichstest: direkte Wirkung gegen Colletotrichum lagenarium

Der formulierte Wirkstoff wird in verschiedenen Konzentrationen (100, 10, 1, 0.1 ppm) mit autoklaviertem und abgekühltem Nährboden (Gemüsesaft), der $10^3$ Sporen/ml enthält, gemischt und in Mikrotiterplatten gegossen. Die Inkubation erfolgt anschliessend bei 22° C im Dunkeln. Nach 2-3 Tagen wird das Pilzwachstum spektrophotometrisch gemessen und die $EC_{50}$-Werte bestimmt.

Bei z.B. der Verbindung 1.1, 1.2, 1.4, 1.34, 1.39, 1.79, 1.81, 1.86, 1.100, 1.101, 1.116, 1.135, 1.136, 1.139, 1.140, 1.144, 2.5, 3.26, 3.29 oder 7.6 wurde keine Hemmung des Pilzwachstums beobachtet. Dagegen trat bei Anwendung des Fungizids Benomyl (Handelsprodukt)

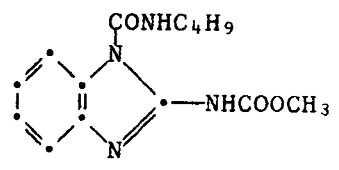

als Vergleichssubstanz bei 0,2 ppm eine 50%ige Hemmung ($EC_{50}$) von Colletotrichum lagenarium auf.

Beispiel 3.3: Immunisierende Wirkung gegen Pyricularia oryzae an Reispflanzen

A) Blattapplikation

Reispflanzen werden nach 3-wöchiger Anzucht mit einer aus Spritzpulver des Wirkstoffes hergestellten Spritzbrühe durch Blattapplikation behandelt (Konzentration: 0,02 % Aktivsubstanz). Nach 2-3 Tagen werden die Pflanzen mit einer Sporensuspension (350000 Sporen/ml) inokuliert und für 7 Tage bei hoher Luftfeuchtigkeit und bei einer Temperatur von 24 °C inkubiert. Die Beurteilung der Schutzwirkung erfolgt aufgrund des Pilzbefalls 7-8 Tage nach Inokulation.

Unbehandelte aber infizierte Kontrollpflanzen weisen in diesem Test einen Befall von 100 % auf.

Verbindungen aus den Tabellen 1 bis 8 bewirken eine gute Immunisierung gegen Pyricularia oryzae. So blieben Pflanzen, die z.B. mit der Verbindung 1.2, 1.34, 1.37, 1.38, 1.39, 1.72, 1.79, 1.86, 1.96, 1.103, 1.119, 1.135, 2.2, 2.3, 3.1, 3.2, 3.8, 3.9, 3.13, 4.2, 5.2 oder 7.2 behandelt wurden, fast völlig frei von Pyricularia oryzae (Befall von 20 bis 0 %).

B) Bodenapplikation

Reispflanzen werden nach 3-wöchiger Anzucht mit einer aus Spritzpulver des Wirkstoffes hergestellten Spritzbrühe durch Bodenapplikation behandelt (Konzentration: 0,002 % Aktivsubstanz auf das Bodenvolumen bezogen). Nach 2-3 Tagen werden die Pflanzen mit einer Sporensuspension ($35 \cdot 10^5$ Sporen/ml) inokuliert und für 7 Tage bei hoher Luftfeuchtigkeit und bei einer Temperatur von 24 °C inkubiert.

Die Beurteilung der Schutzwirkung erfolgt aufgrund des Pilzbefalls 7-8 Tage nach Inokulation.

Unbehandelte aber infizierte Kontrollpflanzen weisen in diesem Test einen Befall von 100 % auf. Verbindungen aus den Tabellen 1 bis 7 zeigten eine gute Wirkung gegen Pyricularia oryzae. So blieben Pflanzen, die z.B. mit den Verbindungen 1.2, 1.34, 1.37, 1.38, 1.39, 1.79, 1.96, 1.103, 1.119, 1.135, 2.2, 2.3, 3.1, 3.9, 3.13 oder 7.3 behandelt wurden, fast völlig frei von Pyricularia oryzae (Befall von 20 bis 0 %).

Beispiel 3.4: Vergleichstest: direkte Wirkung gegen Pyricularia oryzae

Der formulierte Wirkstoff wird bei verschiedenen Konzentrationen (100, 10, 1, 0.1 ppm) mit autoklaviertem und abgekühltem Nährboden (Gemüsesaft), der $10^3$ Sporen/ml enthält, gemischt und in Mikrotiterplatten gegossen. Inkubation erfolgt bei 22 °C im Dunkeln. Nach 2-3 Tagen wird das Pilzwachstum spektrophotometrisch bestimmt.

Bei z.B. der Verbindung 1.1, 1.2, 1.3, 1.4, 1.5, 1.34, 1.37, 1.39, 1.72, 1.86, 1.96, 1.100, 1.101, 1.103, 1.108, 1.140, 2.5, 3.1, 3.9, 3.26, 7.26 oder 7.6 wurde keine Hemmung des Pilzwachstums beobachtet. Dagegen trat bei Anwendung des Fungizids Benomyl (Handelsprodukt/vgl. Beispiel 3.2) als Vergleichssubstanz bei 0,1 ppm eine 50 %ige Hemmung ($EC_{50}$) von Pyricularia oryzae auf.

Beispiel 3.5: Immunisierende Wirkung gegen Pseudomonas lachrymans an Cucumis sativus L.

A) Blattapplikation

Gurkenpflanzen werden nach 2-wöchiger Anzucht mit einer aus Spritzpulver des Wirkstoffes hergestellten Spritzbrühe besprüht (Konzentration: 0,02 % Aktivsubstanz).

Nach 1 Woche werden die Pflanzen mit einer Bakteriensuspension ($10^8$ Bakterien/ml) infiziert und für 7 Tage bei hoher Luftfeuchtigkeit und einer Temperatur von 23 °C inkubiert.

Die Beurteilung der Schutzwirkung erfolgt aufgrund des Bakterienbefalls 7-8 Tage nach der Infektion.

Verbindungen aus den Tabellen 1 bis 7 bewirken eine gute Immunisierung gegen Pseudomonas lachrymans. So blieben Pflanzen, die z.B. mit der Verbindung 1.2, 1.3, 1.4, 1.5, 1.9, 1.34, 1.38, 1.46, 1.72, 1.79, 1.81, 1.119, 1.135, 2.2, 2.3, 3.1, 3.28, 3.29 oder 7.26 behandelt wurden, weitgehend frei von Pseudomonas (Befall 20 bis 0 %).

B) Bodenapplikation

Gurkenpflanzen werden nach 2-wöchiger Anzucht mit einer aus Spritzpulver des Wirkstoffes hergestellten Spritzbrühe durch Bodenapplikation behandelt (Konzentration: 0,002 % Aktivsubstanz bezogen auf das Bodenvolumen).

Nach 1 Woche werden die Pflanzen mit einer Bakteriensuspension ($10^8$ Bakterien/ml) infiziert und für 7 Tage bei hoher Luftfeuchtigkeit und einer Temperatur von 23 °C inkubiert.

Die Beurteilung der Schutzwirkung erfolgt aufgrund des Pilzbefalls 7-8 Tage nach der Infektion.

Verbindungen aus den Tabellen 1 bis 7 bewirken eine gute Immunisierung gegen Pseudomonas lachrymans. So blieben Pflanzen, die z.B. mit der Verbindung 1.1, 1.2, 1.3, 1.4, 1.5, 1.9, 1.34, 1.38, 1.46, 1.72, 1.79, 1.81, 1.119, 1.135, 2.2, 2.3, 3.1, 3.9, 3.28, 3.29 oder 7.26 behandelt wurden, fast völlig frei von Pseudomonas (Befall 20 bis 0 %).

Unbehandelte aber infizierte Kontrollpflanzen weisen in den Tests A und B einen Krankheitsbefall von 100 % auf.

Beispiel 3.6 : Vergleichstest: direkte Wirkung gegen Pseudomonas lachrymans

Der formulierte Wirkstoff wird bei verschiedenen Konzentrationen (100, 10, 1, 0.1 ppm) mit autoklavierter und abgekühlter Nährbrühe (Nutrient broth 0.8 %), die $10^6$ Bakterien/ml enthält, gemischt und in Mikrotiterplatten gegossen. Die Inkubation erfolgt dann bei 22°C im Dunkeln auf einem Schütteltisch (120 rpm). Nach 2-3 Tagen Inkubationsdauer wird das Bakterienwachstum spektrophotometrisch bestimmt.

Bei z.B. der Verbindung 1.1, 1.2, 1.3, 1.4, 1.5, 1.34, 1.38, 1.72, 1.79, 1.81, 1.96, 1.101, 1.119, 1.140, 2.2, 2.3, 2.5, 3.1, 3.6, 3.9, 3.26, 3.28, 3.29 oder 7.26 wurde keine Hemmung des Bakterienwachstums beobachtet. Dagegen trat bei Anwendung des Bakterizids Streptomycin als Vergleichssubstanz bei 0,4 ppm eine 50%ige Hemmung ($EC_{50}$) von Pseudomonas lachrymans auf.

Beispiel 3.7: Immunisierende Wirkung gegen Xanthomonas oryzae an Reispflanzen

A) Blattapplikation

Reispflanzen werden nach 3-wöchiger Anzucht mit einer aus Spritzpulver des Wirkstoffes hergestellten Spritzbrühe durch Blattapplikation behandelt (Konzentration: 0,02 % Aktivsubstanz). Nach 2-3 Tagen werden die Pflanzen mit einer Bakteriensuspension ($10^8$ Bakterien/ml) inokuliert und für 7 Tage bei hoher Luftfeuchtigkeit und bei einer Temperatur von 24°C inkubiert. Die Beurteilung der Schutzwirkung erfolgt aufgrund des Bakterienbefalls 7-8 Tage nach Inokulation.

Verbindungen aus den Tabellen bewirken eine gute Immunisierung gegen Xanthomonas oryzae. So blieben Pflanzen, die z.B. mit der Verbindung 1.3, 1.5, 1.16, 1.37, 1.38, 1.72, 1.81, 1.86, 1.95, 1.102, 1.103, 1.108, 1.136, 1.139, 2.2, 2.5 oder 3.29 behandelt wurden, fast völlig frei von Xanthomonas oryzae (Befall von 20 bis 0 %.

B) Bodenapplikation

Reispflanzen werden nach 3-wöchiger Anzucht mit einer aus Spritzpulver des Wirkstoffes hergestellten Spritzbrühe durch Bodenapplikation behandelt (Konzentration: 0,002 % Aktivsubstanz bezogen auf das Bodenvolumen. Nach 2-3 Tagen werden die Pflanzen mit einer Bakteriensuspension ($10^8$ Bakterien/ml) inokuliert und für 7 Tage bei hoher Luftfeuchtigkeit und bei einer Temperatur von 24°C inkubiert.

Die Beurteilung der Schutzwirkung erfolgt aufgrund des Bakterienbefalls 7-8 Tage nach Inokulation.

Verbindungen aus den Tabellen 1 bis 7 bewirken eine gute Immunisierung gegen Xanthomonas oryzae. So blieben Pflanzen, die z.B. mit der Verbindung 1.2, 1.3, 1.4, 1.5, 1.6, 1.9, 1.16, 1.34, 1.35, 1.38, 1.44, 1.46, 1.68, 1.71, 1.72, 1.81, 1.86, 1.96, 1.102, 1.103, 1.119, 1.135, 1.136, 2.2, 2.3, 2.5, 3.1, 3.13, 3.28, 3.29, 7.2, 7.5 oder 7.26 behandelt wurden, fast völlig frei von Xanthomonas oryzae (Befall 20 bis 0 %).

Unbehandelte aber infizierte Kontrollpflanzen weisen in den Tests A und B einen Befall von 100 % auf.

Beispiel 3.8: Vergleichstest: direkte Wirkung gegen Xanthomonas oryzae

Der formulierte Wirkstoff wird bei verschiedenen Konzentrationen (100, 10, 1, 0.1 ppm) mit autoklavierter und abgekühlter Nährbrühe (Nutrient broth 0,8 %), die $10^6$ Bakterien/ml enthält, gemischt und in Mikrotiterplatten gegossen. Inkubation erfolgt bei 22°C im Dunkeln auf einem Schütteltisch (120 rpm). Nach 2-3 Tagen wird das Bakterienwachstum spektrophotometrisch bestimmt.

Bei z.B. der Verbindung 1.1, 1.2, 1.3, 1.4, 1.5, 1.9, 1.34, 1.38, 1.81, 1.101, 1.119, 1.135, 1.140, 2.3 oder 2.5 wurde keine Hemmung des Bakterienwachstums beobachtet. Dagegen trat bei Anwendung des Bakterizids Streptomycin als Vergleichssubstanz bei 0,4 ppm eine 50%ige Hemmung ($EC_{50}$) von Xanthomonas oryzae auf.

Beispiel 3.9: Immunisierende Wirkung gegen Xanthomonas versicatori an Paprikapflanzen

A) Blattapplikation

Paprikapflanzen werden nach 4-wöchiger Anzucht mit einer aus Spritzpulver des Wirkstoffes hergestellten Spritzbrühe durch Blattapplikation behandelt (Konzentration: 0,02 % ppm). Nach 2-3 Tagen werden die Pflanzen mit einer Bakteriensuspension ($10^8$ Bakterien/ml) inokuliert und für 6 Tage bei hoher Luftfeuchtigkeit und bei einer Temperatur von 25° C inkubiert. Die Beurteilung der Schutzwirkung erfolgt aufgrund des Bakterienbefalls 7-8 Tage nach Inokulation.

Unbehandelte aber infizierte Kontrollpflanzen weisen in dem Test einen Befall von 100 % auf.

Verbindungen aus dem Tabellen 1 bis 7 bewirken eine gute Immunisierung gegen Xanthomonas vesicatoria. So blieben Pflanzen, die z.B. mit der Verbindung 1.2, 1.5, 1.9, 1.16, 1.34, 1.35, 1.37, 1.72, 1.81, 1.86, 1.96, 1.102, 1.103, 1.108, 1.136, 3.1, 3.13, 3.28, 3.29, 5.2, 7.26 oder 7.5 behandelt wurden, fast völlig frei von Xanthomonas vesicatoria (Befall von 20 bis 0 %).

B) Bodenapplikation

Paprikapflanzen werden nach 4-wöchiger Anzucht mit einer aus Spritzpulver des Wirkstoffes hergestellten Spritzbrühe durch Bodenapplikation behandelt (Konzentration: 60 ppm bezogen auf das Bodenvolumen). Nach 2-3 Tagen werden die Pflanzen mit Bakteriensuspension ($10^8$ Bakterien/ml) inokuliert und für 6 Tage bei hoher Luftfeuchtigkeit und bei einer Temperatur von 25° C inkubiert.

Die Beurteilung der Schutzwirkung erfolgt aufgrund des Bakterienbefalls 7-8 Tage nach Inokulation.

Unbehandelte aber infizierte Kontrollpflanzen weisen in dem Test einen Befall von 100 % auf.

Verbindungen aus den Tabellen 1 bis 7 bewirken eine gute Immunisierung gegen Xanthomonas vesicatoria. So blieben Pflanzen, die z.B. mit der Verbindung 1.2, 1.5, 1.6, 1.9, 1.11, 1.16, 1.34, 1.35, 1.36, 1.37, 1.39, 1.68, 1.71, 1.72, 1.81, 1.86, 1.95, 1.100, 1.102, 1.103, 1.108, 1.116, 1.140, 1.144, 2.5, 3.1, 3.13, 3.28, 3.29, 5.2, 7.2 oder 7.26 behandelt wurden, fast völlig frei von Xanthomonas vesicatoria (Befall von 0 bis 20 %).

Beispiel 3.10: Immunisierende Wirkung gegen Phytophthora infestans an Tomatenpflanzen

A) Blattapplikation

Tomatenpflanzen werden nach 3-wöchiger Anzucht mit einer aus Spritzpulver des Wirkstoffes hergestellten Spritzbrühe (0,02 % Aktivsubstanz) besprüht. Nach 2-3 Tagen werden die behandelten Pflanzen mit einer Sporangiensuspension des Pilzes ($5 \cdot 10^4$ Sporangien/ml) infiziert. Die Beurteilung der Schutzwirkung erfolgt nach einer Inkubation der infizierten Pflanzen während 5 Tagen bei 90-100 % relativer Luftfeuchtigkeit und 20° C.

Unbehandelte aber infizierte Kontrollpflanzen weisen in diesem Test einen Befall von 100 % auf.

Verbindungen aus den Tabellen 1 bis 7 bewirken eine gute Immunisierung gegen Phytophthora infestans. So blieben Pflanzen, die z.B. mit der Verbindung 1.6, 1.16, 1.34, 1.44, 1.68, 1.71, 1.72, 1.96, 1.101, 3.9 oder 7.26 behandelt wurden, weitgehend frei von Phytophthora (Befall: 20 bis 0 %).

B) Bodenapplikation

Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kam. Nach 4 Tagen werden die behandelten Pflanzen mit einer Sporangiensuspension ($5 \cdot 10^4$ Sporangien/ml) des Pilzes infiziert. Die Beurteilung der Schutzwirkung erfolgt nach einer Inkubation der infizierten Pflanzen während 5 Tagen bei 90-100 % relativer Luftfeuchtigkeit und 20° C.

Unbehandelte aber infizierte Kontrollpflanzen weisen in diesem Test einen Befall von 100 % auf.

Verbindungen aus den Tabellen 1 bis 7 bewirken eine gute Immunisierung gegen Phytophthora infestans. So blieben Pflanzen, die z.B. mit der Verbindung 1.6, 1.34, 1.44, 1.68, 1.71, 1.72, 1.101, 3.2, 3.4, 3.6, 3.8, 3.9, 3.13 oder 7.26 behandelt wurden, weitgehend frei von Phytophthora (Befall: 20 bis 0 %).

Beispiel 3.11: Vergleichstest: direkte Wirkung gegen Phytophthora infestans

Der formulierte Wirkstoff wird bei verschiedenen Konzentrationen (100, 10, 1, 0.1 ppm) mit steril filtriertem Nährboden (Erbsen/Agar), der $10^6$ Sporangien/ml enthält, gemischt und in Mikrotiterplatten

gegossen. Die Inkubation erfolgt bei 22°C im Dunkeln. Nach 2-3 Tagen wird das Pilzwachstum spektrophotometrisch bestimmt.

Bei z.B. der Verbindung 1.1, 1.2, 1.4, 1.34, 1.72, 1.86, 1.104, 1.108, 1.116, 1.135, 1.140, 1.144, 2.5, 3.1, 3.6, 3.9, 3.13, 3.26, 7.6 oder 7.26 wurde keine Hemmung des Pilzwachstums beobachtet. Dagegen trat bei Anwendung von Ridomil (Handelsprodukt) als Vergleichssubstanz bei 0,2 ppm eine 50%ige Hemmung von Phytophthora infestans auf.

Beispiel 3.12: Immunisierende Wirkung gegen Plasmopara viticola an Reben

Im 4-5 Blattstadium werden Rebensämlinge mit einer aus Spritzpulver des Wirkstoffes hergestellten Spritzbrühe (0,02 % Aktivsubstanz) besprüht. Nach 1 Woche werden die behandelten Pflanzen mit einer Sporangiensuspension ($5 \cdot 10^4$ Sporangien/ml) des Pilzes infiziert. Nach einer Inkubation während 6 Tagen bei 95-100 % relativer Luftfeuchtigkeit und 20°C wird die Schutzwirkung beurteilt.

Unbehandelte aber infizierte Kontrollpflanzen weisen in diesem Test einen Befall von 100 % auf.

Verbindungen aus den Tabellen 1 bis 8 bewirken eine gute Immunisierung gegen Plasmopara viticola. So blieben Reben, die z.B. mit der Verbindung 1.1, 1.2 oder 1.5 behandelt wurden, weitgehend frei von Plasmopara viticola (Befall: 20 - 0 %).

Beispiel 3.13: Immunisierende Wirkung gegen Pseudomonas tomato an Tomatenpflanzen

A) Blattapplikation

Tomatenpflanzen werden nach 3-wöchiger Anzucht mit einer aus Spritzpulver des Wirkstoffes hergestellten Spritzbrühe durch Blattapplikation behandelt (Konzentration: 0,02 % Aktivsubstanz). Nach 2-3 Tagen werden die Pflanzen mit einer Bakteriensuspension ($10^8$ Bakterien/ml) inokuliert und für 6 Tage bei hoher Luftfeuchtigkeit und bei einer Temperatur von 25°C inkubiert. Die Beurteilung der Schutzwirkung erfolgt aufgrund des Bakterienbefalls 7-8 Tage nach Inokulation.

Unbehandelte aber infizierte Kontrollpflanzen weisen in diesem Test einen Befall von 100 % auf.

Verbindungen aus den Tabellen 1 bis 8 bewirken eine gute Immunisierung gegen Pseudomonas tomato. So blieben Pflanzen, die z.B. mit der Verbindung 1.16, 1.95 oder 7.5 behandelt wurden, weitgehend frei von Pseudomonas (Befall: 20 bis 0 %).

B) Bodenapplikation

Tomatenpflanzen werden nach 3-wöchiger Anzucht mit einer aus Spritzpulver des Wirkstoffes hergestellten Spritzbrühe durch Bodenapplikation behandelt (Konzentration: 0,002 % Aktivsubstanz bezogen auf das Bodenvolumen). Nach 2-3 Tagen werden die Pflanzen mit einer Bakteriensuspension ($10^8$ Bakterien/ml) inokuliert und für 6 Tage bei hoher Luftfeuchtigkeit und bei einer Temperatur von 25°C inkubiert.

Die Beurteilung der Schutzwirkung erfolgt aufgrund des Bakterienbefalls 7-8 Tage nach Inokulation.

Unbehandelte aber infizierte Kontrollpflanzen weisen in diesem Test einen Befall von 100 % auf.

Verbindungen aus den Tabellen 1 bis 7 bewirken eine gute Immunisierung gegen Pseudomonas tomato. So blieben Pflanzen, die z.B. mit der Verbindung 1.44, 1.95 oder 7.5 behandelt wurden, fast völlig frei von Pseudomonas (Befall: 20 bis 0 %).

Beispiel 3.14: Immunisierende Wirkung gegen Phytophthora parasitica var. nicotianae an Tabakpflanzen

Bodenapplikation

Tabakpflanzen (8 Wochen alt) werden mit einer formulierten Lösung des Wirkstoffes durch Bodenapplikation behandelt (Konzentration: 2 ppm bezogen auf das Bodenvolumen) oder in das Blatt injiziert (Konzentration: 0,02 % Aktivsubstanz). Nach 4 Tagen werden die Pflanzen mit Phytophthora parasitica infiziert. 2 ml einer Zoosporensuspension ($8 \cdot 10^4$ z/ml) werden um die Stengelbasis pipettiert und mit Wasser in den Boden gewaschen. Die Pflanzen werden 3 Wochen bei 24°-26°C aufbewahrt.

Die Beurteilung der Symptome erfolgt aufgrund des Welkgrades der Pflanzen.

Unbehandelte aber infizierte Pflanzen waren zu 100 % verwelkt.

Verbindungen aus den Tabellen 1 bis 7 zeigen gute Wirkung gegen Phytophthora parasitica. So reduzierte z.B. die Verbindung 1.2 die Welkerscheinungen auf 0-5 %.

Beispiel 3.15: Direkte Wirkung gegen Phytophthora parasitica var. nicotianae

Der Wirkstoff wird mit Nährboden (V-8 Agar) in einer Konzentration von 100 ppm gemischt und in Petrischalen gegossen. Nach dem Abkühlen wird entweder eine Myzelrondelle (8 mm) im Zentrum der Platte plaziert oder 100 µl einer Pilzzoosporensuspension ($10^5$ Sporen/ml) aud die Platte gestrichen. Die Platten werden bei 22°C inkubiert.

Die Verbindung 1.2 zeigte keine hemmende Aktivität auf die Keimung und das Wachstum des Pilzes im Vergleich zu den Kontrollplatten ohne Wirkstoff.

Beispiel 3.16: Immunisierende Wirkung gegen Peronospora tabacina an Tabakpflanzen

A) Blattapplikation

Tabakpflanzen (8 Wochen alt) werden mit einer formulierten Lösung des Wirkungsstoffes besprüht (Konzentration: 0,02 % Aktivsubstanz). Vier Tage nach der Behandlung werden die Pflanzen mit einer Sporangiensuspension von Peronospora tabacina ($10^4$ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

B) Bodenapplikation

Tabakpflanzen (8 Wochen alt) werden mit einer formulierten Lösung des Wirkungsstoffes durch Bodenapplikation behandelt (Konzentration: 0,006 % Aktivsubstanz bezogen auf das Bodenvolumen). Nach 4 Tagen werden die Pflanzen mit einer Sporangiensuspension von Peronospora tabacina ($10^4$ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

Die Beurteilung der Symptome in den Tests A und B erfolgt aufgrund der mit Pilz befallenen Blattoberfläche.

Die Kontrollpflanzen zeigen einen Befall von 90 bis 100%. Pflanzen, welche in Test A und B mit der Verbindung 1.2 behandelt wurden, zeigten einen Befall von 0-35 %.

Beispiel 3.17: Direkte Wirkung gegen Peronospora tabacina

Formulierter Wirkstoff wird in verschiedenen Konzentrationen (10, 1, 0,1 ppm) mit Wasseragar gemischt und in Petrischalen gegossen. Nach dem Abkühlen werden 100 µl einer Sporangiensuspension ($10^6$ Sporen/ml) auf die Platte gestrichen. Die Platten werden 16 Stunden bei 18°C inkubiert.

Bei z.B. der Verbindung 1.2 wurde keine Hemmung der Keimung von Peronospora tabazina beobachtet.

Beispiel 3.18: Immunisierende Wirkung gegen Cercospora nicotianae an Tabakpflanzen

A) Blattapplikation

Tabakpflanzen (8 Wochen alt) werden mit einer formulierten Lösung des Wirkstoffs besprüht (Konzentration: 200 ppm). Vier Tagen nach der Behandlung werden die Pflanzen mit einer Sporensuspension von Cercospora nicotianae ($10^5$ Sporen/ml) inokuliert und für 5 Tage bei hoher Luftfeuchtigkeit und einer Temperatur von 22°-25°C inkubiert. Die Inkubation wird dann bei normaler Luftfeuchtigkeit und bei 20°-22°C weitergeführt.

B) Bodenapplikation

Tabakpflanzen (8 Wochen alt) wurden mit einer formulierten Lösung des Wirkstoffs durch Bodenapplikation behandelt (Konzentration: 0,002 % Aktivsubstanz). Nach 4 Tagen wurden die Pflanzen mit einer Sporensuspension von Cercospora nicotianae ($10^5$ Sporen/ml) inokuliert und für 5 Tage bei hoher Luftfeuchtigkeit und einer Temperatur von 22°-25°C inkubiert. Die Inkubation wurde dann bei normaler Luftfeuchtigkeit und bei 20°-22°C weitergeführt.

Die Beurteilung der Symptome in den Tests A und B erfolgt aufgrund des Pilzbefalls 12 bis 14 Tage nach der Infektion.

Die Kontrollpflanzen zeigten einen Befall von 100 %.

Pflanzen, welche in den Tests A und B mit der Verbindung 1.2 behandelt wurden, zeigten einen Befall von

0-20 %.

Beispiel 3.19: Direkte Wirkung gegen Cercospora nicotianae

Wirkstoff wird in verschiedenen Konzentrationen (100, 10, 1, 0,1 ppm) mit Nährboden (V-8 Agar) gemischt und in Petrischalen gegossen. Nach dem Abkühlen wird entweder eine Myzelrondelle (8 mm) im Zentrum der Platte plaziert oder 100 µl einer Sporensuspension ($5 \cdot 10^4$ Sporen/ml) auf die Platte gestrichen. Die Platten werden bei 22 °C inkubiert.

Die Verbindung 1.2 zeigte keine hemmende Aktivität auf die Keimung und das Wachstum des Pilzes im Vergleich zu den Kontrollplatten ohne Wirkstoff.

Beispiel 3.20: Immunisierende Wirkung gegen Pseudomonas tabaci an Tabakpflanzen

A) Blattapplikation

Tabakpflanzen (8 Wochen alt) werden mit einer formulierten Lösung des Wirkstoffs besprüht (Konzentration: 200 ppm) oder injiziert (Konzentration: 200, 60, 20 ppm). Nach 4 Tagen werden die Pflanzen mit einer Bakteriensuspension ($2 \cdot 10^7$ Bakterien/ml) besprüht und für 3 Tage bei hoher Luftfeuchtigkeit und 22 °-25 °C aufbewahrt. Die Inkubation wird dann 3 Tage bei normaler Luftfeuchtigkeit und 22 °-25 °C weiterverfolgt.

B) Bodenapplikation

Tabakpflanzen (8 Wochen alt) werden mit einer formulierten Lösung des Wirkstoffs durch Bodenapplikation behandelt (Konzentration: 0,002 % bis 0,0002 % Aktivsubstanz). Nach 4 Tagen wurden die Pflanzen mit einer Bakteriensuspension ($2 \cdot 10^7$ Bakterien/ml) besprüht und für 3 Tage bei hoher Luftfeuchtigkeit und 22 °-25 °C aufbewahrt. Die Inkubation wird dann 3 Tage bei normaler Luftfeuchtigkeit und 22-25 °C weiterverfolgt.

Die Beurteilung der Symptome in den Tests A und B erfolgt aufgrund des Bakteriebefalls.

Die Kontrollpflanzen zeigten einen Befall von 100 %.

Pflanzen, welche in den Tests A und B mit der Verbindung 1.2 oder 1.46 behandelt wurden zeigten einen Befall von 0-20 %.

Beispiel 3.21: Direkte Wirkung gegen Pseudomonas tabaci

Der Wirkstoff wird in verschiedenen Konzentrationen (100, 10, 1, 0,1 ppm) mit flüssigem Nährboden (Nutrient broth), der $10^6$ Bakterien/ml enthält, gemischt und in Mikrotiterplatten gegossen. Die Platten werden bei 22 °C inkubiert und das Wachstum der Bakterien wird nach 16 Stunden durch Messung der optischen Dichte bestimmt.

Bei z. B. der Verbindung 1.2 wurde keine Hemmung des Wachstums von Pseudomonas tabaci beobachtet. Streptomycin dagegen verursachte eine 50%ige Hemmung ($EC_{50}$) des Wachstums bei 0,1 ppm

Beispiel 3.22: Immunisierende Wirkung gegen den Tabakmosaikvirus und den Kartoffelvirus Y auf Tabakpflanzen

Tabakpflanzen (8 Wochen alt) wurden mit einer formulierten Lösung des Wirkstoffs besprüht (Konzentration: 200 ppm) oder injiziert (Konzentration: 0,02 % bis 0,0002 % Aktivsubstanz). Nach 4 Tagen werden die Pflanzen mit einer Suspension von Tabakmosaikvirus (0,5 µg/ml + Carborundum) oder von Kartoffelvirus Y (Saft eines infizierten Blattes, 1 g/100 ml $H_2O$ + Carborundum) mechanisch inokuliert und bei einer Temperatur von 20 °-22 °C inkubiert.

Die Beurteilung der Schutzwirkung erfolgt für den Tabakmosaikvirus aufgrund der Anzahl und der Grösse der Lokalläsionen 7 Tage nach der Inokulation und für den Kartoffelvirus Y durch serologische Bestimmung der Virenanzahl 7 und 10 Tage nach der Inokulation.

Pflanzen, welche mit der Verbindung 1.2 behandelt wurden, zeigten im Test beim Tabakmosaikvirus eine 88 bis 100%ige Hemmung der Entwicklung der Läsionen im Vergleich zu den entsprechenden Kontrollen (100 % Schädigung) und beim Kartoffelvirus Y eine 70 bis 100%ige Hemmung der Virusvermehrung im Vergleich zu den entsprechenden Kontrollen (= 100 %-Wert) auf. Die unbehandelten aber infizierten Pflanzen wiesen Läsionen von 100 % auf (Kontrolle).

Beispiel 3.23: Direkte Wirkung gegen den Tabakmosaikvirus

Der formulierte Wirkstoff wird direkt zu dem Tabakmosaikvirus-Inokulum addiert (200 ppm + 0,5 µg/ml Virus + Carborundum). Nach einer Stunde wird die Mischung auf Tabakpflanzen (8 Wochen alt) mechanisch inokuliert.

Pflanzen, die mit dieser Mischung aus Tabakmosaikviren und der Verbindung 1.2 inokuliert wurden, zeigten keine Schutzwirkung.

Beispiel 3.24: Immunisierende Wirkung gegen Erysiphe graminis auf Weizen

Weizenpflanzen werden nach 5-tägiger Anzucht mit einer aus Spritzpulver des Wirkstoffes hergestellten Spritzbrühe besprüht (Konzentration: 0,02 %). Einen Tag später werden die Pflanzen mit Konidien von Erysiphe graminis infiziert und bei 20° C inkubiert.

Die Beurteilung der Schutzwirkung erfolgt aufgrund des Pilzbefalls 8-10 Tage nach der Infektion.

Als Wirkstoff eingesetzte Verbindungen aus den Tabellen 1 bis 8 zeigten in diesem Test eine gute Wirkung gegen Erysiphe graminis. So blieben Pflanzen, die z.B. mit der Verbindung 1.2 behandelt wurden, weitgehend frei von Erysiphe-Befall (0 bis 20 % Schädigung). Unbehandelte aber infizierte Pflanzen (Kontrolle) wiesen dagegen einen Erysiphe-Befall von 100 % auf.

Beispiel 3.25a: In-vitro-Test der direkten Wirkung gegen Pyricularia oryzae, Colletotrichum lagenarium oder Phytophthora infestans

Der Wirkstoff der Testverbindungen wird a) zu einem flüssigen V-8-Medium (Gemüsegemisch) gegeben, das $10^4$ Sporen/ml von Pyricularia oryzae oder Colletotrichum lagenarium enthält, und b) zu einem flüssigen Erbsenmedium gegeben, das $10^4$ Sprangien/ml von Phytophthora infestans enthält, wobei in beiden Fällen die Endkonzentration des Wirkstoffs 60 ppm beträgt. Die präparierten Nährmedien werden auf Mikrotiterplatten gegeben und dort bei 22° C und 100 % relativer Luftfeuchtigkeit im Dunkeln 2 Tage lang gehalten, wobei die Präparate mit Pyricularia oryzae und Colletotrichum geschüttelt werden.

Das Pilzwachstum wird anschliessend durch spektrometrische Absorptionsmessung der Medien bei 595 nm (Trübungsmessung) bestimmt.

Beispiel 3.25b: In-vitro-Test von direkter Wirkung gegen Xanthomonas oryzae und Pseudomonas lachrymans

Der Wirkstoff der Testverbindungen wird zu einem Nährmedium (Bacto-Nutrient Broth Difco) gegeben, das $10^6$ Keime/ml von Xanthomonas oryzae oder Pseudomonas lachrymans enthält, wobei die Endkonzentration des Wirkstoffs 60 ppm beträgt. Das präparierte Nährmedium wird auf Mikrotiterplatten gegeben und dort bei 22° C und 100 % relativer Luftfeuchtigkeit im Dunkeln 2 Tage lang geschüttelt.

Das Bakterienwachstum wird anschliessend durch spekrometrische Absorptionsmessungen der Medien bei 595 nm (Trübungsmessung) bestimmt.

Parallel zur Wirkstoffprüfung in den vorstehend beschriebenen Tests werden Kontrollversuche in der beschriebenen Weise jedoch ohne Verwendung von Wirkstoffen durchgeführt. Der dabei gemessene Trübungsgrad stellt den 100 %-Wert der zur Anwendung gelangenden Bewertungsskala dar.

Die Auswertung erfolgt auf der Basis folgender Notenskala:

| Pilzwachstum (in %) | Note |
|---|---|
| 81 - 100 | 9* |
| 71 - 80 | 8* |
| 61 - 70 | 7* |
| 51 - 60 | 6 |
| 41 - 50 | 5 |
| 31 - 40 | 4 |
| 21 - 30 | 3 |
| 11 - 20 | 2 |
| 0 - 10 | 1 |

* Bei Noten grösser als oder gleich 7 wird auf Abwesenheit einer direkten Mikrobizidwirkung geschlossen.

Fungizide oder bakterizide in-vitro-Wirkung von Verbindungen der Formel I im Vergleich mit bekannten Substanzen

a) Test-Verbindungen der Formel

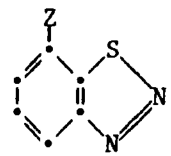

| Verb. Nr. | Z | Colletotrichum lagenarium | Pyricularia oryzae | Phytophthora infestans | Xanthomonas oryzae | Pseudomonas lachrymans |
|---|---|---|---|---|---|---|
| 1.1 | COOH | 9 | 9 | 9 | 9 | 9 |
| 1.2 | COOMethyl | 9 | 9 | 9 | 9 | 9 |
| 1.4 | COO-n-propyl | 9 | 9 | 9 | 9 | 9 |
| 1.135 | COO-cyclohexyl | 6 | - | 9 | 9 | - |
| 1.34 | COO-Benzyl | 9 | 9 | 9 | 9 | 9 |
| 1.96 | COO-$CH_2$-2'Furyl | - | 9 | 4 | - | 9 |
| 2.5 | COS-Benzyl | 9 | 9 | 9 | 9 | 9 |
| 1.101 | $COOCH_2$-2-Pyridinyl | 9 | 9 | 6 | 9 | 9 |
| 1.140 | $COOCH_2$-2-Methylphenyl | 9 | 9 | 9 | 9 | 9 |
| 1.144 | $COOCH_2$-3-Methoxyphenyl | 9 | - | 9 | 9 | - |
| 1.116 | $COOCH_2$-1-Naphthyl | 9 | - | 9 | 6 | - |
| 1.108 | $COOCH_2CH_2P(O)(OMethyl)_2$ | - | 9 | 9 | - | 9 |
| 1.104 | $COOCH_2CH_2Si(Methyl)_3$ | - | 3 | 9 | - | 9 |

(Fortsetzung)

| Verb. Nr. | Z | Colletotrichum lagenarium | Pyricularia oryzae | Phytophthora infestans | Xanthomonas oryzae | Pseudomonas lachrymans |
|---|---|---|---|---|---|---|
| 1.72 | 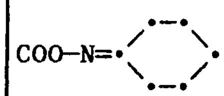 | - | 9 | 9 | - | 9 |
| 1.86 | COO-Diaceton-D-gluco-sidyl | - | 9 | 9 | - | 9 |
| 3.1 | $CONH_2$ | - | 9 | 9 | - | 9 |
| 3.26 | $CON(allyl)_2$ | - | 9 | 9 | - | 8 |
| 3.13 | 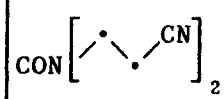 | - | 6 | 9 | - | 9 |
| 1.100 | 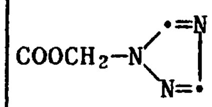 | 9 | 9 | - | 9 | - |
| 3.6 | CONH-Phenyl | - | - | 9 | - | 9 |
| 3.9 | $CONH-CH_2COOH$ | - | 9 | 9 | - | 9 |
| 7.26 * | CN | - | 9 | 9 | - | 8 |

b) Test-Verbindungen der Formeln

| Verb. Nr. | Formel | Colletotrichum lagenarium | Pyricularia oryzae | Phytophthora infestans | Xanthomonas oryzae | Pseudomonas lachrymans |
|---|---|---|---|---|---|---|
| 7.6 | 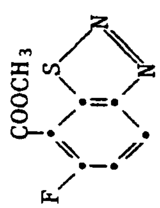 | - | 9 | 9 | - | 8 |
| * | 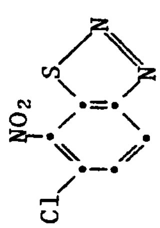 | 1 | 1 | 1 | - | - |
| * | 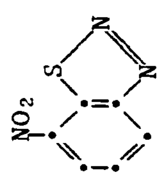 | 1 | 1 | 1 | - | - |

* Verbindungen aus DE-OS 1 695 786

**Patentansprüche**

**Patentansprüche für folgende Vertragsstaaten : AT, BE, CH, DE, FR, GB, GR, IT, LI, LU, NL, SE**

**1.** Verfahren zur Immunisierung von Pflanzen gegen den Befall durch phytopathogene Mikroorganismen, dadurch gekennzeichnet, dass auf Pflanzen und/oder deren Umgebung als Wirkstoff eine Verbindung der nachfolgenden Formel I appliziert wird:

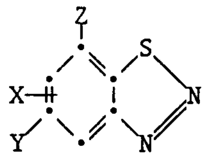

(I)

worin bedeuten:

X Wasserstoff, Halogen, Hydroxy, Methyl, Methoxy, HOOC oder MOOC;

Y Wasserstoff, Halogen, $SO_3H$, $SO_3M$, Nitro, Hydroxy, oder Amino, wobei M das Moläquivalent eines Alkali- oder Erdalkali-Ions ist, das aus einer entsprechenden Base oder basischen Verbindung gebildet wird; und

Z Cyano oder -CO-A, wobei A entweder OH oder SH bedeutet, deren H auch durch das Moläquivalent eines anorganischen oder organischen kationischen Restes ersetzt sein kann.

**2.** Verfahren zur Immunisierung von Pflanzen gegen den Befall durch phytopathogene Mikroorganismus, dadurch gekennzeichnet, dass auf Pflanzen und/oder deren Umgebung als Wirkstoff eine Verbindung der nachfolgenden Formel I appliziert werden:

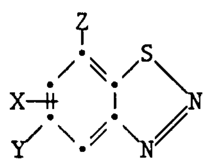

(I)

worin bedeuten:

X Wasserstoff, Halogen, Hydroxy, Methyl, Methoxy, HOOC oder MOOC;

Y Wasserstoff, Halogen, $SO_3H$, $SO_3M$, Nitro, Hydroxy, oder Amino, wobei M das Moläquivalent eines Alkali- oder Erdalkali-Ions ist, das aus einer entsprechenden Base oder basischen Verbindung gebildet wird; und

Z Cyano oder -CO-A,

A UR, $N(R_1)R_2$ oder $U^1N(=C)_n(R_3)R_4$;

M Moläquivalent eines Alkali- oder Erdalkali-Ions, das aus einer entsprechenden Base oder basischen Verbindung gebildet ist;

U Sauerstoff oder Schwefel;

$U^1$ Sauerstoff oder $-N(R_5)-$;

R Wasserstoff, $C_1$-$C_8$-Alkyl, durch Halogen, Cyano, Nitro, Hydroxy, U-$C_1$-$C_3$-Alkyl oder $C_2$-$C_4$-Dialkylamino substituiertes oder durch die CO-Gruppe unterbrochenes $C_1$-$C_8$-Alkyl, (T)-COOH oder (T)-$COOC_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

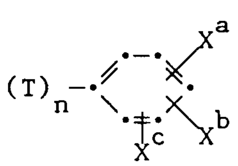

, $(T)_n$-Napht, $(T)_n$-Si($C_1$-$C_8$-Alkyl)$_3$,

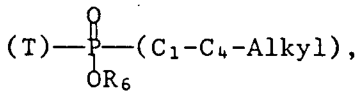

, 

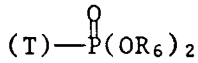

; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder

$X^a$ $C_1$-$C_2$-Halogenalkoxy mit bis zu 5 Halogenatomen, Nitro, Dimethylamino, Phenyl, Phenyloxy,

Benzyloxy, Sulfamoyl und $X^b$ und $X^c$ gleichzeitig Wasserstoff; oder
$X^a$ Phenyl, Phenyloxy oder Benzyloxy und
$X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;
W einen 5- bis 7-gliedrigen gesättigten oder ungesättigten unsubstituierten oder durch Halogen, Trifluormethyl, Cyano, $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$, $-CH(CH_3)-$, $-CCH_3(CH_3)-$, $-CH_2CH_2CH_2-$ oder $-CH_2CH_2O-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, Methoxy, $CF_3$, Cyano, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
wobei in den Verbindungen der Formel I der organische Rest A ein Molgewicht von weniger als 900 besitzt; und umfassen im Falle von U als Sauerstoff oder Schwefel die Salze der pflanzenphysiologisch verträglichen 7-Carbonsäure mit primären, sekundären oder tertiären Aminen oder mit anorganischen Basen.

**3.** Verfahren gemäss Anspruch 1 oder 2, dadurch gekennzeichnet, dass die Aufwandmenge weniger als 1 kg AS/ha beträgt.

**4.** Verfahren gemäss Anspruch 2, dadurch gekennzeichnet, dass Verbindungen der Formel I verwendet werden, in welcher bedeuten:
X und Y für Wasserstoff;
Z für Cyano oder COA;
A für UR oder $U^1N(=C)_n(R_3)R_4$;
U für Sauerstoff;
$U^1$ für Sauerstoff oder $-N(R_5)-$;
R für Wasserstoff, $C_1$-$C_8$-Alkyl, durch Halogen oder $C_1$-$C_3$-Alkoxy substituiertes $C_1$-$C_8$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, Benzyl, halogeniertes Benzyl, Benzylmethoxy, $(T)_n$-$Si(CH_3)_3$, (T)-P(O)($C_1$-$C_4$-Alkyl)$CH_3$, (T)-P(O)(O$C_1$-$C_4$-Alkyl$)_2$ oder die Gruppe $(T)_n$-W;
W für einen 5- bis 7-gliedrigen gesättigten oder ungesättigten unsubstituierten Heterocyclus mit 1 bis 3 Heteroatomen aus der Gruppe O, N und S sowie für Diaceton-D-glucosidyl;
T für die Brückenglieder $-CH_2-$, $-CH_2CH_2-$, $-CH(CH_3)-$, $-CCH_3(CH_3)-$ oder $-CH_2CH_2CH_2-$;
$R_3$ für Wasserstoff, Cyano, $C_1$-$C_6$-Alkyl, Phenyl oder W;
$R_4$ für Wasserstoff, $C_1$-$C_6$-Alkyl, $CONH_2$, CONH-CONH-$C_1$-$C_3$-Alkyl, $C_1$-$C_3$-Alkanoyl oder $C_3$-$C_5$-Alkenoyl;
$R_3$ und $R_4$ zusammen mit dem Atom, an welches sie gebunden sind, W oder W';

W für einen 5- bis 7-gliedrigen gesättigten oder ungesättigten Heterocyclus mit 1 bis 3 Heteroatomen aus der Gruppe O, N und S;
W' für einen Rest aus der Gruppe

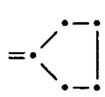 , 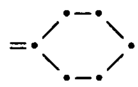 und 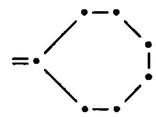 ;

n für Null oder 1.

**5.** Verbindungen der Formel I'

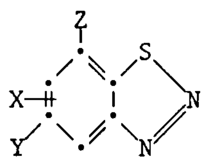 (I')

in welcher bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-COO$C_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

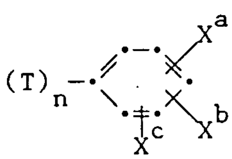 , $(T)_n$-Napht, $(T)_n$-Si($C_1$-$C_8$-Alkyl)$_3$,

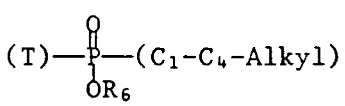 , 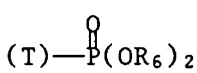 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder
$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder
$X^a$ Phenyl, Phenyloxy oder Benzyloxy und $X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;
W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;

T die Brückenglieder $-CH_2-$, $-CH_2CH_2-$, $-CH(CH_3)-$, $-CH_2CH_2CH_2-$ oder $-CH_2CH_2O-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure.

**6.** Verbindungen der Formel I'

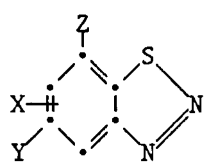

(I')

in welcher bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, Alkoxy oder U-$C_1$-$C_3$-Alkyl substituiertes $C_1$-$C_8$-Alkyl, (T)-COOH oder (T)-COO$C_1$-$C_4$-Alkyl, $C_2$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

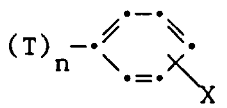 , $(T)_n$-Naphthyl, $(T)_n-Si(C_1-C_8-Alkyl)_3$,

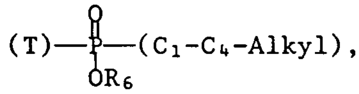 , 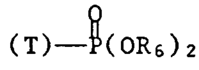 ; oder $(T)_n$—W ;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$ oder $-CH(CH_3)-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure.

**7.** Verbindungen der Formel I' gemäss Anspruch 5, bei denen Z = COA bedeutet und R eine andere Bedeutung hat als Wasserstoff und unsubstituiertes Alkyl.

**8.** Verbindungen der Formel I' gemäss Anspruch 5 aus der Gruppe:
7-n-Pentoxycarbonyl-benzo-1,2,3-thiadiazol;
7-(4-Methoxy-benzyloxycarbonyl)-benzo-1,2,3-thiadiazol;
7-(Cycloheximino-oxycarbonyl)-benzo-1,2,3-thiadiazol;
7-(3-Hydroxy-n-propoxycarbonyl)-benzo-1,2,3-thiadiazol;
1,2,5,6-Di-O-isopropyliden-3-(7-benzo-1,2,3-thiadiazoyl)-D-glucofuranose;
7-Furfuryloxycarbonyl-benzo-1,2,3-thiadiazol;
7-(1,2,4-Triazol-1-yl)-methoxycarbonyl-benzo-1,2,3-thiadiazol;
7-(2-Pyridylmethoxycarbonyl)-benzo-1,2,3-thiadiazol;

7-Trimethylsilyl-methoxycarbonyl-benzo-1,2,3-thiadiazol;
7-[2-(Trimethylsilyl)-ethoxycarbonyl]-benzo-1,2,3-thiadiazol;
7-Dimethylphosphono-ethoxycarbonyl-benzo-1,2,3-thiadiazol;
7-Cyclohexyloxycarbonyl-benzo-1,2,3-thiadiazol;
7-(1-Phenethyloxycarbonyl)-benzo-1,2,3-thiadiazol;
7-(3-Methoxybenzyl)-benzo-1,2,3-thiadiazol;
7-(Ethylthio-carbonyl)-benzo-1,2,3-thiadiazol;
7-(n-Propylthio-carbonyl)-benzo-1,2,3-thiadiazol;
7-(Benzylthio-carbonyl)-benzo-1,2,3-thiadiazol;
7-Carbamoyl-benzo-1,2,3-thiadiazol;
7-N-Phenylcarbamoyl-benzo-1,2,3-thiadiazol;
N-(7-Benzo-1,2,3-thiadiazoyl)-glycin;
7-(N-Diallylcarbamoyl)-benzo-1,2,3-thiadiazol;
6-Fluor-7-methoxycarbonyl-benzo-1,2,3-thiadiazol;
6-Fluor-7-carboxy-benzo-1,2,3-thiadiazol;
5-Fluor-7-benzyloxycarbonyl-benzo-1,2,3-thiadiazol;
5-Fluor-7-carboxy-benzo-1,2,3-thiadiazol;
5-Fluor-7-ethoxycarbonyl-benzo-1,2,3-thiadiazol;

**9.** Verbindungen der Formel I' gemäss Anspruch 6 aus der Gruppe:
7-Ethoxycarbonyl-benzo-1,2,3-thiadiazol;
7-n-Propoxycarbonyl-benzo-1,2,3-thiadiazol;
7-iso-Propoxycarbonyl-benzo-1,2,3-thiadiazol;
7-n-Butoxycarbonyl-benzo-1,2,3-thiadiazol;
7-sek.Butoxycarbonyl-benzo-1,2,3-thiadiazol;
7-tert.Butoxycarbonyl-benzo-1,2,3-thiadiazol;
7-Cyclopropylmethoxycarbonyl-benzo-1,2,3-thiadiazol;
7-(2'-Phenethoxycarbonyl)-benzo-1,2,3-thiadiazol;
7-Benzyloxycarbonyl-benzo-1,2,3-thiadiazol;
7-Allyloxycarbonyl-benzo-1,2,3-thiadiazol;
7-Propin-2-yloxycarbonyl-benzo-1,2,3-thiadiazol;
2-(7-Benzo-1,2,3-thiadiazoyloxy-imino)-2-cyano-N-(ethylaminocarbonyl)-acetamid;
Na-Salz der Benzo-1,2,3-thiadiazol-7-carbonsäure;
K-Salz der Benzo-1,2,3-thiadiazol-7-carbonsäure;
Triethylammoniumsalz der Benzo-1,2,3-thiadiazol-7-carbonsäure;
7-(1'-Phenethoxycarbonyl)-benzo-1,2,3-thiadiazol;
7-(1'-Naphthylmethoxycarbonyl)-benzo-1,2,3-thiadiazol;
7-(Methylthio-carbonyl)-benzo-1,2,3-thiadiazol;
7-(Ethylthio-carbonyl)-benzo-1,2,3-thiadiazol;
7-(Benzylthio-carbonyl)-benzo-1,2,3-thiadiazol;
7-[(Di-cyanomethyl)-amino-carbonyl]-benzo-1,2,3-thiadiazol;
3-[(N-Benzo-1,2,3-thiadiazol-7-ylcarbonyl)-1,3,4-thiadiazol-2-ylamino]-2-methyl-propencarbonsäuremethylester;
3-[(N-Benzo-1,2,3-thiadiazol-7-ylcarbonyl)-1,3,4-thiadiazol-2-ylamino]-2-ethyl-propencarbonsäuremethylester;
1-(Benzo-1,2,3-thiadiazol-7-carbonyl)-2-($\alpha$-methylpropyliden)-hydrazin;
1-(Benzo-1,2,3-thiadiazol-7-carbonyl)-2-(cyclobutyliden)-hydrazin;
1-(Benzo-1,2,3-thiadiazol-7-carbonyl)-2-(cyclopentyliden)-hydrazin;
1-(Benzo-1,2,3-thiadiazol-7-carbonyl)-2-(cyclohexyliden)-hydrazin;
2-(Benzo-1,2,3-thiadiazol-7-carbonyl)-1-(2'-sec-butyl)-hydrazin;
1-(Benzo-1,2,3-thiadiazol-7-carbonyl)-2-(cyclopentyl)-hydrazin;
1-(Benzo-1,2,3-thiadiazol-7-carbonyl)-2-(cyclohexyl)-hydrazin;
1-(Benzo-1,2,3-thiadiazol-7-carbonyl)-2-(cycloheptyl)-hydrazin;
1-(Benzo-1,2,3-thiadiazol-7-carbonyl)-1,2-diacetyl-hydrazin;
1-(Benzo-1,2,3-thiadiazol-7-carbonyl)-2-phenyl-hydrazin;
1-(Benzo-1,2,3-thiadiazol-7-carbonyl)-2-pyridin-2'-yl-hydrazin.

**10.** Verfahren gemäss Anspruch 2, dadurch gekennzeichnet, dass Verbindungen der Formel I' gemäss

Anspruch 5 verwendet werden.

11. Verfahren gemäss Anspruch 2, dadurch gekennzeichnet, dass Verbindungen der Formel I' gemäss Anspruch 6 verwendet werden.

12. Verfahren gemäss Anspruch 2, dadurch gekennzeichnet, dass Verbindungen der Formel I' gemäss Anspruch 7 verwendet werden.

13. Verfahren gemäss Anspruch 2, dadurch gekennzeichnet, dass als Wirkstoff 1,2,3-Benzothiadiazol-7-carbonsäure oder ein Salz davon mit einer basischen Verbindung verwendet wird.

14. Verfahren gemäss Anspruch 13, dadurch gekennzeichnet, dass als Salze solche mit primären, sekundären oder tertiären Aminen oder Alkali- oder Erdalkaliverbindungen verwendet werden.

15. Verfahren gemäss Anspruch 2, dadurch gekennzeichnet, dass als Wirkstoff 7-Methoxycarbonyl-benzo-1,2,3-thiadiazol verwendet wird.

16. Verfahren gemäss Anspruch 2, dadurch gekennzeichnet, dass als Wirkstoff 7-Benzyloxycarbonyl-benzo-1,2,3-thiadiazol verwendet wird.

17. Verfahren gemäss Anspruch 2, dadurch gekennzeichnet, dass als Wirkstoff 7-Cyano-benzo-1,2,3-thiadiazol verwendet wird.

18. Verfahren gemäss Anspruch 2, dadurch gekennzeichnet, dass als Wirkstoffe Verbindungen von 7-$C_2$-$C_4$-Alkoxycarbonyl-benzo-1,2,3-thiadiazol verwendet werden.

19. Mittel zur Immunisierung von Pflanzen gegen den Befall durch phytopathogene Mikroorganismen enthaltend als Wirkstoff mindestens eine Verbindung der Formel I gemäss Anspruch 1 oder 2 mit Ausnahme der Verbindungen 7-Cyano-benzo-1,2,3-thiadiazol; 4-Chlor-7-cyano-benzo-1,2,3-thiadiazol; 6-Chlor-7-cyano-benzo-1,2,3-thiadiazol, 4,6-Dibrom-7-cyano-benzo-1,2,3-thiadiazol und 7-Methoxycarbonyl-benzo-1,2,3-thiadiazol.

20. Mittel zur Immunisierung von Pflanzen gegen den Befall durch phytopathogene Mikroorganismen enthaltend als Wirkstoff mindestens eine Verbindung der Formel I' gemäss Anspruch 5.

21. Mittel zur Immunisierung von Pflanzen gegen den Befall durch phytopathogene Mikroorganismen enthaltend als Wirkstoff mindestens eine Verbindung der Formel I' gemäss Anspruch 6.

22. Mittel gemäss Anspruch 19, dadurch gekennzeichnet, dass es als Wirkstoff mindestens eine Verbindung der Formel I' gemäss Anspruch 7 enthält.

23. Mittel gemäss Anspruch 19, dadurch gekennzeichnet, dass es als Wirkstoff mindestens eine Verbindung gemäss einem der Ansprüche 8 oder 9 enthält.

24. Mittel gemäss Anspruch 19, dadurch gekennzeichnet, dass es als Wirkstoff mindestens eine der Verbindungen Benzo-1,2,3-thiadiazol-7-carbonsäure oder 7-Methoxycarbonyl-benzo-1,2,3-thiadiazol enthält.

25. Mittel gemäss Anspruch 19, dadurch gekennzeichnet, dass es als Wirkstoff 1,2,3-Benzothiadiazol-7-carbonsäure oder ein Salz davon mit einer basischen Verbindung enthält.

26. Mittel gemäss Anspruch 25, dadurch gekennzeichnet, dass als Salze solche mit primären, sekundären oder tertiären Aminen oder Alkali- oder Erdalkaliverbindungen verwendet werden.

27. Verfahren zur Herstellung von Verbindungen der Formel I'

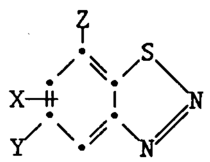

(I')

in welcher bedeuten:

X Wasserstoff, Halogen, Hydroxy, Methyl, Methoxy, HOOC oder MOOC;

Y Wasserstoff, Halogen, $SO_3H$, $SO_3M$, Nitro, Hydroxy oder Amino;

Z Cyano oder COA;

A UR, $N(R_1)R_2$ oder $U^1N(=C)_n(R_3)R_4$;

M Moläquivalent eines Alkali- oder Erdalkali-Ions, das aus einer entsprechenden Base oder basischen Verbindung gebildet ist;

U Sauerstoff oder Schwefel;

$U^1$ Sauerstoff oder $-N(R_5)-$;

R Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-$COOC_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

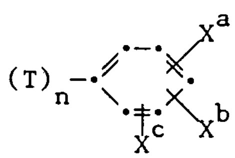

, $(T)_n$-Napht, $(T)_n$-$Si(C_1$-$C_8$-$Alkyl)_3$,

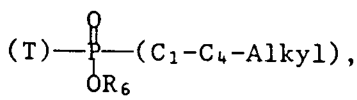

, 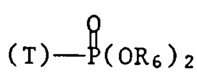 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder

$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder

$X^a$ Phenyl, Phenyloxy oder Benzyloxy und $X^b$ Halogen oder Methyl und $X^c$ Wasserstoff; oder

$X^a$, $X^b$ und $X^c$ zusammen 4 oder 5 Fluoratome;

Napht einen unsubstituierten oder mit Halogen, Methyl, Methoxy oder Nitro substituierten Naphthylrest;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;

T die Brückenglieder $-CH_2-$, $-CH_2CH_2-$, $-CH(CH_3)-$, $-CH_2CH_2CH_2-$ oder $-CH_2CH_2O-$;

$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;

$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;

$R_1$ und $R_2$ zusammen mit dem Atom, an welches sie gebünden sind, einen Heterocyclus W;

$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;

$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebünden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure;
dadurch gekennzeichnet, dass

1. Verbindungen der Formel Ia

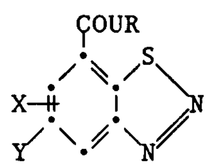

(Ia)

in welcher R, X, Y und U die unter Formel I angegebenen Bedeutungen besitzen, hergestellt werden, indem man umsetzt:

1.1 Verbindungen der Formel II

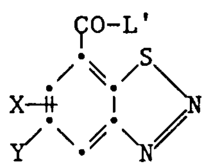

(II)

worin L' eine Abgangsgruppe, z.B. Halogen, O-Acyl, wie z.B. der zum symmetrischen Anhydrid der Säure Ib gehörende Acylrest, oder 1-Imidazoyl bedeutet,
mit Verbindungen der Formel III

RUH (III)

a) im Ueberschuss des Reaktanden RUH oder
b) in Gegenwart einer organischen Base entweder mit oder ohne 4-Dialkylaminopyridin als Katalysator in inerten Lösungsmitteln oder
c) in Gegenwart anorganischer Basen, wobei jeweils im Temperaturbereich von -10° bis 180°C, vorzugsweise 0° bis 100°C, gearbeitet wird; und

1.2 Verbindungen der Formel Ib

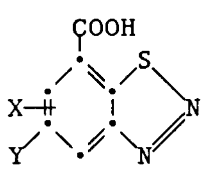

(Ib)

mit Verbindungen der Formel III im Ueberschuss oder in einem inerten Lösungsmittel in Gegenwart einer Säure, wie Schwefelsäure, Salzsäure, p-Toluolsulfonsäure oder Bortrifluorid-Diethylether-Komplex, oder von Dicyclohexylcarbodiimid bei Temperaturen von -10° bis 180°C, vorzugsweise 0° bis 140°C; und

2. Verbindungen der Formel Ic

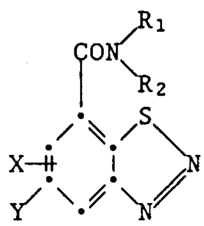

(Ic)

in welcher die Symbole $R_1$, $R_2$, X und Y die unter Formel I angegebenen Bedeutungen besitzen, hergestellt werden, indem man umsetzt:

2.1 Verbindungen der Formel II mit Verbindungen der Formel IV

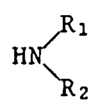

(IV)

a) im Ueberschuss des Reaktanden $HN(R_1)R_2$ oder

b) in Gegenwart einer organischen Base entweder mit oder ohne 4-Dialkylaminopyridin als Katalysator in inerten Lösungsmitteln oder

c) in Gegenwart anorganischer Basen, wobei jeweils im Temperaturbereich von -10° bis 160°C, vorzugsweise 0° bis 100°C, gearbeitet wird; und

3. Verbindungen der Formel Id

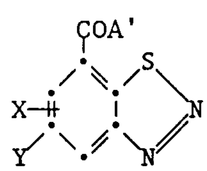

(Id)

worin A' den Rest $U^1N(=C)_n(R_3)R_4$ darstellt und X, Y, $R_3$, $R_4$, $R_5$ und n die unter Formel I angegebenen Bedeutungen besitzen, hergestellt werden, indem man

3.1 Verbindungen der Formel Ie

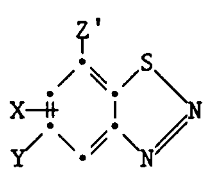

(Ie)

in welcher Z' die Gruppe COOH, COCl, $COOAlk^1$ oder einen Acyloxycarbonylrest, wie z.B.

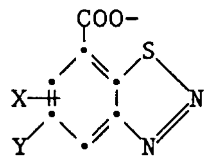

,

Benzoyloxycarbonyl oder Acetyloxycarbonyl darstellt, worin $Alk^1$ $C_1$-$C_4$-Alkyl bedeutet, in Gegenwart einer Base mit Hydrazin-Derivaten der Formeln V oder VI

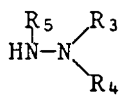 (V) oder 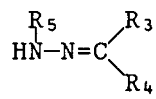 (VI)

in einem inerten Lösungsmittel bei Temperaturen von -10° bis 180°C, bevorzugt 0° bis 100°C; oder

3.2 Verbindungen der Formel Ie schrittweise zuerst mit Hydrazin umsetzt und dann die erhaltenen Hydrazinverbindungen

3.2.1 mit dem Alkylierungsmittel $R_3$-L oder $R_4$-L, worin L eine Abgangsgruppe darstellt, in einem inerten Lösungsmittel bei Temperaturen von 0° bis 160°C, bevorzugt 20° bis 120°C; oder

3.2.2 mit einem Aldehyd oder Keton der Formel $R_3(R_4)C=0$, worin $R_3$ und $R_4$ die unter Formel I angegebenen Bedeutungen haben, mit oder ohne Zusatz einer organischen oder anorganischen Säure bei Temperaturen von -10° bis 150°C, vorzugsweise bei 20° bis 100°C, und anschliessend gewünschtenfalls

3.2.3 mit einem Alkylierungsmittel L-$R_5$, worin L eine Abgangsgruppe darstellt, in Gegenwart einer starken Base in einem inerten Lösungsmittel bei Temperaturen von -80° bis 120°C, vorzugsweise -40° bis 80°C; oder gewünschtenfalls

3.2.4 die unter (3.2.1) hergestellten Hydrazonderivate

a) mit Wasserstoff unter einem Druck von 1 bis $30 \cdot 10^5$ Pa in Gegenwart eines Katalysators im Gemisch mit Aktivkohle in einem inerten Lösungsmittel bei Temperaturen von 0° bis 100°C hydriert oder

b) mit einem komplexen Metallhydrid, wie z.B. Na-Cyanoborhydrid, in einem inerten Lösungsmittel bei Temperaturen von -10° bis 80°, vorzugsweise 0° bis 50°C, behandelt; und

4. Verbindungen der Formel If

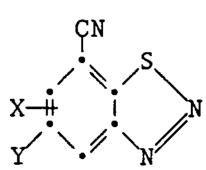

(If)

in welcher die Symbole X und Y die unter Formel I angegebenen Bedeutungen besitzen, hergestellt werden, indem man

Verbindungen der Formel Ig [hergestellt nach Verfahren (2)]

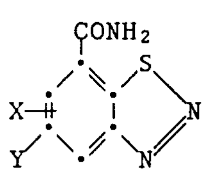

(Ig)

mit einem Dehydratisierungsagens in einem inerten Lösungsmittel oder ohne Lösungsmittel bei Temperaturen von -10° bis 250°C behandelt, wobei als Dehydratisierungsmittel verwendet werden:

a) Trifluoressigsäureanhydrid in Gegenwart einer Base, wie z.B. Pyridin, in einem inerten Lösungsmittel, wie z.B. Tetrahydrofuran oder Dioxan, bei Temperaturen von -10° bis 40°C; oder
b) Chlorsulfonylisocyanat in einem inerten Lösungsmittel, wie z.B. Tetrahydrofuran, bei Temperaturen von 0° bis 65°C und anschliessender Behandlung mit Dimethylformamid;
c) Phosphorpentoxid mit oder ohne inertem Lösungsmittel, wie z.B. 1,2-Dichlorethan, Xylol oder Chlorbenzol, gegebenenfalls im Bombenrohr unter erhöhtem Druck, bei 50° bis 250°C;

5.1 Verbindungen der Formel $IL^1$

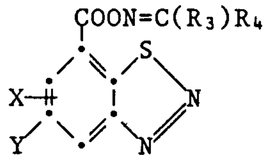

$(IL^1)$,

in welcher $R_3$, $R_4$, X und Y die unter Formel I angegebenen Bedeutungen besitzen, hergestellt werden, indem man Oximderivate der Formel

$HO\text{-}N=C(R_3)R_4$

mit aktivierten Säurederivaten der Formel

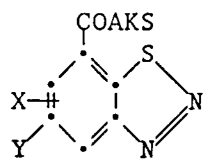

,

worin AKS ein Halogen, ein O-Acyl, wie z.B. der O-Acylrest der freien Säure vorstehender Formel, Acetoxy oder Benzoyloxy, oder 1-Imidazolyl darstellt, in einem inerten Lösungsmittel und einer Base bei -20°C bis 120°C, bevorzugt bei 0° bis 50°C, oder die freie Säure (= Ib) in Gegenwart von Dicyclohexylcarbodiimid unter den gleichen Bedingungen umsetzt;

5.2 Verbindungen der Formel $IL^2$

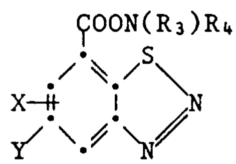

$(IL^2)$

in welcher $R_3$, $R_4$, X und Y die unter Formel I angegebenen Bedeutungen besitzen, hergestellt werden durch Reduktion von Verbindungen der Formel $IL^1$

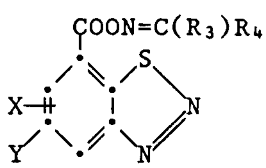

$(IL^1)$

a) mit einem Silan, wie z.B. Triethylsilan, in Gegenwart einer Säure, wie z.B. Trifluoressigsäure,

bei 0° bis 80°C, oder
b) mit Natriumcyanoborhydrid in Gegenwart einer organischen Säure, wie z.B. Essigsäure, bei 0° bis 80°C, oder
c) auf katalytischen Wege, z.B. mit $Pt/H_2$.

**28.** Verfahren zur Herstellung von Verbindungen der Formel Ik

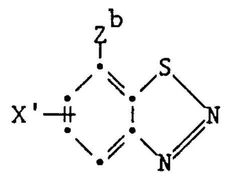

(Ik)

indem man eine Verbindung der Formel XI'

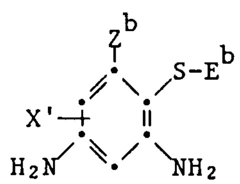

(XI')

in saurem Medium mit einer Nitritverbindung bei -40° bis 30°C diazotiert und im gleichen oder zweiten Reaktionsgefäss mit einem Reduktionsmittel bei -40° bis 80°C, vorzugsweise bei -30° bis 30°C, behandelt, wobei das Reduktionsmittel vor, nach oder gleichzeitig mit der Nitritverbindung hinzugefügt wird, wobei die Substituenten der Verbindungen folgende Bedeutungen haben:
X' Wasserstoff, Halogen, Methyl, Methoxy oder COOH;
$E^b$ eine leicht abspaltbare Gruppe wie z.B. Wasserstoff, $C_1$-$C_{16}$-Alkyl, z.B. Methyl, Ethyl, Isopropyl, n-Dodecyl, oder Benzyl oder Acyl, z.B. Acetyl oder den Sulfonsäure-Rest ($-SO_3H-$) oder Cyano oder darüber hinaus als Teil einer Disulfid-Brücke einen zweiten Rest

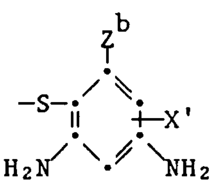

;

$Z^b$ Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-COO$C_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

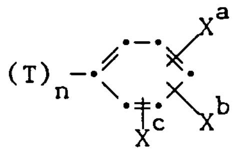, $(T)_n$-Napht, $(T)_n$-Si($C_1$-$C_8$-Alkyl)$_3$,

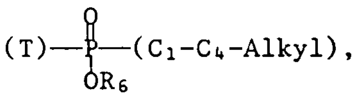, 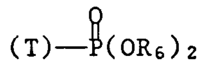 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder

$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder

$X^a$ Phenyl, Phenyloxy oder Benzyloxy und $X^b$ Halogen oder Methyl und $X^c$ Wasserstoff; oder

$X^a$, $X^b$ und $X^c$ zusammen 4 oder 5 Fluoratome;

Napht einen unsubstituierten oder mit Halogen, Methyl, Methoxy oder Nitro substituierten Naphthylrest;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;

T die Brückenglieder $-CH_2-$, $-CH_2CH_2-$, $-CH(CH_3)-$, $-CH_2CH_2CH_2-$ oder $-CH_2CH_2O-$;

$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;

$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;

$R_1$ und $R_2$ zusammen mit dem Atom, an welches sie gebunden sind, einen Heterocyclus W;

$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;

$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;

$R_3$ und $R_4$ zusammen mit dem Atom, an welches sie gebunden sind, einen Heterocyclus W oder einen carbocyclischen Ring W';

W' einen carbocyclischen Rest mit 3- bis 7 Ringkohlenstoffatomen;

$R_5$ Wasserstoff oder Methyl;

$R_6$ Wasserstoff oder $C_1$-$C_4$-Alkyl; und

n Null oder 1;

mit Ausnahme von primären oder sekundären Aminogruppen, UH- oder Nitro-Gruppen, Si($C_1$-$C_8$-Alkyl)$_3$ oder Phosphor-haltigen Resten.

**29.** Verfahren zur Herstellung von Verbindungen der Formel

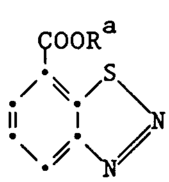

indem man eine Verbindung der Formel

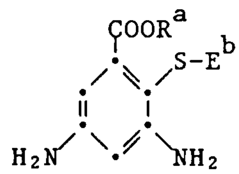

in saurem Medium mit einer Nitritverbindung bei -20° bis 30° C diazotiert und im gleichen Reaktionsgefäss mit einem Reduktionsmittel bei -20° bis 80° C, vorzugsweise bei 20° bis 30° C, behandelt, wobei das Reduktionsmittel vor, nach oder gleichzeitig mit der Nitritverbindung hinzugefügt wird, wobei die Substituenten der Verbindungen folgende Bedeutungen haben:

$R^a$ Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-COO$C_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

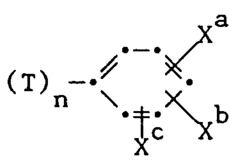 , $(T)_n$-Napht, $(T)_n$-Si($C_1$-$C_8$-Alkyl$)_3$,

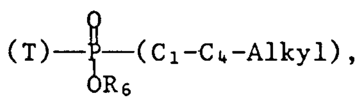, 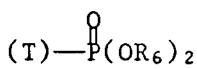 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder

$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder

$X^a$ Phenyl, Phenyloxy oder Benzyloxy und $X^b$ Halogen oder Methyl und $X^c$ Wasserstoff; oder

$X^a$, $X^b$ und $X^c$ zusammen 4 oder 5 Fluoratome;

Napht einen unsubstituierten oder mit Halogen, Methyl, Methoxy oder Nitro substituierten Naphthylrest;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;

T die Brückenglieder $-CH_2-$, $-CH_2CH_2-$, $-CH(CH_3)-$, $-CH_2CH_2CH_2-$ oder $-CH_2CH_2O-$;

$R_1$ Wasserstoff- $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;

$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;

$R_1$ und $R_2$ zusammen mit dem Atom, an welches sie gebunden sind, einen Heterocyclus W;

$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;

$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy

substituiertes $C_3$-$C_5$-Alkenoyl;
$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme von Resten mit UH- oder Nitro-Gruppen sowie Silizium- oder Phosphor-haltigen Resten, und
$E^b$ eine leicht abspaltbare Gruppe wie z.B. Wasserstoff, $C_1$-$C_{16}$-Alkyl, z.B. Methyl, Ethyl, Isopropyl, n-Dodecyl, oder Benzyl oder Acyl, z.B. Acetyl oder den Sulfonsäure-Rest (-$SO_3$H-) oder Cyano.

**30.** Verwendung der Verbindungen der Formel I gemäss Anspruch 1 oder 2

**31.** Verwendung der Verbindungen gemäß Formel I' gemäss Anspruch 8 oder 9 zur Immunisierung von Pflanzen gegen Krankheiten.

**32.** Verwendung von 7-Methoxycarbonyl-benzo-1,2,3-thiadiazol als Wirkstoff zur Herstellung eines Mittels zur Immunisierung von Pflanzen gegen den Befall durch phytopathogene Mikroorganismen.

**Patentansprüche für folgenden Vertragsstaat : ES**

**1.** Verfahren zur Immunisierung von Pflanzen gegen den Befall durch phytopathogene Mikroorganismen, dadurch gekennzeichnet, dass auf Pflanzen und/oder deren Umgebung als Wirkstoff eine Verbindung der nachfolgenden Formel I appliziert wird:

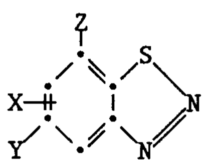

(I)

worin bedeuten:
X Wasserstoff, Halogen, Hydroxy, Methyl, Methoxy, HOOC oder MOOC;
Y Wasserstoff, Halogen, $SO_3$H, $SO_3$M, Nitro, Hydroxy, oder Amino, wobei M das Moläquivalent eines Alkali- oder Erdalkali-Ions ist, das aus einer entsprechenden Base oder basischen Verbindung gebildet wird; und
Z Cyano oder -CO-A, wobei A entweder OH oder SH bedeutet, deren H auch durch das Moläquivalent eines anorganischen oder organischen kationischen Restes ersetzt sein kann.

**2.** Verfahren zur Immunisierung von Pflanzen gegen den Befall durch phytopathogene Mikroorganismus, dadurch gekennzeichnet, dass auf Pflanzen und/oder deren Umgebung als Wirkstoffe eine Verbindung der nachfolgenden Formel I appliziert werden:

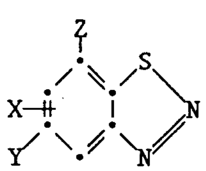

(I)

worin bedeuten:
X Wasserstoff, Halogen, Hydroxy, Methyl, Methoxy, HOOC oder MOOC;
Y Wasserstoff, Halogen, $SO_3$H, $SO_3$M, Nitro, Hydroxy, oder Amino, wobei M das Moläquivalent eines Alkali- oder Erdalkali-Ions ist, das aus einer entsprechenden Base oder basischen Verbindung gebildet

wird; und
Z Cyano oder -CO-A,
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, U-$C_1$-$C_3$-Alkyl oder $C_2$-$C_4$-Dialkylamino substituiertes oder durch die CO-Gruppe unterbrochenes $C_1$-$C_8$-Alkyl, (T)-COOH oder (T)-$COOC_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

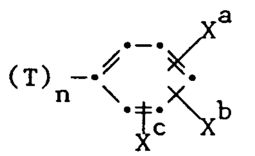 , $(T)_n$-Napht, $(T)_n-Si(C_1-C_8\text{-Alkyl})_3$,

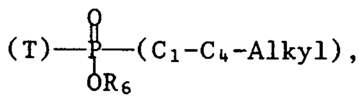, 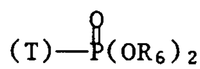 ; oder $(T)_n-W$ ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder
$X^a$ $C_1$-$C_2$-Halogenalkoxy mit bis zu 5 Halogenatomen, Nitro, Dimethylamino, Phenyl, Phenyloxy, Benzyloxy, Sulfamoyl und $X^b$ und $X^c$ gleichzeitig Wasserstoff; oder
$X^a$ Phenyl, Phenyloxy oder Benzyloxy und
$X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;
W einen 5- bis 7-gliedrigen gesättigten oder ungesättigten unsubstituierten oder durch Halogen, Trifluormethyl, Cyano, $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$, $-CH(CH_3)-$, $-CCH_3(CH_3)-$, $-CH_2CH_2CH_2-$ oder $-CH_2CH_2O-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, Methoxy, $CF_3$, Cyano, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und

n Null oder 1;
wobei in den Verbindungen der Formel I der organische Rest A ein Molgewicht von weniger als 900 besitzt; und umfassen im Falle von U als Sauerstoff oder Schwefel die Salze der pflanzenphysiologisch verträglichen 7-Carbonsäure mit primären, sekundären oder tertiären Aminen oder mit anorganischen Basen.

**3.** Verfahren gemäss Anspruch 1 oder 2, dadurch gekennzeichnet, dass die Aufwandmenge weniger als 1 kg AS/ha beträgt.

**4.** Verfahren gemäss Anspruch 2, dadurch gekennzeichnet, dass Verbindungen der Formel I verwendet werden, in welcher bedeuten:
X und Y für Wasserstoff;
Z für Cyano oder COA;
A für UR oder $U^1N(=C)_n(R_3)R_4$;
U für Sauerstoff;
$U^1$ für Sauerstoff oder $-N(R_5)-$;
R für Wasserstoff, $C_1$-$C_8$-Alkyl, durch Halogen oder $C_1$-$C_3$-Alkoxy substituiertes $C_1$-$C_8$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, Benzyl, halogeniertes Benzyl, Benzylmethoxy, $(T)_n$-$Si(CH_3)_3$, $(T)$-$P(O)(C_1$-$C_4$-Alkyl)$CH_3$, $(T)$-$P(O)(OC_1$-$C_4$-Alkyl$)_2$ oder die Gruppe $(T)_n$-W;
W für einen 5- bis 7-gliedrigen gesättigten oder ungesättigten unsubstituierten Heterocyclus mit 1 bis 3 Heteroatomen aus der Gruppe O, N und S sowie für Diaceton-D-glucosidyl;
T für die Brückenglieder $-CH_2-$, $-CH_2CH_2-$, $-CH(CH_3)-$, $-CCH_3(CH_3)-$ oder $-CH_2CH_2CH_2-$;
$R_3$ für Wasserstoff, Cyano, $C_1$-$C_6$-Alkyl, Phenyl oder W;
$R_4$ für Wasserstoff, $C_1$-$C_6$-Alkyl, $CONH_2$, CONH-CONH-$C_1$-$C_3$-Alkyl, $C_1$-$C_3$-Alkanoyl oder $C_3$-$C_5$-Alkenoyl;
$R_3$ und $R_4$ zusammen mit dem Atom, an welches sie gebunden sind, W oder W';
W für einen 5- bis 7-gliedrigen gesättigten oder ungesättigten Heterocyclus mit 1 bis 3 Heteroatomen aus der Gruppe O, N und S;
W' für einen Rest aus der Gruppe

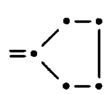 , 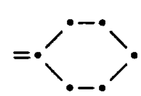 und 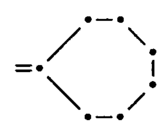 ;

n für Null oder 1.

**5.** Verfahren gemäss Anspruch 2, dadurch gekennzeichnet, dass Verbindungen der Formel I'

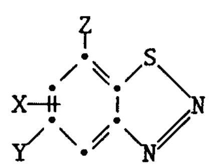 (I')

in welcher bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, oder U-$C_1$-$C_3$-Alkyl substituiertes

$C_1$-$C_8$-Alkyl, (T)-COOH oder (T)-COO$C_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

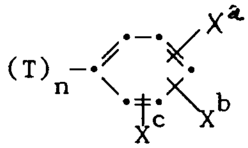 , $(T)_n$-Napht, $(T)_n$-Si($C_1$-$C_8$-Alkyl$)_3$,

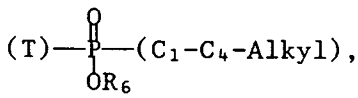 , 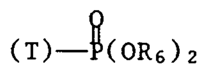 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder

$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder

$X^a$ Phenyl, Phenyloxy oder Benzyloxy und $X^b$ Halogen oder Methyl und $X^c$ Wasserstoff; oder

$X^a$, $X^b$ und $X^c$ zusammen 4 oder 5 Fluoratome;

Napht einen unsubstituierten oder mit Halogen, Methyl, Methoxy oder Nitro substituierten Naphthylrest;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;

T die Brückenglieder $-CH_2-$, $-CH_2CH_2-$, $-CH(CH_3)-$, $-CH_2CH_2CH_2-$ oder $-CH_2CH_2O-$;

$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;

$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;

$R_1$ und $R_2$ zusammen mit dem Atom, an welches sie gebunden sind, einen Heterocyclus W;

$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;

$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;

$R_3$ und $R_4$ zusammen mit dem Atom, an welches sie gebunden sind, einen Heterocyclus W oder einen carbocyclischen Ring W';

W' einen carbocyclischen Rest mit 3- bis 7 Ringkohlenstoffatomen;

$R_5$ Wasserstoff oder Methyl;

$R_6$ Wasserstoff oder $C_1$-$C_4$-Alkyl; und

n Null oder 1;

mit Ausnahme der Verbindungen:

7-Cyano-benzo-1,2,3-thiadiazol;

4-Chlor-7-cyano-benzo-1,2,3-thiadiazol;

4,6-Dibrom-7-cyano-benzo-1,2,3-thiadiazol;

Benzo-1,2,3-thiadiazol-7-carbonsäure;

Benzo-1,2,3-thiadiazol-7-carbonsäuremethylester;

6-Chlor-7-cyano-benzo-1,2,3-thiadiazol;

6-Chlor-benzo-1,2,3-thiadiazol-7-carbonsäure verwendet werden.

**6.** Verfahren gemäss Anspruch 2, dadurch gekennzeichnet, dass Verbindungen der Formel I'

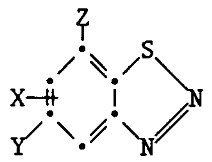

(I')

in welcher bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, Alkoxy oder U-$C_1$-$C_3$-Alkyl substituiertes $C_1$-$C_8$-Alkyl, (T)-COOH oder (T)-$COOC_1$-$C_4$-Alkyl, $C_2$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

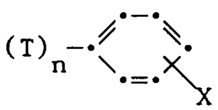, $(T)_n$-Naphthyl, $(T)_n$-Si($C_1$-$C_8$-Alkyl$)_3$,

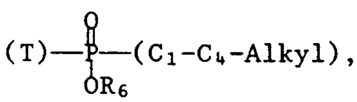, 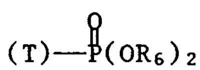 ; oder $(T)_n$—W ;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$ oder $-CH(CH_3)-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;

$R_6$ Wasserstoff oder $C_1$-$C_4$-Alkyl; und
n Null oder 1;
mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure verwendet werden.

**7.** Verfahren gemäss Anspruch 2, dadurch gekennzeichnet, dass Verbindungen der Formel I' gemäss Anspruch 5 verwendet werden, bei denen Z = COA bedeutet und R eine andere Bedeutung hat als Wasserstoff und unsubstituiertes Alkyl.

**8.** Verfahren gemäss Anspruch 2, dadurch gekennzeichnet, dass als Wirkstoff 1,2,3-Benzothiadiazol-7-carbonsäure oder ein Salz davon mit einer basischen Verbindung verwendet wird.

**9.** Verfahren gemäss Anspruch 8, dadurch gekennzeichnet, dass als Salze solche mit primären, sekundären oder tertiären Aminen oder Alkali- oder Erdalkaliverbindungen verwendet werden.

**10.** Verfahren gemäss Anspruch 2, dadurch gekennzeichnet, dass als Wirkstoff 7-Methoxycarbonyl-benzo-1,2,3-thiadiazol verwendet wird.

**11.** Verfahren gemäss Anspruch 2, dadurch gekennzeichnet, dass als Wirkstoff 7-Benzyloxycarbonyl-benzo-1,2,3-thiadiazol verwendet wird.

**12.** Verfahren gemäss Anspruch 2, dadurch gekennzeichnet, dass als Wirkstoff 7-Cyano-benzo-1,2,3-thiadiazol verwendet wird.

**13.** Verfahren gemäss Anspruch 2, dadurch gekennzeichnet, dass als Wirkstoffe Verbindungen von 7-$C_2$-$C_4$-Alkoxycarbonyl-benzo-1,2,3-thiadiazol verwendet werden.

**14.** Mittel zur Immunisierung von Pflanzen gegen den Befall durch phytopathogene Mikroorganismen enthaltend als Wirkstoff mindestens eine Verbindung der Formel I gemäss Anspruch 1 oder 2 mit Ausnahme der Verbindungen 7-Cyano-benzo-1,2,3-thiadiazol; 4-Chlor-7-cyano-benzo-1,2,3-thiadiazol; 6-Chlor-7-cyano-benzo-1,2,3-thiadiazol, 4,6-Dibrom-7-cyano-benzo-1,2,3-thiadiazol und 7-Methoxycarbonyl-benzo-1,2,3-thiadiazol.

**15.** Benzo-1,2,3-thiadiazol-7-carbonsäuremethylester zur Verwendung in einem Verfahren zur Immunisierung von Pflanzen gegen den Befall durch phytopathogene Mikroorganismen.

**16.** Benzo-1,2,3-thiadiazol-7-carbonsäuremethylester zur Verwendung für die Herstellung eines Mittels zur Immunisierung von Pflanzen gegen den Befall durchh phytopathogene Mikroorganismen.

**17.** Mittel zur Immunisierung von Pflanzen gegen den Befall durch phytopathogene Mikroorganismen enthaltend als Wirkstoff mindestens eine Verbindung der Formel I'

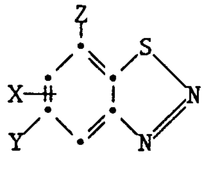

(I')

in welcher bedeuten:
X Wasserstoff, Halogen, Hydroxy, Methyl, Methoxy, HOOC oder MOOC;

Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-$COOC_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

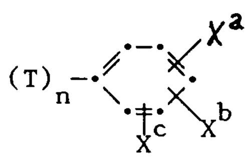 , $(T)_n$-Napht, $(T)_n$-Si($C_1$-$C_8$-Alkyl$)_3$,

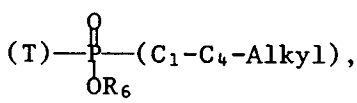 , 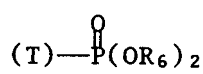 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder
$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder
$X^a$ Phenyl, Phenyloxy oder Benzyloxy und $X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;
W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$, $-CH(CH_3)-$, $-CH_2CH_2CH_2-$ oder $-CH_2CH_2O-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;

$R_5$ Wasserstoff oder Methyl;
$R_6$ Wasserstoff oder $C_1$-$C_4$-Alkyl; und
n Null oder 1;
mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure.

**18.** Mittel zur Immunisierung von Pflanzen gegen den Befall durch phytopathogene Mikroorganismen enthaltend als Wirkstoff mindestens eine Verbindung der Formel I'

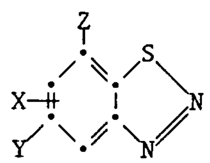

(I')

in welcher bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;
A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, Alkoxy oder U-$C_1$-$C_3$-Alkyl substituiertes $C_1$-$C_8$-Alkyl, (T)-COOH oder (T)-COO$C_1$-$C_4$-Alkyl, $C_2$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

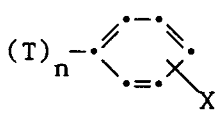 , $(T)_n$-Naphthyl, $(T)_n$-Si($C_1$-$C_8$-Alkyl$)_3$,

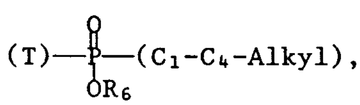 , $(T)-P(=O)(OR_6)_2$ ; oder $(T)_n$—W ;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$ oder $-CH(CH_3)-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;

$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme der Verbindungen:
7-Cyano-benzo-1,2,3-thiadiazol;
4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure.

**19.** Mittel gemäss Anspruch 14, dadurch gekennzeichnet, dass es als Wirkstoff mindestens eine Verbindung der Formel I' gemäss Anspruch 17 enthält, bei denen Z = COA bedeutet und R eine andere Bedeutung hat als Wasserstoff und unsubstituiertes Alkyl.

**20.** Mittel gemäss Anspruch 14, dadurch gekennzeichnet, dass es als Wirkstoff mindestens eine Verbindung der Formel I' aus der Gruppe:
7-n-Pentoxycarbonyl-benzo-1,2,3-thiadiazol;
7-(4-Methoxy-benzyloxycarbonyl)-benzo-1,2,3-thiadiazol;
7-(Cycloheximino-oxycarbonyl)-benzo-1,2,3-thiadiazol;
7-(3-Hydroxy-n-propoxycarbonyl)-benzo-1,2,3-thiadiazol;
1,2,5,6-Di-O-isopropyliden-3-(7-benzo-1,2,3-thiadiazoyl)-D-glucofuranose;
7-Furfuryloxycarbonyl-benzo-1,2,3-thiadiazol;
7-(1,2,4-Triazol-1-yl)-methoxycarbonyl-benzo-1,2,3-thiadiazol;
7-(2-Pyridylmethoxycarbonyl)-benzo-1,2,3-thiadiazol;
7-Trimethylsilyl-methoxycarbonyl-benzo-1,2,3-thiadiazol;
7-[2-(Trimethylsilyl)-ethoxycarbonyl]-benzo-1,2,3-thiadiazol;
7-Dimethylphosphono-ethoxycarbonyl-benzo-1,2,3-thiadiazol;
7-Cyclohexyloxycarbonyl-benzo-1,2,3-thiadiazol;
7-(1-Phenethyloxycarbonyl)-benzo-1,2,3-thiadiazol;
7-(3-Methoxybenzyl)-benzo-1,2,3-thiadiazol;
7-(Ethylthio-carbonyl)-benzo-1,2,3-thiadiazol;
7-(n-Propylthio-carbonyl)-benzo-1,2,3-thiadiazol;
7-(Benzylthio-carbonyl)-benzo-1,2,3-thiadiazol;
7-Carbamoyl-benzo-1,2,3-thiadiazol;
7-N-Phenylcarbamoyl-benzo-1,2,3-thiadiazol;
N-(7-Benzo-1,2,3-thiadiazoyl)-glycin;
7-(N-Diallylcarbamoyl)-benzo-1,2,3-thiadiazol;
6-Fluor-7-methoxycarbonyl-benzo-1,2,3-thiadiazol;
6-Fluor-7-carboxy-benzo-1,2,3-thiadiazol;
5-Fluor-7-benzyloxycarbonyl-benzo-1,2,3-thiadiazol;
5-Fluor-7-carboxy-benzo-1,2,3-thiadiazol;
5-Fluor-7-ethoxycarbonyl-benzo-1,2,3-thiadiazol; oder aus der Gruppe:
7-Ethoxycarbonyl-benzo-1,2,3-thiadiazol;
7-n-Propoxycarbonyl-benzo-1,2,3-thiadiazol;

7-iso-Propoxycarbonyl-benzo-1,2,3-thiadiazol;
7-n-Butoxycarbonyl-benzo-1,2,3-thiadiazol;
7-sek.Butoxycarbonyl-benzo-1,2,3-thiadiazol;
7-tert.Butoxycarbonyl-benzo-1,2,3-thiadiazol;
7-Cyclopropylmethoxycarbonyl-benzo-1,2,3-thiadiazol;
7-(2'-Phenethoxycarbonyl)-benzo-1,2,3-thiadiazol;
7-Benzyloxycarbonyl-benzo-1,2,3-thiadiazol;
7-Allyloxycarbonyl-benzo-1,2,3-thiadiazol;
7-Propin-2-yloxycarbonyl-benzo-1,2,3-thiadiazol;
N-Ethylaminocarbonyl-2-cyano-2-oximinocarbonyl-benzo-1,2,3-thiadiazol-7-yl-acetamid;
Na-Salz der Benzo-1,2,3-thiadiazol-7-carbonsäure;
K-Salz der Benzo-1,2,3-thiadiazol-7-carbonsäure;
Triethylammoniumsalz der Benzo-1,2,3-thiadiazol-7-carbonsäure;
7-(1'-Phenethoxycarbonyl)-benzo-1,2,3-thiadiazol;
7-(1'-Naphthylmethoxycarbonyl)-benzo-1,2,3-thiadiazol;
7-(Methylthio-carbonyl)-benzo-1,2,3-thiadiazol;
7-(Ethylthio-carbonyl)-benzo-1,2,3-thiadiazol;
7-(Benzylthio-carbonyl)-benzo-1,2,3-thiadiazol;
7-[(Di-cyanomethyl)-amino-carbonyl]-benzo-1,2,3-thiadiazol;
3-[(N-Benzo-1,2,3-thiadiazol-7-ylcarbonyl)-1,3,4-thiadiazol-2-ylamino]-2-methyl-propencarbonsäuremethylester;
3-[(N-Benzo-1,2,3-thiadiazol-7-ylcarbonyl)-1,3,4-thiadiazol-2-ylamino]-2-ethyl-propencarbonsäuremethylester;
1-(Benzo-1,2,3-thiadiazol-7-carbonyl)-2-($\alpha$-methylpropyliden)-hydrazin;
1-(Benzo-1,2,3-thiadiazol-7-carbonyl)-2-(cyclobutyliden)-hydrazin;
1-(Benzo-1,2,3-thiadiazol-7-carbonyl)-2-(cyclopentyliden)-hydrazin;
1-(Benzo-1,2,3-thiadiazol-7-carbonyl)-2-(cyclohexyliden)-hydrazin;
2-(Benzo-1,2,3-thiadiazol-7-carbonyl)-1-(2'-sec-butyl)-hydrazin;
1-(Benzo-1,2,3-thiadiazol-7-carbonyl)-2-(cyclopentyl)-hydrazin;
1-(Benzo-1,2,3-thiadiazol-7-carbonyl)-2-(cyclohexyl)-hydrazin;
1-(Benzo-1,2,3-thiadiazol-7-carbonyl)-2-(cycloheptyl)-hydrazin;
1-(Benzo-1,2,3-thiadiazol-7-carbonyl)-1,2-diacetyl-hydrazin;
1-(Benzo-1,2,3-thiadiazol-7-carbonyl)-2-phenyl-hydrazin;
1-(Benzo-1,2,3-thiadiazol-7-carbonyl)-2-pyridin-2'-yl-hydrazin enthält.

**21.** Mittel gemäss Anspruch 14, dadurch gekennzeichnet, dass es als Wirkstoff mindestens eine der Verbindungen Benzo-1,2,3-thiadiazol-7-carbonsäure oder 7-Methoxycarbonyl-benzo-1,2,3-thiadiazol enthält.

**22.** Mittel gemäss Anspruch 14, dadurch gekennzeichnet, dass es als Wirkstoff 1,2,3-Benzothiadiazol-7-carbonsäure oder ein Salz davon mit einer basischen Verbindung enthält.

**23.** Mittel gemäss Anspruch 22, dadurch gekennzeichnet, dass als Salze solche mit primären, sekundären oder tertiären Aminen oder Alkali- oder Erdalkaliverbindungen verwendet werden.

**24.** Verfahren zur Herstellung von Verbindungen der Formel I'

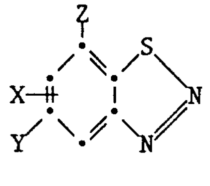

(I')

in welcher bedeuten:
X Wasserstoff, Halogen, Hydroxy, Methyl, Methoxy, HOOC oder MOOC;
Y Wasserstoff, Halogen, $SO_3H$, $SO_3M$, Nitro, Hydroxy oder Amino;
Z Cyano oder COA;

A UR, $N(R_1)R_2$ oder $U^1N(=C)_n(R_3)R_4$;
M Moläquivalent eines Alkali- oder Erdalkali-Ions, das aus einer entsprechenden Base oder basischen Verbindung gebildet ist;
U Sauerstoff oder Schwefel;
$U^1$ Sauerstoff oder $-N(R_5)-$;
R Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-$COOC_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

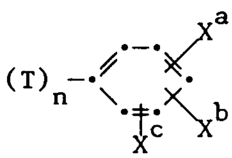, $(T)_n$-Napht, $(T)_n$-Si($C_1$-$C_8$-Alkyl$)_3$,

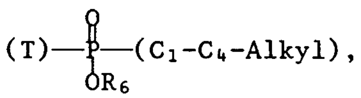, 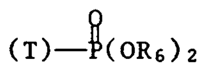 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder
$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder
$X^a$ Phenyl, Phenyloxy oder Benzyloxy und $X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;
W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$, $-CH(CH_3)-$, $-CH_2CH_2CH_2-$ oder $-CH_2CH_2O-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme der Verbindungen:
7-Cyano-benzo-1,2,3-thiadiazol;

4-Chlor-7-cyano-benzo-1,2,3-thiadiazol;
4,6-Dibrom-7-cyano-benzo-1,2,3-thiadiazol;
Benzo-1,2,3-thiadiazol-7-carbonsäure;
Benzo-1,2,3-thiadiazol-7-carbonsäuremethylester;
6-Chlor-7-cyano-benzo-1,2,3-thiadiazol;
6-Chlor-benzo-1,2,3-thiadiazol-7-carbonsäure;
dadurch gekennzeichnet, dass

1. Verbindungen der Formel Ia

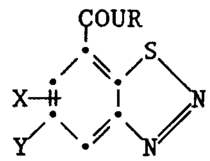

(Ia)

in welcher R, X, Y und U die unter Formel I angegebenen Bedeutungen besitzen, hergestellt werden, indem man umsetzt:

1.1 Verbindungen der Formel II

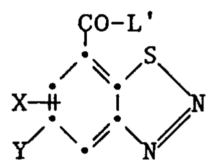

(II)

worin L' eine Abgangsgruppe, z.B. Halogen, O-Acyl, wie z.B. der zum symmetrischen Anhydrid der Säure Ib gehörende Acylrest, oder 1-Imidazoyl bedeutet,
mit Verbindungen der Formel III

RUH (III)

a) im Ueberschuss des Reaktanden RUH oder
b) in Gegenwart einer organischen Base entweder mit oder ohne 4-Dialkylaminopyridin als Katalysator in inerten Lösungsmitteln oder
c) in Gegenwart anorganischer Basen, wobei jeweils im Temperaturbereich von -10° bis 180°C, vorzugsweise 0° bis 100°C, gearbeitet wird; und

1.2 Verbindungen der Formel Ib

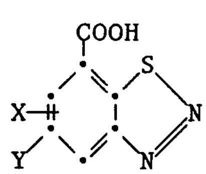

(Ib)

mit Verbindungen der Formel III im Ueberschuss oder in einem inerten Lösungsmittel in Gegenwart einer Säure, wie Schwefelsäure, Salzsäure, p-Toluolsulfonsäure oder Bortrifluorid-Diethylether-Komplex, oder von Dicyclohexylcarbodiimid bei Temperaturen von -10° bis 180°C, vorzugsweise 0° bis 140°C; und

2. Verbindungen der Formel Ic

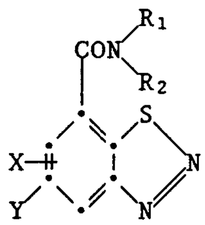

(Ic)

in welcher die Symbole $R_1$, $R_2$, X und Y die unter Formel I angegebenen Bedeutungen besitzen, hergestellt werden, indem man umsetzt:

2.1 Verbindungen der Formel II mit Verbindungen der Formel IV

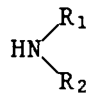

(IV)

a) im Ueberschuss des Reaktanden $HN(R_1)R_2$ oder

b) in Gegenwart einer organischen Base entweder mit oder ohne 4-Dialkylaminopyridin als Katalysator in inerten Lösungsmitteln oder

c) in Gegenwart anorganischer Basen, wobei jeweils im Temperaturbereich von -10° bis 160°C, vorzugsweise 0° bis 100°C, gearbeitet wird; und

3. Verbindungen der Formel Id

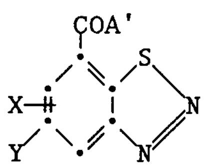

(Id)

worin A' den Rest $U^1N(=C)_n(R_3)R_4$ darstellt und X, Y, $R_3$, $R_4$, $R_5$ und n die unter Formel I angegebenen Bedeutungen besitzen, hergestellt werden, indem man

3.1 Verbindungen der Formel Ie

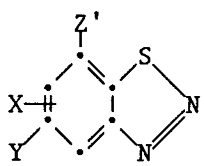

(Ie)

in welcher Z' die Gruppe COOH, COCl, $COOAlk^1$ oder einen Acyloxycarbonylrest, wie z.B.

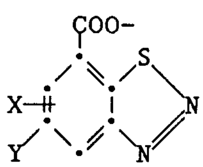

,

Benzoyloxycarbonyl oder Acetyloxycarbonyl darstellt, worin $Alk^1$ $C_1$-$C_4$-Alkyl bedeutet, in Gegenwart einer Base mit Hydrazin-Derivaten der Formeln V oder VI

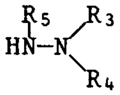 (V) oder 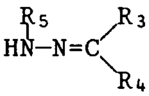 (VI)

in einem inerten Lösungsmittel bei Temperaturen von -10° bis 180°C, bevorzugt 0° bis 100°C; oder

3.2 Verbindungen der Formel Ie schrittweise zuerst mit Hydrazin umsetzt und dann die erhaltenen Hydrazinverbindungen

3.2.1 mit dem Alkylierungsmittel $R_3$-L oder $R_4$-L, worin L eine Abgangsgruppe darstellt, in einem inerten Lösungsmittel bei Temperaturen von 0° bis 160°C, bevorzugt 20° bis 120°C; oder

3.2.2 mit einem Aldehyd oder Keton der Formel $R_3(R_4)C=O$, worin $R_3$ und $R_4$ die unter Formel I angegebenen Bedeutungen haben, mit oder ohne Zusatz einer organischen oder anorganischen Säure bei Temperaturen von -10° bis 150°C, vorzugsweise bei 20° bis 100°C, und anschliessend gewünschtenfalls

3.2.3 mit einem Alkylierungsmittel L-$R_5$, worin L eine Abgangsgruppe darstellt, in Gegenwart einer starken Base in einem inerten Lösungsmittel bei Temperaturen von -80° bis 120°C, vorzugsweise -40° bis 80°C; oder gewünschtenfalls

3.2.4 die unter (3.2.1) hergestellten Hydrazonderivate

a) mit Wasserstoff unter einem Druck von 1 bis $30 \cdot 10^5$ Pa in Gegenwart eines Katalysators im Gemisch mit Aktivkohle in einem inerten Lösungsmittel bei Temperaturen von 0° bis 100°C hydriert oder

b) mit einem komplexen Metallhydrid, wie z.B. Na-Cyanoborhydrid, in einem inerten Lösungsmittel bei Temperaturen von -10° bis 80°, vorzugsweise 0° bis 50°C, behandelt; und

4. Verbindungen der Formel If

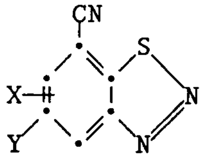 (If)

in welcher die Symbole X und Y die unter Formel I angegebenen Bedeutungen besitzen, hergestellt werden, indem man

Verbindungen der Formel Ig [hergestellt nach Verfahren (2)]

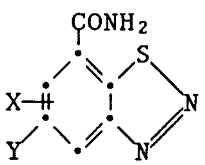 (Ig)

mit einem Dehydratisierungsagens in einem inerten Lösungsmittel oder ohne Lösungsmittel bei Temperaturen von -10° bis 250°C behandelt, wobei als Dehydratisierungsmittel verwendet werden:

a) Trifluoressigsäureanhydrid in Gegenwart einer Base, wie z.B. Pyridin, in einem inerten Lösungsmittel, wie z.B. Tetrahydrofuran oder Dioxan, bei Temperaturen von -10° bis 40°C; oder

b) Chlorsulfonylisocyanat in einem inerten Lösungsmittel, wie z.B. Tetrahydrofuran, bei Temperaturen von 0° bis 65°C und anschliessender Behandlung mit Dimethylformamid;

c) Phosphorpentoxid mit oder ohne inertem Lösungsmittel, wie z.B. 1,2-Dichlorethan, Xylol oder Chlorbenzol, gegebenenfalls im Bombenrohr unter erhöhtem Druck, bei 50° bis 250°C;

5.1 Verbindungen der Formel IL[1]

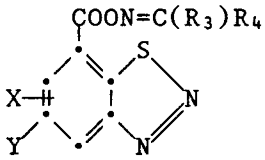

(IL[1]),

in welcher $R_3$, $R_4$, X und Y die unter Formel I angegebenen Bedeutungen besitzen, hergestellt werden, indem man Oximderivate der Formel

HO-N = $C(R_3)R_4$

mit aktivierten Säurederivaten der Formel

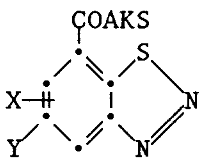

,

worin AKS ein Halogen, ein O-Acyl, wie z.B. der O-Acylrest der freien Säure vorstehender Formel, Acetoxy oder Benzoyloxy, oder 1-Imidazolyl darstellt, in einem inerten Lösungsmittel und einer Base bei -20 °C bis 120 °C, bevorzugt bei 0 ° bis 50 °C, oder die freie Säure (= Ib) in Gegenwart von Dicyclohexylcarbodiimid unter den gleichen Bedingungen umsetzt;
5.2 Verbindungen der Formel $IL^2$

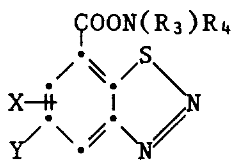

($IL^2$)

in welcher $R_3$, $R_4$, X und Y die unter Formel I angegebenen Bedeutungen besitzen, hergestellt werden durch Reduktion von Verbindungen der Formel $IL^1$

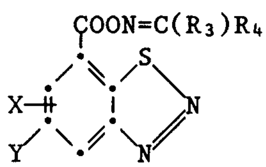

($IL^1$)

a) mit einem Silan, wie z.B. Triethylsilan, in Gegenwart einer Säure, wie z.B. Trifluoressigsäure, bei 0 ° bis 80 °C, oder
b) mit Natriumcyanoborhydrid in Gegenwart einer organischen Säure, wie z.B. Essigsäure, bei 0 ° bis 80 °C, oder
c) auf katalytischen Wege, z.B. mit $Pt/H_2$.

**25.** Verfahren zur Herstellung von Verbindungen der Formel Ik

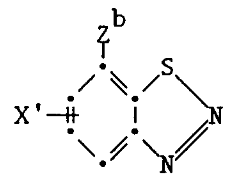

(Ik)

indem man eine Verbindung der Formel XI'

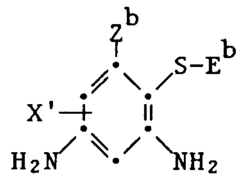

(XI')

in saurem Medium mit einer Nitritverbindung bei -40° bis 30°C diazotiert und im gleichen oder zweiten Reaktionsgefäss mit einem Reduktionsmittel bei -40° bis 80°C, vorzugsweise bei -30° bis 30°C, behandelt, wobei das Reduktionsmittel vor, nach oder gleichzeitig mit der Nitritverbindung hinzugefügt wird, wobei die Substituenten der Verbindungen folgende Bedeutungen haben:

X' Wasserstoff, Halogen, Methyl, Methoxy oder COOH;

$E^b$ eine leicht abspaltbare Gruppe wie z.B. Wasserstoff, $C_1$-$C_{16}$-Alkyl, z.B. Methyl, Ethyl, Isopropyl, n-Dodecyl, oder Benzyl oder Acyl, z.B. Acetyl oder den Sulfonsäure-Rest ($-SO_3H-$) oder Cyano oder darüber hinaus als Teil einer Disulfid-Brücke einen zweiten Rest

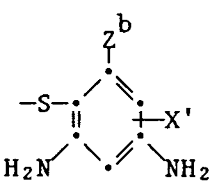

;

$Z^b$ Cyano oder COA;

A UR, $N(R_1)R_2$ oder $U^1N(=C)_n(R_3)R_4$;

M Moläquivalent eines Alkali- oder Erdalkali-Ions, das aus einer entsprechenden Base oder basischen Verbindung gebildet ist;

U Sauerstoff oder Schwefel;

$U^1$ Sauerstoff oder $-N(R_5)-$;

R Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-COO$C_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

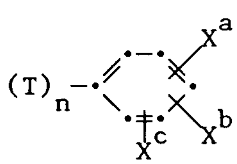 , $(T)_n$-Napht, $(T)_n$-Si($C_1$-$C_8$-Alkyl$)_3$,

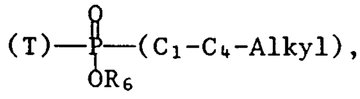 , 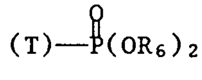 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-

Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder
$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder
$X^a$ Phenyl, Phenyloxy oder Benzyloxy und $X^b$ Halogen oder Methyl und $X^c$ Wasserstoff; oder
$X^a$, $X^b$ und $X^c$ zusammen 4 oder 5 Fluoratome;
Napht einen unsubstituierten oder mit Halogen, Methyl, Methoxy oder Nitro substituierten Naphthylrest;
W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;
T die Brückenglieder $-CH_2-$, $-CH_2CH_2-$, $-CH(CH_3)-$, $-CH_2CH_2CH_2-$ oder $-CH_2CH_2O-$;
$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;
$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;
$R_1$ und $R_2$ zusammen mit dem Atom, an welches sie gebunden sind, einen Heterocyclus W;
$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;
$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;
$R_3$ und $R_4$ zusammen mit dem Atom, an welches sie gebunden sind, einen Heterocyclus W oder einen carbocyclischen Ring W';
W' einen carbocyclischen Rest mit 3- bis 7 Ringkohlenstoffatomen;
$R_5$ Wasserstoff oder Methyl;
$R_6$ Wasserstoff oder $C_1$-$C_4$-Alkyl; und
n Null oder 1;
mit Ausnahme von primären oder sekundären Aminogruppen, UH- oder Nitro-Gruppen, $Si(C_1$-$C_8$-$Alkyl)_3$ oder Phosphor-haltigen Resten.

**26.** Verfahren zur Herstellung von Verbindungen der Formel

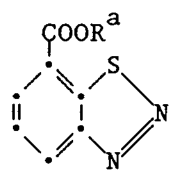

indem man eine Verbindung der Formel

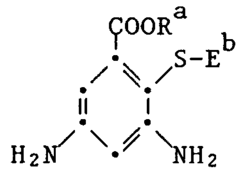

in saurem Medium mit einer Nitritverbindung bei -20° bis 30°C diazotiert und im gleichen Reaktionsgefäss mit einem Reduktionsmittel bei -20° bis 80°C, vorzugsweise bei 20° bis 30°C, behandelt, wobei das Reduktionsmittel vor, nach oder gleichzeitig mit der Nitritverbindung hinzugefügt wird, wobei die Substituenten der Verbindungen folgende Bedeutungen haben:

$R^a$ Wasserstoff, $C_1$-$C_8$-Alkyl, durch Halogen, Cyano, Nitro, Hydroxy, oder U-$C_1$-$C_3$-Alkyl substituiertes $C_1$-$C_8$-Alkyl, (T)-COOH oder (T)-$COOC_1$-$C_4$-Alkyl, $C_3$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

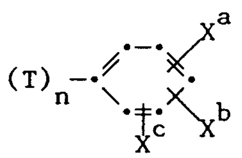 , $(T)_n$-Napht, $(T)_n$-Si($C_1$-$C_8$-Alkyl$)_3$,

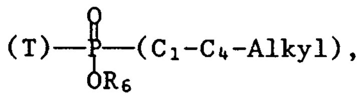, 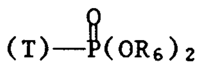 ; oder $(T)_n$—W ;

$X^a$, $X^b$ und $X^c$ unabhängig voneinander Wasserstoff, Halogen, Hydroxy, Cyano, HOOC, MOOC, $C_1$-$C_3$-Alkyl-OOC, $C_1$-$C_4$-Alkyl, $C_1$-$C_4$-Alkoxy, $C_1$-$C_2$-Halogenalkyl mit bis zu 5 Halogenatomen, insbesondere Fluoratomen; oder

$X^a$ $C_1$-$C_2$-Halogenalkoxy mit bis zu 5 Halogenatomen, Nitro, Dimethylamino, Phenyl, Phenyloxy, Benzyloxy, Sulfamoyloxy und $X^b$ und $X^c$ gleichzeitig Wasserstoff; oder

$X^a$ Phenyl, Phenyloxy oder Benzyloxy und $X^b$ Halogen oder Methyl und $X^c$ Wasserstoff; oder

$X^a$, $X^b$ und $X^c$ zusammen 4 oder 5 Fluoratome;

Napht einen unsubstituierten oder mit Halogen, Methyl, Methoxy oder Nitro substituierten Naphthylrest;

W einen 5- bis 7-gliedrigen gesättigten oder ungesättigten unsubstituierten oder durch Halogen, Trifluormethyl, Cyano oder $C_1$-$C_2$-Alkyl oder einen $C_1$-$C_2$-Alkoxycarbonyl-$C_2$-$C_4$-alkylen-imino-Rest substituierten Heterocyclus mit 1 bis 3 Heteroatomen aus der Gruppe O, N und S sowie darüber hinaus einen Monosaccharid-Rest;

T die Brückenglieder $-CH_2-$, $-CH_2CH_2-$, $-CH(CH_3)-$, $-CH_2CH_2CH_2-$ oder $-CH_2CH_2O-$;

$R_1$ Wasserstoff, $C_1$-$C_5$-Alkyl, durch ein Sauerstoff- oder Schwefelatom unterbrochenes $C_1$-$C_5$-Alkyl, durch Halogen, Cyano, HOOC oder $C_1$-$C_2$-Alkyl-OOC substituiertes $C_1$-$C_5$-Alkyl, durch ein Sauerstoff oder ein Schwefelatom unterbrochenes und durch Halogen, Cyano, HOOC oder $C_1$-$C_2$-Alkyl-OOC substituiertes $C_1$-$C_5$-Alkyl, $C_3$-$C_5$-Alkenyl, durch $C_1$-$C_3$-Alkyl-OOC substituiertes $C_3$-$C_5$-Alkenyl, $C_3$-$C_5$-Alkinyl, durch $C_1$-$C_3$-Alkyl-OOC substituiertes $C_3$-$C_5$-Alkinyl, $(T)_n$-$C_3$-$C_6$-Cycloalkyl, durch $C_1$-$C_3$-Alkyl-OOC substituiertes $(T)_n$-$C_3$-$C_6$-Cycloalkyl, $(T)_n$-Phenyl oder im Phenylteil durch Halogen, Hydroxy, Methyl, $CF_3$, Cyano, Methoxy, HOOC oder MOOC substituiertes $(T)_n$-Phenyl;

$R_2$ Wasserstoff, Hydroxy, $C_1$-$C_3$-Alkyl, durch Cyano oder $C_1$-$C_3$-Alkoxy substituiertes $C_1$-$C_3$-Alkyl, $C_1$-$C_4$-Alkoxy, einen 3- bis 6-gliedrigen gesättigten oder ungesättigten Heterocyclus mit O, N oder S als Heteroatome;

$R_1$ und $R_2$ zusammen mit dem Atom, an welches sie gebunden sind, einen Heterocyclus W;

$R_3$ Wasserstoff, Cyano, $C_1$-$C_6$-Alkyl, Phenyl, durch Halogen, Hydroxy, Methyl, Methoxy, HOOC oder MOOC substituiertes Phenyl, einen Heterocyclus W;

$R_4$ Wasserstoff, $C_1$-$C_6$-Alkyl, $CONH_2$, CONH-CONH-$C_1$-$C_3$-Alkyl, $C_1$-$C_6$-Alkanoyl, durch Halogen oder $C_1$-$C_3$-Alkoxy substituiertes $C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl;

$R_3$ und $R_4$ zusammen einen Heterocyclus W oder einen carbocyclischen Ring W';

W' einen carbocyclischen Rest mit 3- bis 7 Ringkohlenstoffatomen;

$R_5$ Wasserstoff oder Methyl;

$R_6$ Wasserstoff oder $C_1$-$C_4$-Alkyl; und

n Null oder 1;

mit Ausnahme von Resten mit UH- oder Nitro-Gruppen sowie Silizium- oder Phosphor-haltigen Resten, und

$E^b$ eine leicht abspaltbare Gruppe wie z.B. Wasserstoff, $C_1$-$C_{16}$-Alkyl, z.B. Methyl, Ethyl, Isopropyl, n-Dodecyl, oder Benzyl oder Acyl, z.B. Acetyl oder den Sulfonsäure-Rest ($-SO_3H-$) oder Cyano.

**27.** Verwendung der Verbindungen der Formel I gemäss Anspruch 1 oder 2 zur Immunisierung von

Pflanzen gegen Krankheiten.

**28.** Verwendung der Verbindungen der Formel I' gemäss Anspruch 20 zur Immunisierung von Pflanzen gegen Krankheiten.

**29.** Verwendung von 7-Methoxycarbonyl-benzo-1,2,3-thiadiazol als Wirkstoff zur Herstellung eines Mittels zur Immunisierung von Pflanzen gegen den Befall durch phytopathogene Mikroorganismen.

## Claims

**Claims for the following Contracting States : AT, BE, CH, DE, FR, GB, GR, IT, LI, LU, NL, SE**

**1.** A process for immunising plants against attack by phytopathogenic microorganisms which comprises applying as active ingredient to said plants and/or to the locus thereof a compound of the following formula I

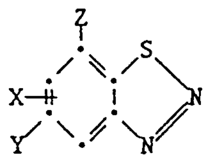

(I)

wherein:
X is hydrogen, halogen, hydroxy, methyl, methoxy, HOOC or MOOC;
Y is hydrogen, halogen, $SO_3H$, $SO_3M$, nitro, hydroxy or amino, M being the molar equivalent of an alkali metal or alkaline earth metal ion that is formed from a corresponding base or basic compound; and
Z is cyano or -CO-A, A being either OH or SH, the H of which may also have been replaced by the molar equivalent of an inorganic or organic cationic radical.

**2.** A process for immunising plants against attack by phytopathogenic microorganisms which comprises applying as active ingredient to said plants and/or to the locus thereof a compound of the following formula I

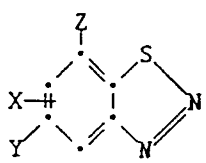

(I)

wherein:
X is hydrogen, halogen, hydroxy, methyl, methoxy, HOOC or MOOC;
Y is hydrogen, halogen, $SO_3H$, $SO_3M$, nitro, hydroxy or amino, M being the molar equivalent of an alkali metal or alkaline earth metal ion that is formed from a corresponding base or basic compound; and
Z is cyano or -CO-A,
A is UR, $N(R_1)R_2$ or $U^1N(=C)_n(R_3)R_4$;
M is the molar equivalent of an alkali metal or alkaline earth metal ion that has been formed from a corresponding base or basic compound;
U is oxygen or sulfur;
$U^1$ is oxygen or $-N(R_5)-$;
R is hydrogen, $C_1$-$C_8$alkyl, $C_1$-$C_8$alkyl that is substituted by halogen, cyano, nitro, hydroxy, U-$C_1$-$C_3$alkyl or by $C_2$-$C_4$dialkylamino or is interrupted by the CO group, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

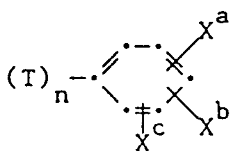 , (T)$_n$-naphth, (T)$_n$-Si($C_1$-$C_8$alkyl)$_3$,

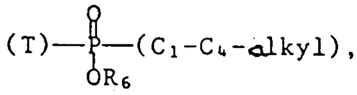 , 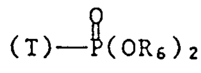 ; and (T)$_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms; or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or
$X^a$ is phenyl, phenoxy or benzyloxy and $X^b$ is halogen or methyl and $X^c$ is hydrogen; or
$X^a$, $X^b$ and $X^c$ together are 4 or 5 fluorine atoms;
naphth is a naphthyl radical that is unsubstituted or is substituted by halogen, methyl, methoxy or by nitro;
W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CCH_3(CH_3)-$, $-CH_2CH_2CH_2-$ or $-CH_2CH_2O-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, (T)$_n$-$C_3$-$C_6$cycloalkyl, (T)$_n$-$C_3$-$C_6$cycloalkyl substituted by $C_1$-$C_3$alkyl-OOC, (T)$_n$-phenyl, or (T)$_n$-phenyl substituted in the phenyl moiety by halogen, hydroxy, methyl, methoxy, $CF_3$, cyano, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_3$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
and in a compound of formula I the organic radical A has a molecular weight of less than 900; or in the case where U is oxygen or sulfur, a salt of the phytophysiologically tolerable 7-carboxylic acid with a primary, secondary or tertiary amine or with an inorganic base.

**3.** A process according to claim 1 or claim 2, wherein the application rate is less than 1 kg of active ingredient/hectare.

**4.** A process according to claim 2, wherein there is used a compound of formula I wherein:
X and Y are hydrogen;
Z is cyano or COA;
A is UR or $U^1N(=C)_n(R_3)R_4$;
U is oxygen;
$U^1$ is oxygen or $-N(R_5)-$;

R is hydrogen, $C_1$-$C_8$alkyl, $C_1$-$C_8$alkyl substituted by halogen or by $C_1$-$C_3$alkoxy, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, benzyl, halogenated benzyl, methoxybenzyl, $(T)_n$-$Si(CH_3)_3$, (T)-P(O)($C_1$-$C_4$alkyl)$CH_3$, (T)-P(O)($OC_1$-$C_4$alkyl)$_2$ or the group $(T)_n$-W;
W is a 5- to 7-membered saturated or unsaturated unsubstituted heterocycle having from 1 to 3 hetero atoms from the group O, N and S, or is diacetone-D-glucosidyl;
T is a bridge member $-CH_2-$, $-CH_2CH_2-$, $-CH(CH_3)-$, $-CCH_3(CH_3)-$ or $-CH_2CH_2CH_2-$;
$R_3$ is hydrogen, cyano, $C_1$-$C_6$alkyl, phenyl or W;
$R_4$ is hydrogen, $C_1$-$C_6$alkyl, $CONH_2$, CONH-CONH-$C_1$-$C_3$alkyl, $C_1$-$C_3$alkanoyl or $C_3$-$C_5$alkenoyl;
$R_3$ and $R_4$ together with the atom to which they are bonded are W or W';
W is a 5- to 7-membered saturated or unsaturated heterocycle having from 1 to 3 hetero atoms from the group O, N and S;
W' is a radical from the group:

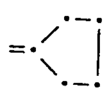 , 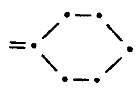 and 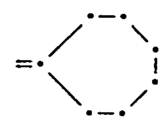 ;

n is 0 or 1.

**5.** A compound of formula I'

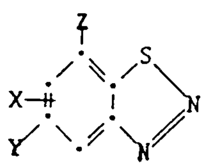 (I')

wherein:
X is hydrogen, halogen, hydroxy, methyl, methoxy, HOOC or MOOC;
Y is hydrogen, halogen, $SO_3H$, $SO_3M$, nitro, hydroxy or amino;
Z is cyano or COA;
A is UR, $N(R_1)R_2$ or $U^1N(=C)_n(R_3)R_4$;
M is the molar equivalent of an alkali metal or alkaline earth metal ion that has been formed from a corresponding base or basic compound;
U is oxygen or sulfur;
$U^1$ is oxygen or $-N(R_5)-$;
R is hydrogen, $C_1$-$C_8$alkyl, $C_1$-$C_8$alkyl substituted by halogen, cyano, nitro, hydroxy or by U-$C_1$-$C_3$alkyl, (T)-COOH or (T)-$COOC_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

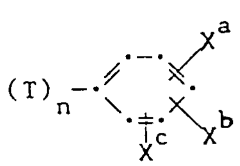 , $(T)_n$-naphth, $(T)_n$-Si($C_1$-$C_8$alkyl)$_3$,

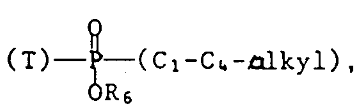 , 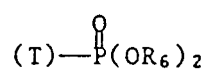 ; and $(T)_n$—W;

each of $X^a$, $X^b$ and $X^c$, independently of the others, is hydrogen, halogen, hydroxy, cyano, HOOC,

MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms; or $X^a$ is $C_1$-$C_2$haloalkoxy having up to 5 halogen atoms, nitro, dimethylamino, phenyl, phenoxy, benzyloxy or sulfamoyloxy and $X^b$ and $X^c$ are both hydrogen; or
$X^a$ is phenyl, phenoxy or benzyloxy and $X^b$ is halogen or methyl and $X^c$ is hydrogen; or
$X^a$, $X^b$ and $X^c$ together are 4 or 5 fluorine atoms;
naphth is a naphthyl radical that is unsubstituted or is substituted by halogen, methyl, methoxy or by nitro;
W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CH_2CH_2CH_2-$ or $-CH_2CH_2O-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, or a 3- to 6-membered saturated or unsaturated heterocycle having O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
with the exception of the compounds:
7-cyanobenzo-1,2,3-thiadiazole;
4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid.

**6.** A compound of formula I'

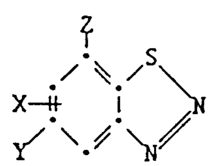

(I')

wherein:
X is hydrogen, halogen, hydroxy, methyl, methoxy, HOOC or MOOC;
Y is hydrogen, halogen, $SO_3H$, $SO_3M$, nitro, hydroxy or amino;
Z is cyano or COA;
A is UR, $N(R_1)R_2$ or $U^1N(=C)_n(R_3)R_4$;
M is the molar equivalent of an alkali metal or alkaline earth metal ion that has been formed from a corresponding base or basic compound;
U is oxygen or sulfur;
$U^1$ is oxygen or $-N(R_5)-$;

R is hydrogen, $C_1$-$C_8$alkyl, $C_1$-$C_8$alkyl that is substituted by halogen, cyano, nitro, hydroxy, alkoxy or by U-$C_1$-$C_3$alkyl, (T)-COOH or (T)-$COOC_1$-$C_4$alkyl, $C_2$-$C_6$alkenyl, halo-substituted $C_3$-$C_6$alkenyl, $C_3$-$C_6$-alkynyl, halo-substituted $C_3$-$C_6$alkynyl, $(T)_n$-$C_3$-$C_8$cycloalkyl, or a group selected from the following:

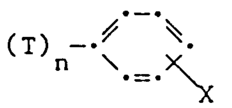 , $(T)_n$-naphthyl, $(T)_n$-Si($C_1$-$C_8$alkyl$)_3$,

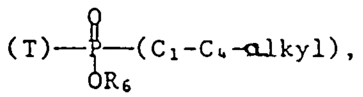 , 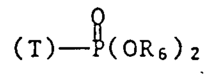 ; and $(T)_n$—W;

W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$ or $-CH(CH_3)-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, or a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_3$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
with the exception of the compounds:
7-cyanobenzo-1,2,3-thiadiazole;
4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid.

**7.** A compound of formula I' according to claim 5 wherein Z = COA and R has a meaning other than hydrogen and unsubstituted alkyl.

**8.** A compound of formula I' according to claim 5 from the group:
7-n-pentyloxycarbonylbenzo-1,2,3-thiadiazole;
7-(4-methoxybenzyloxycarbonyl)-benzo-1,2,3-thiadiazole;
7-(cycloheximino-oxycarbonyl)-benzo-1,2,3-thiadiazole;
7-(3-hydroxy-n-propoxycarbonyl)-benzo-1,2,3-thiadiazole;
1,2,5,6-di-O-isopropylidene-3-(7-benzo-1,2,3-thiadiazoyl)-D-glucofuranose;
7-furfuryloxycarbonylbenzo-1,2,3-thiadiazole;
7-(1,2,4-triazol-1-yl)-methoxycarbonylbenzo-1,2,3-thiadiazole;

7-(2-pyridylmethoxycarbonyl)-benzo-1,2,3-thiadiazole;
7-trimethylsilylmethoxycarbonylbenzo-1,2,3-thiadiazole;
7-[2-(trimethylsilyl)-ethoxycarbonyl]-benzo-1,2,3-thiadiazole;
7-dimethylphosphono-ethoxycarbonylbenzo-1,2,3-thiadiazole;
7-cyclohexyloxycarbonylbenzo-1,2,3-thiadiazole;
7-(1-phenethyloxycarbonyl)-benzo-1,2,3-thiadiazole;
7-(3-methoxybenzyl)-benzo-1,2,3-thiadiazole;
7-(ethylthiocarbonyl)-benzo-1,2,3-thiadiazole;
7-(n-propylthiocarbonyl)-benzo-1,2,3-thiadiazole;
7-(benzylthiocarbonyl)-benzo-1,2,3-thiadiazole;
7-carbamoylbenzo-1,2,3-thiadiazole;
7-N-phenylcarbamoylbenzo-1,2,3-thiadiazole;
N-(7-benzo-1,2,3-thiadiazoyl)-glycine;
7-(N-diallylcarbamoyl)-benzo-1,2,3-thiadiazole;
6-fluoro-7-methoxycarbonylbenzo-1,2,3-thiadiazole;
6-fluoro-7-carboxybenzo-1,2,3-thiadiazole;
5-fluoro-7-benzyloxycarbonylbenzo-1,2,3-thiadiazole;
5-fluoro-7-carboxybenzo-1,2,3-thiadiazole;
5-fluoro-7-ethoxycarbonylbenzo-1,2,3-thiadiazole.

**9.** A compound of formula I' according to claim 6 from the group:
7-ethoxycarbonylbenzo-1,2,3-thiadiazole;
7-n-propoxycarbonylbenzo-1,2,3-thiadiazole;
7-isopropoxycarbonylbenzo-1,2,3-thiadiazole;
7-n-butoxycarbonylbenzo-1,2,3-thiadiazole;
7-sec-butoxycarbonylbenzo-1,2,3-thiadiazole;
7-tert-butoxycarbonylbenzo-1,2,3-thiadiazole;
7-cyclopropylmethoxycarbonylbenzo-1,2,3-thiadiazole;
7-(2'-phenethoxycarbonyl)-benzo-1,2,3-thiadiazole;
7-benzyloxycarbonylbenzo-1,2,3-thiadiazole;
7-allyloxycarbonylbenzo-1,2,3-thiadiazole;
7-propyn-2-yloxycarbonylbenzo-1,2,3-thiadiazole;
2-(7-benzo-1,2,3-thiadiazoyloxy-imino)-2-cyano-N-(ethylaminocarbonyl)-acetamide;
sodium salt of benzo-1,2,3-thiadiazole-7-carboxylic acid;
potassium salt of benzo-1,2,3-thiadiazole-7-carboxylic acid;
triethylammonium salt of benzo-1,2,3-thiadiazole-7-carboxylic acid;
7-(1'-phenethoxycarbonyl)-benzo-1,2,3-thiadiazole;
7-(1'-naphthylmethoxycarbonyl)-benzo-1,2,3-thiadiazole;
7-(methylthiocarbonyl)-benzo-1,2,3-thiadiazole;
7-(ethylthiocarbonyl)-benzo-1,2,3-thiadiazole;
7-(benzylthiocarbonyl)-benzo-1,2,3-thiadiazole;
7-[(dicyanomethyl)-aminocarbonyl]-benzo-1,2,3-thiadiazole;
3-[(N-benzo-1,2,3-thiadiazol-7-ylcarbonyl)-1,3,4-thiadiazol-2-ylamino]-2-methylpropenecarboxylic acid methyl ester;
3-[(N-benzo-1,2,3-thiadiazol-7-ylcarbonyl)-1,3,4-thiadiazol-2-ylamino]-2-ethylpropenecarboxylic acid methyl ester;
1-(benzo-1,2,3-thiadiazole-7-carbonyl)-2-($\alpha$-methylpropylidene)-hydrazine;
1-(benzo-1,2,3-thiadiazole-7-carbonyl)-2-(cyclobutylidene)-hydrazine;
1-(benzo-1,2,3-thiadiazole-7-carbonyl)-2-(cyclopentylidene)-hydrazine;
1-(benzo-1,2,3-thiadiazole-7-carbonyl)-2-(cyclohexylidene)-hydrazine;
2-(benzo-1,2,3-thiadiazole-7-carbonyl)-1-(2'-sec-butyl)-hydrazine;
1-(benzo-1,2,3-thiadiazole-7-carbonyl -2-(cyclopentyl)-hydrazine;
1-(benzo-1,2,3-thiadiazole-7-carbonyl)-2-(cyclohexyl)-hydrazine;
1-(benzo-1,2,3-thiadiazole-7-carbonyl)-2-(cycloheptyl)-hydrazine;
1-(benzo-1,2,3-thiadiazole-7-carbonyl)-1,2-diacetylhydrazine;
1-(benzo-1,2,3-thiadiazole-7-carbonyl)-2-phenylhydrazine;
1-(benzo-1,2,3-thiadiazole-7-carbonyl)-2-pyridin-2'-ylhydrazine.

**10.** A process according to claim 2, wherein a compound of formula I' as defined in claim 5 is used.

**11.** A process according to claim 2, wherein a compound of formula I' as defined in claim 6 is used.

**12.** A process according to claim 2, wherein a compound of formula I' as defined in claim 7 is used.

**13.** A process according to claim 2, wherein 1,2,3-benzothiadiazole-7-carboxylic acid or a salt thereof with a basic compound is used as active ingredient.

**14.** A process according to claim 13, wherein the salt used is one with a primary, secondary or tertiary amine or an alkali metal or alkaline earth metal compound.

**15.** A process according to claim 2, wherein 7-methoxycarbonylbenzo-1,2,3-thiadiazole is used as active ingredient.

**16.** A process according to claim 2, wherein 7-benzyloxycarbonylbenzo-1,2,3-thiadiazole is used as active ingredient.

**17.** A process according to claim 2, wherein 7-cyanobenzo-1,2,3-thiadiazole is used as active ingredient.

**18.** A process according to claim 2, wherein a compound of 7-$C_2$-$C_4$alkoxycarbonylbenzo-1,2,3-thiadiazole is used as active ingredient.

**19.** A composition for the immunisation of plants against attack by phytopathogenic microorganisms, comprising as active ingredient at least one compound of formula I defined in claim 1 or claim 2 with the exception of the compounds 7-cyanobenzo-1,2,3-thiadiazole; 4-chloro-7-cyanobenzo-1,2,3-thiadiazole; 6-chloro-7-cyanobenzo-1,2,3-thiadiazole; 4,6-dibromo-7-cyanobenzo-1,2,3-thiadiazole and 7-methoxycarbonylbenzo-1,2,3-thiadiazole.

**20.** A composition for the immunisation of plants against attack by phytopathogenic microorganisms, comprising as active ingredient at least one compound of formula I' defined in claim 5.

**21.** A composition for the immunisation of plants against attack by phytopathogenic microorganisms, comprising as active ingredient at least one compound of formula I' defined in claim 6.

**22.** A composition according to claim 19, comprising as active ingredient at least one compound of formula I' defined in claim 7.

**23.** A composition according to claim 19, comprising as active ingredient at least one compound according to either claim 8 or claim 9.

**24.** A composition according to claim 19, comprising as active ingredient at least one of the compounds: benzo-1,2,3-thiadiazole-7-carboxylic acid and 7-methoxycarbonylbenzo-1,2,3-thiadiazole.

**25.** A composition according to claim 19, comprising as active ingredient 1,2,3-benzothiadiazole-7-carboxylic acid or a salt thereof with a basic compound.

**26.** A composition according to claim 25, wherein the salt used is one with a primary, secondary or tertiary amine or an alkali metal or alkaline earth metal compound.

**27.** A process for the preparation of a compound of formula I'

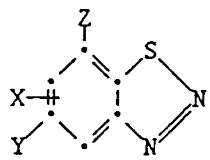

(I')

wherein:

X is hydrogen, halogen, hydroxy, methyl, methoxy, HOOC or MOOC;

Y is hydrogen, halogen, $SO_3H$, $SO_3M$, nitro, hydroxy or amino;

Z is cyano or COA;

A is UR, $N(R_1)R_2$ or $U^1N(=C)_n(R_3)R_4$;

M is the molar equivalent of an alkali metal or alkaline earth metal ion that has been formed from a corresponding base or basic compound;

U is oxygen or sulfur;

$U^1$ is oxygen or $-N(R_5)-$;

R is hydrogen, $C_1$-$C_8$alkyl, $C_1$-$C_8$alkyl that is substituted by halogen, cyano, nitro, hydroxy or by U-$C_1$-$C_3$alkyl, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

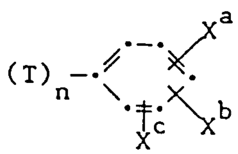 , $(T)_n$-naphth, $(T)_n$-Si($C_1$-$C_8$alkyl$)_3$,

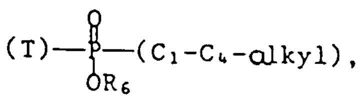 , 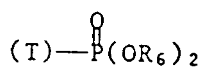 ; and $(T)_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;

or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or

$X^a$ is phenyl, phenoxy or benzyloxy and $X^b$ is halogen or methyl and $X^c$ is hydrogen; or

$X^a$, $X^b$ and $X^c$ together are 4 or 5 fluorine atoms;

naphth is a naphthyl radical that is unsubstituted or is substituted by halogen, methyl, methoxy or by nitro;

W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;

T is a bridge member $-CH_2-$, $-CH_2CH_2-$, $-CH(CH_3)-$, $-CH_2CH_2CH_2-$ or $-CH_2CH_2O-$;

$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl, or $(T)_n$-phenyl substituted in the phenyl moiety by halogen, hydroxy, methyl, $CF_3$, cyano, methoxy, HOOC or by MOOC;

$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;

$R_1$ and $R_2$ together with the atom to which they are bonded are a heterocycle W;

$R_3$ is hydrogen, cyano, $C_1$-$C_6$alkyl, phenyl, phenyl substituted by halogen, hydroxy, methyl, methoxy, HOOC or by MOOC, or a heterocycle W;

$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;

$R_3$ and $R_4$ together with the atom to which they are bonded are a heterocycle W or a carbocyclic ring W';

W' is a carbocyclic radical having from 3 to 7 ring carbon atoms;

$R_5$ is hydrogen or methyl;

$R_6$ is hydrogen or $C_1$-$C_4$alkyl; and

n is 0 or 1;

with the exception of the compounds:

7-cyanobenzo-1,2,3-thiadiazole;

4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid;
in which process

1. a compound of formula Ia

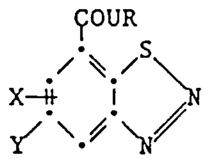

(Ia)

in which R, X, Y and U have the meanings given under formula I, is prepared by reacting:

1.1 a compound of formula II

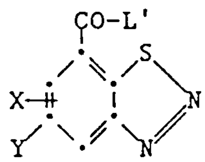

(II)

in which L' is a leaving group, for example halogen, O-acyl, for example the acyl radical belonging to the symmetric anhydride of acid Ib, or 1-imidazolyl, with a compound of formula III

RUH (III)

a) in an excess of the reactant RUH or
b) in the presence of an organic base either with or without 4-dialkylaminopyridine as catalyst in an inert solvent or
c) in the presence of an inorganic base,
the reaction in each case being carried out in a temperature range of from -10° to 180°C, preferably from 0° to 100°C; or

1.2 a compound of formula Ib

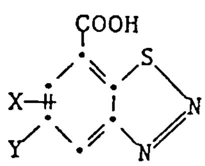

(Ib)

with a compound of formula III in excess or in an inert solvent in the presence of an acid, such as sulfuric acid, hydrochloric acid, p-toluenesulfonic acid or boron trifluoride/diethyl ether complex, or in the presence of dicyclohexylcarbodiimide at a temperature of from -10° to 180°C, preferably from 0° to 140°C; and

2. a compound of formula Ic

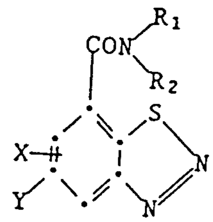

(Ic)

in which the symbols $R_1$, $R_2$, X and Y have the meanings given under formula I, is prepared by reacting:

2.1 a compound of formula II with a compound of formula IV

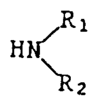

(IV)

a) in an excess of the reactant $HN(R_1)R_2$ or

b) in the presence of an organic base either with or without 4-dialkylaminopyridine as catalyst in an inert solvent or

c) in the presence of an inorganic base,

the reaction in each case being carried out in a temperature range of from -10° to 160°C, preferably from 0° to 100°C; and

3. a compound of formula Id

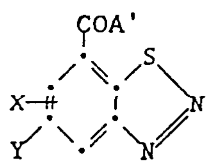

(Id),

in which A' is the radical $U^1N(=C)_n(R_3)R_4$ and X, Y, $R_3$, $R_4$, $R_5$ and n have the meanings given under formula I, is prepared by:

3.1 reacting a compound of formula Ie

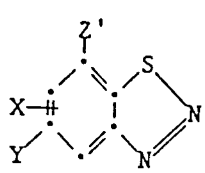

(Ie)

in which Z' is a group COOH, COCl, $COOAlk^1$ or an acyloxycarbonyl radical, for example

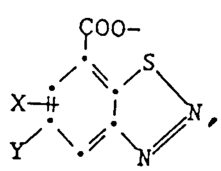

benzoyloxycarbonyl or acetoxycarbonyl, in which $Alk^1$ is $C_1$-$C_4$alkyl, in the presence of a base, with a hydrazine derivative of formula V or VI

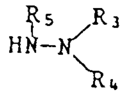 (V) or 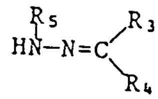 (VI)

in an inert solvent at a temperature of from -10° to 180°C, preferably from 0° to 100°C; or

3.2 reacting a compound of formula Ie stepwise first with hydrazine, and then reacting the resulting hydrazine compound

3.2.1 with the alkylating agent $R_3$-L or $R_4$-L in which L is a leaving group, in an inert solvent at a temperature of from 0° to 160°C, preferably from 20° to 120°C; or

3.2.2 with an aldehyde or ketone of formula $R_3(R_4)C=O$ in which $R_3$ and $R_4$ have the meanings given under formula I, with or without the addition of an organic or inorganic acid, at a temperature of from -10° to 150°C, preferably from 20° to 100°C, and subsequently, if desired,

3.2.3 with an alkylating agent L-$R_5$ in which L is a leaving group, in the presence of a strong base in an inert solvent at a temperature of from -80° to 120°C, preferably from -40° to 80°C; or, if desired,

3.2.4 a) hydrogenating the hydrazone derivative prepared under (3.2.1) with hydrogen at a pressure of from 1 to $30x10^5$ Pa in the presence of a catalyst in admixture with activated carbon in an inert solvent at a temperature of from 0° to 100°C, or

3.2.4 b) treating the hydrazone derivative prepared under (3.2.1) with a complex metal hydride, for example sodium cyanoborohydride, in an inert solvent at a temperature of from -10° to 80°C, preferably from 0° to 50°C; and

4. a compound of formula If

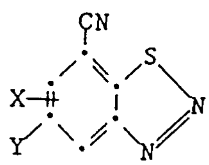 (If),

in which the symbols X and Y have the meanings given under formula I, is prepared by treating a compound of formula Ig [prepared according to process (2)]

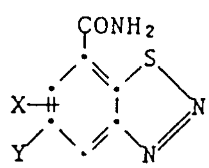 (Ig)

with a dehydrating agent in an inert solvent or without a solvent at a temperature of from -10° to 250°C, suitable dehydrating agents being:

a) trifluoroacetic acid anhydride in the presence of a base, for example pyridine, in an inert solvent, for example tetrahydrofuran or dioxane, at a temperature of from -10° to 40°C; or

b) chlorosulfonyl isocyanate in an inert solvent, for example tetrahydrofuran, at a temperature of from 0° to 65°C, with subsequent treatment with dimethylformamide;

c) phosphorus pentoxide with or without an inert solvent, for example 1,2-dichloroethane, xylene or chlorobenzene, optionally in a bomb tube under elevated pressure, at from 50° to 250°C;

5.1 a compound of formula $IL^1$

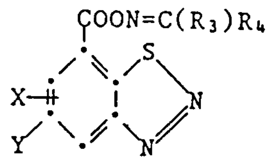

(IL[1])

in which $R_3$, $R_4$, X and Y have the meanings given under formula I, is prepared by reacting an oxime derivative of formula

HO-N = C($R_3$)$R_4$

with an activated acid derivative of formula

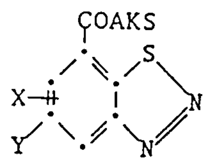

,

in which AKS is a halogen, an O-acyl, for example the O-acyl radical of the free acid of the formula above, acetoxy or benzoyloxy, or 1-imidazolyl, in an inert solvent and a base at from -20 °C to 120 °C, preferably from 0 ° to 50 °C, or by reacting the free acid (= Ib) in the presence of dicyclohexylcarbodiimide under the same conditions;
5.2 a compound of formula IL[2]

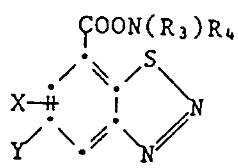

(IL[2])

in which $R_3$, $R_4$, X and Y have the meanings given under formula I, is prepared by reduction of a compound of formula IL[1]

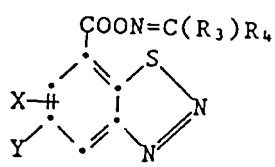

(IL[1])

a) with a silane, for example triethylsilane, in the presence of an acid, for example trifluoroacetic acid, at from 0 ° to 80 °C, or
b) with sodium cyanoborohydride in the presence of an organic acid, for example acetic acid, at from 0 ° to 80 °C, or
c) by catalytic methods, for example with Pt/$H_2$.

**28.** A process for the preparation of a compound of formula Ik

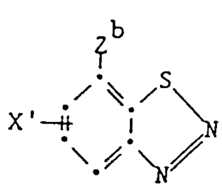

(Ik)

by diazotisation of a compound of formula XI'

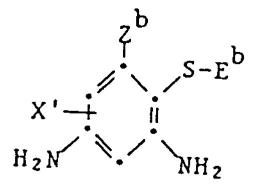

(XI')

in an acidic medium with a nitrite compound at from -40° to 30°C and, in the same reaction vessel or in a second reaction vessel, treatment with a reducing agent at from -40° to 80°C, preferably at from -30° to 30°C, the reducing agent being added before, after or at the same time as the nitrite compound, the substituents of the compounds having the following meanings:

X' is hydrogen, halogen, methyl, methoxy or COOH;

$E^b$ is a readily removable group, for example hydrogen, $C_1$-$C_{16}$alkyl, for example methyl, ethyl, isopropyl, n-dodecyl, or benzyl or acyl, for example acetyl or the sulfonic acid radical ($-SO_3H-$) or cyano, or as part of a disulfide bridge, is a second radical

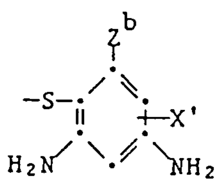

;

$Z^b$ is cyano or COA;

A is UR, $N(R_1)R_2$ or $U^1N(=C)_n(R_3)R_4$;

M is the molar equivalent of an alkali metal or alkaline earth metal ion that has been formed from a corresponding base or basic compound;

U is oxygen or sulfur;

$U^1$ is oxygen or $-N(R_5)-$;

R is hydrogen, $C_1$-$C_8$alkyl, $C_1$-$C_8$alkyl that is substituted by halogen, cyano, nitro, hydroxy or by U-$C_1$-$C_3$alkyl, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

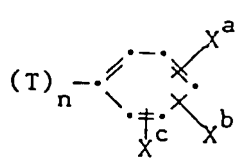

, $(T)_n$-naphth, $(T)_n$-Si($C_1$-$C_8$alkyl$)_3$,

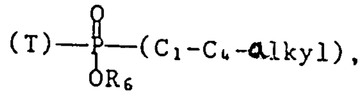

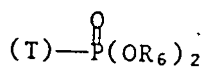

; and $(T)_n$—W;

each of $X^a$, $X^b$ and $X^c$, independently of the others, is hydrogen, halogen, hydroxy, cyano, HOOC, MOOC, $C_1$-$C_3$-alkyl-OOC, $C_1$-$C_4$alkyl, $C_1$-$C_4$alkoxy, $C_1$-$C_2$haloalkyl having up to 5 halogen atoms, especially fluorine atoms;

or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or

$X^a$ is phenyl, phenoxy or benzyloxy and $X^b$ is halogen or methyl and $X^c$ is hydrogen; or

$X^a$, $X^b$ and $X^c$ together are 4 or 5 fluorine atoms;

naphth is a naphthyl radical that is unsubstituted or is substituted by halogen, methyl, methoxy or by nitro;

W is a 5- to 7-membered saturated or unsaturated heterocycle having from 1 to 3 hetero atoms from the group O, N and S that is unsubstituted or is substituted by halogen, trifluoromethyl, cyano, $C_1$-

$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CH_2CH_2CH_2-$ or $-CH_2CH_2O-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl, or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
with the exception of primary or secondary amino groups, UH or nitro groups, $Si(C_1$-$C_8alkyl)_3$ or phosphorus-containing radicals.

**29.** A process for the preparation of a compound of formula

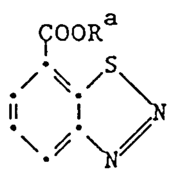

by diazotisation of a compound of formula

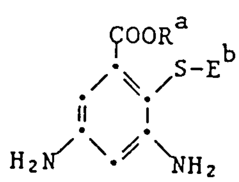

in an acidic medium with a nitrite compound at from -20° to 30°C, and in the same reaction vessel treatment with a reducing agent at from -20° to 80°C, preferably at from 20° to 30°C, the reducing agent being added before, after or at the same time as the nitrite compound, the substituents of the compounds having the following meanings:
$R^a$ is hydrogen, $C_1$-$C_8$alkyl, $C_1$-$C_8$alkyl that is substituted by halogen, cyano, nitro, hydroxy or by U-$C_1$-$C_3$-alkyl, (T)-COOH or (T)-$COOC_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

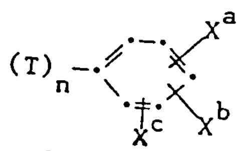 , $(T)_n$-naphth, $(T)_n$-Si($C_1$-$C_8$alkyl)$_3$,

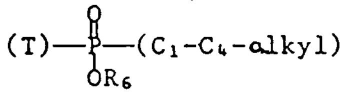 , 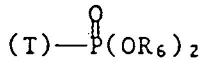 ; and $(T)_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;
or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or
$X^a$ is phenyl, phenoxy or benzyloxy and $X^b$ is halogen or methyl and $X^c$ is hydrogen; or
$X^a$, $X^b$ and $X^c$ together are 4 or 5 fluorine atoms;
naphth is a naphthyl radical that is unsubstituted or is substituted by halogen, methyl, methoxy or by nitro;
W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CH_2CH_2CH_2-$ or $-CH_2CH_2O-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl, or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
with the exception of radicals containing UH or nitro groups and silicon- or phosphorus-containing radicals, and
$E^b$ is a readily removable group, for example hydrogen, $C_1$-$C_{16}$alkyl, for example methyl, ethyl, isopropyl, n-dodecyl, or benzyl or acyl, for example acetyl or the sulfonic acid radical ($-SO_3H-$) or cyano.

**30.** The use of a compound of formula I according to claim 1 or claim 2, for the immunisation of plants against diseases.

**31.** Use of a compound of formula I' according to claim 8 or claim 9 for the immunisation of plants against diseases.

**32.** Use of 7-methoxycarbonylbenzo-1,2,3-thiadiazole as active ingredient in the preparation of a composition for the immunisation of plants against attack by phytopathogenic microorganisms.

**Claims for the following Contracting State : ES**

1. A process for immunising plants against attack by phytopathogenic microorganisms which comprises applying as active ingredient to said plants and/or to the locus thereof a compound of the following formula I

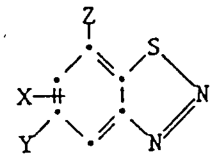

(I)

wherein:
X is hydrogen, halogen, hydroxy, methyl, methoxy, HOOC or MOOC;
Y is hydrogen, halogen, $SO_3H$, $SO_3M$, nitro, hydroxy or amino, M being the molar equivalent of an alkali metal or alkaline earth metal ion that is formed from a corresponding base or basic compound; and
Z is cyano or -CO-A, A being either OH or SH, the H of which may also have been replaced by the molar equivalent of an inorganic or organic cationic radical.

2. A process for immunising plants against attack by phytopathogenic microorganisms which comprises applying as active ingredient to said plants and/or to the locus thereof a compound of the following formula I

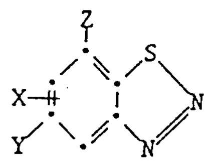

(I)

wherein:
X is hydrogen, halogen, hydroxy, methyl, methoxy, HOOC or MOOC;
Y is hydrogen, halogen, $SO_3H$, $SO_3M$, nitro, hydroxy or amino, M being the molar equivalent of an alkali metal or alkaline earth metal ion that is formed from a corresponding base or basic compound; and
Z is cyano or -CO-A,
A is UR, $N(R_1)R_2$ or $U^1N(=C)_n(R_3)R_4$;
M is the molar equivalent of an alkali metal or alkaline earth metal ion that has been formed from a corresponding base or basic compound;
U is oxygen or sulfur;
$U^1$ is oxygen or $-N(R_5)-$;
R is hydrogen, $C_1$-$C_8$alkyl, $C_1$-$C_8$alkyl that is substituted by halogen, cyano, nitro, hydroxy, U-$C_1$-$C_3$alkyl or by $C_2$-$C_4$dialkylamino or is interrupted by the CO group, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

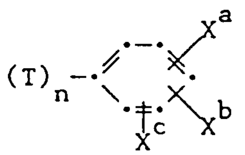 , (T)$_n$-naphth, (T)$_n$-Si($C_1$-$C_8$alkyl)$_3$,

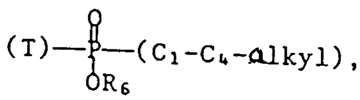 , 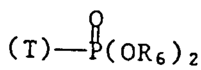 ; and (T)$_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;
or $X^a$ is $C_1$-$C_2$haloalkoxy having up to 5 halogen atoms, nitro, dimethylamino, phenyl, phenoxy, benzyloxy, sulfamoyl and $X^b$ and $X^c$ are both hydrogen; or
$X^a$ is phenyl, phenoxy or benzyloxy and $X^b$ is halogen or methyl and $X^c$ is hydrogen; or
$X^a$, $X^b$ and $X^c$ together are 4 or 5 fluorine atoms;
naphth is a naphthyl radical that is unsubstituted or is substituted by halogen, methyl, methoxy or by nitro;
W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CCH_3(CH_3)-$, $-CH_2CH_2CH_2-$ or $-CH_2CH_2O-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, (T)$_n$-$C_3$-$C_6$cycloalkyl, (T)$_n$-$C_3$-$C_6$cycloalkyl substituted by $C_1$-$C_3$alkyl-OOC, (T)$_n$-phenyl, or (T)$_n$-phenyl substituted in the phenyl moiety by halogen, hydroxy, methyl, methoxy, $CF_3$, cyano, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_3$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
and in a compound of formula I the organic radical A has a molecular weight of less than 900; or in the case where U is oxygen or sulfur, a salt of the phytophysiologically tolerable 7-carboxylic acid with a primary, secondary or tertiary amine or with an inorganic base.

**3.** A process according to claim 1 or claim 2, wherein the application rate is less than 1 kg of active ingredient/hectare.

**4.** A process according to claim 2, wherein there is used a compound of formula I wherein:
X and Y are hydrogen;
Z is cyano or COA;
A is UR or $U^1N(=C)_n(R_3)R_4$;
U is oxygen;
$U^1$ is oxygen or $-N(R_5)-$;

R is hydrogen, $C_1$-$C_8$alkyl, $C_1$-$C_8$alkyl substituted by halogen or by $C_1$-$C_3$alkoxy, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, benzyl, halogenated benzyl, methoxybenzyl, $(T)_n$-$Si(CH_3)_3$, (T)-P(O)($C_1$-$C_4$alkyl)$CH_3$, (T)-P(O)($OC_1$-$C_4$alkyl)$_2$ or the group $(T)_n$-W;
W is a 5- to 7-membered saturated or unsaturated unsubstituted heterocycle having from 1 to 3 hetero atoms from the group O, N and S, or is diacetone-D-glucosidyl;
T is a bridge member $-CH_2-$, $-CH_2CH_2-$, $-CH(CH_3)-$, $-CCH_3(CH_3)-$ or $-CH_2CH_2CH_2-$;
$R_3$ is hydrogen, cyano, $C_1$-$C_6$alkyl, phenyl or W;
$R_4$ is hydrogen, $C_1$-$C_6$alkyl, $CONH_2$, CONH-CONH-$C_1$-$C_3$alkyl, $C_1$-$C_3$alkanoyl or $C_3$-$C_5$alkenoyl;
$R_3$ and $R_4$ together with the atom to which they are bonded are W or W';
W is a 5- to 7-membered saturated or unsaturated heterocycle having from 1 to 3 hetero atoms from the group O, N and S;
W' is a radical from the group:

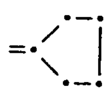 , 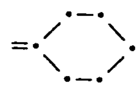 and 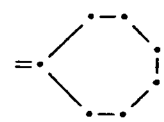 ;

n is 0 or 1.

**5.** A process according to claim 2, wherein there is used a compound of formula I'

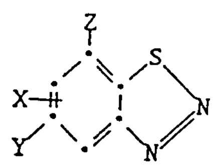 (I')

wherein:
X is hydrogen, halogen, hydroxy, methyl, methoxy, HOOC or MOOC;
Y is hydrogen, halogen, $SO_3H$, $SO_3M$, nitro, hydroxy or amino;
Z is cyano or COA;
A is UR, $N(R_1)R_2$ or $U^1N(=C)_n(R_3)R_4$;
M is the molar equivalent of an alkali metal or alkaline earth metal ion that has been formed from a corresponding base or basic compound;
U is oxygen or sulfur;
$U^1$ is oxygen or $-N(R_5)-$;
R is hydrogen, $C_1$-$C_8$alkyl, $C_1$-$C_8$alkyl substituted by halogen, cyano, nitro, hydroxy or by U-$C_1$-$C_3$alkyl, (T)-COOH or (T)-$COOC_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

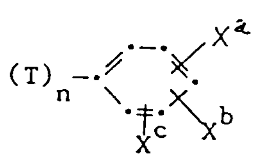 , $(T)_n$-naphth, $(T)_n$-$Si(C_1$-$C_8alkyl)_3$,

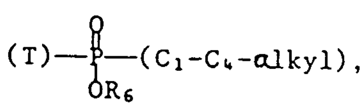 , 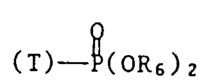 ; and $(T)_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;
or $X^a$ is $C_1$-$C_2$haloalkoxy having up to 5 halogen atoms, nitro, dimethylamino, phenyl, phenoxy, benzyloxy or sulfamoyloxy and $X^b$ and $X^c$ are both hydrogen; or
$X^a$ is phenyl, phenoxy or benzyloxy and $X^b$ is halogen or methyl and $X^c$ is hydrogen; or
$X^a$, $X^b$ and $X^c$ together are 4 or 5 fluorine atoms;
naphth is a naphthyl radical that is unsubstituted or is substituted by halogen, methyl, methoxy or by nitro;
W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CH_2CH_2CH_2-$ or $-CH_2CH_2O-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, or a 3- to 6-membered saturated or unsaturated heterocycle having O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
with the exception of the compounds:
7-cyanobenzo-1,2,3-thiadiazole;
4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid.

6. A process according to claim 2, wherein there is used a compound of formula I'

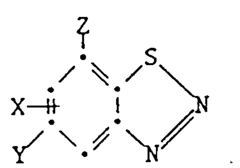

(I')

wherein:
X is hydrogen, halogen, hydroxy, methyl, methoxy, HOOC or MOOC;
Y is hydrogen, halogen, $SO_3H$, $SO_3M$, nitro, hydroxy or amino;
Z is cyano or COA;
A is UR, $N(R_1)R_2$ or $U^1N(=C)_n(R_3)R_4$;
M is the molar equivalent of an alkali metal or alkaline earth metal ion that has been formed from a

corresponding base or basic compound;
U is oxygen or sulfur;
$U^1$ is oxygen or $-N(R_5)-$;
R is hydrogen, $C_1$-$C_8$alkyl, $C_1$-$C_8$alkyl that is substituted by halogen, cyano, nitro, hydroxy, alkoxy or by U-$C_1$-$C_3$alkyl, (T)-COOH or (T)-COO$C_1$-$C_4$alkyl, $C_2$-$C_6$alkenyl, halo-substituted $C_3$-$C_6$alkenyl, $C_3$-$C_6$-alkynyl, halo-substituted $C_3$-$C_6$alkynyl, $(T)_n$-$C_3$-$C_8$cycloalkyl, or a group selected from the following:

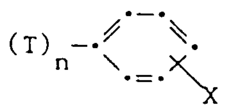 , $(T)_n$-naphthyl, $(T)_n$-Si($C_1$-$C_8$alkyl$)_3$,

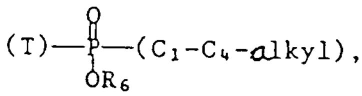 , 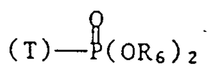 ; and $(T)_n$—W;

W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$ or $-CH(CH_3)-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, or a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_3$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
with the exception of the compounds:
7-cyanobenzo-1,2,3-thiadiazole;
4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid.

7. A process according to claim 2, wherein a compound of formula I' according to claim 5 is used wherein Z = COA and R has a meaning other than hydrogen and unsubstituted alkyl.

8. A process according to claim 2, wherein 1,2,3-benzothiadiazole-7-carboxylic acid or a salt thereof with a basic compound is used as active ingredient.

9. A process according to claim 8, wherein the salt used is one with a primary, secondary or tertiary amine or an alkali metal or alkaline earth metal compound.

**10.** A process according to claim 2, wherein 7-methoxycarbonylbenzo-1,2,3-thiadiazole is used as active ingredient.

**11.** A process according to claim 2, wherein 7-benzyloxycarbonylbenzo-1,2,3-thiadiazole is used as active ingredient.

**12.** A process according to claim 2, wherein 7-cyanobenzo-1,2,3-thiadiazole is used as active ingredient.

**13.** A process according to claim 2, wherein a compound of 7-$C_2$-$C_4$alkoxycarbonylbenzo-1,2,3-thiadiazole is used as active ingredient.

**14.** A composition for the immunisation of plants against attack by phytopathogenic microorganisms, comprising as active ingredient at least one compound of formula I defined in claim 1 or claim 2 with the exception of the compounds 7-cyanobenzo-1,2,3-thiadiazole; 4-chloro-7-cyanobenzo-1,2,3-thiadiazole; 6-chloro-7-cyanobenzo-1,2,3-thiadiazole; 4,6-dibromo-7-cyanobenzo-1,2,3-thiadiazole and 7-methoxycarbonylbenzo-1,2,3-thiadiazole.

**15.** Benzo-1,2,3-thiadiazole-7-carboxylic acid methyl ester for use in a process for the immunisation of plants against attack by phytopathogenic microorganisms.

**16.** Benzo-1,2,3-thiadiazole-7-carboxylic acid methyl ester for use in the preparation of a composition for the immunisation of plants against attack by phytopathogenic microorganisms.

**17.** A composition for the immunisation of plants against attack by phytopathogenic microorganisms comprising as active ingredient at least one compound of formula I'

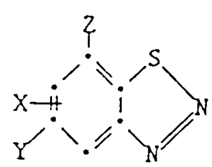

(I')

wherein:
X is hydrogen, halogen, hydroxy, methyl, methoxy, HOOC or MOOC;
Y is hydrogen, halogen, $SO_3H$, $SO_3M$, nitro, hydroxy or amino;
Z is cyano or COA;
A is UR, $N(R_1)R_2$ or $U^1N(=C)_n(R_3)R_4$;
M is the molar equivalent of an alkali metal or alkaline earth metal ion that has been formed from a corresponding base or basic compound;
U is oxygen or sulfur;
$U^1$ is oxygen or $-N(R_5)-$;
R is hydrogen, $C_1$-$C_8$alkyl, $C_1$-$C_8$alkyl that is substituted by halogen, cyano, nitro, hydroxy or by U-$C_1$-$C_3$alkyl, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

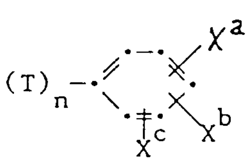

, $(T)_n$-naphth, $(T)_n$-Si($C_1$-$C_8$alkyl)$_3$,

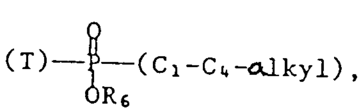

,

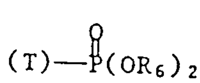

; and $(T)_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;
or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or
$X^a$ is phenyl, phenoxy or benzyloxy and $X^b$ is halogen or methyl and $X^c$ is hydrogen; or
$X^a$, $X^b$ and $X^c$ together are 4 or 5 fluorine atoms;
naphth is a naphthyl radical that is unsubstituted or is substituted by halogen, methyl, methoxy or by nitro;
W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CH_2CH_2CH_2-$ or $-CH_2CH_2O-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl, or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
$\underline{n}$ is 0 or 1;
with the exception of the compounds:
7-cyanobenzo-1,2,3-thiadiazole;
4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid.

**18.** A composition for the immunisation of plants against attack by phytopathogenic microorganisms comprising as active ingredient at least one compound of formula I'

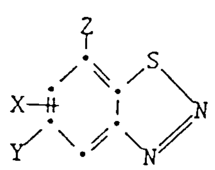

(I')

wherein:
X is hydrogen, halogen, hydroxy, methyl, methoxy, HOOC or MOOC;
Y is hydrogen, halogen, $SO_3H$, $SO_3M$, nitro, hydroxy or amino;
Z is cyano or COA;
A is UR, $N(R_1)R_2$ or $U^1N(=C)_n(R_3)R_4$;
M is the molar equivalent of an alkali metal or alkaline earth metal ion that has been formed from a

corresponding base or basic compound;
U is oxygen or sulfur;
$U^1$ is oxygen or $-N(R_5)-$;
R is hydrogen, $C_1$-$C_8$alkyl, $C_1$-$C_8$alkyl that is substituted by halogen, cyano, nitro, hydroxy, alkoxy or by U-$C_1$-$C_3$alkyl, (T)-COOH or (T)-$COOC_1$-$C_4$alkyl, $C_2$-$C_6$alkenyl, halo-substituted $C_3$-$C_6$alkenyl, $C_3$-$C_6$-alkynyl, halo-substituted $C_3$-$C_6$alkynyl, $(T)_n$-$C_3$-$C_8$cycloalkyl, or a group selected from the following:

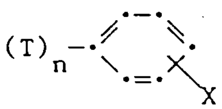 , $(T)_n$-naphthyl, $(T)_n$-$Si(C_1$-$C_8alkyl)_3$,

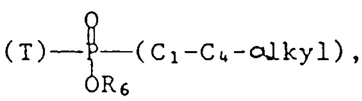 , 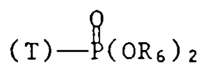 ; and $(T)_n$—W;

W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$ or $-CH(CH_3)-$;
$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, or a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_3$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
n is 0 or 1;
with the exception of the compounds:
7-cyanobenzo-1,2,3-thiadiazole;
4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid.

**19.** A composition according to claim 14, comprising as active ingredient at least one compound of formula I' according to claim 17, wherein Z = COA and R has a meaning other than hydrogen and unsubstituted alkyl.

**20.** A composition according to claim 14, comprising as active ingredient at least one compound of formula I' from the group:
7-n-pentyloxycarbonylbenzo-1,2,3-thiadiazole;
7-(4-methoxybenzyloxycarbonyl)-benzo-1,2,3-thiadiazole;

7-(cycloheximino-oxycarbonyl)-benzo-1,2,3-thiadiazole;
7-(3-hydroxy-n-propoxycarbonyl)-benzo-1,2,3-thiadiazole;
1,2,5,6-di-O-isopropylidene-3-(7-benzo-1,2,3-thiadiazoyl)-D-glucofuranose;
7-furfuryloxycarbonylbenzo-1,2,3-thiadiazole;
7-(1,2,4-triazol-1-yl)-methoxycarbonylbenzo-1,2,3-thiadiazole;
7-(2-pyridylmethoxycarbonyl)-benzo-1,2,3-thiadiazole;
7-trimethylsilylmethoxycarbonylbenzo-1,2,3-thiadiazole;
7-[2-(trimethylsilyl)-ethoxycarbonyl]-benzo-1,2,3-thiadiazole;
7-dimethylphosphono-ethoxycarbonylbenzo-1,2,3-thiadiazole;
7-cyclohexyloxycarbonylbenzo-1,2,3-thiadiazole;
7-(1-phenethyloxycarbonyl)-benzo-1,2,3-thiadiazole;
7-(3-methoxybenzyl)-benzo-1,2,3-thiadiazole;
7-(ethylthiocarbonyl)-benzo-1,2,3-thiadiazole;
7-(n-propylthiocarbonyl)-benzo-1,2,3-thiadiazole;
7-(benzylthiocarbonyl)-benzo-1,2,3-thiadiazole;
7-carbamoylbenzo-1,2,3-thiadiazole;
7-N-phenylcarbamoylbenzo-1,2,3-thiadiazole;
N-(7-benzo-1,2,3-thiadiazoyl)-glycine;
7-(N-diallylcarbamoyl)-benzo-1,2,3-thiadiazole;
6-fluoro-7-methoxycarbonylbenzo-1,2,3-thiadiazole;
6-fluoro-7-carboxybenzo-1,2,3-thiadiazole;
5-fluoro-7-benzyloxycarbonylbenzo-1,2,3-thiadiazole;
5-fluoro-7-carboxybenzo-1,2,3-thiadiazole;
5-fluoro-7-ethoxycarbonylbenzo-1,2,3-thiadiazole;
or from the group:
7-ethoxycarbonylbenzo-1,2,3-thiadiazole;
7-n-propoxycarbonylbenzo-1,2,3-thiadiazole;
7-isopropoxycarbonylbenzo-1,2,3-thiadiazole;
7-n-butoxycarbonylbenzo-1,2,3-thiadiazole;
7-sec-butoxycarbonylbenzo-1,2,3-thiadiazole;
7-tert-butoxycarbonylbenzo-1,2,3-thiadiazole;
7-cyclopropylmethoxycarbonylbenzo-1,2,3-thiadiazole;
7-(2'-phenethoxycarbonyl)-benzo-1,2,3-thiadiazole;
7-benzyloxycarbonylbenzo-1,2,3-thiadiazole;
7-allyloxycarbonylbenzo-1,2,3-thiadiazole;
7-propyn-2-yloxycarbonylbenzo-1,2,3-thiadiazole;
2-(7-benzo-1,2,3-thiadiazolyloxy-imino)-2-cyano-N-(ethylaminocarbonyl)-acetamide;
sodium salt of benzo-1,2,3-thiadiazole-7-carboxylic acid;
potassium salt of benzo-1,2,3-thiadiazole-7-carboxylic acid;
triethylammonium salt of benzo-1,2,3-thiadiazole-7-carboxylic acid;
7-(1'-phenethoxycarbonyl)-benzo-1,2,3-thiadiazole;
7-(1'-naphthylmethoxycarbonyl)-benzo-1,2,3-thiadiazole;
7-(methylthiocarbonyl)-benzo-1,2,3-thiadiazole;
7-(ethylthiocarbonyl)-benzo-1,2,3-thiadiazole;
7-(benzylthiocarbonyl)-benzo-1,2,3-thiadiazole;
7-[(dicyanomethyl)-aminocarbonyl]-benzo-1,2,3-thiadiazole;
3-[(N-benzo-1,2,3-thiadiazol-7-ylcarbonyl)-1,3,4-thiadiazol-2-ylamino]-2-methylpropenecarboxylic acid methyl ester;
3-[(N-benzo-1,2,3-thiadiazol-7-ylcarbonyl)-1,3,4-thiadiazol-2-ylamino]-2-ethylpropenecarboxylic acid methyl ester;
1-(benzo-1,2,3-thiadiazole-7-carbonyl)-2-(α-methylpropylidene)-hydrazine;
1-(benzo-1,2,3-thiadiazole-7-carbonyl)-2-(cyclobutylidene)-hydrazine;
1-(benzo-1,2,3-thiadiazole-7-carbonyl)-2-(cyclopentylidene)-hydrazine;
1-(benzo-1,2,3-thiadiazole-7-carbonyl)-2-(cyclohexylidene)-hydrazine;
2-(benzo-1,2,3-thiadiazole-7-carbonyl)-1-(2'-sec-butyl)-hydrazine;
1-(benzo-1,2,3-thiadiazole-7-carbonyl)-2-(cyclopentyl)-hydrazine;
1-(benzo-1,2,3-thiadiazole-7-carbonyl)-2-(cyclohexyl)-hydrazine;
1-(benzo-1,2,3-thiadiazole-7-carbonyl)-2-(cycloheptyl)-hydrazine;

1-(benzo-1,2,3-thiadiazole-7-carbonyl)-1,2-diacetylhydrazine;
1-(benzo-1,2,3-thiadiazole-7-carbonyl)-2-phenylhydrazine;
1-(benzo-1,2,3-thiadiazole-7-carbonyl)-2-pyridin-2'-yl-hydrazine.

**21.** A composition according to claim 14, comprising as active ingredient at least one of the compounds: benzo-1,2,3-thiadiazole-7-carboxylic acid and 7-methoxycarbonylbenzo-1,2,3-thiadiazole.

**22.** A composition according to claim 14, comprising as active ingredient 1,2,3-benzothiadiazole-7-carboxylic acid or a salt thereof with a basic compound.

**23.** A composition according to claim 22, wherein the salt used is one with a primary, secondary or tertiary amine or an alkali metal or alkaline earth metal compound.

**24.** A process for the preparation of a compound of formula I'

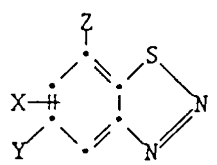

(I')

wherein:
X is hydrogen, halogen, hydroxy, methyl, methoxy, HOOC or MOOC;
Y is hydrogen, halogen, $SO_3H$, $SO_3M$, nitro, hydroxy or amino;
Z is cyano or COA;
A is UR, $N(R_1)R_2$ or $U^1N(=C)_n(R_3)R_4$;
M is the molar equivalent of an alkali metal or alkaline earth metal ion that has been formed from a corresponding base or basic compound;
U is oxygen or sulfur;
$U^1$ is oxygen or $-N(R_5)-$;
R is hydrogen, $C_1$-$C_8$alkyl, $C_1$-$C_8$alkyl that is substituted by halogen, cyano, nitro, hydroxy or by U-$C_1$-$C_3$alkyl, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

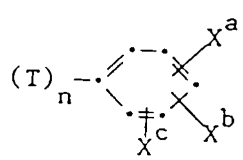

, $(T)_n$-naphth, $(T)_n$-Si($C_1$-$C_8$alkyl$)_3$,

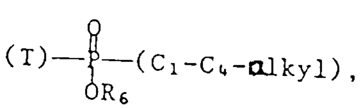

,

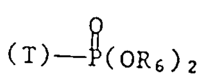

; and $(T)_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;
or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or
$X^a$ is phenyl, phenoxy or benzyloxy and $X^b$ is halogen or methyl and $X^c$ is hydrogen; or
$X^a$, $X^b$ and $X^c$ together are 4 or 5 fluorine atoms;
naphth is a naphthyl radical that is unsubstituted or is substituted by halogen, methyl, methoxy or by nitro;
W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;
T is a bridge member $-CH_2-$, $-CH_2CH_2-$, $-CH(CH_3)-$, $-CH_2CH_2CH_2-$ or $-CH_2CH_2O-$;

$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl, or $(T)_n$-phenyl substituted in the phenyl moiety by halogen, hydroxy, methyl, $CF_3$, cyano, methoxy, HOOC or by MOOC;
$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;
$R_1$ and $R_2$ together with the atom to which they are bonded are a heterocycle W;
$R_3$ is hydrogen, cyano, $C_1$-$C_6$alkyl, phenyl, phenyl substituted by halogen, hydroxy, methyl, methoxy, HOOC or by MOOC, or a heterocycle W;
$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;
$R_3$ and $R_4$ together with the atom to which they are bonded are a heterocycle W or a carbocyclic ring W';
W' is a carbocyclic radical having from 3 to 7 ring carbon atoms;
$R_5$ is hydrogen or methyl;
$R_6$ is hydrogen or $C_1$-$C_4$alkyl; and
$\underline{n}$ is 0 or 1;
with the exception of the compounds:
7-cyanobenzo-1,2,3-thiadiazole;
4-chloro-7-cyanobenzo-1,2,3-thiadiazole;
4,6-dibromo-7-cyanobenzo-1,2,3-thiadiazole;
benzo-1,2,3-thiadiazole-7-carboxylic acid;
benzo-1,2,3-thiadiazole-7-carboxylic acid methyl ester;
6-chloro-7-cyanobenzo-1,2,3-thiadiazole;
6-chlorobenzo-1,2,3-thiadiazole-7-carboxylic acid;
in which process

1. a compound of formula Ia

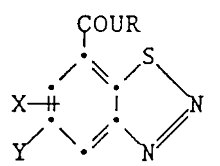

(Ia)

in which R, X, Y and U have the meanings given under formula I, is prepared by reacting:

1.1 a compound of formula II

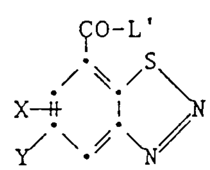

(II)

in which L' is a leaving group, for example halogen, O-acyl, for example the acyl radical belonging to the symmetric anhydride of acid Ib, or 1-imidazoyl, with a compound of formula III

RUH (III)

a) in an excess of the reactant RUH or
b) in the presence of an organic base either with or without 4-dialkylaminopyridine as catalyst in an inert solvent or
c) in the presence of an inorganic base,
the reaction in each case being carried out in a temperature range of from -10° to 180°C,

preferably from 0° to 100°C; or

1.2 a compound of formula Ib

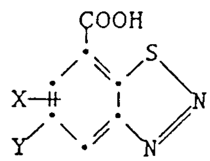

(Ib)

with a compound of formula III in excess or in an inert solvent in the presence of an acid, such as sulfuric acid, hydrochloric acid, p-toluenesulfonic acid or boron trifluoride/diethyl ether complex, or in the presence of dicyclohexylcarbodiimide at a temperature of from -10° to 180°C, preferably from 0° to 140°C; and

2. a compound of formula Ic

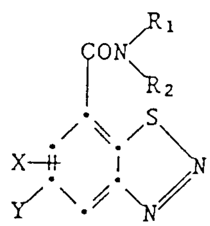

(Ic)

in which the symbols $R_1$, $R_2$, X and Y have the meanings given under formula I, is prepared by reacting:

2.1 a compound of formula II with a compound of formula IV

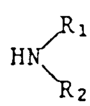

(IV)

a) in an excess of the reactant $HN(R_1)R_2$ or

b) in the presence of an organic base either with or without 4-dialkylaminopyridine as catalyst in an inert solvent or

c) in the presence of an inorganic base,

the reaction in each case being carried out in a temperature range of from -10° to 160°C, preferably from 0° to 100°C; and

3. a compound of formula Id

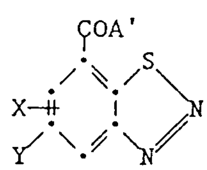

(Id),

in which A' is the radical $U^1N(=C)_n(R_3)R_4$ and X, Y, $R_3$, $R_4$, $R_5$ and <u>n</u> have the meanings given under formula I, is prepared by:

3.1 reacting a compound of formula Ie

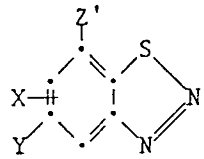 (Ie)

in which Z' is a group COOH, COCl, COOAlk[1] or an acyloxycarbonyl radical, for example

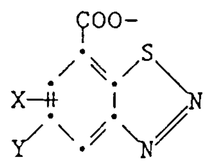 ,

benzoyloxycarbonyl or acetoxycarbonyl, in which Alk[1] is $C_1$-$C_4$alkyl, in the presence of a base, with a hydrazine derivative of formula V or VI

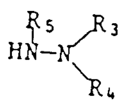 (V) or 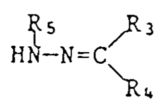 (VI)

in an inert solvent at a temperature of from -10° to 180°C, preferably from 0° to 100°C; or

3.2 reacting a compound of formula Ie stepwise first with hydrazine, and then reacting the resulting hydrazine compound

3.2.1 with the alkylating agent $R_3$-L or $R_4$-L in which L is a leaving group, in an inert solvent at a temperature of from 0° to 160°C, preferably from 20° to 120°C; or

3.2.2 with an aldehyde or ketone of formula $R_3(R_4)C=O$ in which $R_3$ and $R_4$ have the meanings given under formula I, with or without the addition of an organic or inorganic acid, at a temperature of from -10° to 150°C, preferably from 20° to 100°C, and subsequently, if desired,

3.2.3 with an alkylating agent L-$R_5$ in which L is a leaving group, in the presence of a strong base in an inert solvent at a temperature of from -80° to 120°C, preferably from -40° to 80°C; or, if desired,

3.2.4 a) hydrogenating the hydrazone derivative prepared under (3.2.1) with hydrogen at a pressure of from 1 to $30x10^5$ Pa in the presence of a catalyst in admixture with activated carbon in an inert solvent at a temperature of from 0° to 100°C, or

3.2.4 b) treating the hydrazone derivative prepared under (3.2.1) with a complex metal hydride, for example sodium cyanoborohydride, in an inert solvent at a temperature of from -10° to 80°C, preferably from 0° to 50°C; and

4. a compound of formula If

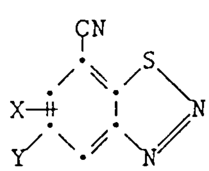 (If),

in which the symbols X and Y have the meanings given under formula I, is prepared by treating a compound of formula Ig [prepared according to process (2)]

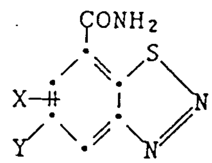

(Ig)

with a dehydrating agent in an inert solvent or without a solvent at a temperature of from -10° to 250°C, suitable dehydrating agents being:

a) trifluoroacetic acid anhydride in the presence of a base, for example pyridine, in an inert solvent, for example tetrahydrofuran or dioxane, at a temperature of from -10° to 40°C; or

b) chlorosulfonyl isocyanate in an inert solvent, for example tetrahydrofuran, at a temperature of from 0° to 65°C, with subsequent treatment with dimethylformamide;

c) phosphorus pentoxide with or without an inert solvent, for example 1,2-dichloroethane, xylene or chlorobenzene, optionally in a bomb tube under elevated pressure, at from 50° to 250°C;

5.1 a compound of formula $IL^1$

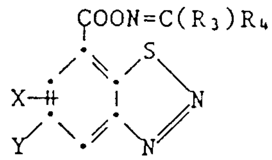

($IL^1$)

in which $R_3$, $R_4$, X and Y have the meanings given under formula I, is prepared by reacting an oxime derivative of formula

$HO\text{-}N=C(R_3)R_4$

with an activated acid derivative of formula

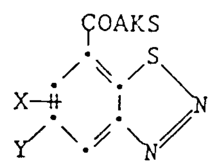

,

in which AKS is a halogen, an O-acyl, for example the O-acyl radical of the free acid of the formula above, acetoxy or benzoyloxy, or 1-imidazolyl, in an inert solvent and a base at from -20°C to 120°C, preferably from 0° to 50°C, or by reacting the free acid (= Ib) in the presence of dicyclohexylcarbodiimide under the same conditions;

5.2 a compound of formula $IL^2$

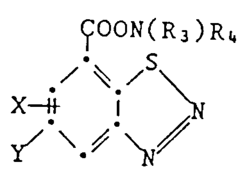

($IL^2$)

in which $R_3$, $R_4$, X and Y have the meanings given under formula I, is prepared by reduction of a compound of formula $IL^1$

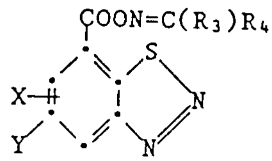

(IL[1])

a) with a silane, for example triethylsilane, in the presence of an acid, for example trifluoroacetic acid, at from 0° to 80°C, or

b) with sodium cyanoborohydride in the presence of an organic acid, for example acetic acid, at from 0° to 80°C, or

c) by catalytic methods, for example with Pt/$H_2$.

**25.** A process for the preparation of a compound of formula Ik

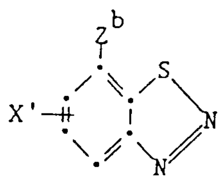

(Ik)

by diazotisation of a compound of formula XI'

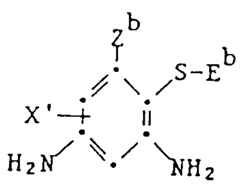

(XI')

in an acidic medium with a nitrite compound at from -40° to 30°C and, in the same reaction vessel or in a second reaction vessel, treatment with a reducing agent at from -40° to 80°C, preferably at from -30° to 30°C, the reducing agent being added before, after or at the same time as the nitrite compound, the substituents of the compounds having the following meanings:

X' is hydrogen, halogen, methyl, methoxy or COOH;

$E^b$ is a readily removable group, for example hydrogen, $C_1$-$C_{16}$alkyl, for example methyl, ethyl, isopropyl, n-dodecyl, or benzyl or acyl, for example acetyl or the sulfonic acid radical (-$SO_3$H-) or cyano, or as part of a disulfide bridge, is a second radical

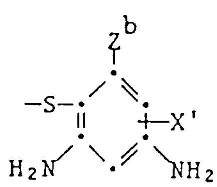

;

$Z^b$ is cyano or COA;

A is UR, $N(R_1)R_2$ or $U^1N(=C)_n(R_3)R_4$;

M is the molar equivalent of an alkali metal or alkaline earth metal ion that has been formed from a corresponding base or basic compound;

U is oxygen or sulfur;

$U^1$ is oxygen or -$N(R_5)$-;

R is hydrogen, $C_1$-$C_8$alkyl, $C_1$-$C_8$alkyl that is substituted by halogen, cyano, nitro, hydroxy or by U-$C_1$-$C_3$alkyl, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

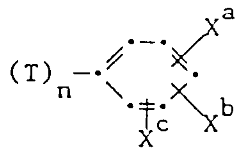 , $(T)_n$-naphth, $(T)_n$-Si($C_1$-$C_8$alkyl)$_3$,

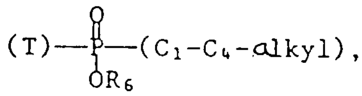 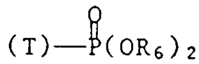 ; and $(T)_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;

or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or

$X^a$ is phenyl, phenoxy or benzyloxy and $X^b$ is halogen or methyl and $X^c$ is hydrogen; or

$X^a$, $X^b$ and $X^c$ together are 4 or 5 fluorine atoms;

naphth is a naphthyl radical that is unsubstituted or is substituted by halogen, methyl, methoxy or by nitro;

W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;

T is a bridge member $-CH_2-$, $-CH_2CH_2-$, $-CH(CH_3)-$, $-CH_2CH_2CH_2-$ or $-CH_2CH_2O-$;

$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl, or $(T)_n$-phenyl substituted in the phenyl moiety by halogen, hydroxy, methyl, $CF_3$, cyano, methoxy, HOOC or by MOOC;

$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;

$R_1$ and $R_2$ together with the atom to which they are bonded are a heterocycle W;

$R_3$ is hydrogen, cyano, $C_1$-$C_6$alkyl, phenyl, phenyl substituted by halogen, hydroxy, methyl, methoxy, HOOC or by MOOC, or a heterocycle W;

$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;

$R_3$ and $R_4$ together with the atom to which they are bonded are a heterocycle W or a carbocyclic ring W';

W' is a carbocyclic radical having from 3 to 7 ring carbon atoms;

$R_5$ is hydrogen or methyl;

$R_6$ is hydrogen or $C_1$-$C_4$alkyl; and

n is 0 or 1;

with the exception of primary or secondary amino groups, UH or nitro groups, Si($C_1$-$C_8$alkyl)$_3$ or phosphorus-containing radicals.

**26.** A process for the preparation of a compound of formula

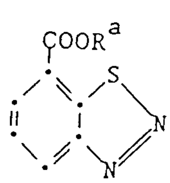

by diazotisation of a compound of formula

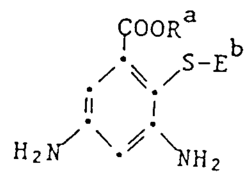

in an acidic medium with a nitrite compound at from -20° to 30°C, and in the same reaction vessel treatment with a reducing agent at from -20° to 80°C, preferably at from 20° to 30°C, the reducing agent being added before, after or at the same time as the nitrite compound, the substituents of the compounds having the following meanings:

$R^a$ is hydrogen, $C_1$-$C_8$alkyl, $C_1$-$C_8$alkyl that is substituted by halogen, cyano, nitro, hydroxy or by U-$C_1$-$C_3$alkyl, (T)-COOH or (T)-COO$C_1$-$C_4$alkyl, $C_3$-$C_6$alkenyl, halo-substituted $C_3$-$C_6$alkenyl, $C_3$-$C_6$alkynyl, halo-substituted $C_3$-$C_6$alkynyl, $(T)_n$-$C_3$-$C_8$cycloalkyl, or a group selected from the following:

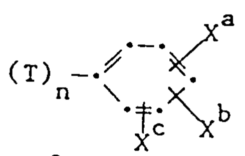 , $(T)_n$-naphth, $(T)_n$-Si($C_1$-$C_8$alkyl)$_3$,

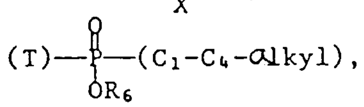 , 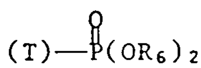 ; and $(T)_n$—W;

each of $X^a$, $X^b$ and $X^c$, independently of the others, is hydrogen, halogen, hydroxy, cyano, HOOC, MOOC, $C_1$-$C_3$alkyl-OOC, $C_1$-$C_4$alkyl, $C_1$-$C_4$alkoxy, $C_1$-$C_2$haloalkyl having up to 5 halogen atoms, especially fluorine atoms;

or $X^a$ is $C_1$-$C_2$haloalkoxy having up to 5 halogen atoms, nitro, dimethylamino, phenyl, phenoxy, benzyloxy, sulfamoyloxy and $X^b$ and $X^c$ are both hydrogen; or

$X^a$ is phenyl, phenoxy or benzyloxy and $X^b$ is halogen or methyl and $X^c$ is hydrogen; or

$X^a$, $X^b$ and $X^c$ together are 4 or 5 fluorine atoms;

naphth is a naphthyl radical that is unsubstituted or is substituted by halogen, methyl, methoxy or by nitro;

W is a 5- to 7-membered saturated or unsaturated heterocycle having from 1 to 3 hetero atoms from the group O, N and S that is unsubstituted or is substituted by halogen, trifluoromethyl, cyano, $C_1$-$C_2$alkyl or by a $C_1$-$C_2$alkoxycarbonyl-$C_2$-$C_4$alkyleneimino radical, or is a monosaccharide radical;

T is a bridge member $-CH_2-$, $-CH_2CH_2-$, $-CH(CH_3)-$, $-CH_2CH_2CH_2-$ or $-CH_2CH_2O-$;

$R_1$ is hydrogen, $C_1$-$C_5$alkyl, $C_1$-$C_5$alkyl interrupted by an oxygen or sulfur atom, $C_1$-$C_5$alkyl substituted by halogen, cyano, HOOC or by $C_1$-$C_2$alkyl-OOC, $C_1$-$C_5$alkyl interrupted by an oxygen or sulfur atom and substituted by halogen, cyano, HOOC or by $C_1$-$C_2$alkyl-OOC, $C_3$-$C_5$alkenyl, $C_3$-$C_5$alkenyl substituted by $C_1$-$C_3$alkyl-OOC, $C_3$-$C_5$alkynyl, $C_3$-$C_5$alkynyl substituted by $C_1$-$C_3$alkyl-OOC, $(T)_n$-$C_3$-$C_6$cycloalkyl, $(T)_n$-$C_3$-$C_6$cycloalkyl substituted by $C_1$-$C_3$alkyl-OOC, $(T)_n$-phenyl, or $(T)_n$-phenyl substituted in the phenyl moiety by halogen, hydroxy, methyl, $CF_3$, cyano, methoxy, HOOC or by MOOC;

$R_2$ is hydrogen, hydroxy, $C_1$-$C_3$alkyl, $C_1$-$C_3$alkyl substituted by cyano or by $C_1$-$C_3$alkoxy, $C_1$-$C_4$alkoxy, a 3- to 6-membered saturated or unsaturated heterocycle containing O, N or S as hetero atoms;

$R_1$ and $R_2$ together with the atom to which they are bonded are a heterocycle W;

$R_3$ is hydrogen, cyano, $C_1$-$C_6$alkyl, phenyl, phenyl substituted by halogen, hydroxy, methyl, methoxy, HOOC or by MOOC, or a heterocycle W;

$R_4$ is hydrogen, $C_1$-$C_6$alkyl, $CONH_2$, CONH-CONH-$C_1$-$C_3$alkyl, $C_1$-$C_6$alkanoyl, $C_1$-$C_3$alkanoyl substituted by halogen or by $C_1$-$C_3$alkoxy, $C_3$-$C_5$alkenoyl, or $C_3$-$C_5$alkenoyl substituted by halogen or by $C_1$-$C_3$alkoxy;

$R_3$ and $R_4$ together with the atom to which they are bonded are a heterocycle W or a carbocyclic ring W';

W' is a carbocyclic radical having from 3 to 7 ring carbon atoms;

$R_5$ is hydrogen or methyl;

$R_6$ is hydrogen or $C_1$-$C_4$alkyl; and

n is 0 or 1;

with the exception of radicals containing UH or nitro groups and silicon- or phosphorus-containing

radicals, and
$E^b$ is a readily removable group, for example hydrogen, $C_1$-$C_{16}$alkyl, for example methyl, ethyl, isopropyl, n-dodecyl, or benzyl or acyl, for example acetyl or the sulfonic acid radical ($-SO_3H-$) or cyano.

**27.** The use of a compound of formula I according to claim 1 or claim 2, for the immunisation of plants against diseases.

**28.** Use of a compound of formula I' according to claim 20 for the immunisation of plants against diseases.

**29.** Use of 7-methoxycarbonylbenzo-1,2,3-thiadiazole as active ingredient in the preparation of a composition for the immunisation of plants against attack by phytopathogenic microorganisms.

**Revendications**
**Revendications pour les Etats contractants suivants : AT, BE, CH, DE, FR, GB, GR, IT, LI, LU, NL, SE**

**1.** Procédé d'immunisation de végétaux contre une infestation par des micro-organismes phytopathogènes, caractérisé en ce qu'on applique sur les végétaux et/ou sur leur environnement, comme matière active, un composé ayant la formule I ci-après :

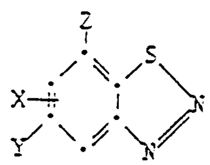

(I)

dans laquelle :

X est un hydrogène, un halogène ou un radical hydroxy, méthyle, méthoxy, HOOC ou MOOC ;

Y est un hydrogène, un halogène ou un radical $SO_3H$, $SO_3M$, nitro, hydroxy ou amino, M étant l'équivalent molaire d'un ion d'un métal alcalin ou alcalino-terreux, formé à partir d'une base ou d'un composé basique correspondants ; et

Z est le radical cyano ou -CO-A, où A est OH ou SH, H pouvant aussi être remplacé par l'équivalent molaire d'un résidu cationique minéral ou organique.

**2.** Procédé d'immunisation de végétaux contre une infestation par des micro-organismes phytopathogènes, caractérisé en ce qu'on applique sur les végétaux et/ou sur leur environnement, comme matière active, un composé ayant la formule I ci-après :

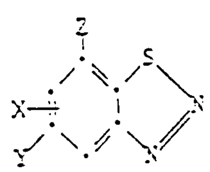

(I)

dans laquelle :

X est un hydrogène, un halogène ou un radical hydroxy, méthyle, méthoxy, HOOC ou MOOC ;

Y est un hydrogène, un halogène ou un radical $SO_3H$, $SO_3M$, nitro, hydroxy ou amino, M étant l'équivalent molaire d'un ion d'un métal alcalin ou alcalino-terreux, formé à partir d'une base ou d'un composé basique correspondants ; et

Z est le radical cyano ou -CO-A,

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par

halogène, cyano, nitro, hydroxy, U-(alkyle en $C_1$-$C_3$) ou di-(alkyle en $C_2$-$C_4$)-amino ou interrompu par le groupe CO ; (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

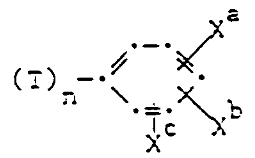

, $(T)_n$-Napht, $(T)_n$-Si($C_1$-$C_8$-Alkyl)$_3$,

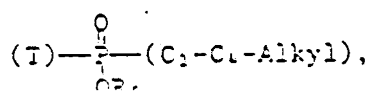

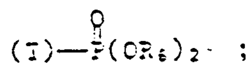

; ou $(T)_n$-W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyleoxy, et $X^b$ et $X^c$ sont simultanément des atomes d'hydrogène : ou bien

$X^a$ est le radical phényle, phényloxy ou benzyloxy, et $X^b$ est un halogène ou le radical méthyle et $X^c$ est un hydrogène ; ou bien

$X^a$, $X^b$ et $X^c$ sont ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CCH_3(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$ ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le cycle ;

$R_5$ est un hydrogène ou le radical méthyle ;

$R_6$ est un hydrogène ou un radical alkyle en $C_1$-$C_4$ ; et

n vaut zéro ou 1 ;

où dans les composés de formule I, le résidu organique A a une masse moléculaire inférieure à 900 ; et, quand U est un oxygène ou un soufre, ces composés englobent les sels de l'acide 7-carboxylique, compatibles du point de vue phytosanitaire, avec des amines primaires, secondaires ou tertiaires ou des bases minérales.

**3.** Procédé selon les revendications 1 ou 2, caractérisé en ce que la quantité appliquée est inférieure à 1 kg SA/ha.

**4.** Procédé selon la revendication 2, caractérisé en ce qu'on l'utilise des composés de formule I dans laquelle :

X et Y représentent chacun un hydrogène ;

Z est le radical cyano ou COA ;

A est UR ou $U^1N(=C)_n(R_3)R_4$ ;

U est l'oxygène ;

$U^1$ est l'oxygène ou $-N(R_5)-$ ;

R est un hydrogène ou un radical alkyle en $C_1$-$C_8$, alkyle en $C_1$-$C_8$ substitué par halogéno ou alcoxy en $C_1$-$C_3$, alcényle en $C_3$-$C_6$, alcényle en $C_3$-$C_6$ substitué par halogéno, alcynyle en $C_3$-$C_6$, alcynyle en $C_3$-$C_6$ substitué par halogéno, $(T)_n$-cyclo(alkyle en $C_3$-$C_8$), benzyle, benzyle halogéné, benzylméthoxy, $(T)_n$-$Si(CH_3)_3$, (T)-P(O)(alkyle en $C_1$-$C_4$)$CH_3$, (T)-P(O)(O-alkyle en $C_1$-$C_4$)$_2$ ou le groupe $(T)_n$-W ;

W est un hétérocycle à 5-7 chaînons, saturé ou insaturé, non substitué et ayant 1 à 3 hétéro-atomes du groupe O, N et S, ou encore le diacétone-D-glucosidyle ;

T représente les chaînons pontants $-CH_2-$, $-CH_2CH_2-$, $-CH(CH_3)-$, $-CCH_3(CH_3)-$ ou $-CH_2CH_2CH_2-$ ;

$R_3$ est un hydrogène ou un radical cyano, alkyle en $C_1$-$C_6$, phényle ou W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$ ou alcénoyle en $C_3$-$C_5$ ;

$R_3$ et $R_4$, avec l'atome auquel ils sont liés, représentent W ou W' ;

W est un hétérocycle à 5-7 chaînons, saturé ou insaturé, ayant 1 à 3 hétéro-atomes du groupe O, N et S ;

W' est un résidu du groupe

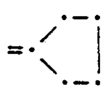 , 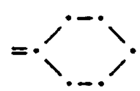 et 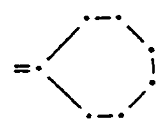 ;

n vaut zéro ou 1.

**5.** Composés de formule I'

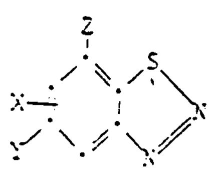 (I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

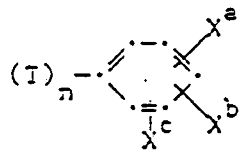 , $(T)_n$-Napht, $(T)_n-Si(C_1-C_6\text{-Alkyl})_3$,

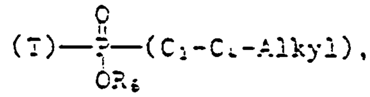 , 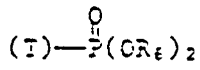 ; ou $(T)_n$—W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyloxy, et $X^b$ et $X^c$ sont simultanément des atomes d'hydrogène ; ou bien

$X^a$ est le radical phényle, phényloxy ou benzyloxy, et $X^b$ est un halogène ou le radical méthyle et $X^c$ est un hydrogène ; ou bien

$X^a$, $X^b$ et $X^c$ sont ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou de soufre et substitué par halogéno, cyano, HYOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cycloalkyle en $C_3$-$C_6$ ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des composés suivants :
7-cyano-benzo-1,2,3-thiadiazole ;
4-chloro-7-cyano-benzo-1,2,3-thiadiazole ;
4,6-dibromo-7-cyano-benzo-1,2,3-thiadiazole ;
acide benzo-1,2,3-thiadiazole-7-carboxylique ;
ester méthylique de l'acide benzo-1,2,3-thiadiazole-7-carboxylique ;
6-chloro-7-cyano-benzo-1,2,3-thiadiazole ;
acide 6-chloro-benzo-1,2,3-thiadiazole-7-carboxylique.

**6.** Composés de formule I'

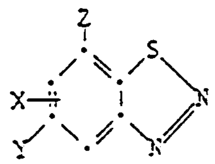

(I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy, alcoxy ou U-(alkyle en $C_1$-$C_3$), (T)-COOH ou (T)-COO(alkyle en $C_1$-$C_4$), alcényle en C2-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

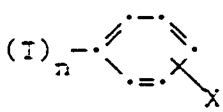, $(T)_n$-Naphthyl, $(T)_n$-Si($C_1$-$C_8$-Alkyl)$_3$,

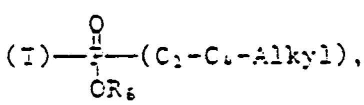, 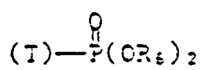 ; ou $(T)_n$-W ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, ou $CH(CH_3)-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$) ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;
$R_6$ est un hydrogène ou un radical alkyle en $C_1$-$C_4$ ; et
n vaut zéro ou 1 ;
à l'exception des composés suivants :
7-cyano-benzo-1,2,3-thiadiazole ;
4-chloro-7-cyano-benzo-1,2,3-thiadiazole ;
4,6-dibromo-7-cyano-benzo-1,2,3-thiadiazole ;
acide benzo-1,2,3-thiadiazole-7-carboxylique ;
ester méthylique de l'acide benzo-1,2,3-thiadiazole-7-carboxylique ;
6-chloro-7-cyano-benzo-1,2,3-thiadiazole ;
acide 6-chloro-benzo-1,2,3-thiadiazole-7-carboxylique.

**7.** Composés de formule I' selon la revendication 5, dans lesquels Z = COA et R a une signification autre qu'hydrogène et alkyle non substitué.

**8.** Composés de formule I' selon la revendication 5, appartenant au groupe suivant :
7-n-pentoxycarbonyl-benzo-1,2,3-thiadiazole ;
7-(4-méthoxy-benzyloxycarbonyl)-benzo-1,2,3-thiadiazole ;
7-(cycloheximino-oxycarbonyl)-benzo-1,2,3-thiadiazole ;
7-(3-hydroxy-n-propoxycarbonyl)-benzo-1,2,3-thiadiazole ;
1,2,5,6-di-O-isopropylidène-3-(7-benzo-1,2,3-thiadiazol)D-glucofurannose ;
7-fulfuryloxycarbonyl-benzo-1,2,3-thiadiazole ;
7-(1,2,4-triazole-1-yl)-méthoxycarbonyl-benzo-1,2,3-thiadiazole ;
7-(2-pyridylméthoxycarbonyl)-benzo-1,2,3-thiadiazole ;
7-triméthylsilyl-méthoxycarbonyl-benzo-1,2,3-thiadiazole ;
7[2-(triméthylsilyl)-éthoxycarbonyl]-benzo-1,2,3-thiadiazole ;
7-diméthylphosphono-éthoxycarbonyl-benzo-1,2,3-thiadiazole ;
7-cyclohexyloxycarbonyl-benzo-1,2,3-thiadiazole ;
7-(1-phénéthyloxycarbonyl)-benzo-1,2,3-thiadiazole ;
7-(3-méthoxybenzyl)-benzo-1,2,3-thiadiazole ;
7-(éthylthio-carbonyl)-benzo-1,2,3-thiadiazole ;
7-(n-propylthio-carbonyl)-benzo-1,2,3-thiadiazole ;
7-(benzylthio-carbonyl)-benzo-1,2,3-thiadiazole ;
7-carbamoyl-benzo-1,2,3-thiadiazole ;
7-N-phénylcarbamoyl-benzo-1,2,3-thiadiazole ;
N-(7-benzo-1,2,3-thiadiazolyle)-glycine ;
7-(N-diallylcarbamoyl)-benzo-1,2,3-thiadiazole ;
6-fluoro-7-méthoxycarbonyl-benzo-1,2,3-thiadiazole ;
6-fluoro-7-carboxy-benzo-1,2,3-thiadiazole ;
5-fluoro-7-benzyloxycarbonyl-benzo-1,2,3-thiadiazole ;
5-fluoro-7-carboxy-benzo-1,2,3-thiadiazole ;
5-fluoro-7-éthoxycarbonyl-benzo-1,2,3-thiadiazole.

**9.** Composés de formule I' selon la revendication 6, appartenant au groupe suivant :
7-éthoxycarbonyl-benzo-1,2,3-thiadiazole ;
7-n-propoxycarbonyl-benzo-1,2,3-thiadiazole ;
7-iso-propoxycarbonyl-benzo-1,2,3-thiadiazole ;
7-n-butoxycarbonyl-benzo-1,2,3-thiadiazole ;
7-sec-butoxycarbonyl-benzo-1,2,3-thiadiazole ;
7-tert-butoxycarbonyl-benzo-1,2,3-thiadiazole ;
7-cyclopropylméthoxycarbonyl-benzo-1,2,3-thiadiazole ;
7-(2'-phénéthoxycarbonyl)-benzo-1,2,3-thiadiazole ;
7-benzyloxycarbonyl-benzo-1,2,3-thiadiazole ;
7-allyloxycarbonyl-benzo-1,2,3-thiadiazole ;
7-propyn-2-yloxycarbonyl-benzo-1,2,3-thiadiazole ;
2-(7-benzo-1,2,3-thiadiazolyloxy-imino)-2-cyano-N-(éthylaminocarbonyl)-acétamide ;
Sel de Na de l'acide benzo-1,2,3-thiadiazole-7-carboxylique ;
Sel de K de l'acide benzo-1,2,3-thiadiazole-7-carboxylique ;

Sel de triéthylammonium de l'acide benzo-1,2,3-thiadiazole-7-carboxylique ;
7-(1'-phénéthoxycarbonyl)-benzo-1,2,3-thiadiazole ;
7-(1'-naphthylméthoxycarbonyl)-benzo-1,2,3-thiadiazole ;
7-(méthylthio-carbonyl)-benzo-1,2,3-thiadiazole ;
7-(éthylthio-carbonyl)-benzo-1,2,3-thiadiazole ;
7-(benzylthio-carbonyl)-benzo-1,2,3-thiadiazole ;
7-[(di-cyanométhyl)-amino-carbonyl]-benzo-1,2,3-thiadiazole ;
Ester méthylique de l'acide 3-[(N-benzo-1,2,3-thiadiazole-7-ylcarbonyl)-1,3,4-thiadiazole-2-ylamino]-2-méthylpropènecarboxylique ;
Ester méthylique de l'acide 3-[(N-benzo-1,2,3-thiadiazole-7-ylcarbonyl)-1,3,4-thiadiazole-2-ylamino]-2-éthylpropènecarboxylique ;
1-(Benzo-1,2,3-thiadiazole-7-carbonyl)-2-(A-méthylpropylidène)-hydrazine ;
1-(Benzo-1,2,3-thiadiazole-7-carbonyl)-2-(cyclobutylidène)-hydrazine;
1-(Benzo-1,2,3-thiadiazole-7-carbonyl)-2-(cyclopentylidène)-hydrazine ;
1-(Benzo-1,2,3-thiadiazole-7-carbonyl)-2-(cyclohexylidène)-hydrazine;
2-(Benzo-1,2,3-thiadiazole-7-carbonyl)-1-(2'-sec-butyl)-hydrazine;
1-(Benzo-1,2,3-thiadiazole-7-carbonyl)-2-(cyclopentyl)-hydrazine ;
1-(Benzo-1,2,3-thiadiazole-7-carbonyl)-2-(cyclohexyl)-hydrazine ;
1-(Benzo-1,2,3-thiadiazole-7-carbonyl)-2-(cycloheptyl)-hydrazine ;
1-(Benzo-1,2,3-thiadiazole-7-carbonyl)-1,2-diacétyl-hydrazine ;
1-(Benzo-1,2,3-thiadiazole-7-carbonyl)-2-phényl-hydrazine ;
1-(Benzo-1,2,3-thiadiazole-7-carbonyl)-2-pyridine-2'-yl-hydrazine.

**10.** Procédé selon la revendication 2, caractérisé en ce qu'on utilise des composés de formule I' selon la revendication 5.

**11.** Procédé selon la revendication 2, caractérisé en ce qu'on utilise des composés de formule I' selon la revendication 6.

**12.** Procédé selon la revendication 2, caractérisé en ce qu'on utilise des composés de formule I' selon la revendication 7.

**13.** Procédé selon la revendication 2, caractérisé en ce qu'on utilise comme matière active l'acide 1,2,3-benzothiadiazole-7-carboxylique ou un de ses sels avec un composé basique.

**14.** Procédé selon la revendication 13, caractérisé en ce qu'on utilise comme sels des sels obtenus avec des amines primaires, secondaires ou tertiaires ou des composés de métaux alcalins ou alcalino-terreux.

**15.** Procédé selon la revendication 2, caractérisé en ce qu'on utilise comme matière active le 7-méthoxycarbonyl-benzo-1,2,3-thiadiazole.

**16.** Procédé selon la revendication 2, caractérisé en ce qu'on utilise comme matière active le 7-benzyloxycarbonyl-benzo-1,2,3-thiadiazole.

**17.** Procédé selon la revendication 2, caractérisé en ce qu'on utilise comme matière active le 7-cyano-benzo-1,2,3-thiadiazole.

**18.** Procédé selon la revendication 2, caractérisé en ce qu'on utilise comme matières actives des composés 7-(alcoxy en $C_2$-$C_4$)-carbonyl-benzo-1,2,3-thiadiazole.

**19.** Produit destiné à immuniser les végétaux contre une infestation par des micro-organismes phytopathogènes, contenant comme matière active au moins un composé de formule I selon la revendication 1 ou 2 à l'exception des composés 7-cyano-benzo-1,2,3-thiadiazole, 4-chloro-7-cyano-benzo-1,2,3-thiadiazole, 6-chloro-7-cyano-benzo-1,2,3-thiadiazole, 4,6-dibromo-7-cyano-benzo-1,2,3-thiadiazole et 7-méthoxycarbonyl-benzo-1,2,3-thiadiazole.

**20.** Produit destiné à l'immunisation des végétaux contre une infestation par des micro-organismes

phytopathogènes, contenant comme matière active au moins un composé de formule I' selon la revendication 5.

**21.** Produit destiné à l'immunisation des végétaux contre une infestation par des micro-organismes phytopathogènes, contenant comme matière active au moins un composé de formule I' selon la revendication 6.

**22.** Produit selon la revendication 19, caractérisé en ce qu'il contient comme matière active au moins un composé de formule I' selon la revendication 7.

**23.** Produit selon la revendication 19, caractérisé en ce qu'il contient comme matière active au moins un composé selon l'une des revendications 8 ou 9.

**24.** Produit selon la revendication 19, caractérisé en ce qu'il contient comme matière active au moins l'un des composés acide benzo-1,2,3-thiadiazole-7-carboxylique ou 7-méthoxycarbonyl-benzo-1,2,3-thiadiazole.

**25.** Produit selon la revendication 19, caractérisé en ce qu'il contient comme matière active de l'acide 1,2,3-benzothiadiazole-7-carboxylique ou un de ses sels avec un composé basique.

**26.** Produit selon la revendication 25, caractérisé en ce qu'on utilise comme sels des sels avec des amines primaires, secondaires ou tertiaires ou avec des composés de métaux alcalins ou alcalino-terreux.

**27.** Procédé de préparation de composés de formule I'

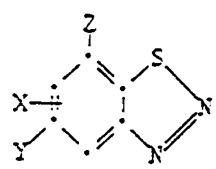

(I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

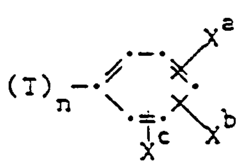 , $(T)_n$-Napht, $(T)_n$-Si($C_1$-$C_8$-Alkyl)$_3$,

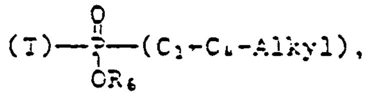, 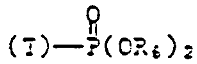 ; ou $(T)_n$—W ;

$X^a$, $X^b$ et $X^c$, chacun indépendamment l'un de l'autre, représentent un hydrogène, un halogène ou

un radical hydroxy, cyano, HOOC, MOOC, (alkyle en $C_1$-$C_3$)-OOC, alkyle en $C_1$-$C_4$, alcoxy en $C_1$-$C_4$; halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyloxy, et $X^b$ et $X^c$ sont simultanément des atomes d'hydrogène ; ou bien

$X^a$ est le radical phényle, phényloxy ou benzyloxy, et

$X^b$ est un halogène ou le radical méthyle et $X^c$ est un hydrogène ; ou bien

$X^a$, $X^b$ et $X^c$ sont ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$) ; $(T)_n$-cyclo(alkyle en $C_3$-$C_6$) substitué par alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons saturé ou insaturé comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_6$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des composés suivants :
7-cyano-benzo-1,2,3-thiadiazole ;
4-chloro-7-cyano-benzo-1,2,3-thiadiazole ;
4,6-dibromo-7-cyano-benzo-1,2,3-thiadiazole ;
acide benzo-1,2,3-thiadiazole-7-carboxylique ;
ester méthylique de l'acide benzo-1,2,3-thiadiazole-7-carboxylique ;
6-chloro-7-cyano-benzo-1,2,3-thiadiazole ;
acide 6-chloro-benzo-1,2,3-thiadiazole-7-carboxylique,
caractérisé en ce que :

1. on prépare des composés de formule Ia

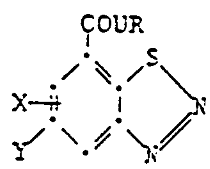

(Ia)

dans laquelle R, X, Y et U ont les significations données pour la formule I, en faisant réagir :

1.1. des composés de formule II

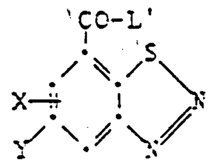

(II)

dans laquelle L' est un groupe éliminable, par exemple un halogène, un groupe O-acyle comme par exemple le résidu acyle appartenant à l'anhydride symétrique de l'acide Ib, ou encore le groupe 1-imidazolyle,
avec des composés de formule III

RUH (III)

a) en présence d'un excès du réactif RUH, ou bien
b) en présence d'une base organique, avec ou sans 4-dialkylaminopyridine servant de catalyseur, dans des solvants inertes, ou bien
c) en présence de bases minérales, en travaillant dans chaque cas dans l'intervalle de températures de -10° à 180°C et de préférence de 0° à 100°C ; et

1.2. des composés de formule Ib

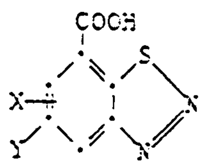

(Ib)

avec des composés de formule III en excès ou dans un solvant inerte, en présence d'un acide tel que l'acide sulfurique, l'acide chlorhydrique, l'acide p-toluènesulfonique ou le complexe trifluorure de borediéthyléther, ou encore de dicyclohexylcarbodiimide, à des températures de -10° à 180°C et de préférence de 0° à 140°C ; et

2. on préparé des composés de formule Ic

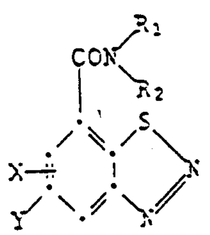

(Ic)

dans laquelle les symboles $R_1$, $R_2$, X et Y ont les significations données pour la formule I, en faisant réagir :

2.1. des composés de formule II avec des composés de formule IV

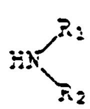

(IV)

a) en présence d'un excès de réactif $HN(R_1)R_2$ ou bien
b) en présence d'une base organique, avec ou sans 4-dialkylaminopyridine servant de catalyseur dans des solvants inertes, ou bien
c) en présence de bases minérales, en travaillant dans chaque cas dans l'intervalle de températures de -10° à 160°C et de préférence de 0° à 100°C ; et

3. on prépare des composés de formule Id

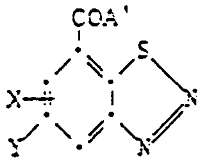

(Id)

dans laquelle A' est le résidu $U^1N(=C)_n(R_3)R_4$, et X, Y, $R_3$, $R_4$, $R_5$ et n ont les significations données pour la formule I, en faisant réagir

3.1. des composés de formule Ie

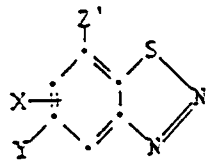

(Ie)

dans laquelle Z' est le groupe COOH, COCl, $COOAlk^1$ ou un résidu acyloxycarbonyle, comme par exemple

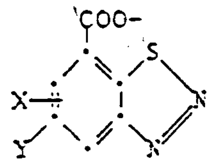

benzoyloxycarbonyle ou acétyloxycarbonyle, $Alk^1$ représentant un radical alkyle en $C_1$-$C_4$, en présence d'une base, avec des dérivés de l'hydrazine de formules V ou VI

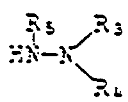

(V) ou

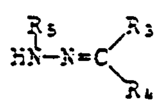

(VI)

dans un solvant inerte à des températures de -10° à 180°C et de préférence de 0° à 100°C ; ou

3.2. on fait réagir par étapes des composés de formule Ie, d'abord avec l'hydrazine, puis on fait réagir les dérivés obtenus de l'hydrazine

3.2.1. avec l'agent d'alkylation $R_3$-L ou $R_4$-L, où L est un groupe éliminable, dans un solvant inerte, à des températures de 0° à 160°C et de préférence de 20° à 120°C ; ou bien

3.2.2. avec un aldéhyde ou une cétone de formule $R_3(R_4)C=O$, où $R_3$ et $R_4$ ont les significations données pour la formule I, avec ou sans addition d'un acide minéral ou organique, à des températures de -10° à 150°C et de préférence de 20° à 100°C, puis, si on le souhaite,

3.2.3. avec un agent d'alkylation L-$R_5$, où L est un groupe éliminable, en présence d'une base forte, dans un solvant inerte, à des températures de -80° à 120° et de préférence de -40° à 80°C ; ou encore, si on le souhaite

3.2.4.

a) on hydrogène les dérivés hydrazones préparés en 3.2.1. à l'aide d'hydrogène sous une pression de 1 à $30.10^5$ Pa, en présence d'un catalyseur en mélange avec du charbon activé, dans un solvant inerte, à des températures de 0° à 100°C, ou bien

b) on traite les dérivés hydrazones préparés en 3.2.1. avec un hydrure métallique complexe tel que le cyanoborohydrure de Na, dans un solvant inerte, à des températures de -10° à 80° et de préférence de 0° à 50°C ; et

4. on prépare des composés de formule If

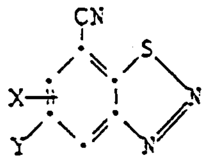

**(If)**

dans laquelle les symboles X et Y ont les significations données pour la formule I, en traitant des composés de formule Ig [préparés par le procédé (2)]

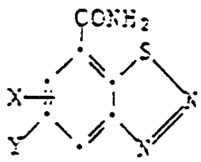

**(Ig)**

avec un agent de déshydratation, dans un solvant inerte ou sans solvant, à des températures de -10° à 250°C, auquel cas on utilise comme agent de déshydratation :

a) de l'anhydride trifluoracétique en présence d'une base comme par exemple la pyridine, dans un solvant inerte tel par exemple que le tétrahydrofuranne ou le dioxanne, à des températures de -10° à 40°C ; ou bien

b) de l'isocyanate de chlorosulfonyle dans un solvant inerte, comme par exemple le tétrahydrofuranne, à des températures de 0° à 65°C, opération suivie d'un traitement au diméthylformamide ;

c) du pentoxyde de phosphore, avec ou sans solvant inerte comme par exemple le 1,2-dichlorométhane, le xylène ou le chlorobenzène, éventuellement dans une bombe tubulaire sous pression élevée, à une température de 50° à 250°C ;

5.1. on prépare des composés de formule $IL_1$

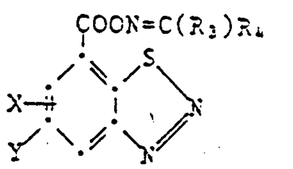

**($IL_1$)**

dans laquelle $R_3$, $R_4$, X et Y ont les significations données pour la formule I, en faisant réagir des dérivés oximes de formule

$HO\text{-}N=C(R_3)R_4$

avec des dérivés d'acide activés de formule

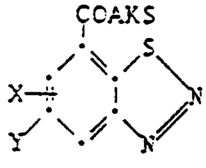

où AKS désigne un halogène, un radical O-acyle comme par exemple le résidu O-acyle de l'acide libre ayant la formule ci-dessus, le radical acétoxy ou benzoyloxy, ou encore 1-imidazolyle, dans un solvant inerte et une base, à une température de -20° à 120° et de préférence de 0° à 50°C, ou

encore l'acide libre (= Ib) en présence de dicyclohexylcarbodiimide dans les mêmes conditions ;
5.2. on prépare des composés de formule $IL_2$

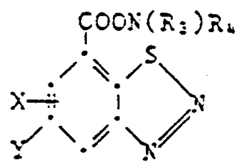

(IL₂)

dans laquelle $R_3$, $R_4$, X et Y ont les significations données pour la formule I, par réduction de composés de formule $IL_1$

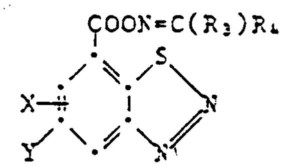

($IL_1$)

a) avec un silane, par exemple le triéthylsilane, en présence d'un acide tel que l'acide trifluoracétique, à une température de 0° à 80°C, ou bien
b) avec du cyanoborohydrure de sodium en présence d'un acide organique tel que l'acide acétique, à une température de 0° à 80°C, ou bien
c) par voie catalytique, par exemple avec $Pt/H_2$.

**28.** Procédé de préparation de composés de formule Ik

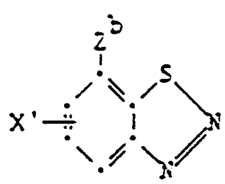

(Ik)

qui consiste à diazoter un composé de formule XI'

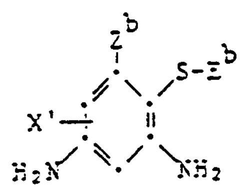

(XI')

en milieu acide, avec un composé nitrite, à une température de -40° à 30°C, et à le traiter, dans le même réacteur ou dans un autre réacteur, avec un réducteur à une température de -40° à 80° et de préférence de -30° à 30°C, le réducteur étant ajouté avant ou après le composé nitrite ou en même temps que ce dernier, les substituants des composés ayant les significations suivantes :

X' est un hydrogène, un halogène ou le radical méthyle, méthoxy ou COOH ;

$E^b$ est un groupe facilement éliminable comme par exemple un hydrogène ou un radical alkyle en $C_1$-$C_{16}$, par exemple méthyle, éthyle, isopropyle ou n-dodécyle, ou benzyle ou encore acyle, par exemple acétyle, ou un résidu acide sulfonique ($-SO_3H$) ou cyano, ou encore, comme élément d'un pont disulfure, un deuxième résidu

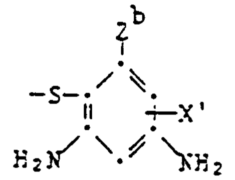

;

$Z^b$ est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

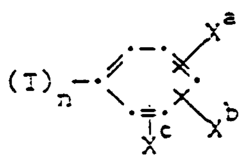 , $(T)_n$-Napht, $(T)_n$-Si($C_1$-$C_8$-Alkyl)$_3$,

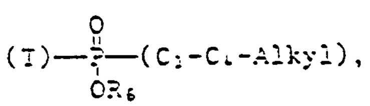 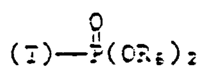 ; ou $(T)_n$—W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyle, et $X^b$ et $X^c$ sont simultanément des atomes d' hydrogène ; ou bien

$X^a$ est le radical phényle, phényloxy ou benzyloxy, et

$X^b$ est un halogène ou le radical méthyle et $X^c$ est un hydrogène ; ou bien

$X^a$, $X^b$ et $X^c$ forment ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo (alkyle en $C_3$-$C_6$) ; $(T)_n$-cyclo(alkyle en $C_3$-$C_6$) substitué par alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par

halrogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_6$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des groupes amino primaires ou secondaires, des groupes UH- ou nitro, des radicaux Si-(alkyle en $C_1$-$C_8$)$_3$ ou des résidus contenant du phosphore.

**29.** Procédé de préparation de composés de formule

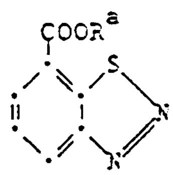

qui consiste à diazoter un composé de formule

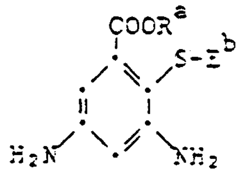

en milieu acide avec un composé nitrite, à une température de -20° à 30°C et, dans le même réacteur, à le traiter avec un réducteur à une température de -20° à 80° et de préférence de -20° à 30°C, le réducteur étant ajouté avant ou après le composé nitrite ou en même temps que ce dernier, les substituants des composés ayant les significations suivantes :

$R^a$ est un hydrogène ou un radical alkyle en $C_1$-$C_8$, un radical alkyle en $C_1$-$C_8$ substitué par halogéno, ou ; cyano, nitro, hydroxy ou U-(alkyle en $C_1$-$C_3$) ; (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

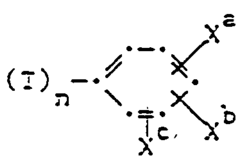 , $(T)_n$-Napht, $(T)_n$-Si($C_1$-$C_8$-Alkyl)$_3$,

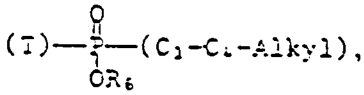 , 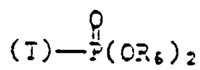 ; ou $(T)_n$—W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en' particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyle, et $X^b$ et $X^c$ sont simultanément des atomes d'hydrogène ; ou bien

$X^a$ est le radical phényle, phényloxy ou benzyloxy, et

$X^b$ est un halogène ou le radical méthyle et $X^c$ est un hydrogène ; ou bien

$X^a$, $X^b$ et $X^c$ forment ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$) ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_6$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone sur le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des résidus comportant des groupes UH- ou nitro, ainsi que des résidus contenant du silicium ou du phosphore, et

$E^b$ est un groupe facilement éliminable comme par exemple l'hydrogène ou un radical alkyle en $C_1$-$C_{16}$, par exemple méthyle, éthyle, isopropyle ou n-dodécyle, ou benzyle ou encore acyle, par exemple acétyle, ou un résidu acide sulfonique ($-SO_3H$) ou cyano.

**30.** Utilisation des composés de formule I selon la revendication 1 ou 2 pour l'immunisation des végétaux contre les maladies.

**31.** Utilisation des composés de formule I' selon la revendication 8 ou 9 pour l'immunisation des végétaux contre les maladies.

**32.** Utilisation du 7-méthoxycarbonyl-benzo-1,2,3-thiadiazole en tant que matière active pour la préparation d'un produit destiné à immuniser les végétaux contre une infestation par des micro-organismes phytopathogènes.

**Revendications pour l'Etat contractant suivant : ES**

**1.** Procédé d'immunisation de végétaux contre une infestation par des micro-organismes phytopathogènes, caractérisé en ce qu'on applique sur les végétaux et/ou sur leur environnement, comme matière active, un composé ayant la formule I ci-après :

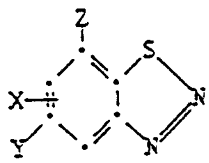

(I)

dans laquelle :

X est un hydrogène, un halogène ou un radical hydroxy, méthyle, méthoxy, HOOC ou MOOC ;

Y est un hydrogène, un halogène ou un radical $SO_3H$, $SO_3M$, nitro, hydroxy ou amino, M étant l'équivalent molaire d'un ion d'un métal alcalin ou alcalino-terreux, formé à partir d'une base ou d'un composé basique correspondants ; et

Z est le radical cyano ou -CO-A, où A est OH ou SH, H pouvant aussi être remplacé par l'équivalent molaire d'un résidu cationique minéral ou organique.

**2.** Procédé d'immunisation de végétaux contre une infestation par des micro-organismes phytopathogènes, caractérisé en ce qu'on applique sur les végétaux et/ou sur leur environnement, comme matière active, un composé ayant la formule I ci-après :

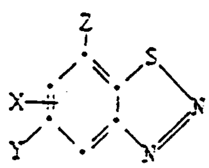

(I)

dans laquelle :

X est un hydrogène, un halogène ou un radical hydroxy, méthyle, méthoxy, HOOC ou MOOC ;

Y est un hydrogène, un halogène ou un radical $SO_3H$, $SO_3M$, nitro, hydroxy ou amino, M étant l'équivalent molaire d'un ion d'un métal alcalin ou alcalino-terreux, formé à partir d'une base ou d'un composé basique correspondants ; et

Z est le radical cyano ou -CO-A,

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogène, cyano, nitro, hydroxy, U-(alkyle en $C_1$-$C_3$) ou di-(alkyle en $C_2$-$C_4$)-amino ou interrompu par le groupe CO ; (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

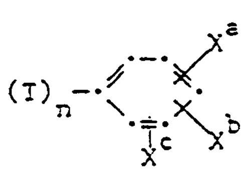

, $(T)_n$-Napht, $(T)_n$-Si($C_1$-$C_8$-Alkyl)$_3$,

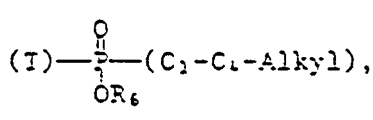

,

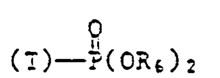

; ou $(T)_n$—W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou

bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyle, et $X^b$ et $X^c$ sont simultanément des atomes d'hydrogène ; ou bien

$X^a$ est le radical phényle, phényloxy ou benzyloxy, et $X^b$ est un halogène ou le radical méthyle et $X^c$ est un hydrogène ; ou bien

$X^a$, $X^b$ et $X^c$ sont ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CCH_3(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$ ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par des radicaux halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le cycle ;

$R_5$ est un hydrogène ou le radical méthyle ;

$R_6$ est un hydrogène ou un radical alkyle en $C_1$-$C_4$ ; et

n vaut zéro ou 1 ;

où dans les composés de formule I, le résidu organique A a une masse moléculaire inférieure à 900 ; et, quand U est un oxygène ou un soufre, ces composés englobent les sels de l'acide 7-carboxylique, compatibles du point de vue phytosanitaire, avec des amines primaires, secondaires ou tertiaires ou des bases minérales.

**3.** Procédé selon les revendications 1 ou 2, caractérisé en ce que la quantité appliquée est inférieure à 1 kg SA/ha.

**4.** Procédé selon la revendication 2, caractérisé en ce qu'on l'utilise des composés de formule I dans laquelle :

X et Y représentent chacun un hydrogène ;

Z est le radical cyano ou COAl ;

A est UR ou $U^1N(=C)_n(R_3)R_4$ ;

U est l'oxygène ;

$U^1$ est l'oxygène ou $-N(R_5)-$ ;

R est un hydrogène ou un radical alkyle en $C_1$-$C_8$, alkyle en $C_1$-$C_8$ substitué par halogéno ou alcoxy en $C_1$-$C_3$, alcényle en $C_3$-$C_6$, alcényle en $C_3$-$C_6$ substitué par halogéno, alcynyle en $C_3$-$C_6$, alcynyle en $C_3$-$C_6$ substitué par halogéno, $(T)_n$-cyclo(alkyle en $C_3$-$C_8$), benzyle, benzyle halogéné, benzylméthoxy, $(T)_n$-$Si(CH_3)_3$, (T)-P(O)(alkyle en $C_1$-$C_4$)$CH_3$, (T)-P(O)(O-alkyle en $C_1$-$C_4$)$_2$ ou le groupe $(T)_n$-W ;

W est un hétérocycle à 5-7 chaînons, saturé ou insaturé, non substitué et ayant 1 à 3 hétéro-atomes du groupe O, N et S, ou encore le diacétone-D-glucosidyle ;

T représente les chaînons pontants $-CH_2-$, $-CH_2CH_2-$, $-CH(CH_3)-$, $-CCH_3(CH_3)-$ ou $-CH_2CH_2CH_2-$ ;

$R_3$ est un hydrogène ou un radical cyano, alkyle en $C_1$-$C_6$, phényle ou W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$ ou alcénoyle en $C_3$-$C_5$ ;

$R_3$ et $R_4$, avec l'atome auquel ils sont liés, représentent W ou W' ;

W est un hétérocycle à 5-7 chaînons, saturé ou insaturé, ayant 1 à 3 hétéro-atomes du groupe O, N et S ;

W' est un résidu du groupe

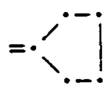 , 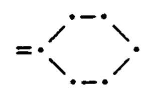 et 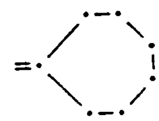 ;

n vaut zéro ou 1.

**5.** Procédé selon la revendication 2, caractérisé en ce qu'on utilise des composés de formule I'

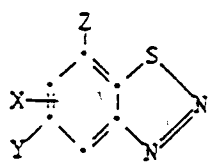 (I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

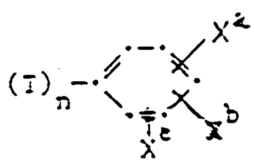 , $(T)_n$-Napht, $(T)_n$-Si($C_1$-$C_8$-Alkyl)$_3$,

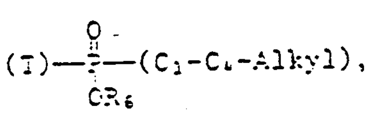 , 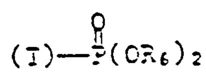 ; ou $(T)_n$-W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyloxy, et $X^b$ et $X^c$ sont simultanément des atomes d'hydrogène ; ou bien

$X^a$ est le radical phényle, phényloxy ou benzyloxy, et $X^b$ est un halogène ou le radical méthyle et

$X^c$ est un hydrogène ; ou bien

$X^a$, $X^b$ et $X^c$ sont ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, M et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou de soufre et substitué par halogéno, cyano, HYOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cycloalkyle en $C_3$-$C_6$ ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par des radicaux halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, M ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des composés suivants :

7-cyano-benzo-1,2,3-thiadiazole ;

4-chloro-7-cyano-benzo-1,2,3-thiadiazole ;

4,6-dibromo-7-cyano-benzo-1,2,3-thiadiazole ;

acide benzo-1,2,3-thiadiazole-7-carboxylique ;

ester méthylique de l'acide benzo-1,2,3-thiadiazole-7-carboxylique ;

6-chloro-7-cyano-benzo-1,2,3-thiadiazole ;

acide 6-chloro-benzo-1,2,3-thiadiazole-7-carboxylique.

**6.** Procédé selon la revendication 2, caractérisé en ce qu'on utilise des composés de formule I'

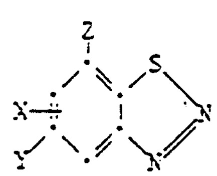

(I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy, alcoxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$),ou encore un groupe de la série :

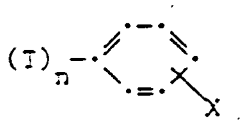 , $(T)_n$-Naphthyl, $(T)_n$-Si($C_1$-$C_8$-Alkyl)$_3$,

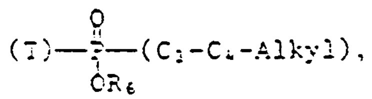 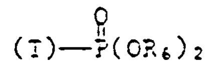 ; ou $(T)_n$—W ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, ou $-CH(CH_3)-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$) ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons saturé ou insaturé comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des composés suivants :
7-cyano-benzo-1,2,3-thiadiazole ;
4-chloro-7-cyano-benzo-1,2,3-thiadiazole ;
4,6-dibromo-7-cyano-benzo-1,2,3-thiadiazole ;
acide benzo-1,2,3-thiadiazole-7-carboxylique ;
ester méthylique de l'acide benzo-1,2,3-thiadiazole-7-carboxylique ;
6-chloro-7-cyano-benzo-1,2,3-thiadiazole ;
acide 6-chloro-benzo-1,2,3-thiadiazole-7-carboxylique.

7. Procédé selon la revendication 2, caractérisé en ce qu'on utilise des composés de formule I' selon la revendication 5, dans lesquels Z = COA et R a une signification autre qu'hydrogène et alkyle substitué.

8. Procédé selon la revendication 2, caractérisé en ce qu'on utilise comme matière active l'acide 1,2,3-benzothiadiazole-7-carboxylique ou un de ses sels avec un composé basique.

**9.** Procédé selon la revendication 8, caractérisé en ce qu'on utilise comme sels des sels obtenus avec des amines primaires, secondaires ou tertiaires ou des composés de métaux alcalins ou alcalino-terreux.

**10.** Procédé selon la revendication 2, dans lequel on utilise comme matière active le 7-méthoxycarbonyl-benzo-1,2,3-thiadiazole.

**11.** Procédé selon la revendication 2, caractérisé en ce qu'on utilise comme matière active le 7-benzyloxycarbonyl-benzo-1,2,3-thiadiazole.

**12.** Procédé selon la revendication 2, caractérisé en ce qu'on utilise comme matière active le 7-cyano-benzo-1,2,3-thiadiazole.

**13.** Procédé selon la revendication 2, caractérisé en ce qu'on utilise comme matières actives des composés 7-(alcoxy en $C_2$-$C_4$)-carbonyl-benzo-1,2,3-thiadiazole.

**14.** Produit destiné à immuniser les végétaux contre une infestation par des micro-organismes phytopathogènes, contenant comme matière active au moins un composé de formule I selon la revendication 1 ou 2 à l'exception des composés 7-cyano-benzo-1,2,3-thiadiazole, 4-chloro-7-cyano-benzo-1,2,3-thiadiazole, 6-chloro-7-cyano-benzo-1,2,3-thiadiazole, 4,6-dibromo-7-cyano-benzo-1,2,3-thiadiazole et 7-méthoxycarbonyl-benzo-1,2,3-thiadiazole.

**15.** Ester méthylique de l'acide benzo-1,2,3-thiadiazole-7-carboxylique, à utiliser dans un procédé d'immunisation des végétaux contre une infestation par des micro-organismes phytopathogènes.

**16.** Ester méthylique de l'acide benzo-1,2,3-thiadiazole-7-carboxylique, à utiliser dans la préparation d'un produit destiné à l'immunisation de végétaux contre une infestation par des micro-organismes phytopathogènes.

**17.** Produit destiné à l'immunisation de végétaux contre une infestation par des micro-organismes phytopathogènes, contenant comme matière active au moins un composé de formule I'

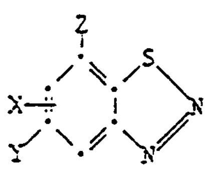

(I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

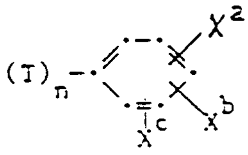 , $(T)_n$-Napht, $(T)_n-Si(C_1-C_6-Alkyl)_3$,

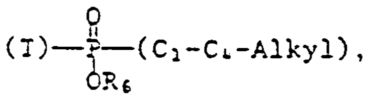 , 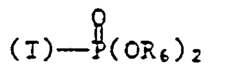 ; ou $(T)_n-W$ ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyloxy, et $X^b$ et $X^c$ sont simultanément des atomes d'hydrogène ; ou bien

$X^a$ est le radical phényle, phényloxy ou benzyloxy, et $X^b$ est un halogène ou le radical méthyle et $X^c$ est un hydrogène ; ou bien

$X^a$, $X^b$ et $X^c$ sont ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou de soufre et substitué par halogéno, cyano, HYOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cycloalkyle en $C_3$-$C_6$ ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des composés suivants :
7-cyano-benzo-1,2,3-thiadiazole ;
4-chloro-7-cyano-benzo-1,2,3-thiadiazole ;
4,6-dibromo-7-cyano-benzo-1,2,3-thiadiazole ;
acide benzo-1,2,3-thiadiazole-7-carboxylique ;
ester méthylique de l'acide benzo-1,2,3-thiadiazole-7-carboxylique ;
6-chloro-7-cyano-benzo-1,2,3-thiadiazole ;
acide 6-chloro-benzo-1,2,3-thiadiazole-7-carboxylique.

**18.** Produit destiné à immuniser des végétaux contre une infestation par des micro-organismes phytosanitaires, contenant comme matière active au moins un composé de formule I'

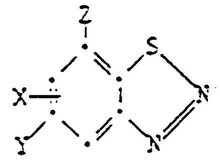

(I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy, alcoxy ou U-(alkyle en $C_1$-$C_3$), (T)-COOH ou (T)-COO(alkyle en $C_1$-$C_4$), alcényle en $C_2$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$),ou encore un groupe de la série :

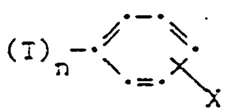 , $(T)_n$-Naphthyl, $(T)_n$-Si($C_1$-$C_6$-Alkyl)$_3$,

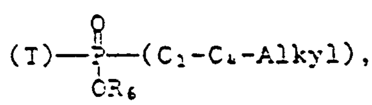 , 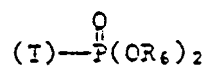 ; ou $(T)_n$—W ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, ou $CH(CH_3)-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$) ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par des radicaux halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_3$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;
$R_5$ est un hydrogène ou le radical méthyle ;
$R_6$ est un hydrogène ou un radical alkyle en $C_1$-$C_4$ ; et
n vaut zéro ou 1 ;
à l'exception des composés suivants :
7-cyano-benzo-1,2,3-thiadiazole ;
4-chloro-7-cyano-benzo-1,2,3-thiadiazole ;
4,6-dibromo-7-cyano-benzo-1,2,3-thiadiazole ;
acide benzo-1,2,3-thiadiazole-7-carboxylique ;
ester méthylique de l'acide benzo-1,2,3-thiadiazole-7-carboxylique ;
6-chloro-7-cyano-benzo-1,2,3-thiadiazole ;
acide 6-chloro-benzo-1,2,3-thiadiazole-7-carboxylique.

**19.** Produit selon la revendication 14, caractérisé en ce qu'il contient comme matière active au moins un composé de formule I' selon la revendication 17, où Z = COA et R a une signification autre qu'hydrogène ou radical alkyle non substitué.

**20.** Produit selon la revendication 14, caractérisé en ce qu'il contient comme matière active au moins un composé de formule I' appartenant au groupe suivant :
7-n-pentoxycarbonyl-benzo-1,2,3-thiadiazole ;
7-(4-méthoxy-benzyloxycarbonyl)-benzo-1,2,3-thiadiazole ;
7-(cycloheximino-oxycarbonyl)-benzo-1,2,3-thiadiazole ;
7-(3-hydroxy-n-propoxycarbonyl)-benzo-1,2,3-thiadiazole ;
1,2,5,6-di-O-isopropylidène-3-(7-benzo-1,2,3-thiadiazol)D-glucofurannose ;
7-fulfuryloxycarbonyl-benzo-1,2,3-thiadiazole ;
7-(1,2,4-triazole-1-yl)-méthoxycarbonyl-benzo-1,2,3-thiadiazole ;
7-(2-pyridylméthoxycarbonyl)-benzo-1,2,3-thiadiazole ;
7-triméthylsilyl-méthoxycarbonyl-benzo-1,2,3-thiadiazole ;
7[2-(triméthylsilyl)-éthoxycarbonyl]-benzo-1,2,3-thiadiazole ;
7-diméthylphosphono-éthoxycarbonyl-benzo-1,2,3-thiadiazole ;
7-cyclohexyloxycarbonyl-benzo-1,2,3-thiadiazole ;
7-(1-phénéthyloxycarbonyl)-benzo-1,2,3-thiadiazole ;
7-(3-méthoxybenzyl)-benzo-1,2,3-thiadiazole ;
7-(éthylthio-carbonyl)-benzo-1,2,3-thiadiazole ;
7-(n-propylthio-carbonyl)-benzo-1,2,3-thiadiazole ;
7-(benzylthio-carbonyl)-benzo-1,2,3-thiadiazole ;
7-carbamoyl-benzo-1,2,3-thiadiazole ;
7-N-phénylcarbamoyl-benzo-1,2,3-thiadiazole ;
N-(7-benzo-1,2,3-thiadiazolyle)-glycine ;
7-(N-diallylcarbamoyl)-benzo-1,2,3-thiadiazole ;
6-fluoro-7-méthoxycarbonyl-benzo-1,2,3-thiadiazole ;
6-fluoro-7-carboxy-benzo-1,2,3-thiadiazole ;
5-fluoro-7-benzyloxycarbonyl-benzo-1,2,3-thiadiazole ;
5-fluoro-7-carboxy-benzo-1,2,3-thiadiazole ;
5-fluoro-7-éthoxycarbonyl-benzo-1,2,3-thiadiazole ; ou
appartenant au groupe suivant :
7-éthoxycarbonyl-benzo-1,2,3-thiadiazole ;
7-n-propoxycarbonyl-benzo-1,2,3-thiadiazole ;
7-iso-propoxycarbonyl-benzo-1,2,3-thiadiazole ;
7-n-butoxycarbonyl-benzo-1,2,3-thiadiazole ;
7-sec-butoxycarbonyl-benzo-1,2,3-thiadiazole ;
7-tert-butoxycarbonyl-benzo-1,2,3-thiadiazole ;
7-cyclopropylméthoxycarbonyl-benzo-1,2,3-thiadiazole ;
7-(2'-phénéthoxycarbonyl)-benzo-1,2,3-thiadiazole ;
7-benzyloxycarbonyl-benzo-1,2,3-thiadiazole ;
7-allyloxycarbonyl-benzo-1,2,3-thiadiazole ;
7-propyn-2-yloxycarbonyl-benzo-1,2,3-thiadiazole ;
2-(7-benzo-1,2,3-thiadiazolyloxy-imino)-2-cyano-N-(éthylaminocarbonyl)-acétamide ;

Sel de Na de l'acide benzo-1,2,3-thiadiazole-7-carboxylique ;
Sel de K de l'acide benzo-1,2,3-thiadiazole-7-carboxylique ;
Sel de triéthylammonium de l'acide benzo-1,2,3-thiadiazole-7-carboxylique ;
7-(1'-phénéthoxycarbonyl)-benzo-1,2,3-thiadiazole ;
7-(1'-naphthylméthoxycarbonyl)-benzo-1,2,3-thiadiazole ;
7-(méthylthio-carbonyl)-benzo-1,2,3-thiadiazole ;
7-(éthylthio-carbonyl)-benzo-1,2,3-thiadiazole ;
7-(benzylthio-carbonyl)-benzo-1,2,3-thiadiazole ;
7-[(di-cyanométhyl)-amino-carbonyl]-benzo-1,2,3-thiadiazole ;
Ester méthylique de l'acide 3-[(N-benzo-1,2,3-thiadiazole-7-ylcarbonyl)-1,3,4-thiadiazole-2-ylamino]-2-méthylpropènecarboxylique ;
Ester méthylique de l'acide 3-[(N-benzo-1,2,3-thiadiazole-7-ylcarbonyl)-1,3,4-thiadiazole-2-ylamino]-2-éthylpropènecarboxylique ;
1-(Benzo-1,2,3-thiadiazole-7-carbonyl)-2-(A-méthylpropylidène)-hydrazine ;
1-(Benzo-1,2,3-thiadiazole-7-carbonyl)-2-(cyclobutylidène)-hydrazine;
1-(Benzo-1,2,3-thiadiazole-7-carbonyl)-2-(cyclopentylidène)-hydrazine ;
1-(Benzo-1,2,3-thiadiazole-7-carbonyl)-2-(cyclohexylidène)-hydrazine;
2-(Benzo-1,2,3-thiadiazole-7-carbonyl)-1-(2'-sec-butyl)-hydrazine;
1-(Benzo-1,2,3-thiadiazole-7-carbonyl)-2-(cyclopentyl)-hydrazine ;
1-(Benzo-1,2,3-thiadiazole-7-carbonyl)-2-(cyclohexyl)-hydrazine ;
1-(Benzo-1,2,3-thiadiazole-7-carbonyl)-2-(cycloheptyl)-hydrazine ;
1-(Benzo-1,2,3-thiadiazole-7-carbonyl)-1,2-diacétylhydrazine ;
1-(Benzo-1,2,3-thiadiazole-7-carbonyl)-2-phényl-hydrazine ;
1-(Benzo-1,2,3-thiadiazole-7-carbonyl)-2-pyridine-2'-ylhydrazine.

**21.** Produit selon la revendication 14, caractérisé en ce qu'il contient comme matière active au moins l'un des composés acide benzo-1,2,3-thiadiazole-7-carboxylique ou 7-méthoxycarbonyl-benzo-1,2,3-thiadiazole.

**22.** Produit selon la revendication 14, caractérisé en ce qu'il contient comme matière active de l'acide 1,2,3-benzothiadiazole-7-carboxylique ou un de ses sels avec un composé basique.

**23.** Produit selon la revendication 22, caractérisé en ce qu'on utilise comme sels des sels avec des amines primaires, secondaires ou tertiaires ou avec des composés de métaux alcalins ou alcalino-terreux.

**24.** Procédé de préparation de composés de formule I'

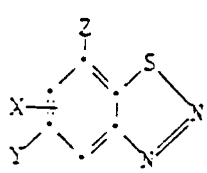

(I')

dans laquelle :

X est un hydrogène ou un radical halogéno, hydroxy, méthyle, méthoxy, HOOC ou MOOC ;

Y est un hydrogène ou un radical halogéno, $SO_3H$, $SO_3M$, nitro, hydroxy ou amino ;

Z est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou $-N(R_5)-$ ;

R est un hydrogène ou un radical alkyle en $C_1$-$C_8$, un radical alkyle en $C_1$-$C_8$ substitué par halogéno, cyano, nitro, hydroxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

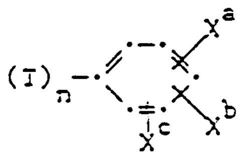 , $(T)_n$-Napht, $(T)_n$-Si($C_1$-$C_6$-Alkyl)$_3$,

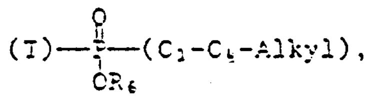 , 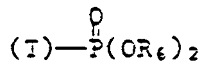 ; ou $(T)_n$—W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor : ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyloxy, et $X^b$ et $X^c$ sont simultanément des atomes d'hydrogène ; ou bien

$X^a$ est le radical phényle, phényloxy ou benzyloxy, et

$X^b$ est un halogène ou le radical méthyle et $X^c$ est un hydrogène ; ou bien

$X^a$, $X^b$ et $X^c$ sont ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$) ; $(T)_n$-cyclo(alkyle en $C_3$-$C_6$) substitué-par alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons saturé ou insaturé comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_6$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des composés suivants :
7-cyano-benzo-1,2,3-thiadiazole ;
4-chloro-7-cyano-benzo-1,2,3-thiadiazole ;
4,6-dibromo-7-cyano-benzo-1,2,3-thiadiazole ;
acide benzo-1,2,3-thiadiazole-7-carboxylique ;
ester méthylique de l'acide benzo-1,2,3-thiadiazole-7-carboxylique ;
6-chloro-7-cyano-benzo-1,2,3-thiadiazole ;
acide 6-chloro-benzo-1,2,3-thiadiazole-7-carboxylique,
caractérisé en ce que :

1. on prépare des composés de formule Ia

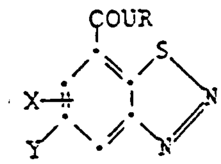

(Ia)

dans laquelle R, X, Y et U ont les significations données pour la formule I, en faisant réagir :

1.1. des composés de formule II

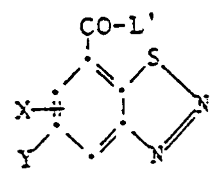

(II)

dans laquelle L' est un groupe éliminable, par exemple un halogène, un groupe O-acyle comme par exemple le résidu acyle appartenant à l'anhydride symétrique de l'acide Ib, ou encore le groupe 1-imidazyle,
avec des composés de formule III

RUH (III)

a) en présence d'un excès du réactif RUH, ou bien

b) en présence d'une base organique, avec ou sans 4-dialkylaminopyridine servant de catalyseur dans des solvants inertes, ou bien

c) en présence de bases minérales, en travaillant dans chaque cas dans l'intervalle de températures de -10° à 180°C et de préférence de 0° à 100°C ; et

1.2. des composés de formule Ib

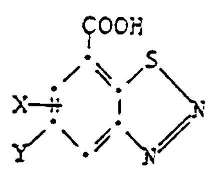

(Ib)

avec des composés de formule III en excès ou dans un solvant inerte, en présence d'un acide tel que l'acide sulfurique, l'acide chlorhydrique, l'acide p-toluènesulfonique ou le complexe trifluorure de borediéthyléther, ou encore de dicyclohexylcarbodiimide à des températures de -10° à 180°C et de préférence de 0° à 140°C ; et

2. on prépare des composés de formule Ic

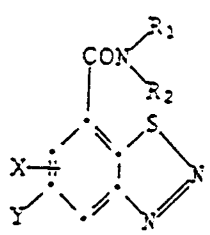

(Ic)

dans laquelle les symboles $R_1$, $R_2$, X et Y ont les significations données pour la formule I, en faisant réagir :

2.1. des composés de formule II avec des composés de formule IV

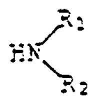

(IV)

a) en présence d'un excès de réactif $HN(R_1)R_2$ ou bien

b) en présence d'une base organique, avec ou sans 4-dialkylaminopyridine servant de catalyseur dans des solvants inertes, ou bien

c) en présence de bases minérales, en travaillant dans chaque cas dans l'intervalle de températures de -10° à 160°C et de préférence de 0° à 100°C ; et

3. on prépare des composés de formule Id

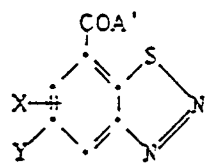

(Id)

dans laquelle A' est le résidu $U^1N(=C)_n(R_3)R_4$, et X, Y, $R_3$, $R_4$, $R_5$ et n ont les significations données pour la formule I, en faisant réagir

3.1. des composés de formule Ie

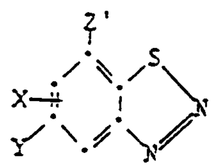

(Ie)

dans laquelle Z' est le groupe COOH, COCl, $COOAlk^1$ ou un résidu acyloxycarbonyle, comme par exemple

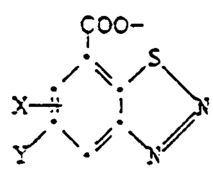

,

benzoyloxycarbonyle ou acétyloxycarbonyle, $Alk^1$ représentant un radical alkyle en $C_1$-$C_4$, en présence d'une base, avec des dérivés de l'hydrazine de formules V ou VI

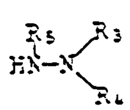

(V) ou

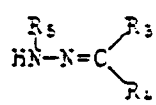

(VI)

dans un solvant inerte à des températures de -10° à 180°C et de préférence de 0° à 100°C ; ou

3.2. on fait réagir par étapes des composés de formule Ie, d'abord avec l'hydrazine, puis on fait réagir les dérivés obtenus de l'hydrazine

3.2.1. avec l'agent d'alkylation $R_3$-L ou $R_4$-L, où L est un groupe éliminable, dans un solvant inerte, à des températures de 0° à 160°C et de préférence de 20° à 120°C ; ou bien

3.2.2. avec un aldéhyde ou une cétone de formule $R_3(R_4)C=O$, où $R_3$ et $R_4$ ont les significations données pour la formule I, avec ou sans addition d'un acide minéral ou

organique, à des températures de -10° à 150°C et de préférence de 20° à 100°C, puis, si on le souhaite,

3.2.3. avec un agent d'alkylation L-$R_5$, où L est un groupe éliminable, en présence d'une base forte, dans un solvant inerte, à des températures de -80° à 120° et de préférence de -40° à 80°C ; ou encore, si on le souhaite

3.2.4.

a) on hydrogène les dérivés hydrazones préparés en 3.2.1. à l'aide d'hydrogène sous une pression de 1 à $30.10^5$ Pa, en présence d'un catalyseur en mélange avec du charbon activé, dans un solvant inerte, à des températures de 0° à 100°C, ou bien

b) on traite les dérivés hydrazones préparés en 3.2.1. avec un hydrure métallique complexe tel que le cyanoborohydrure de Na, dans un solvant inerte, à des températures de -10° à 80° et de préférence de 0° à 50°C ; et

4. on prépare des composés de formule If

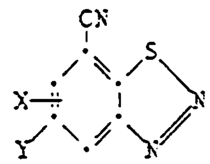

(If)

dans laquelle les symboles X et Y ont les significations données pour la formule I, en traitant des composés de formule Ig [préparés par le procédé (2)]

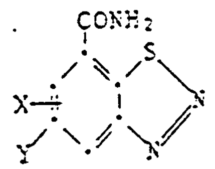

(Ig)

avec un agent de déshydratation, dans un solvant inerte ou sans solvant, à des températures de -10° à 250°C, auquel cas on utilise comme agent de déshydratation :

a) de l'anhydride trifluoracétique en présence d'une base comme par exemple la pyridine, dans un solvant inerte tel par exemple que le tétrahydrofuranne ou le dioxanne, à des températures de -10° à 40°C ; ou bien

b) de l'isocyanate de chlorosulfonyle dans un solvant inerte, comme par exemple le tétrahydrofuranne, à des températures de 0° à 65°C, opération suivie d'un traitement au diméthylformamide ;

c) du pentoxyde de phosphore, avec ou sans solvant inerte comme par exemple le 1,2-dichlorométhane, le xylène ou le chlorobenzène, éventuellement dans une bombe tubulaire sous pression élevée, à une température de 50° à 250°C ;

5.1. on prépare des composés de formule $IL_1$

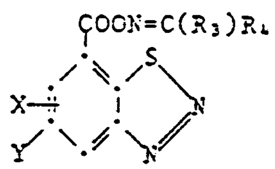

($IL_1$)

dans laquelle $R_3$, $R_4$, X et Y ont les significations données pour la formule I, en faisant réagir des dérivés oximes de formule

HO-N=C($R_3$)$R_4$

avec des dérivés d'acide activés de formule

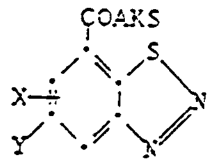

,

où AKS désigne un halogène, un radical O-acyle comme par exemple le résidu O-acyle de l'acide libre ayant la formule ci-dessus, le radical acétoxy ou benzoyloxy, ou encore 1-imidazolyle, dans un solvant inerte et une base, à une température de -20° à 120° et de préférence de 0° à 50°C, ou encore l'acide libre (= Ib) en présence de dicyclohexylcarbodiimide dans les mêmes conditions ;
5.2. on prépare des composés de formule $IL_2$

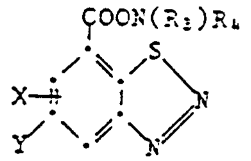

$(IL_2)$

dans laquelle $R_3$, $R_4$, X et Y ont les significations données pour la formule I, par réduction de composés de formule $IL_1$

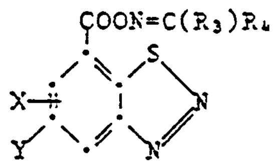

$(IL_1)$

a) avec un silane, par exemple le triéthylsilane, en présence d'un acide tel que l'acide trifluoracétique, à une température de 0° à 80°C, ou bien
b) avec du cyanoborohydrure de sodium, en présence d'un acide organique tel que l'acide acétique, à une température de 0° à 80°C, ou bien
c) par voie catalytique, par exemple avec $Pt/H_2$.

**25.** Procédé de préparation de composés de formule Ik

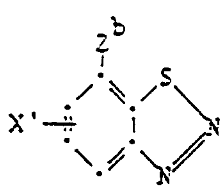

(Ik)

qui consiste à diazoter un composé de formule XI'

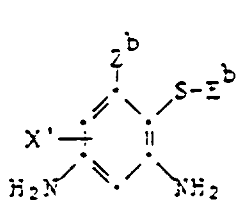

(XI')

en milieu acide, avec un composé nitrite, à une température de -40° à 30°C, et à le traiter, dans le même réacteur ou dans un autre réacteur, avec un réducteur à une température de -40° à 80° et de préférence de -30° à 30°C, le réducteur étant ajouté avant ou après le composé nitrite ou en même temps que ce dernier, les substituants des composés ayant les significations suivantes :

X' est un hydrogène, un halogène ou le radical méthyle, méthoxy ou COOH ;

$E^b$ est un groupe facilement éliminable comme par exemple un hydrogène ou un radical alkyle en $C_1$-$C_{16}$, par exemple méthyle, éthyle, isopropyle ou n-dodécyle, ou benzyle ou encore acyle, par exemple acétyle, ou un résidu acide sulfonique (-$SO_3H$) ou cyano, ou encore, comme élément d'un pont disulfure, un deuxième résidu

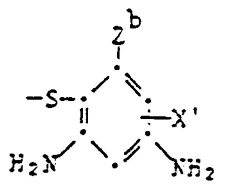 ;

$Z^b$ est le radical cyano ou COA ;

A est UR, $N(R_1)R_2$ ou $U^1N(=C)_n(R_3)R_4$ ;

M est l'équivalent molaire d'un ion d'un métal alcalin ou alcalino-terreux, formé à partir d'une base ou d'un composé basique correspondants ;

U est l'oxygène ou le soufre ;

$U^1$ est l'oxygène ou -$N(R_5)$- ;

R est un hydrogène ou un radical alkyle en $C_1$-$C_8$, un radical alkyle en $C_1$-$C_8$ substitué par halogéno, cyano, nitro, hydroxy ou U-(alkyle en $C_1$-$C_3$), (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

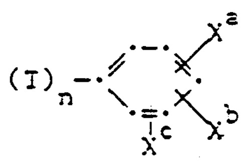 , $(T)_n$-Napht, $(T)_n$-Si($C_1$-$C_8$-Alkyl)$_3$,

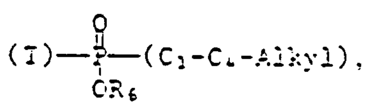 , 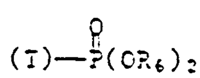 ; ou $(T)_n$-W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyle, et $X^b$ et $X^c$ sont simultanément des atomes d'hydrogène ; ou bien

$X^a$ est le radical phényle, phényloxy ou benzyloxy, et

$X^b$ est un halogène ou le radical méthyle et $X^c$ est un hydrogène ; ou bien

$X^a$, $X^b$ et $X^c$ forment ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants -$CH_2$-, -$CH_2CH_2$-, -$CH(CH_3)$-, -$CH_2CH_2CH_2$- ou -$CH_2CH_2O$- ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué

par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC : alcynyle en $C_3$-$C_5$ ; alcynyle en $C_3$-$C_5$ substitué par (alkyle en $C_1$-$C_3$)-OOC ; $(T)_n$-cyclo (alkyle en $C_3$-$C_6$) ; $(T)_n$-cyclo(alkyle en $C_3$-$C_6$) substitué par alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_6$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$, avec l'atome auquel ils sont liés, forment un hétérocycle W ou un noyau carbocyclique W' ;

W' est un résidu carbocyclique ayant 3 à 7 atomes de carbone dans le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des groupes amino primaires ou secondaires, des groupes UH- ou nitro, des radicaux Si-(alkyle en $C_1$-$C_8$)$_3$ ou des résidus contenant du phosphore.

**26.** Procédé de préparation de composés de formule

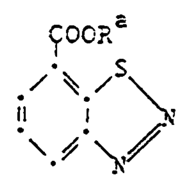

qui consiste à diazoter un composé de formule

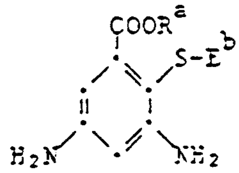

en milieu acide, avec un composé nitrite, à une température de -20° à 30°C et, dans le même réacteur, à le traiter avec un réducteur à une température de -20° à 80° et de préférence de -20° à 30°C, le réducteur étant ajouté avant ou après le composé nitrite ou en même temps que ce dernier, les substituants des composés ayant les significations suivantes :

$R^a$ est un hydrogène ou un radical alkyle en $C_1$-$C_8$, un radical alkyle en $C_1$-$C_8$ substitué par halogéno cyano, nitro, hydroxy ou U-(alkyle en $C_1$-$C_3$) ; (T)-COOH ou (T)-COO(alkyle en $C_1$-$C_4$), alcényle en $C_3$-$C_6$, alcényle en $C_3$-$C_6$ à substituant halogène, alcynyle en $C_3$-$C_6$, alcynyle en $C_3$-$C_6$ à substituant halogène, $(T)_n$-cyclo(alkyle en $C_3$-$C_8$), ou encore un groupe de la série :

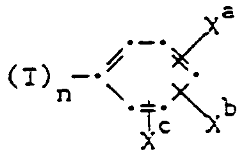 , $(T)_n$-Napht, $(T)_n$-Si($C_1$-$C_8$-Alkyl)$_3$,

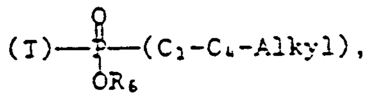 , 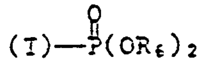 ; ou $(T)_n$—W ;

$X^a$, $X^b$ et $X^c$, chacun indépendamment l'un de l'autre, représentent un hydrogène, un halogène ou un radical hydroxy, cyano, HOOC, MOOC, (alkyle en $C_1$-$C_3$)-OOC, alkyle en $C_1$-$C_4$, alcoxy en $C_1$-$C_4$, halogéno-(alkyle en $C_1$-$C_2$) ayant jusqu'à 5 atomes d'halogène, en particulier des atomes de fluor ; ou bien $X^a$ est un radical halogéno-(alcoxy en $C_1$-$C_2$) ayant jusqu'à 5 atomes d'halogène ou un radical nitro, diméthylamino, phényle, phényloxy, benzyloxy, sulfamoyle, et $X^b$ et $X^c$ sont simultanément des atomes d'hydrogène ; ou bien

$X^a$ est le radical phényle, phényloxy ou benzyloxy, et

$X^b$ est un halogène ou le radical méthyle et $X^c$ est un hydrogène ; ou bien

$X^a$, $X^b$ et $X^c$ forment ensemble 4 ou 5 atomes de fluor ;

Napht est un résidu naphtyle non substitué, ou substitué par halogéno, méthyle, méthoxy ou nitro ;

W est un hétérocycle ayant 1 à 3 hétéroatomes du groupe O, N et S, à 5-7 chaînons, saturé ou insaturé, non substitué ou encore substitué par halogéno, trifluorométhyle, cyano ou alkyle en $C_1$-$C_2$ ou par un radical (alcoxy en $C_1$-$C_2$)-carbonyle-(alkylène en $C_2$-$C_4$)-imino, ou encore un résidu de monosaccharide ;

T représente les chaînons pontants $-CH_2-$, $-CH_2CH_2-$, $-CH(CH_3)-$, $-CH_2CH_2CH_2-$ ou $-CH_2CH_2O-$ ;

$R_1$ est un hydrogène ou un radical alkyle en $C_1$-$C_5$, un radical alkyle en $C_1$-$C_5$ interrompu par un atome d'oxygène ou de soufre ; alkyle en $C_1$-$C_5$ substitué par halogéno, cyano, HOOC ou (alkyle en $C_1$-$C_2$)-OOC ; alkyle en $C_1$-$C_5$ interrompu par un atome d'oxygène ou un atome de soufre et substitué par halogéno, cyano, HOOC ou (alkyle en $C_1$-$C_2$)-OOC ; alcényle en $C_3$-$C_5$ ; alcényle en $C_3$-$C_5$ substitué par (alkyle en $C_1$-$C_3$)-OOC ; alcynyle en $C_3$-$C_5$ ; alcynyle en $C_3$-$C_5$ substitué par (alkyle en $C_1$-$C_3$)-OOC ; $(T)_n$-cyclo(alkyle en $C_3$-$C_6$) ; $(T)_n$-cyclo(alkyle en $C_3$-$C_6$) substitué par (alkyle en $C_1$-$C_3$)-OOC ; $(T)_n$-phényle ou $(T)_n$-phényle substitué dans la partie phényle par halogéno, hydroxy, méthyle, méthoxy, $CF_3$, cyano, HOOC ou MOOC ;

$R_2$ est un hydrogène ou un radical hydroxy, alkyle en $C_1$-$C_3$, alkyle en $C_1$-$C_3$ substitué par cyano ou alcoxy en $C_1$-$C_3$, alcoxy en $C_1$-$C_4$, ou encore un hétérocycle à 3-6 chaînons, saturé ou insaturé, comportant O, N ou S comme hétéro-atomes ;

$R_1$ et $R_2$, avec l'atome auquel ils sont liés, forment un hétérocycle W ;

$R_3$ est un hydrogène ou un radical cyano, alkyle en $C_1$-$C_6$, phényle, phényle substitué par halogéno, hydroxy, méthyle, méthoxy, HOOC ou MOOC, ou encore un hétérocycle W ;

$R_4$ est un hydrogène ou un radical alkyle en $C_1$-$C_6$, $CONH_2$, CONH-CONH-(alkyle en $C_1$-$C_3$), alcanoyle en $C_1$-$C_6$, alcanoyle en $C_1$-$C_3$ substitué par halogéno ou alcoxy en $C_1$-$C_3$, alcénoyle en $C_3$-$C_5$ ou alcénoyle en $C_3$-$C_5$ substitué par halogéno ou alcoxy en $C_1$-$C_3$ ;

$R_3$ et $R_4$ avec l'atome auquel ils sont liés, forment un hétérocycle W ou un noyau carbocyclique W' ;

W' est un résidu carbocyclique ayant 3 à 7 atomes de carbone sur le noyau ;

$R_5$ est un hydrogène ou le radical méthyle ;

$R_6$ est un hydrogène ou un radical alkyle en $C_1$-$C_4$ ; et

n vaut zéro ou 1 ;

à l'exception des résidus comportant des groupes UH- ou nitro, ainsi que des résidus contenant du silicium ou du phosphore, et

$E^b$ est un groupe facilement éliminable comme par exemple l'hydrogène ou un radical alkyle en $C_1$-$C_{16}$, par exemple méthyle, éthyle, isopropyle ou n-dodécyle, ou benzyle ou encore acyle, par exemple acétyle, ou un résidu acide sulfonique ($-SO_3H$) ou cyano.

**27.** Utilisation des composés de formule I selon la revendication 1 ou 2 pour l'immunisation des végétaux contre les maladies.

**28.** Utilisation des composés de formule I' selon la revendication 20 pour l'immunisation des végétaux contre les maladies.

**29.** Utilisation du 7-méthoxycarbonyl-benzo-1,2,3-thiadiazole en tant que matière active pour la préparation d'un produit destiné à immuniser les végétaux contre une infestation par des micro-organismes phytopathogènes.